# EUROPÄISCHE PATENTSCHRIFT

(11) **EP 2 170 877 B1**
(45) Veröffentlichungstag und Bekanntmachung des Hinweises auf die Patenterteilung: **21.11.2012**
(21) Anmeldenummer: 08773372.1
(22) Anmeldetag: 07.06.2008
(51) Int. Cl.: C07D 413/14, A61K 31/4439, A61P 7/00

(54) **SUBSTITUIERTE (OXAZOLIDINON-5-YL-METHYL) -2-THIOPHEN-CARBOXAMIDE UND IHRE VERWENDUNG IM GEBIET DER BLUTGERINNUNG**
SUBSTITUTED (OXAZOLIDINON-5-YL-METHYL) -2-THIOPHENE-CARBOXAMIDES AND USE THEREOF IN THE FIELD OF BLOOD COAGULATION
(OXAZOLIDINON-5-YL-MÉTHYL)-2-THIOPHÈNE-CARBOXAMIDES SUBSTITUÉS ET LEUR UTILISATION DANS LE DOMAINE DE LA COAGULATION SANGUINE

(30) Priorität: 20.06.2007 DE 102007028319
(43) Veröffentlichungstag der Anmeldung: 07.04.2010
(73) Patentinhaber: Bayer Intellectual Property GmbH, 40789 Monheim (DE)
(72) Erfinder: ALLERHEILIGEN, Swen, 45259 Essen (DE); BAUSER, Marcus, 10439 Berlin (DE); HEIMBACH, Dirk, 40549 Düsseldorf (DE); HEITMEIER, Stefan, 42489 Wülfrath (DE); GNOTH, Mark, Jean, 40822 Mettmann (DE); GERDES, Christoph, 51063 Köln (DE); VON DEGENFELD, Georges, 51373 Leverkusen (DE); RÖHRIG, Susanne, 40724 Hilden (DE); RESTER, Ulrich, 42115 Wuppertal (DE); DITTRICH-WENGENROTH, Elke, 42113 Wuppertal (DE); SAATMANN, Uwe, 42117 Wuppertal (DE); TERSTEEGEN, Adrian, 42111 Wuppertal (DE); KRÜGER, Joachim, 40489 Düsseldorf (DE); PAULSEN, Holger, 40724 Hilden (DE)
(74) Vertreter: Bayer Intellectual Property GmbH
(86) Internationale Anmeldenummer: PCT/EP2008/004562
(87) Internationale Veröffentlichungsnummer: WO 2008/155032

(56) Entgegenhaltungen:
- WO-A-01/47919
- DE-A1- 10 129 725
- US-A1- 2006 069 260

## Beschreibung

Die Erfindung betrifft neue substituierte Oxazolidinone, Verfahren zu ihrer Herstellung, ihre Verwendung zur Herstellung eines Arzneimittels zur Behandlung und/oder Prophylaxe von Krankheiten sowie ihre Verwendung zur Herstellung von Arzneimitteln zur Behandlung und/oder Prophylaxe von Krankheiten, insbesondere von thromboembolischen Erkrankungen.

Die Blutgerinnung ist ein Schutzmechanismus des Organismus, mit dessen Hilfe Defekte in der Gefäßwand rasch und zuverlässig "abgedichtet" werden können. So kann ein Blutverlust vermieden bzw. minimiert werden. Die Blutstillung nach Gefäßverletzung erfolgt im wesentlichen durch das Gerinnungssystem, bei dem eine enzymatische Kaskade komplexer Reaktionen von Plasmaproteinen ausgelöst wird. Hierbei sind zahlreiche Blutgerinnungsfaktoren beteiligt, von denen jeder, sobald aktiviert, die jeweils nächste inaktive Vorstufe in ihre aktive Form überführt. Am Ende der Kaskade steht die Umwandlung des löslichen Fibrinogens in das unlösliche Fibrin, so dass es zu einem Blutgerinnsel kommt. Traditionell unterscheidet man bei der Blutgerinnung zwischen dem intrinsischen und dem extrinsischen System, die in einem abschließenden gemeinsamen Reaktionsweg münden. Hierbei kommen den Faktoren Xa und IIa (Thrombin) Schlüsselrollen zu.

Faktor Xa bündelt die Signale der beiden Gerinnungswege, da er sowohl über Faktor VIIa/Tissue Factor (extrinsischer Weg) wie auch den Tenase Komplex (intrisischer Weg) durch Umsetzung von Faktor X entsteht. Die aktivierte Serinprotease Xa spaltet Prothrombin zu Thrombin.

Thrombin überträgt über einen Strauss von Umsetzungen die Signale aus der Kaskade auf den Gerinnungsstatus des Blutes. Thrombin spaltet direkt Fibrinogen zu Fibrin. Es aktiviert den zur Stabilisierung des Fibringerinnsels notwendigen Faktor XIII zu Faktor XIIIa. Darüber hinaus ist Thrombin ein potenter Auslöser der Thrombozytenaggregation (über PAR-1 Aktivierung), die ebenfalls einen erheblichen Beitrag bei der Hämostase leistet. Mittels Aktivierung von TAFI (Thrombin-aktivierbarer Fibrinolyse Inhibitor) zu TAFIa hemmt Thrombin im Komplex mit Thrombomodulin die Auflösung des Gerinnsels. Aktivierung der Faktoren V und VIII führt zur Potenzierung der Thrombinproduktion und damit wiederum zur Verstärkung der Gerinnungsreaktion, das im Komplex mit Thrombomodulin hergestellte aktivierte Protein C wirkt dieser verstärkten Thrombinproduktion entgegen und verhindert so das Überschießen der Hämostase (Thrombose).

Neben frei im Blut befindlichem Faktor X und Thrombin sind auch gebundene Formen bekannt. Während der Entstehung eines Fibringerinnsels werden Thrombin und Prothrombinase (Faktor Xa im Komplex) an das Fibringerüst gebunden. Diese Enzymmoleküle besitzen weiterhin Aktivität und können durch das körpereigene Anti-Thrombin III nicht inhibiert werden. Gerinnsel besitzen also auf diese Weise ein generelles koagulatives Potenzial.

Im Verlauf vieler Herzkreislauf- und Stoffwechselerkrankungen kommt es infolge systemischer Faktoren, wie z.B. Hyperlipidämie, Diabetes oder Rauchen, infolge von Blutflußveränderungen mit Stase, wie z.B. beim Vorhofflimmern, oder infolge pathologischer Gefäßwandveränderungen, z.B. endothelialer Dysfunktionen oder Atherosklerose, zu einer erhöhten Neigung von Gerinnungs- und Thrombozytenaktivierung. Diese unerwünschte und überschießende Hämostase kann durch Bildung fibrin- und plättchenreicher Thromben zu thromboembolischen Erkrankungen und thrombotischen Komplikationen mit lebensbedrohlichen Zuständen führen.

Die Hämostase unterliegt einem komplexen Regulationsmechanismus. Eine unkontrollierte Aktivierung des Gerinnungssystems oder eine defekte Hemmung der Aktivierungsprozesse kann die Bildung von lokalen Thrombosen oder Embolien in Gefäßen (Arterien, Venen, Lymphgefäßen) oder Herzhöhlen bewirken. Dies kann zu schwerwiegenden thrombotischen oder thromboembolischen Erkrankungen führen. Darüber hinaus kann eine systemische Hyperkoagulabilität zu einer Verbrauchskoagulopathie im Rahmen einer disseminierten intravasalen Gerinnung führen. Thromboembolische Komplikationen treten ferner auf bei mikroangiopathischen hämolytischen Anämien, extrakorporalen Blutkreisläufen, wie Hämodialyse, sowie Herzklappenprothesen und Stents.

Thromboembolische Erkrankungen sind die häufigste Ursache von Morbidität und Mortalität in den meisten industrialisierten Ländern [Heart Disease: A Textbook of Cardiovascular Medicine, Eugene Braunwald, 5. Auflage, 1997, W.B. Saunders Company, Philadelphia].

In der Therapie und Prophylaxe von thromboembolischen und thrombotischen Erkrankungen spielen Antikoagulantien, d.h. Stoffe zur Hemmung oder Verhinderung der Blutgerinnung, eine wesentliche Rolle. Zur Behandlung von akuten thromboembolischen bzw. thrombotischen Erkrankungen - insbesondere derjenigen, bei denen ein schnelles und einfaches Absetzen bzw. Anpassen der Antikoagulation erforderlich ist (z.B. akutes Koronarsyndrom, Sepsis) - oder Erkrankungen, die eine bedarfsgerechte, kurzzeitige prophylaktische Hemmung des Gerinnungssystems erfordern (z.B. Hämodialyse, Kardiokonversion), werden Antikoagulatien parenteral appliziert eingesetzt.

Die aus dem Stand der Technik bekannten Antikoagulantien, d.h. Stoffe zur Hemmung oder Verhinderung der Blutgerinnung, weisen verschiedene, oftmals gravierende Nachteile auf. Eine effiziente und sichere Behandlungsmethode bzw. Prophylaxe von akuten thrombotischen/thromboembolischen Erkrankungen erweist sich in der Praxis deshalb oft als schwierig und unbefriedigend.

In der akuten Therapie und Prophylaxe von thromboembolischen Erkrankungen finden zum einen Heparin und niedermolekulare Heparine (NMH) Verwendung, die intravenös oder subkutan appliziert werden. Heparin ist eine Mischung hochgradig sulfatierter Glucosaminoglucane tierischem Ursprungs (Molmasse 3-40 kDa mit der größten Häufigkeit bei 15 kDa; beispielsweise extrahiert aus Rinderlungen), von denen je nach Präparation ca. 30% antikoagulativ sind. Die Dosierung erfolgt deshalb in Units und muss im Patienten genau kontrolliert werden. Eine unkontrollierte Gabe ohne Kontrolle führt zu einem hohen Risiko der Unterdosierung bzw. Überdosierung und damit Blutungen. Niedermolekulare Heparine (NMH) werden aus unfraktioniertem Heparin (UFH) hergestellt. Sie sind bezogen auf die Größe besser definiert und zeigen aufgrund ihrer geringeren Größe ein verändertes Nebenwirkungsprofil.

Die Heparine wirken nicht direkt am Enzym, sondern führen durch Bindung an Antithrombin III (AT(III)) zur Beschleunigung der Bindung von AT(III) an Koagulationsfaktoren mit einer AT(III)-Bindestelle. Zu diesen gehören vor allem Faktor Xa und Thrombin (Faktor IIa). Während UFHs auf beiden Enzymen ungefähr gleich potent sind, zeigen die NMHs ein zugunsten Faktor Xa verschobenes Wirkspektrum, das ultimativ in der reinen Faktor Xa-Hemmung des Pentasaccharids Fondaparinux kulminiert. Dieser indirekte Mechanismus ist unter bestimmten Gesichtspunkten nachteilig:

Im Rahmen von Krankheiten, die mit einer Verbrauchskoagulopathie einhergehen, führt die Depletion von AT(III) dazu, dass die Heparine nicht mehr wirksam sind. Im Gegenteil: durch Gabe von Heparinen wird dem Organismus die letzte Reserve an freiem AT(III) entzogen.

Im Rahmen der Entstehung eines Thrombus wird Prothrombinase (Faktor Xa) und Thrombin an diesen gebunden. Diese Koagulationsenzyme sind aktiv und tragen zur Weiterentwickung des Thrombus bei. Hemmung von Thrombus-gebundenem Thrombin bzw. Faktor Xa gilt daher bei der Risikoreduktion von arteriellen Erkrankungen eine besondere Aufmerksamkeit. AT(III) als Protein besitzt aufgrund seiner Größe nicht die Fähigkeit, Thrombus-gebundene Koagulationsfaktoren zu hemmen. Deshalb können Heparine nicht zu dieser Risikoreduktion beitragen

Im Rahmen des Einsatzes zur Behandlungen von Krankheiten wie zum Beispiel Akutes Koronarsyndrom oder Sepsis ist es von besonderem Vorteil, wenn die Therapie kurzfristig abgesetzt werden kann. UFHs haben eine relativ kurze Halbwertszeit von 30 - 150 min. Außerdem kann ihre Wirkung durch Protaminsulfat antagonisiert werden. Nachteilig ist hier die lange Halbwertszeit der verschiedenen NMHs insbesondere des Fondaparinux, die ein einfaches Anpassen der Therapie an akute Situationen fast unmöglich macht. Dazu kommt noch, dass die Wirkung dieser Substanzen nur partiell durch Protaminsulfat zu antagonisieren ist.

Generell besteht bei Gabe von UFH ein hohes Blutungsrisiko, insbesondere können Hirnblutungen und Blutungen im Gastrointestinaltrakt auftreten, und es kann zu Thrombopenie, Alopecia medicomentosa oder Osteoporose kommen [Pschyrembel, Klinisches Wörterbuch, 257. Auflage, 1994, Walter de Gruyter Verlag, Seite 610, Stichwort "Heparin"; Römpp Lexikon Chemie, Version 1.5, 1998, Georg Thieme Verlag Stuttgart, Stichwort "Heparin"]. Niedermolekulare Heparine zeigen - wenn auch mit geringerer Inzidenz - ein ähnliches Nebenwirkungsspektrum - insbesondere in Bezug auf das Auftreten von HIT-II.

Im Rahmen von Herzerkrankungen und auch septischen Erkrankungen kann es zu gleichzeitiger - manchmal akuter - Einschränkung der Nierenfunktion kommen. Substanzen, die über die Niere eliminiert werden, können in diesen Situationen im Körper akkumulieren und zu einem deutlich erhöhten Blutungsrisiko führen. Während bei UFHs keine Dosisanpassung notwendig ist, müssen die NMHs in der Dosis adaptiert werden. Fondaparinux wird bei Patienten mit schwerer Nierenfunktionsstörung nicht empfohlen.

Daneben werden - wenn auch in deutlich geringerem Ausmaß - Hirudin und das daraus abgeleitete synthetisch hergestellte Peptid Bivalirudin sowie die niedermolekulare Substanz Argatroban eingesetzt. Hierbei handelt es sich um direkte Thrombin-Inhibitoren, die ein hohes Blutungsrisiko zeigen. Hirudin und Bivalirudin müssen bei Auftreten einer Nierenfunktionsstörung in ihrer Dosierung angepasst werden. Argatroban zeigt eine vergleichsweise schwache antithrombotische Wirkung und ist nicht gut wasserlöslich. Aufgrund der fehlenden Inhibition von Faktor Xa kann die Thrombinherstellung innerhalb und außerhalb eines Thrombus nicht gehemmt werden, d.h. bei stark überschießender Gerinnungsreaktion besteht die Gefahr, dass diese Substanzen "austitriert" werden.

Weitere Substanzen, die parenteral applizierbar sind, sind in der Entwicklung. Dabei handelt es sich um Substanzen, die entweder Faktor Xa oder Thrombin inhibieren und somit entweder die Thrombinherstellung oder -aktivität hemmen.

Daneben gibt es Substanzen, die für die orale Therapie verwendet bzw. entwickelt werden. Aufgrund ihres Wirk- und physikochemischen Profils (schlechte Löslichkeit, langsamer Wirkeintritt, lange Halbwertszeit) sind sie für die Behandlung in akuten Situationen nicht oder nur wenig geeignet. Eine derartige Klasse von Antikoagulantien stellen die Vitamin K-Antagonisten dar. Hierzu gehören beispielsweise 1,3-Indandione, vor allem aber Verbindungen wie Warfarin, Phenprocoumon, Dicumarol und andere Cumarin-Derivate, die unselektiv die Synthese verschiedener Produkte bestimmter Vitamin K-abhängiger Gerinnungsfaktoren in der Leber hemmen. Durch den Wirkmechanismus bedingt, setzt die Wirkung nur sehr langsam ein (Latenzzeit bis zum Wirkeintritt 36 bis 48 Stunden). Die Verbindungen können zwar oral appliziert werden, aufgrund des hohen Blutungsrisikos und des engen therapeutischen Indexes ist aber eine aufwendige individuelle Einstellung und Beobachtung des Patienten notwendig. Darüber hinaus sind weitere Nebenwirkungen wie gastrointestinale Störungen, Haarausfall und Hautnekrosen beschrieben.

Andere anti-Thrombin- bzw. anti-Faktor Xa-Substanzen, die für die orale Therapie entwickelt werden, zeigen eine zu geringe Löslichkeit für die parenterale Therapie oder haben eine - für die orale Therapie gewünschte - lange Halbwertszeit, die ein schnelles Reagieren auf eine Veränderung nicht ermöglichen.

In jüngster Zeit sind Ansätze beschrieben worden, in denen niedermolekulare Thrombin- und Faktor Xa Inhibitoren in verschiedenen Mischungsverhältnissen in-vitro und in-vivo getestet wurden. Dabei zeigte sich ein starkes synergistisches Potential. Mit Tanogitran ist eine niedermolekulare Substanz beschrieben worden, die in-vitro sowohl Thrombin als auch Faktor Xa hemmt, aber eine starke Präferenz auf Thrombinhemmung besitzt. Tanogitran wird zu einem wesentlichen Teil unverändert über die Niere ausgeschieden, was eine Dosisanpassung im Falle einer Niereninsuffizienz wahrscheinlich macht.

Bei antithrombotischen Arzneimitteln ist die therapeutische Breite von zentraler Bedeutung: Der Abstand zwischen der therapeutisch wirksamen Dosis zur Gerinnungshemmung und der Dosis, bei der Blutungen auftreten können, sollte möglichst groß sein, so dass eine maximale therapeutische Wirksamkeit bei minimalem Risikoprofil erreicht wird.

Wie die Experimente mit Mischungen von niedermolekularen Thrombin- und Faktor Xa Inhibitoren zeigen, würden Verbindungen, die sowohl Thrombin als auch Faktor Xa hemmen, durch ihren dualen Charakter einen besonders starken Synergismus besitzen und dadurch besonders effektiv die Entstehung von Thromben bekämpfen können. Damit hemmen die Verbindungen die beiden entscheidenden Enzyme der Koagulationskaskade, ohne die einzelnen Enzyme vollständig blocken zu müssen. Der übriggebliebene Rest an Faktor Xa und Thrombin führt zu einer intakten Blutstillung und somit zu einer besonders vorteilhaften therapeutischen Breite. In einem Arteriovenösem-Shunt Modell im Kaninchen konnte gezeigt werden, dass Co-Administration von nur schwach antithrombotisch wirksamen Dosierungen des selektiven FXa Inhibitors PD0313052 und des selektiven Thrombin Inhibitors Argatroban zu einem starken, überadditiven antithrombotischen Effekt führen. Zudem wurde bei der Kombination der Einzeldosen mit dem maximalen synergistischen Effekt keine Verstärkung von Blutungen beobachtet. Diese Beobachtungen lassen den Schluss zu, dass die simultane Inhibition von Thrombin und FXa die therapeutische Breite in Bezug auf Abstand der antithrombotischen Wirkung zum Blutungsrisiko vergrößert (Journal of Thrombosis and Haemostasis, 4: 834-841).

Dieser Synergismus zeigt sich besonders deutlich bei Betrachtung der Entwicklung der Prothrombinzeit in Abhängigkeit von der Substanzkonzentration im direkten Vergleich zu reinen Faktor Xa- und Thrombin-Inhibitoren. Diese starke Wirkung auf die beiden entscheidenden Enzyme der Gerinnungskaskade wird als besonders vorteilhaft angesehen, wenn ein hohes Risiko für Thrombenbildungen besteht bzw. die Bildung von Thromben zu einer fatalen Erkrankung führen kann. Beides trifft beispielsweise auf die atherothiombotischen Erkrankungen aus dem Bereich des Akuten Koronarsyndroms bzw. die Situation nach einem akuten Myokardinfarkt zu.

Desweiteren würden Verbindungen, die sowohl Thombin als auch Faktor Xa hemmen, im Gegensatz zu Heparinen, Hirudin und Vitamin K-Antagonisten auch gegen Fibringerinnselgebundene Koagulationsfaktoren aktiv Die Begrenzung des thrombotischen Potentials eines schon vorhandenen Gerinnsels ist ein kritischer Punkt in der Prävention eines arteriellen Verschlusses Dieses geschieht besonders effektiv durch Hemmung sowohl der vorhandenen Thrombin-Aktivität als auch der Bildung von neuem Thrombin im Gerinnsel. Während ein reiner Thrombin-Inhibitor die lawinenartige Thrombinproduktion durch den Gerinnsel-gebundenen Faktor Xa-enthaltenen Prothrombinase-Komplex nicht verhindern kann und der inhibitorische Effekt damit bei einer stark stimulierten Koagulation von der großen Menge produziertem Thrombin überkompensiert werden kann, können reine Faktor Xa-Inhibitoren die schon vorhandene Thrombin-Aktivität nicht hemmen. Da diese auch durch physiologische Mechanismen nicht mehr hemmbat ist, stellt dies Gerinnsel-gebundene Thrombin ein besonders großes Risiko dar. Im Gegensatz dazu sind duale Verbindungen, d. h. Verbindungen, die sowohl Thrombin als auch Faktor Xa hemmen, in der Lage, sowohl die Thrombinproduktion als auch die Thrombinaktivität auf Gerinnseln zu hemmen und damit auch potentielles Gerinnselwachstum zu verhindern

Oxazolidinone als nicht-peptidische, niedermolekulare Faktor Xa-Inhibitoren sind beschrieben in WO 01/47919 US 2006/069260 offenbart die Verbindung 5-Chlor-thiophen-2-carbonsäure-{2-oxo-3-[4-(2-oxo-2*H*-pyridin-1-yl)-phenyl]-oxazolidin-5-yl-methyl}amid als Faktor Xa Inhibitor

Eine Aufgabe der vorliegenden Erfindung ist daher die Bereitstellung von dualen Verbindungen, d h. Verbindungen, die sowohl Thrombin als auch Faktor Xa hemmen und durch Hemmung der Thrombinproduktion und -aktivität auf Gerinnseln deren potentielles Wachstum verhindern, mit einem breiten therapeutischen Fenster und guter Löslichkeit in Wasser und physiologischen Medien, zur Bekämpfung von Erkrankungen, insbesondere von thromboembolischen Erkrankungen, bei Menschen und Tieren.

Gegenstand der Erfindung sind Verbindungen der Formel 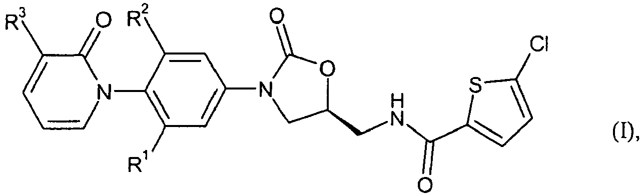 in welcher
- R¹: für Chlor, Trifluormethoxy, Methyl, Ethyl, n-Propyl, Methoxy, Methoxymethyl oder Ethoxymethyl steht,
- R²: für Wasserstoff oder Methyl steht,
und
- R³: für eine Gruppe der Formel 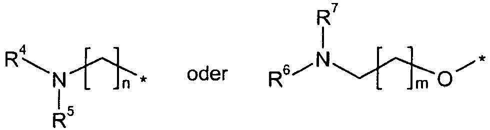
steht,
wobei
- *: die Anknüpfstelle an den Oxopyridin-Ring ist,
- n: für die Zahl 1, 2, 3 oder 4 steht,
- m: für die Zahl 1 oder 2 steht,
- R⁴: für Wasserstoff, Methyl, Ethyl, Cyclopropyl, Cyclobutyl, 2-Hydroxyeth-1-yl, 3-Hydroxyprop-1-yl, 2-Methoxyethy-1-yl, 3-Methoxyprop-1-yl, 4-Hydroxycyclohex-1-yl, Tetrahydrofuran-2-ylmethyl oder 1,4-Dioxan-2-ylmethyl steht,
- R⁵: für Wasserstoff, Methyl oder Ethyl steht,
oder
- R⁴ und R⁵: zusammen mit dem Stickstoffatom an das sie gebunden sind einen Pyrrolidin-1-yl-Ring, einen 2-Methoxymethyl-pyrrolidin-1-yl-Ring, einen Morpholin-4-yl-Ring, einen 1,1-Dioxo-thiomorpholin-4-yl-Ring, einen 1,4-Oxazepan-4-yl-Ring, einen 4-Methyl-piperazin-1-yl oder einen 4-Hydroxy-piperidin-1-yl-Ring bilden,
- R⁶: für Wasserstoff, Methyl, Ethyl, Cyclopropyl, Cyclobutyl, 2-Hydroxyeth-1-yl, 3-Hydroxyprop-1-yl, 2-Methoxyethy-1-yl, 3-Methoxyprop-1-yl, 4-Hydroxycyclohex-1-yl, Tetrahydrofuran-2-ylmethyl oder 1,4-Dioxan-2-ylmethyl steht,
- R⁷: für Wasserstoff, Methyl oder Ethyl steht,
oder
- R⁶ und R⁷: zusammen mit dem Stickstoffatom an das sie gebunden sind einen Pyrrolidin-1-yl-Ring, einen 2-Methoxymethyl-pyrrolidin-1-yl-Ring, einen Morpholin-4-yl-Ring, einen 1,1-Dioxo-thiomorpholin-4-yl-Ring, einen 1,4-Oxazepan-4-yl-Ring, einen 4-Methyl-piperazin-1-yl oder einen 4-Hydroxy-piperidin-1-yl-Ring bilden,
und ihre Salze, ihre Solvate und die Solvate ihrer Salze

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formeln

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt

In den Formeln der Gruppe, die für R³ stehen kann, steht der Endpunkt der Linie, neben der jeweils ein * steht, nicht für ein Kohlenstoffatom beziehungsweise eine CH₂-Gruppe sondern ist Bestandteil der Bindung zu dem Atom, an das R³ gebunden ist.

Bevorzugt sind Verbindungen der Formel (I), in welcher
- R¹: für Chlor, Trifluormethoxy, Methyl, Ethyl, n-Propyl, Methoxy oder Methoxymethyl steht,
- R²: für Wasserstoff oder Methyl steht,
und
- R³: für eine Gruppe der Formel 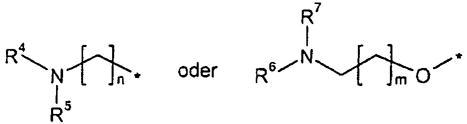
steht,
wobei
- *: die Anknüpfstelle an den Oxopyridin-Ring ist,
- n: für die Zahl 1, 2 oder 3 steht,
- m: für die Zahl 1 oder 2 steht,
- R⁴: für Wasserstoff, Methyl, Ethyl, Cyclopropyl, Cyclobutyl, 2-Hydroxyeth-1-yl, 3-Hydroxyprop-1-yl, 2-Methoxyethy-1-yl, 3-Methoxyprop-1-yl, 4-Hydroxycyclohex-1-yl, Tetrahydrofuran-2-ylmethyl oder 1,4-Dioxan-2-ylmethyl steht,
- R⁵: für Wasserstoff, Methyl oder Ethyl steht,
oder
- R⁴ und R⁵: zusammen mit dem Stickstoffatom an das sie gebunden sind einen Pynolidin-1-yl-Ring, einen 2-Methoxymethyl-pyrrolidin-1-yl-Ring, einen Morpholin-4-yl-Ring, einen 1,1-Dioxo-thiomorpholin-4-yl-Ring, einen 1,4-Oxazepan-4-yl-Ring, einen 4-Methyl-piperazin-1-yl oder einen 4-Hydroxy-piperidin-1-yl-Ring bilden,
- R⁶: für Wasserstoff, Methyl, Ethyl, Cyclopropyl, Cyclobutyl, 2-Hydroxyeth-1-yl, 3-Hydroxyprop-1-yl, 2-Methoxyethy-1-yl, 3-Methoxyprop-1-yl, 4-Hydroxycyclohex-1-yl, Tetrahydrofuran-2-ylmethyl oder 1,4-Dioxan-2-ylmethyl steht,
- R⁷: für Wasserstoff, Methyl oder Ethyl steht,
oder
- R⁶ und R⁷: zusammen mit dem Stickstoffatom an das sie gebunden sind einen Pyrrolidin-1-yl-Ring, einen 2-Methoxymethyl-pyrrolidin-1-yl-Ring, einen Morpholin-4-yl-Ring, einen 1,1-Dioxo-thiomorpholin-4-yl-Ring, einen 1,4-Oxazepan-4-yl-Ring, einen 4-Methyl-piperazin-1-yl oder einen 4-Hydroxy-piperidin-1-yl-Ring bilden,
und ihre Salze, ihre Solvate und die Solvate ihrer Salze.

Bevorzugt sind auch Verbindungen der Formen (I), in welcher
- R¹: für Methyl, Ethyl, n-Propyl, Methoxy oder Methoxymethyl steht,
- R²: für Wasserstoff oder Methyl steht,
und
- R³: für eine Gruppe der Formel 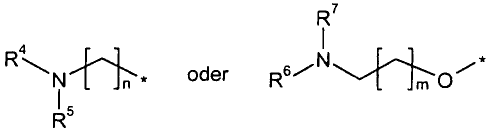
steht,
wobei
- *: die Anknüpfstelle an den Oxopyridin-Ring ist,
- n: für die Zahl 1, 2 oder 3 steht,
- m: für die Zahl 1 oder 2 steht,
- R⁴: für Wasserstoff, Methyl, Ethyl, Cyclopropyl, Cyclobutyl, 2-Hydroxyeth-1-yl, 3-Hydroxyprop-1-yl, 2-Methoxyethy-1-yl, 3-Methoxyprop-1-yl, 4-Hydroxycyclohex-1-yl, Tetrahydrofuran-2-ylmethyl oder 1,4-Dioxan-2-ylmethyl steht,
- R⁵: für Wasserstoff, Methyl oder Ethyl steht,
- R⁶: für Wasserstoff, Methyl, Ethyl, Cyclopropyl, Cyclobutyl, 2-Hydroxyeth-1-yl, 3-Hydroxyprop-1-yl, 2-Methoxyethy-1-yl, 3-Methoxyprop-1-yl, 4-Hydroxycyclohex-1-yl, Tetrahydrofuran-2-ylmethyl oder 1,4-Dioxan-2-ylmethyl steht,
- R⁷: für Wasserstoff, Methyl oder Ethyl steht,
und ihre Salze, ihre Solvate und die Solvate ihrer Salze.

Bevorzugt sind auch Verbindungen der Formel (I), in welcher
- R¹: für Methyl, Ethyl, n-Propyl, Methoxy oder Methoxymethyl steht,
- R²: für Wasserstoff steht,
oder
- R¹: für Methyl steht,
- R²: für Methyl steht,
und
- R³: für eine Gruppe der Formel 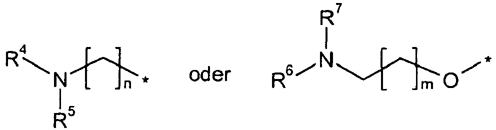
steht,
wobei
- *: die Anknüpfstelle an den Oxopyridin-Ring ist,
- n: für die Zahl 1, 2 oder 3 steht,
- m: für die Zahl 1 oder 2 steht,
- R⁴: für Wasserstoff, Cyclopropyl, Cyclobutyl, 2-Hydroxyeth-1-yl, 3-Hydroxyprop-1-yl oder 4-Hydroxycyclohex-1-yl steht,
- R⁵: für Wasserstoff steht,
- R⁶: für Wasserstoff, Cyclopropyl, Cyclobutyl, 2-Hydroxyeth-1-yl, 3-Hydroxyprop-1-yl oder 4-Hydroxycyclohex-1-yl steht,
- R⁷: für Wasserstoff steht,
und ihre Salze, ihre Solvate und die Solvate ihrer Salze.

Bevorzugt sind auch Verbindungen der Formel (I), in welcher
- R¹: für Methyl, Methoxy oder Methoxymethyl steht,
- R²: für Wasserstoff steht,
oder
- R¹: für Methyl steht,
- R²: für Methyl steht,
und
- R³: für eine Gruppe der Formel 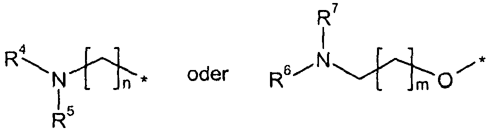
steht,
wobei
- *: die Anknüpfstelle an den Oxopyridin-Ring ist,
- n: für die Zahl 2 steht,
- m: für die Zahl 1 steht,
- R⁴: für 2-Hydroxyeth-1-yl oder 4-Hydroxycyclohex-1-yl steht,
- R⁵: für Wasserstoff steht,
- R⁶: für 2-Hydroxyeth-1-yl oder 4-Hydroxycyclohex-1-yl steht,
- R⁷: für Wasserstoff steht,
und ihre Salze, ihre Solvate und die Solvate ihrer Salze.

Bevorzugt sind auch Verbindungen der Formel (I), in welcher
- R¹: für Methyl oder Methoxy steht,
- R²: für Wasserstoff steht,
oder
- R¹: für Methyl steht,
- R²: für Methyl steht,
und
- R³: für eine Gruppe der Formel 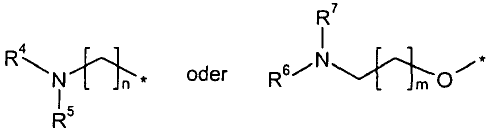
steht,
wobei
- *: die Anknüpfstelle an den Oxopyridin-Ring ist,
- n: für die Zahl 2 steht,
- m: für die Zahl 1 steht,
- R⁴: für 2-Hydroxyeth-1-yl oder 4-Hydroxycyclohex-1-yl steht,
- R⁵: für Wasserstoff steht,
- R⁶: für 2-Hydroxyeth-1-yl oder 4-Hydroxycyclohex-1-yl steht,
- R⁷: für Wasserstoff steht,
und ihre Salze, ihre Solvate und die Solvate ihrer Salze.

Bevorzugt sind auch Verbindungen der Formel (I), in welcher
- R¹: für Methyl, Methoxy oder Methoxymethyl steht,
- R²: für Wasserstoff steht,
oder
- R¹: für Methyl steht,
- R²: für Methyl steht,
und
- R¹: für eine Gruppe der Formel 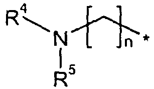
steht,
wobei
- *: die Anknüpfstelle an den Oxopyridin-Ring ist,
- n: für die Zahl 2 steht,
- R⁴: für 2-Hydroxyeth-1-yl oder 4-Hydroxycyclohex-1-yl steht,
- R⁵: für Wasserstoff steht,
und ihre Salze, ihre Solvate und die Solvate ihrer Salze.

Bevorzugt sind auch Verbindungen der Formel (I), in welcher
- R¹: für Methoxymethyl steht,
- R²: für Wasserstoff steht,
oder
- R¹: für Methyl steht,
- R²: für Methyl steht,
und
- R³: für eine Gruppe der Formel 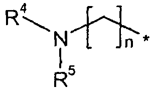
steht,
wobei
- *: die Anknüpfstelle an den Oxopyridin-Ring ist,
- n: für die Zahl 2 steht,
- R⁴: für 2-Hydroxyeth-1-yl oder 4-Hydroxycyclohex-1-yl steht,
- R⁵: für Wasserstoff steht,
und ihre Salze, ihre Solvate und die Solvate ihrer Salze.

Bevorzugt sind auch Verbindungen der Formel (I), in welcher
- R¹: für Methyl steht,
- R²: für Wasserstoff steht,
oder
- R¹: für Methyl steht,
- R²: für Methyl steht,
und
- R³: für eine Gruppe der Formel 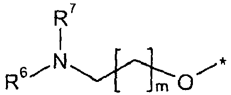
steht,
wobei
- *: die Anknüpfstelle an den Oxopyridin-Ring ist,
- m: für die Zahl 1 steht,
- R⁶: für 2-Hydroxyeth-1-yl oder 4-Hydroxycyclohex-1-yl steht,
- R⁷: für Wasserstoff steht,
und ihre Salze, ihre Solvate und die Solvate ihrer Salze.

Bevorzugt sind auch Verbindungen der Formel (I), in welcher n für die Zahl 2 steht.

Bevorzugt sind auch Verbindungen der Formel (I), in welcher m für die Zahl 1 steht.

Bevorzugt sind auch Verbindungen der Formel (I), in welcher R¹ für Methyl, Methoxy oder Methoxymethyl steht.

Bevorzugt sind auch Verbindungen der Formel (I), in welcher R² für Wasserstoff steht.

Bevorzugt sind auch Verbindungen der Formel (I), in welcher R¹ für Methyl und R² für Wasserstoff steht.

Bevorzugt sind auch Verbindungen der Formel (I), in welcher R¹ für Methyl und R² für Methyl steht.

Bevorzugt sind auch Verbindungen der Formel (I), in welcher R⁴ für 2-Hydroxyeth-1-yl oder 4-Hydroxycyclohex-1-yl steht.

Bevorzugt sind auch Verbindungen der Formel (I), in welcher R⁵ für Wasserstoff steht.

Bevorzugt sind auch Verbindungen der Formel (I), in welcher R⁶ für 2-Hydroxyeth-1-yl oder 4-Hydroxycyclohex-1-yl steht.

Bevorzugt sind auch Verbindungen der Formel (I), in welcher R⁷ für Wasserstoff steht.

Bevorzugt sind auch Verbindungen der Formel (I), in welcher
- R¹: für Methyl, Ethyl, n-Propyl, Methoxy oder Methoxymethyl steht,
- R²: für Wasserstoff oder Methyl steht,
und
- R³: für eine Gruppe der Formel 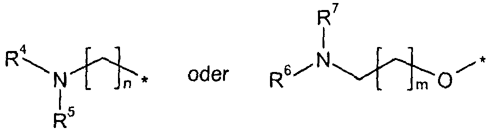
steht,
wobei
- *: die Anknüpfstelle an den Oxopyridin-Ring ist,
- n: für die Zahl 1, 2 oder 3 steht,
- m: für die Zahl 1 oder 2 steht,
- R⁴: für Methyl, Ethyl, Cyclopropyl, Cyclobutyl, 2-Methoxyethy-1-yl, 3-Methoxyprop-1-yl, Tetrahydrofuran-2-ylmethyl oder 1,4-Dioxan-2-ylmethyl steht,
- R⁵: für Wasserstoff, Methyl oder Ethyl steht,
oder
- R⁴ und R⁵: zusammen mit dem Stickstoffatom an das sie gebunden sind einen Pyrrolidin-1-yl-Ring, einen 2-Methoxymethyl-pyrrolidin-1-yl-Ring, einen Morpholin-4-yl-Ring, einen 1,1-Dioxo-thiomorpholin-4-yl-Ring, einen 1,4-Oxazepan-4-yl-Ring, einen 4-Methyl-piperazin-1-yl oder einen 4-Hydroxy-piperidin-1-yl-Ring bilden,
- R⁶: für Methyl, Ethyl, Cyclopropyl, Cyclobutyl, 2-Methoxyethy-1-yl, 3-Methoxyprop-1-yl, Tetrahydrofuran-2-ylmethyl oder 1,4-Dioxan-2-ylmethyl steht,
- R⁷: für Wasserstoff, Methyl oder Ethyl steht,
- R⁶ und R⁷: zusammen mit dem Stickstoffatom an das sie gebunden sind einen Pyrrolidin-1-yl-Ring, einen 2-Methoxymethyl-pyrrolidin-1-yl-Ring, einen Morpholin-4-yl-Ring, einen 1,1-Dioxo-thiomorpholin-4-yl-Ring, einen 1,4-Oxazepan-4-yl-Ring, einen 4-Methyl-piperazin-1-yl oder einen 4-Hydroxy-piperidin-1-yl-Ring bilden,
und ihre Salze, ihre Solvate und die Solvate ihrer Salze.

Bevorzugt sind auch Verbindungen der Formel (I), in welcher
- R¹: für Methyl, Ethyl, n-Propyl, Methoxy oder Methoxymethyl steht,
- R²: für Wasserstoff steht,
oder
- R¹: für Methyl steht,
- R²: für Methyl steht,
und
- R³: für eine Gruppe der Formel 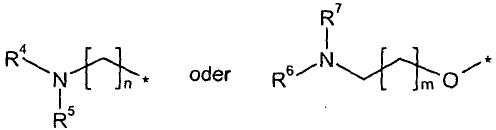 steht,
wobei
* die Anknüpfstelle an den Oxopyridin-Ring ist,
n für die Zahl 1, 2 oder 3 steht,
m für die Zahl 1 oder 2 steht,
R⁴ für Methyl, Ethyl oder Cyclopropyl steht,
R⁵ für Wasserstoff, Methyl oder Ethyl steht, oder
R⁴ und R⁵ zusammen mit dem Stickstoffatom an das sie gebunden sind einen Morpholin-4-yl-Ring, einen 4-Methyl-piperazin-1-yl oder einen 4-Hydroxy-piperidin-1-yl-Ring bilden,
R⁶ für Cyclopropyl, Cyclobutyl, 2-Methoxyethy-1-yl, Tetrahydrofuran-2-ylmethyl oder 1,4-Dioxan-2-ylmethyl steht,
R⁷ für Wasserstoff, Methyl oder Ethyl steht,
R⁶ und R⁷ zusammen mit dem Stickstoffatom an das sie gebunden sind einen Pyrrolidin-1-yl-Ring, einen 2-Methoxymethyl-pyrrolidin-1-yl-Ring, einen 1,1-Dioxothiomorpholin-4-yl-Ring, einen 1,4-Oxazepan-4-yl-Ring oder einen 4-Methylpiperazin-1-yl bilden,
und ihre Salze, ihre Solvate und die Solvate ihrer Salze.

Bevorzugt sind auch Verbindungen der Formel (I), in welcher
- R¹: für Methyl, Methoxy oder Methoxymethyl steht,
- R²: für Wasserstoff steht,
oder
- R¹: für Methyl steht,
- R²: für Methyl steht,
und
- R³: für eine Gruppe der Formel 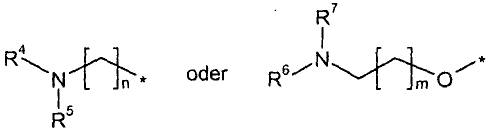
steht,
wobei
- *: die Anknüpfstelle an den Oxopyridin-Ring ist,
- n: für die Zahl 1, 2 oder 3 steht,
- m: für die Zahl 1 oder 2 steht,
- R⁴: für Methyl oder Cyclopropyl steht,
- R⁵: für Wasserstoff oder Methyl steht,
oder
- R⁴ und R⁵: zusammen mit dem Stickstoffatom an das sie gebunden sind einen 4-Methylpiperazin-1-yl oder einen 4-Hydroxy-piperidin-1-yl-Ring bilden,
- R⁶: für Cyclopropyl, Cyclobutyl oder 2-Methoxyethy-1-yl steht,
- R⁷: für Wasserstoff oder Methyl steht,
- R⁶ und R⁷: zusammen mit dem Stickstoffatom an das sie gebunden sind einen Pyrrolidin-1-yl-Ring, einen 2-Methoxymethyl-pyrrolidin-1-yl-Ring oder einen 4-Methylpiperazin-1-yl bilden,
und ihre Salze, ihre Solvate und die Solvate ihrer Salze.

Besonders bevorzugt ist auch die Verbindung 5-Chlor-N-{[(5S)-3-{4-[3-{2-[(trans-4-hydroxy-cyclohexyl)amino]ethyl}-2-oxopyridin-1(2H)-yl]-3-methoxyphenyl}-2-oxo-1,3-oxazolidin-5-yl]-methyl}thiophen-2-carboxamid der Formel 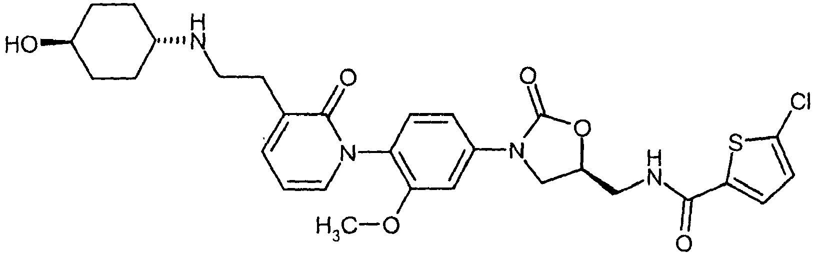 sowie ihre Salze, ihre Solvate und die Solvate ihrer Salze. Die Verbindung ist in Beispiel 6 beschrieben.

Besonders bevorzugt ist auch die Verbindung 5-Chlor-N-{[(5S)-3-{4-[3-{2-[(2-hydroxyethyl)-amino]ethyl}-2-oxopyridin-1(2H)-yl]-3-(methoxy-methyl)phenyl}-2-oxo-1,3-oxazolidin-5-yl]-ethyl}thiophen-2-carboxamid der Formel 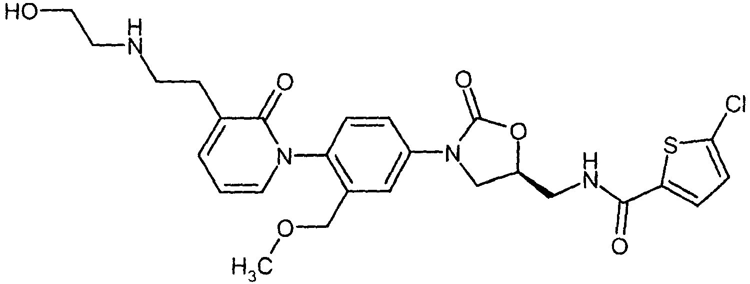 sowie ihre Salze, ihre Solvate und die Solvate ihrer Salze. Die Verbindung ist in Beispiel 9 beschrieben.

Besonders bevorzugt ist auch die Verbindung 5-Chlor-N-{[(5S)-3-{4-[3-{2-[(trans-4-hydroxy-cyclohexyl)amino]ethyl}-2-oxopyridin-1(2H)-yl]-3-(methoxymethyl)phenyl}-2-oxo-1,3-oxazolidin-5-yl]methyl}thiophen-2-carboxamid der Formel 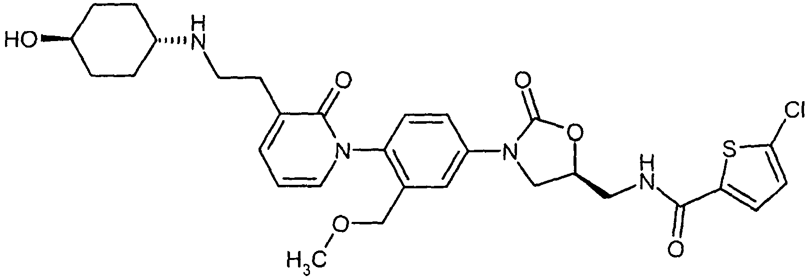 sowie ihre Salze, ihre Solvate und die Solvate ihrer Salze. Die Verbindung ist in Beispiel 10 beschrieben.

Besonders bevorzugt ist auch die Verbindung 5-chlor-N-{[(5S)-3-{4-[3-{2-[(trans-4-hydroxy-cyclohexyl)amino]ethoxy}-2-oxopyridin-1(2H)-yl]-3-methylphenyl}-2-oxo-1,3-oxazolidin-5-yl]-methyl}-thiophen-2-carboxamid der Formel 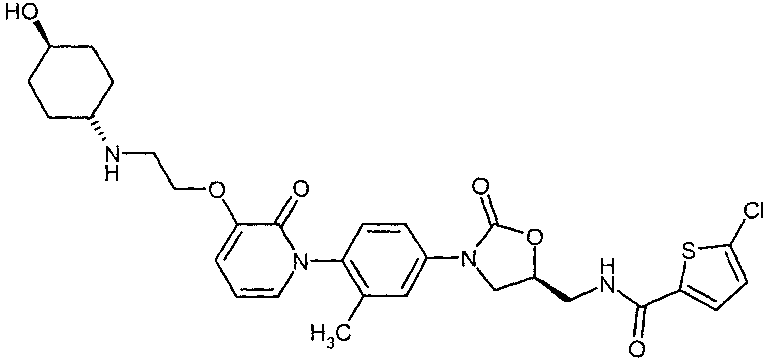 sowie ihre Salze, ihre Solvate und die Solvate ihrer Salze. Die Verbindung ist in Beispiel 21 beschrieben.

Besonders bevorzugt ist auch die Verbindung 5-Chlor-N-{[(5S)-3-{4-[3-{2-[(2-hydroxyethyl)-amino]ethyl}-2-oxopyridin-1(2H)-yl]-3,5-dimethylphenyl}-2-oxo-1,3-oxazolidin-5-yl]methyl}-thiophen-2-carboxamid der Formel 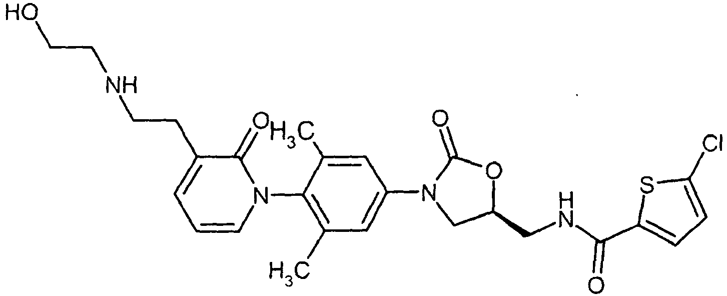 sowie ihre Salze, ihre Solvate und die Solvate ihrer Salze. Die Verbindung ist in Beispiel 37 beschrieben.

Besonders bevorzugt ist auch die Verbindung 5-Chlor-N-{[(5S)-3-{4-[3-{2-[(trans-4-hydroxy-cyclohexyl)amino]ethyl}-2-oxopyridin-1(2H)-yl]-3,5-dimethylphenyl}-2-oxo-1,3-oxazolidin-5-yl]-methyl}thiophen-2-carboxamid der Formel 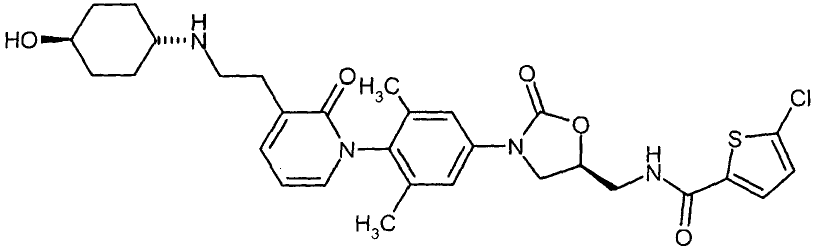 sowie ihre Salze, ihre Solvate und die Solvate ihrer Salze. Die Verbindung ist in Beispiel 38 beschrieben.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Gegenstand der Erfindung ist weiterhin ein Verfahren zur Herstellung der Verbindungen der Formel (I), oder ihrer Salze, ihrer Solvate oder der Solvate ihrer Salze, wobei
[A] die Verbindungen der Formel 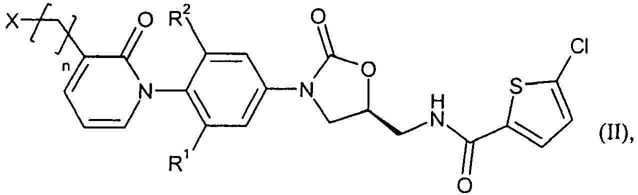 in welcher n, R¹ und R² die oben angegebene Bedeutung haben, und
- X: für Hydroxy oder Brom steht,
mit Verbindungen der Formel 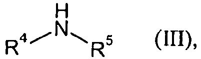 in welcher R⁴ und R⁵ die oben angegebene Bedeutung haben,
zu Verbindungen der Formel 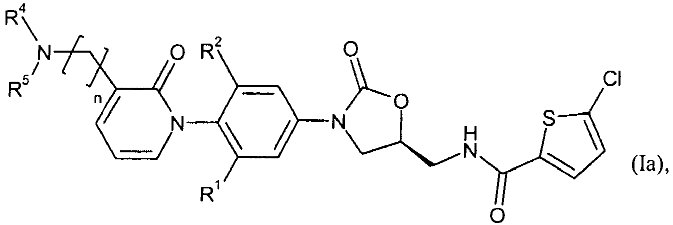 in welcher n, R¹, R², R⁴ und R⁵ die oben angegebene Bedeutung haben,
umgesetzt werden,
oder
[B] die Verbindungen der Formel 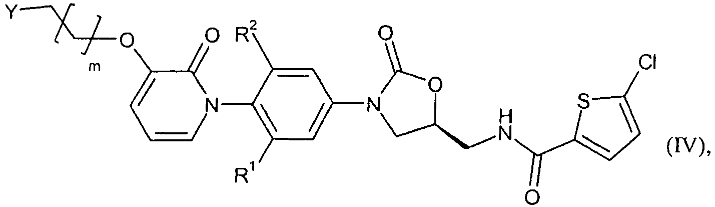 in welcher m, R¹ und R² die oben angegebene Bedeutung haben, und
- Y: für Hydroxy oder Chlor steht,
mit Verbindungen der Formel 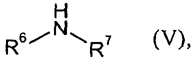 in welcher R⁶ und R⁷ die oben angegebene Bedeutung haben,
zu Verbindungen der Formel 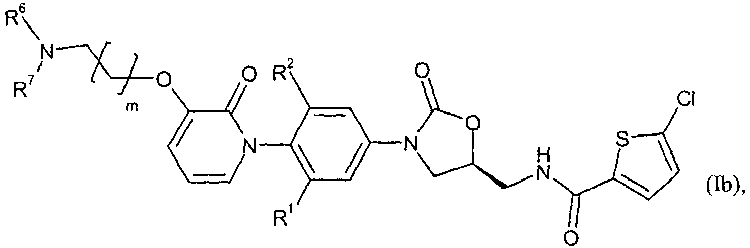 in welcher m, R¹, R², R⁶ und R⁷ die oben angegebene Bedeutung haben,
umgesetzt werden.

Die Verbindungen der Formeln (Ia) und (Ib) bilden die Verbindungen der Formel (I).

Die freie Base der Salze kann zum Beispiel durch Chromatographie an einer Reversed Phase Säule mit einem Acetonitril-Wasser-Gradienten unter Zusatz einer Base erhalten werden, insbesondere durch Verwendung einer RP18 Phenomenex Luna C18(2) Säule und Diethylamin als Base, oder durch Lösen der Salze in einem organischen Lösungsmittel und Ausschütteln mit wässrigen Lösungen von basischen Salzen wie Natriumhydrogencarbonat.

Ebenfalls beschrieben ist ein Verfahren zur Herstellung der Verbindungen der Formel (I) oder ihrer Solvate, bei dem Salze der Verbindungen oder Solvate der Salze der Verbindungen durch Chromatographie unter Zusatz einer Base in die Verbindungen überführt werden.

Für den Fall, dass X Hydroxy ist, erfolgt die Umsetzung nach Verfahren [A] im Allgemeinen in inerten Lösungsmitteln, in Gegenwart von Trifluormethansulfonsäureanhydrid, gegebenenfalls in Gegenwart einer Base, bevorzugt in einem Temperaturbereich von -78°C bis Raumtemperatur bei Normaldruck.

Inerte Lösungsmittel sind beispielsweise Dichlormethan, Diethylether, Nitromethan, 1,2-Dichlorethan oder Acetonitril, bevorzugt ist Dichlormethan.

Basen sind beispielsweise 2,6-Dimethylpyridin, Pyridin, 2,6-Di-tert.-butyl-4-methyl-pyridin, Diisopropylethylamin, 2,6-Lutidin, Trimethylamin, bevorzugt ist 2,6-Dimethylpyridin.

Für den Fall, dass X Brom ist, erfolgt die Umsetzung nach Verfahren [A] im Allgemeinen in inerten Lösungsmitteln, bevorzugt in einem Temperaturbereich von -78°C bis Raumtemperatur bei Normaldruck.

Inerte Lösungsmittel sind beispielsweise Dimethylformamid.

Für den Fall, dass Y Hydroxy ist, erfolgt die Umsetzung nach Verfahren [B] im Allgemeinen in inerten Lösungsmitteln, in Gegenwart von Trifluormethansulfonsäureanhydrid, gegebenenfalls in Gegenwart einer Base, bevorzugt in einem Temperaturbereich von -78°C bis Raumtemperatur bei Normaldruck.

Inerte Lösungsmittel sind beispielsweise Dichlormethan, Diethylether, Nitromethan, 1,2-Dichlorethan oderAcetonitril, bevorzugt ist Dichlormethan.

Basen sind beispielsweise 2,6-Dimethylpyridin, Pyridin, 2,6-Di-tert.-butyl-4-methyl-pyridin, Diisopropylethylamin, 2,6-Lutidin, Trimethylamin, bevorzugt ist 2,6-Dimethylpyridin.

Für den Fall, dass Y Chlor ist, erfolgt die Umsetzung nach Verfahren [B] im Allgemeinen in inerten Lösungsmitteln, gegebenenfalls in Gegenwart eines Halogensalzes, bevorzugt in einem Temperaturbereich von Raumtemperatur bis zum Rückfluss des Lösungsmittels bei Normaldruck.

Inerte Lösungsmittel sind beispielsweise 1,2-Dimethoxyethan.

Halogensalze sind beispielsweise Natriumiodid.

Die Verbindungen der Formeln (III) und (V) sind bekannt oder lassen sich nach bekannten Verfahren aus den entsprechenden Ausgangsverbindungen synthetisieren.

Die Verbindungen der Formel (II), in denen X für Hydroxy steht, sind bekannt oder können hergestellt werden, indem die Verbindung der Formel 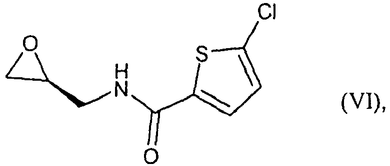 in der ersten Stufe mit Verbindungen der Formel 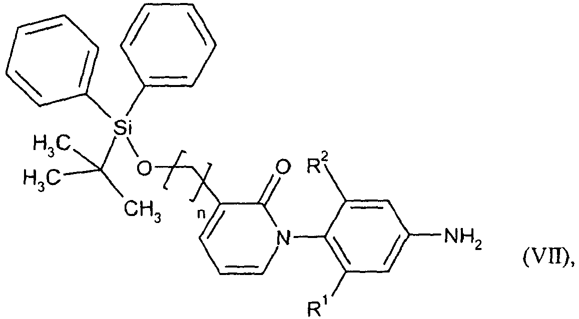 in welcher n, R¹ und R² die oben angegebene Bedeutung haben,
zu Verbindungen der Formel 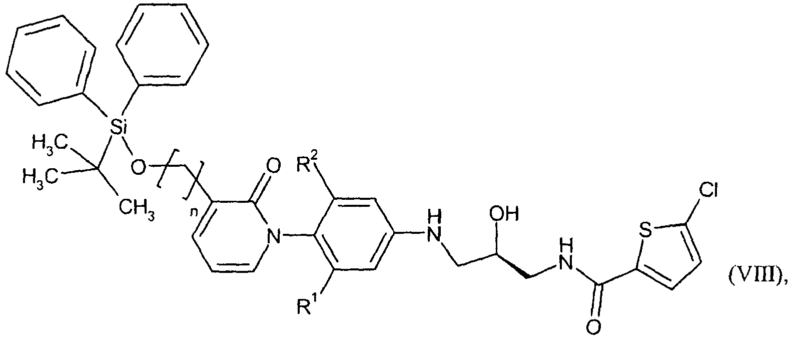 in welcher n, R¹ und R² die oben angegebene Bedeutung haben,
umgesetzt werden,
in der zweiten Stufe in Anwesenheit von Phosgen oder Phosgenäquivalenten wie z.B. Carbonyldiimidazol (CDI) cyclisiert werden,
und in der dritten Stufe die Silylgruppe abgespalten wird und man zu den Verbindungen der Formel (II) gelangt.

Die Umsetzung der ersten Stufe erfolgt im Allgemeinen in inerten Lösungsmitteln, in Gegenwart einer Lewissäure, bevorzugt in einem Temperaturbereich von Raumtemperatur bis zum Rückfluss des Lösungsmittels bei Normaldruck.

Inerte Lösungsmittel sind beispielsweise polare, aprotische Lösungsmittel wie beispielsweise Acetonitril, Butyronitril, Dichlormethan oder Chloroform, bevorzugt ist Acetonitril.

Lewis-Säuren sind beispielsweise Magnesiumperchlorat, Ytterbium(III)trifluormethansulfonat, Lithiumbromid, Magnesiumtriflat oder Aluminiumtrichlorid, bevorzugt ist Magnesiumperchlorat.

Die Umsetzung der zweiten Stufe erfolgt im Allgemeinen in inerten Lösungsmitteln, in Gegenwart einer Base, bevorzugt in einem Temperaturbereich von Raumtemperatur bis zum Rückfluss des Lösungsmittels bei Normaldruck.

Inerte Lösungsmittel sind beispielsweise polare, aprotische Lösungsmittel wie beispielsweise Acetonitril oder Butyronitril.

Basen sind beispielsweise starke, tertiäre Aminbasen wie beispielsweise 4-N,N-Dimethylaminopyridin.

Bevorzugt ist die Reaktion mit N,N'-Carbonyldiimidazol als Kohlensäure-Äquivalent unter Zusatz von 4-N,N-Dimethylaminopyridin als Base.

Die Umsetzung der dritten Stufe erfolgt nach dem Fachmann bekannten Methoden, z. B. durch Umsetzung mit Tetrabutylammoniumfluorid in einem Lösungsmittel, wie z. B. Tetrahydrofuran, oder durch Umsetzung mit Chlorwasserstoff in Methanol, bevorzugt in einem Temperaturbereich von Raumtemperatur bis zum Rückfluss des Lösungsmittels bei Normaldruck.

Die Verbindung der Formel (VI) ist bekannt oder läßt sich nach bekannten Verfahren aus den entsprechenden Ausgangsverbindungen synthetisieren.

In einem alternativen Verfahren können Verbindungen der Formel (II), in denen X für Hydroxy steht, hergestellt werden, indem die Verbindungen der Formel 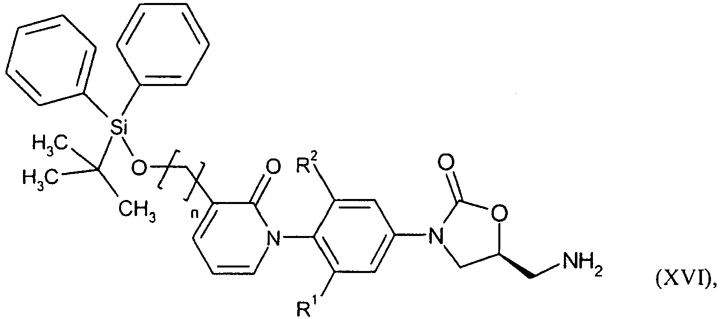 in welcher n, R¹ und R² die oben angegebene Bedeutung haben,
mit Verbindungen der Formel 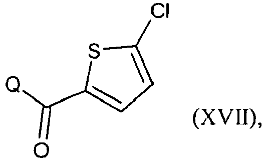 in welcher
Q für Halogen, bevorzugt Brom oder Chlor, oder Hydroxy steht,
umgesetzt werden und anschließend die Silylgruppe abgespalten wird.

Für den Fall, dass Q Halogen ist, erfolgt die Umsetzung im Allgemeinen in inerten Lösungsmitteln, gegebenenfalls in Gegenwart einer Base, bevorzugt in einem Temperaturbereich von -30°C bis 50°C bei Normaldruck.

Inerte Lösungsmittel sind beispielsweise Tetrahydrofuran, Methylenchlorid, Pyridin, Dioxan oder Dimethylformamid, bevorzugt ist Tetrahydrofuran oder Methylenchlorid.

Basen sind beispielsweise Triethylamin, Diisopropylethylamin oder N-Methylmorpholin, bevorzugt ist Diisopropylethylamin.

Für den Fall, dass Q Hydroxy ist, erfolgt die Umsetzung im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Als Dehydratisierungsreagenzien eignen sich hierbei beispielsweise Carbodiimide wie z.B. *N,N'-*Diethyl-, *N,N,*'-Dipropyl-, *N,N*'-Diisopropyl-, *N,N*'-Dicyclohexylcarbodiimid, *N*-(3-Di-methylaminoisopropyl)-*N*'-ethylcarbodiimid-hydrochlorid (EDC), *N*-Cyclohexylcarbodiimid-*N*'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert-*Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)-phosphoniumhexafluorophosphat, oder *O*-(Benzotriazol-1-yl)-*N,N,N',N'*-tetra-methyluronium-hexafluorophosphat (HBTU), 2-(2-Oxo-1-(2H)-pyridyl)-1,1,3,3-tetramethyluroniumtetrafluoro-borat (TPTU) oder *O*-(7-Azabenzotriazol-1-yl)-*N,N,N,N'*-tetramethyl-uroniumhexafluorophosphat (HATU), oder 1-Hydroxybenzotriazol (HOBt), oder Benzotriazol-1-yloxytris(dimethylamino)-phosphoniumhexafluorophosphat (BOP), oder *N*-Hydroxysuccinimid, oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4-Dimethylaminopyridin oder Diisopropylethylamin.

Vorzugsweise wird die Kondensation mit HATU oder mit EDC in Gegenwart von HOBt durchgeführt.

Die Abspaltung der Silylgruppe erfolgt nach dem Fachmann bekannten Methoden, z. B. durch Umsetzung mit Tetrabutylammoniumfluorid in einem Lösungsmittel, wie z. B. Tetrahydrofuran, oder durch Umsetzung mit Chlorwasserstoff in Methanol, bevorzugt in einem Temperaturbereich von Raumtemperatur bis zum Rückfluss des Lösungsmittels bei Normaldruck.

Die Verbindungen der Formel (XVII) sind bekannt oder lassen sich nach bekannten Verfahren aus den entsprechenden Ausgangsverbindungen synthetisieren.

Die Verbindungen der Formel (II), in denen X für Brom steht, sind bekannt oder können hergestellt werden, indem die Verbindungen der Formel (II), in denen X für Hydroxy steht, mit Thionylbromid umgesetzt werden, wie in Beispiel 11A beschrieben.

Die Verbindungen der Formel (VII) sind bekannt oder können hergestellt werden, indem in Verbindungen der Formel 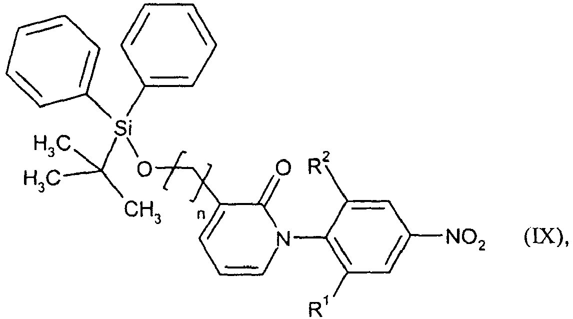 in welcher n, R¹ und R² die oben angegebene Bedeutung haben,
die Nitrogruppe reduziert wird.

Die Umsetzung erfolgt im Allgemeinen mit einem Reduktionsmittel in inerten Lösungsmitteln, bevorzugt in einem Temperaturbereich von Raumtemperatur bis zum Rückfluss der Lösungsmittel bei Normaldruck bis 3 bar.

Reduktionsmittel sind beispielsweise Palladium auf Aktivkohle und Wasserstoff, Zinndichlorid, Titantrichlorid oder Ammoniumformiat und Palladium auf Aktivkohle in einem Gemisch aus Ethanol und Essigsäureethylester, bevorzugt ist Palladium auf Aktivkohle und Wasserstoff oder Zinndichlorid.

Inerte Lösungsmittel sind beispielsweise Ether wie Diethylether, Methyl-*tert*.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder *tert.-*Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Pyridin, als Lösungsmittel sind bevorzugt Tetrahydrofuran, Methanol, Ethanol, iso-Propanol oder im Falle von Zinndichlorid in Dimethylformamid.

Die Verbindungen der Formel (IX) sind bekannt, lassen sich nach bekannten Verfahren aus den entsprechenden Ausgangsverbindungen synthetisieren oder können analog den im Beispielteil beschriebenen Verfahren hergestellt werden.

Die Verbindungen der Formel (IV), in denen Y für Chlor steht, sind bekannt oder können hergestellt werden, indem Verbindungen der Formel 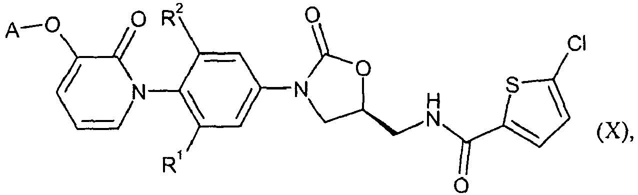 in welcher R¹ und R² die oben angegebene Bedeutung haben, und
- A: für Methyl oder für (2-Methoxyethoxy)methyl steht,
in der ersten Stufe durch Spaltung der Methoxy- oder (2-Methoxyethoxy)methoxy-Gruppe zu Verbindungen der Formel 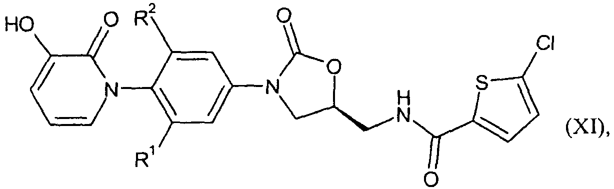 in welcher R¹ und R² die oben angegebene Bedeutung haben,
und in der zweiten Stufe mit Verbindungen der Formel 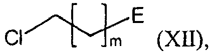 in welcher m die oben angegebene Bedeutung hat, und
- E: für Brom oder Iod steht,
umgesetzt werden.

Für den Fall, dass A für Methyl steht, erfolgt die Umsetzung der ersten Stufe im Allgemeinen in inerten Lösungsmitteln, in Gegenwart von Bromtribromid, bevorzugt in einem Temperaturbereich von -78°C bis Raumtemperatur bei Normaldruck.

Inerte Lösungsmittel sind beispielsweise Dichlormethan oder 1,2-Dichlorethan, bevorzugt ist Dichlormethan.

Für den Fall, dass A für (2-Methoxyethoxy)methyl steht, erfolgt die Umsetzung der ersten Stufe im Allgemeinen in inerten Lösungsmitteln, in Gegenwart von Trifluoressigsäure, bevorzugt in einem Temperaturbereich von Raumtemperatur bis zum Rückfluss des Lösungsmittels bei Normaldruck.

Inerte Lösungsmittel sind beispielsweise Dichlormethan oder 1,2-Dichlorethan, bevorzugt ist Dichlormethan.

Die Umsetzung der zweiten Stufe erfolgt im Allgemeinen in inerten Lösungsmitteln, in Gegenwart einer Base, bevorzugt in einem Temperaturbereich von Raumtemperatur bis zum Rückfluss des Lösungsmittels bei Normaldruck. Gegebenenfalls wird diese Reaktion in einer Mikrowelle durchgeführt.

Inerte Lösungsmittel sind beispielsweise N,N-Dimethylformamid oder 1-Methyl-2-pyrrolidin, bevorzugt ist N,N-Dimethylformamid.

Basen sind beispielsweise Alkalicarbonate wie Cäsiumcarbonat, Natrium- oder Kaliumcarbonat.

Die Verbindungen der Formel (XII) sind bekannt oder lassen sich nach bekannten Verfahren aus den entsprechenden Ausgangsverbindungen synthetisieren.

Die Verbindungen der Formel (IV), in denen Y für Hydroxy steht, sind bekannt oder können hergestellt werden, indem in der zweiten Stufe der oben angegebenen Synthese die Verbindungen der Formel (XI) mit (2-Bromethoxy)(tert.-butyl)dimethylsilan umgesetzt werden und anschließend die Silylgruppe abgespalten wird.

Die Verbindungen der Formel (X) sind bekannt oder können hergestellt werden, indem die Verbindung der Formel (VI) in der ersten Stufe mit Verbindungen der Formel 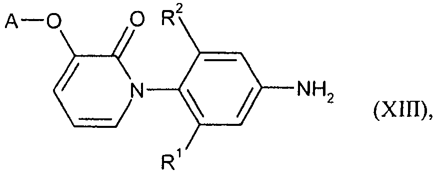 in welcher R¹ und R² die oben angegebene Bedeutung haben, und
- A: für Methyl oder für (2-Methoxyethoxy)methyl steht,
zu Verbindungen der Formel 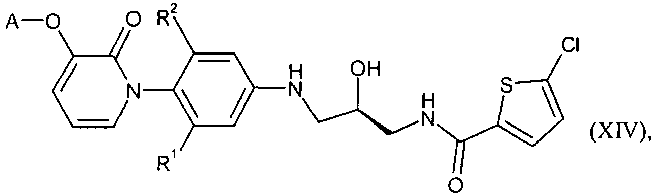 in welcher R¹ und R² die oben angegebene Bedeutung haben, und
- A: für Methyl oder für (2-Methoxyethoxy)methyl steht,
umgesetzt werden,
in der zweiten Stufe in Anwesenheit von Phosgen oder Phosgenäquivalenten wie z.B. Carbonyldiimidazol (CDI) zu den Verbindungen der Formel (X) cyclisiert werden.

Die Umsetzungen der ersten Stufe erfolgt nach dem Verfahren was für die Umsetzung der Verbindung der Formel (VI) mit Verbindungen der Formel (VII) zu Verbindungen der Formel (VIII) beschrieben ist.

Die Umsetzungen der zweiten Stufe erfolgt nach dem Verfahren was für die Umsetzung von Verbindungen der Formel (VIII) zu Verbindungen der Formel (II) beschrieben ist.

Die Verbindungen der Formel (XIII) sind bekannt oder können hergestellt werden, indem in Verbindungen der Formel 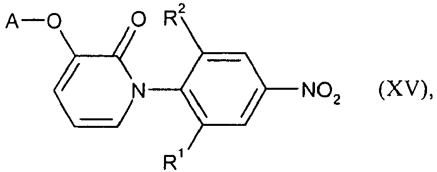 in welcher R¹ und R² die oben angegebene Bedeutung haben, und
- A: für Methyl oder für (2-Methoxyethoxy)methyl steht,
die Nitrogruppe reduziert wird.

Die Umsetzungen erfolgt nach dem Verfahren was für die Umsetzung von Verbindungen der Formel (IX) zu Verbindungen der Formel (VII) beschrieben ist.

Die Verbindungen der Formel (XV) sind bekannt oder lassen sich nach bekannten Verfahren aus den entsprechenden Ausgangsverbindungen synthetisieren.

Die Verbindungen der Formel (XVI) sind bekannt oder können hergestellt werden, indem Verbindungen der Formel 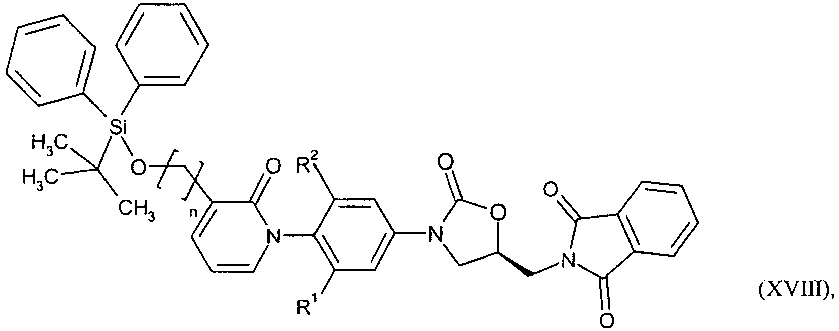 in welcher n, R¹ und R² die oben angegebene Bedeutung haben,
die Phthalimid-Schutzgruppe gespalten wird.

Die Umsetzung erfolgt im Allgemeinen mit einer wässrigen Methylamin-Lösung oder einer Hydrazinhydrat-Lösung in Ethanol, bevorzugt mit einer wässrigen Methylamin-Lösung unter Rückfluss der Lösungsmittel bei Normaldruck.

Die Verbindungen der Formel (XVIII) sind bekannt oder können hergestellt werden, indem in der ersten Stufe Verbindungen der Formel (VII) mit einer Verbindung der Formel 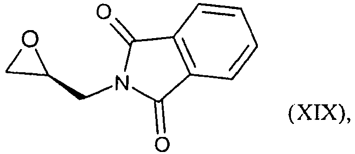 zu Verbindungen der Formel 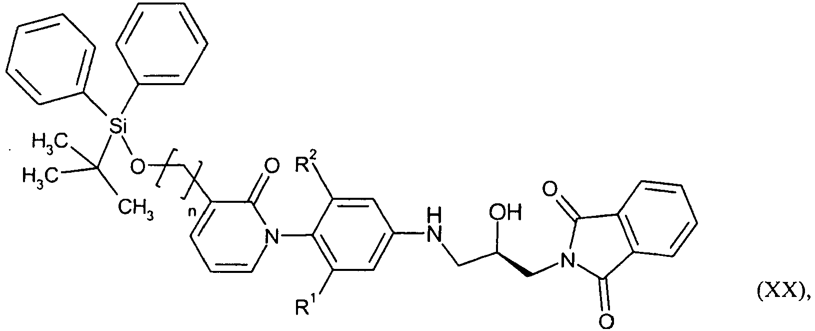 in welcher n, R¹ und R² die oben angegebene Bedeutung haben,
umgesetzt werden,
und in der zweiten Stufe in Anwesenheit von Phosgen oder Phosgenäquivalenten wie z.B. Carbonyldiimidazol (CDI) zu den Verbindungen der Formel (XVIII) cyclisiert werden.

Die Umsetzungen der ersten Stufe erfolgt nach dem Verfahren was für die Umsetzung der Verbindung der Formel (VI) mit Verbindungen der Formel (VII) zu Verbindungen der Formel (VIII) beschrieben ist.

Die Umsetzungen der zweiten Stufe erfolgt nach dem Verfahren was für die Umsetzung von Verbindungen der Formel (VIII) zu Verbindungen der Formel (II) beschrieben ist.

Die Verbindung der Formel (XIX) ist bekannt oder läßt sich nach bekannten Verfahren aus den entsprechenden Ausgangsverbindungen synthetisieren.

Die Herstellung der erfindungsgemäßen Verbindungen kann durch die folgenden Syntheseschemata veranschaulicht werden:

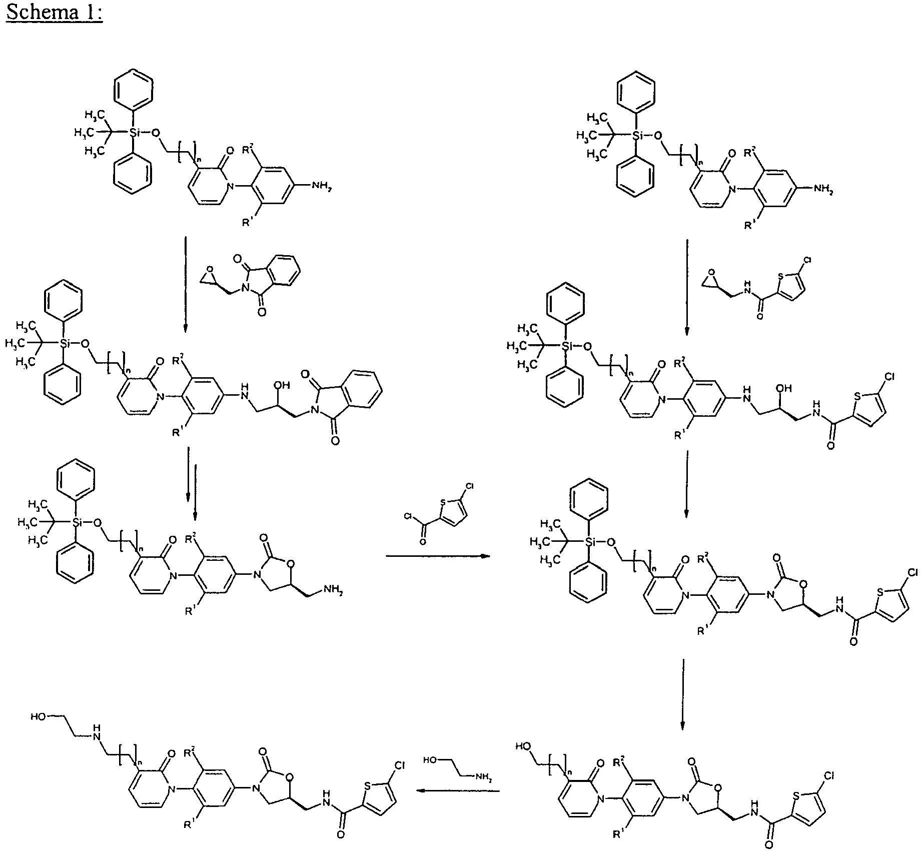

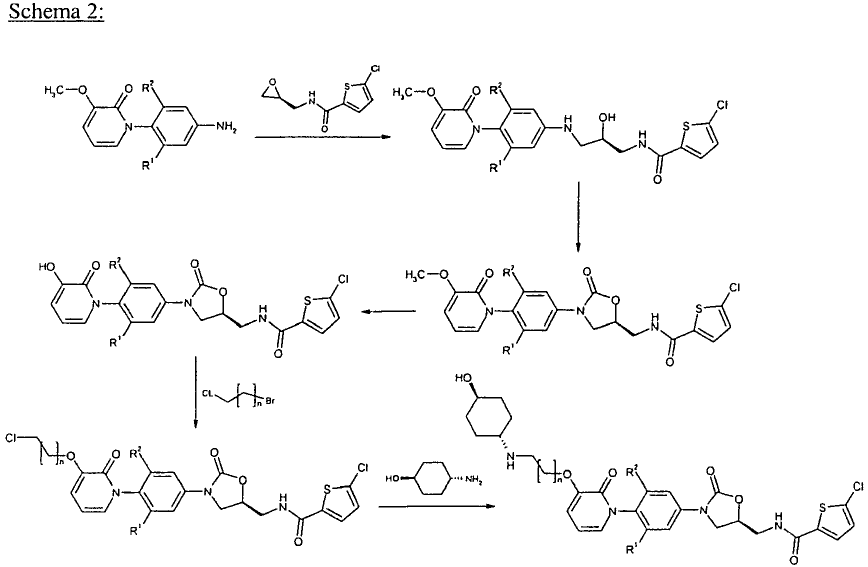

Die erfindungsgemäßen Verbindungen zeigen ein nicht vorhersehbares, wertvolles pharmakologisches Wirkspektrum.

Sie eignen sich daher zur Verwendung als Arzneimittel zur Behandlung und/oder Prophylaxe von Krankheiten bei Menschen und Tieren.

Bei den erfindungsgemäßen Verbindungen handelt es sich um duale Inhibitoren der Blutgerinnungsfaktoren Xa und Thrombin (Faktor IIa), die insbesondere als Antikoagulantien wirken. Die Verbindungen hemmen sowohl Thrombin als auch Faktor Xa, verhindern durch Hemmung der Thrombinproduktion und -aktivität auf Gerinnseln deren potentielles Wachstum und weisen ein breites therapeutisches Fenster auf.

Darüber hinaus verfügen die erfindungsgemäßen Verbindungen über günstige physikochemische Eigenschaften, wie beispielsweise eine gute Löslichkeit in Wasser und physiologischen Medien, was für ihre therapeutische Anwendung von Vorteil ist.

Weitere Gegenstand der vorliegenden Erfindung ist der Einsatz der erfindungsgemäßen Verbindunger zur Herstellung von Arzneimitlein zur Behandlung und/oder Prophylaxe von Erkrankungen, vorzugsweise von thromboembolischen Erkrankungen und/oder thromboembolischen Komplikationen.

Zu den "thromboembolischen Erkrankungen" im Sinne der vorliegenden Erfindung zählen insbesondere Erkrankungen wie Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie oder aortokoronarem Bypass, periphere arterielle Verschlusskrankheiten, Lungenembolien, tiefe venöse Thrombosen und Nierenvenenthrombosen, transitorische ischämische Attacken sowie thrombotischer und thromboembolischer Hirnschlag.

Die die erfindungsgemäßen Verbindungen eignen sich daher auch zur Prävention und Behandlung von kardiogenen Thromboembolien, wie beispielsweise Hirn-Ischämien, Schlaganfall und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, ferner bei Patienten mit Herzklappen-Erkrankungen oder mit künstlichen Herzklappen.

Thromboembolische Komplikationen treten ferner auf bei mikroangiopathischen hämolytischen Anämien, extrakorporalen Blutkreisläufen, wie Hämodialyse, sowie Herzklappenprothesen. Außerdem kommen die erfindungsgemäßen Verbindungen auch für die Prophylaxe und/oder Behandlung von atherosklerotischen Gefäßerkrankungen und entzündlichen Erkrankungen wie rheumatische Erkrankungen des Bewegungsapparats in Betracht, darüber hinaus ebenso für die Prophylaxe und/oder Behandlung der Alzheimer'schen Erkrankung. Außerdem können die erfindungsgemäßen Verbindungen zur Inhibition des Tumorwachstums und der Metastasenbildung, bei Mikroangiopathien, altersbedingter Makula-Degeneration, diabetischer Retinopathie, diabetischer Nephropathie und anderen mikrovaskulären Erkrankungen sowie zur Prävention und Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Außerdem kommen die erfindungsgemäßen Verbindungen auch für die Prophylaxe und/oder Behandlung von pulmonaler Hypertonie in Betracht.

Der Begriff "pulmonale Hypertonie" umfasst bestimmte Formen der pulmonalen Hypertonie, wie sie z.B. von der Weltgesundheitsorganisation (WHO) festgelegt worden sind (Clinical Classification of Pulmonary Hypertension, Venedig 2003). Als Beispiele seien genannt, die pulmonale arterielle Hypertonie, die pulmonale Hypertonie bei Erkrankungen des linken Herzens, die pulmonale Hypertonie bei Lungenerkrankung und/oder Hypoxie und die Pulmonale Hypertonie aufgrund chronischer Thrombembolien (CTEPH).

Die "pulmonale arterielle Hypertonie" beinhaltet die Idiopathische Pulmonale Arterielle Hypertonie (IPAH, früher auch als primäre pulmonale Hypertonie bezeichnet), die Familiär bedingte Pulmonale Arterielle Hypertonie (FPAH) und die Assoziierte Pulmonal-Arterielle Hypertonie (APAH), die assoziiert ist mit Kollagenosen, kongenitalen systemisch-pulmonalen Shuntvitien, portaler Hypertension, HIV-Infektionen, der Einnahme bestimmter Drogen und Medikamente, mit anderen Erkrankungen (Schilddrüsenerkrankungen, Glykogenspeicherkrankheiten, Morbus Gaucher, hereditäre Teleangiektasie, Hämoglobinopathien, myeloproliferative Erkrankungen, Splenektomie), mit Erkrankungen mit einer signifikanten venösen/kapillären Beteiligung wie der pulmonal-venookklusiven Erkrankung und der pulmonal-kapillären Hämangiomatose, sowie die persistierende pulmonale Hypertonie der Neugeborenen.

Die pulmonale Hypertonie bei Erkrankungen des linken Herzens beinhaltet die Erkrankung des linken Vorhofes oder Ventrikels und Mitral- oder Aortenklappenfehler.

Die pulmonale Hypertonie bei Lungenerkrankung und/oder Hypoxie beinhaltet chronisch obstruktive Lungenerkrankungen, interstitielle Lungenerkrankung, Schlafapnoe-Syndrom, alveolärer Hypoventilation, chronische Höhenkrankheit und anlagebedingte Fehlbildungen.

Die Pulmonale Hypertonie aufgrund chronischer Thrombembolien (CTEPH) beinhaltet den thrombembolischen Verschluss proximaler Lungenarterien, den thrombembolischen Verschluss distaler Lungenarterien und nicht-thrombotische Lungenembolien (Tumor, Parasiten, Fremdkörper).

Weiterer Gegenstand der vorliegenden Erfindung ist die Verwendung der erfindungsgemäßen Verbindungen zur Herstellung von Arzneimitteln zur Behandlung und/oder Prophylaxe von pulmonaler Hypertonie bei Sarkoidose, Histiozytose X und Lymphangiomatosis.

Außerdem kommen die erfindungsgemäßen Substanzen auch zur Behandlung von pulmonalen und hepatischen Fibrosen in Betracht.

Außerdem kommen die erfindungsgemäßen Verbindungen auch für die Behandlung und/oder Prophylaxe von Sepsis (oder Septikämie), Systemic Inflammatory Syndrome (SIRS), septische Organdysfunktion, septisches Organversagen und Muliorganversagen, Acute Respiratory Distress Syndrome (ARDS), Acute Lung Injury (ALI), Septischer Schock, DIC ("Disseminated Intravascular coagulation", oder "Verbrauchskoagulopathie") und/oder des septischen Organversagens in Betracht.

"Sepsis" wird definiert als das Vorliegen einer Infektion und eines "systemic inflammatory response syndrome" (nachfolgend mit "SIRS" bezeichnet). SIRS tritt im Rahmen von Infekten, aber auch von anderen Zuständen wie Verletzungen, Verbrennungen, Schock, Operationen, Ischämie, Pankreatitis, Reanimation oder Tumoren auf. Nach der Definition des ACCP/SCCM Consensus Conference Committee von 1992 (Crit Care Med 1992;20:864-874) werden die zur Diagnose "SIRS" erforderlichen Symptome zur Diagnose und Meßparameter beschrieben (u.a. veränderte Körpertemperatur, erhöhte Herzfrequenz, Atemschwierigkeiten und verändertes Blutbild). In der späteren (2001) SCCM/ESICM/ACCP/ATS/SIS International Sepsis Definitions Conference wurden die Kriterien im Wesentlichen beibehalten, in Details jedoch verfeinert (Levy et al., Crit Care Med 2003;31:1250-1256).

Im Verlauf einer Sepsis kann es zur generalisierten Aktivierung des Gerinnungssystems kommen ("Disseminated Intravascular coagulation", oder "Verbrauchskoagulopathie", nachfolgend als "DIC" bezeichnet) mit Mikrothrombosierung in verschiedenen Organen und sekundärer Blutungskomplikationen. Außerdem kann es es zur endothelialen Schädigung mit Erhöhung der Gefäßpermeabilität und Austritt von Flüssigkeit und Proteinen in den Extravasalraum kommen. Im weiteren Verlauf kann ein Versagen eines Organs (z.B. Nierenversagen, Leberversagen, Atemversagen, zentralnervöse Defizite und Herz-/Kreislaufversagen) oder zum Multiorganversagen kommen. "Septischer Schock" bezeichnet das Auftreten einer behandlungspflichtigen Blutdruckerniedrigung, die eine weitere Organschädigung begünstigt und mit einer Verschlechterung der Prognose einhergeht.

Krankheitserreger können Bakterien (gram-negativ und gram-positiv), Pilze, Viren und/oder Eukaryonten sein. Eintrittspforte bzw. Primärinfektion können z.B. Pneumonie, Harnwegsinfekt, Peritonitis sein. Die Infektion kann, muß aber nicht zwingend, mit einer Bakteriämie einhergehen.

DIC und/oder SIRS können im Rahmen einer Sepsis, aber auch infolge von Operationen, Tumorerkrankungen, Verbrennungen oder anderen Verletzungen auftreten. Bei der DIC kommt es an der Oberfäche von geschädigten Endothelzellen, Fremdkörperoberflächen oder veretztem extravaskulärem Gewebe zur massiven Aktivierung des Gerinnungssystems. Als Folge kommt es zur Gerinnung in kleinen Gefäßen verschiedener Organe mit Hypoxie und anschließender Organdysfunktion. Sekundär kommt es zum Verbrauch von Gerinnungsfaktoren (z.B. Faktor X, Prothrombin und Fibrinogen) und Plättchen, wodurch die Gerinnungsfähigkeit des Blutes herabgesetzt wird und schwere Blutungen auftreten können.

Die Therapie der Sepsis besteht einerseits in der konsequenten Beseitigung der infektiösen Ursache, z.B. durch operative Herdsanierung und Antibiose. Andererseits besteht sie in der temporären intensivmedizinischen Unterstützung der beeinträchtigten Organsysteme. Therapien der verschiedenen Stadien dieser Erkrankung sind z.B. in folgender Publikation beschrieben (Dellinger et al., Crit Care Med 2004;32:858-873). Für die DIC existieren keine erwiesenermaßen effektive Therapien.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- Antibiotische Therapie
   Verschiedene Antibiotika oder antifungale Medikamenten-Kombinationen kommen in Frage, entweder als kalkulierte Therapie (vor Vorliegen des mikrobiellen Befundes) oder als spezifische Therapie.
- Flüssigkeitstherapie
   z.B. Kristalloide oder kolloidale Flüssigkeiten.
- Vasopressoren
   z.B. Norepinephrine, Dopamine oder Vasopressin
- Inotrope Therapie
   z.B. Dobutamin
- Kortikosteroide
   z.B. Hydrokortison, oder Fludrokortison
- rekombinantes humanes aktivierte Protein C
   Xigris
- Blutprodukte
   z.B. Erythrozytenkonzentrate, Thrombozytenkonzentrate, Erythropietin oder Fresh Frozen Plasma
- Maschinelle Beatmung bei sepsis-induziertem Acute Lung Injury (ALI)
   bzw. Acute Respiratory Distress Syndrome (ARDS)
   z.B. Permissive Hyperkapnie, niedrigere Tidalvolumina
- Sedierung, Analgesierung und neuromuskuläre Blockade
   Sedierung: z.B. Diazepam, Lorazepam, Midazolam oder Propofol. Opioide: z.B. Fentanyl, Hydromorphon, Morphin, Meperidin oder Remifentanil. NSAIDs: z.B. Ketorolac, Ibuprofen oder Acetaminophen. Neuromuskuläre Blockade: z.B. Pancuronium
- Glukose-Kontrolle
   z.B. Insulin, Glukose
- Nierenersatzverfahren
   z.B. kontinuierliche veno-venöse-Hämofiltration oder intermittierende Hämodialyse. Dopamin niedrig-dosiert zur renalen Protektion.
- Antikoagulantien
   z.B. zur Thromboseprophylaxe oder bei Nierenersatzverfahren, z.B. unfraktionierte Heparine, low molecular weight Heparine, Heparinoide, Hirudin, Bivalirudin oder Argatroban.
- Bikarbonat-Therapie
- Streßulkusprophylaxe
   z.B. H2-Rezeptorinhibitoren, Antazida

Die erfindungsgemäßen Verbindungen können darüber hinaus auch zur Verhinderung von Koagulation *ex vivo* eingesetzt werden, z.B. zur Konservierung von Blut- und Plasmaprodukten, zur Reinigung/Vorbehandlung von Kathetern und anderen medizinischen Hilfsmitteln und Getäten, zur Beschichtung künstlicher Oberflächen von *in vivo* oder *ex vivo* eingesetzten medizinischen Hilfsmitteln und Geräten oder bei biologischen Proben, die Faktor Xa und/oder Faktor IIa enthalten.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Verhinderung der Blutkoagulation *in vitro*, insbesondere bei Blutkonserven oder biologischen Proben, die Faktor Xa und/oder Faktor IIa enthalten, das dadurch gekennzeichnet ist, dass eine antikoagulatorisch wirksame Menge der erfindungsgemäßen Verbindung zugegeben wird.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- Lipidsenker, insbesondere HMG-CoA-(3-Hydroxy-3-methylglutaryl-Coenzym A)-Reduktase-Inhibitoren wie beispielsweise Lovastatin (Mevacor; US 4,231,938), Simvastatin (Zocor; US 4,444,784), Pravastatin (Pravachol; US 4,346,227), Fluvastatin (Lescol; US 5,354,772) und Atorvastatin (Lipitor; US 5,273,995);
- Koronartherapeutika/Vasodilatatoren, insbesondere ACE-(Angiotensin-Converting-Enzyme)-Inhibitoren wie beispielsweise Captopril, Lisinopril, Enalapril, Ramipril, Cilazapril, Benazepril, Fosinopril, Quinapril und Perindopril, oder AII-(Angiotensin II)-Rezeptor-Antagonisten wie beispielsweise Embusartan (US 5,863,930), Losartan, Valsartan, Irbesartan, Candesartan, Eprosartan und Temisarta, oder β-Adrenozeptor-Antagonisten wie beispielsweise Carvedilol, Alprenolol, Bisoprolol, Acebutolol, Atenolol, Betaxolol, Carteolol, Metoprolol, Nadolol, Penbutolol, Pindolol, Propanolol und Timolol, oder alpha-1-Adrenozeptor-Antagonisten wie beispielsweise Prazosin, Bunazosin, Doxazosin und Terazosin, oder Diuretika wie beispielsweise Hydrochlorothiazid, Furosemid, Bumetanid, Piretanid, Torasemid, Amilorid und Dihydralazin, oder Calciumkanal-Blocker wie beispielsweise Verapamil und Diltiazem, oder Dihydropyridin-Derivate wie beispielsweise Nifedipin (Adalat) und Nitrendipin (Bayotensin), oder Nitropräparate wie beispielsweise Isosorbid-5-mononitrat, Isosorbid-dinitrat und Glyceroltrinitrat, oder Substanzen, die eine Erhöhung von cyclischem Guanosinmonophosphat (cGMP) bewirken, wie beispielsweise Stimulatoren der löslichen Guanylatcyclase (WO 98/16223, WO 98/16507, WO 98/23619, WO 00/06567, WO 00/06568, WO 00/06569, WO 00/21954, WO 00/66582, WO 01/17998, WO 01/19776, WO 01/19355, WO 01/19780, WO 01/19778, WO 07/045366, WO 07/045367, WO 07/045369, WO 07/045370, WO 07/045433);

- Plasminogen-Aktivatoren (Thrombolytika/Fibrinolytika) und die Thrombolyse/Fibrinolyse steigernde Verbindungen wie Inhibitoren des Plasminogen-Aktivator-Inhibitors (PAI-Inhibitoren) oder Inhibitoren des Thrombin-aktivierten Fibrinolyse-Inhibitors (TAFI-Inhibitoren) wie beispielsweise Gewebsplasminogen-Aktivator (t-PA), Streptokinase, Reteplase und Urokinase;
- antikoagulatorisch wirksame Substanzen (Antikoagulantien) wie beispielsweise Heparin (UFH), niedermolekulare Heparine (NMH) wie beispielsweise Tinzaparin, Certoparin, Parnaparin, Nadroparin, Ardeparin, Enoxaparin, Reviparin, Dalteparin, Danaparoid,
   **AVE 5026** (Sanofi-Aventis, *Company Presentation* 2008, February 12 ),
   **M118** (Momenta Pharmaceuticals Inc, *Press Release* 2008, February 14),
   **ORG42675** (Organon International Inc, *Company World Wide Website* 2007, April),
   und direkte Thrombin Inhibitoren (DTI) wie beispielsweise
   **Exanta (Ximelagatran)** 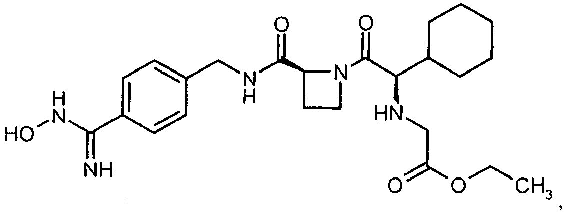 **Rendix (Dabigatran)** 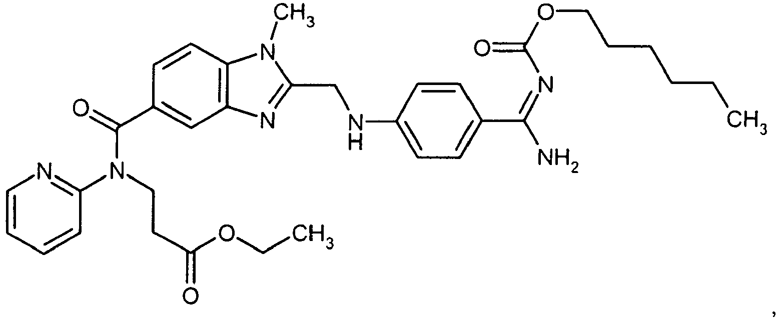 **AZD-0837** [AstraZeneca Annual Report 2006, 19. März 2007] 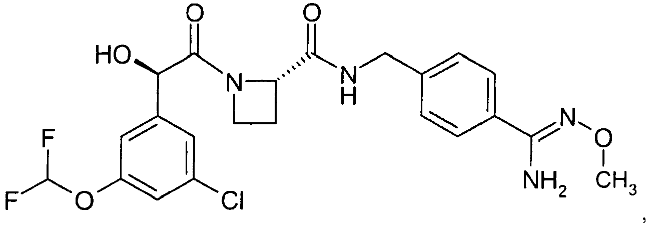 **SSR-182289A** [J. Lorrain et al. Journal of Pharmacology and Experimental Therapeutics 2003, 304, 567-574; J-M Altenburger et al. Bioorg.Med.Chem. 2004, 12, 1713-1730] 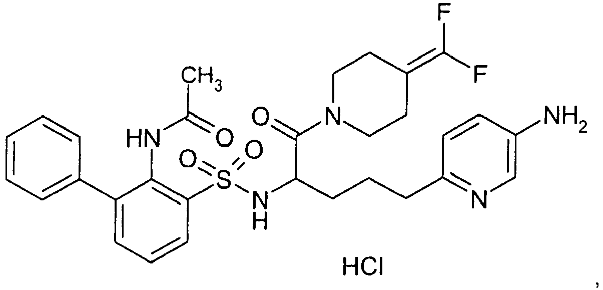 **TGN-167** [S. Combe et al. Blood 2005, 106, abstract 1863 (ASH 2005)],
   ***N*-[(Benzyloxy)carbonyl]-L-phenylalanyl-N-[(1S)-1-(dihydroxyboryl)-4-methoxybutyl]-*D*-prolinamid** [WO 2005/084685] 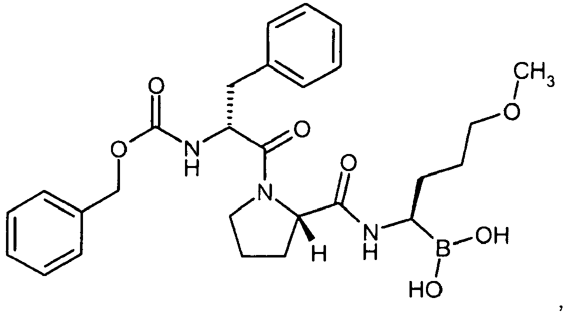 **Sofigatran *[***WHO Drug Information 2007, 21, 77] 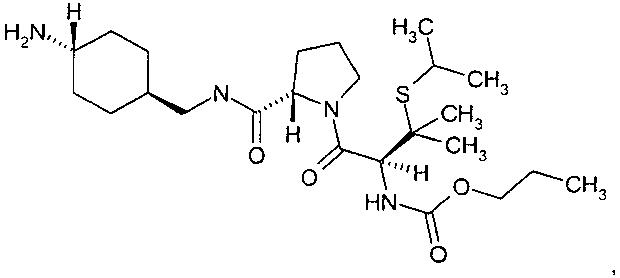 **MCC-977** [Mitsubishi Pharma website pipeline 2006, 25. Juli 2006],
   **MPC-0920** [Press Release: "Myriad Genetics Begins Phase 1 Trial of Anti-Thrombin Drug MPC-0920", Myriad Genetics Inc, 02. Mai 2006] und
   **TGN-255** (Flovagatran) 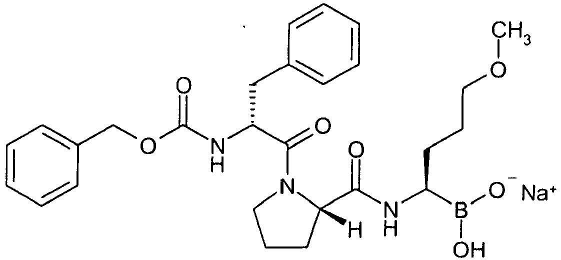 und direkte Faktor Xa-Inhibitoren wie beispielsweise
   **Rivaroxaban (BAY 59-7939)**: 5-Chlor-*N*-({(5*S*)-2-oxo-3-[4-(3-oxomorpholin-4-yl)phenyl]-1,3-oxazolidin-5-yl}methyl)thiophen-2-carboxamid [WO 2001/47919] 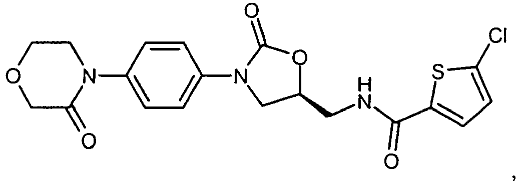 **AX-1826** [S. Takehana et al. Japanese Journal of Pharmacology 2000, 82 (Suppl. 1), 213P; T. Kayahara et al. Japanese Journal of Pharmacology 2000, 82 (Suppl. 1), 213P],
   **Tanogitran (BIBT-986, prodrug: BIBT-1011):** *N*-[(1*R*)-1-{2-[({4-[Amino(imino)methyl]-phenyl}amino)methyl]-1-methyl-1*H*-benzimidazol-5-yl}-1-methyl-2-oxo-2-pyrrolidin-1-ylethyl]glycin [American Chemical Society - 226th National Meeting, New York City, NY, USA, 2003] 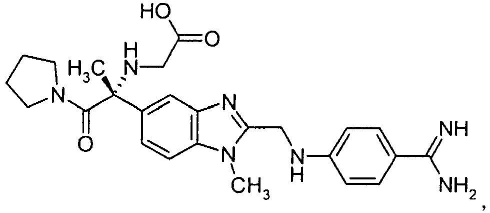 Verbindungen, die mit WO 2004/056784 offengelegt wurden.,
   **YM-150** [Y. Iwatsuki et al. Blood 2006, 108, abstract 911 (ASH 2006)],
   *N*-{4-Brom-2-[(5-chlorpyridin-2-yl)carbamoyl]-6-hydroxyphenyl}-1-isopropylpiperidin-4-carboxamid [JP 2005/179272] 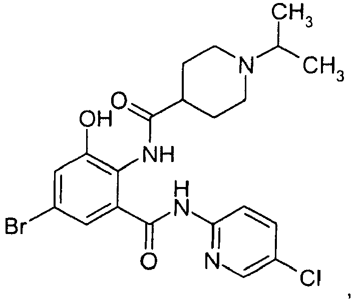 Verbindungen, die mit WO 2000/242270 offengelegt wurden.,
   **AZ12300547:** 6-[4-({(2*S*)-4-[(3-Chlor-1*H*-indol-6-yl)sulfonyl]-2-methyl-6-oxopiperazin-1-yl}methyl)-phenyl]-2-methylpyridazin-3(2*H*)-on [K.L Granberg et al. American Chemical Society - 232th National Meeting, San Francisco, USA, 2006, MEDI 391] 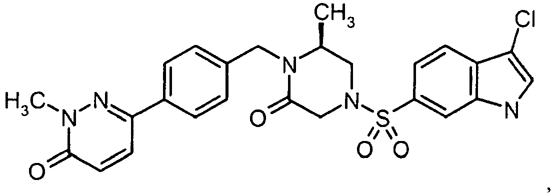 Verbindungen, die mit WO 2007/008142 offengelegt wurden.,
   **Razaxaban (DPC-906)**: 1-(3-Amino-1,2-benzisoxazol-5-yl)-*N*-(4-{2-[(dimethylamino)-methyl]-1*H*-imidazol-1-yl}-2-fluorphenyl)-3-(trifluormethyl)-1*H*-pyrazol-5-carboxamid [J.Med.Chem. 2005, 48, 1729-1744] 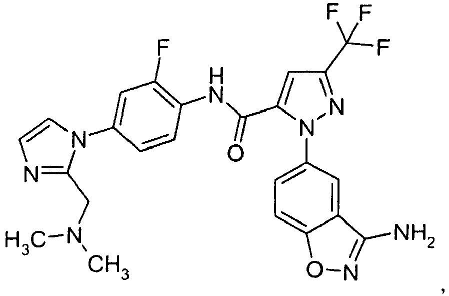 **Apixaban (BMS-562247)**: 1-(4-Methoxyphenyl)-7-oxo-6-[4-(2-oxopiperidin-1-yl)phenyl]-4,5,6,7-tetrahydro-1*H*-pyrazolo[3,4-c]pyridin-3-carboxamid [WO 2003/026652, WO 2003/049681] 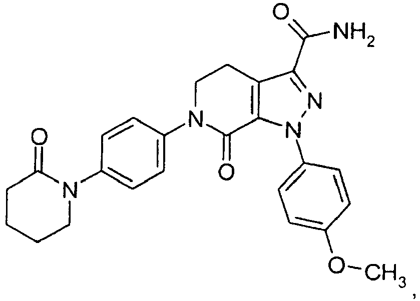 **BMS-691648**: 3-Chlor-N-[(3*S*,4*R*)-1-(methylsulfonyl)-4-{[4-(2-oxopyridin-1(2*H*)-yl)benzoyl]-amino}piperidin-3-yl]-1*H*-indol-6-carboxamid [T. Güngör et al. Drugs Fut. 2006, 31(Suppl A): abstract P118; WO 2004/082687] 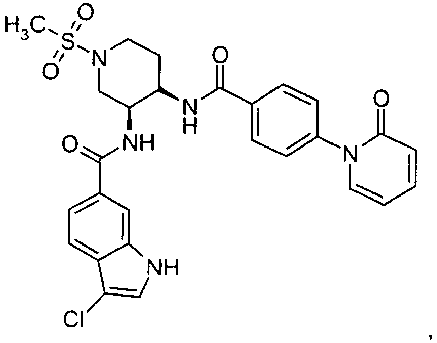 **DX-9065a:** (2*S*)-3-(7-[Amino(imino)methyl]-2-naphthyl)-2-(4-{[(3*S*)-1-ethanimidoyl-pyrrolidin-3-yl]oxy}phenyl)propansäure [T. Nagahara et al. J.Med.Chem. 1994, 37, 1200-1207] 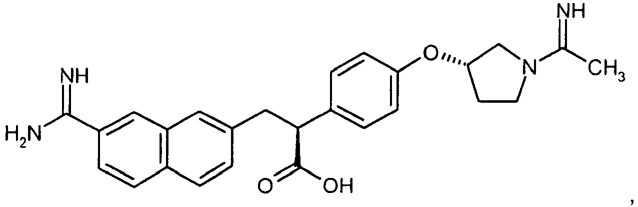 **DU-176b** [Y. Morishima et al. Blood 2004, 104, abstract 1862 (ASH 2004); T. Fukuda et al. Blood 2004, 104, abstract 1852 (ASH 2004); T. Furugohri et al. Blood 2004, 104, abstract 1851 (ASH 2004)],
   *N*-(5-Chlorpyridin-2-yl)-*N*'-[(1*S*,2*R*,4*S*)-4-(dimethylcarbamoyl)-2-{[(5-methyl-4,5,6,7-tetrahydro[1,3]thiazolo[5,4-c]pyridin-2-yl)carbonyl]amino}cyclohexyl]ethandiamid [US 2005/0020645, WO 2005/47296] 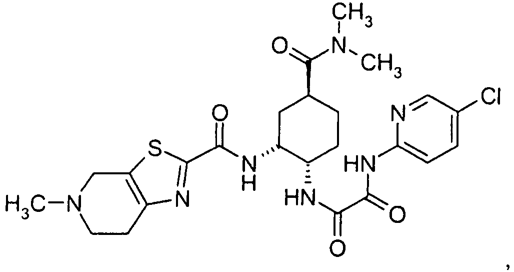 Verbindungen, die mit US 2005/0020645 offengelegt wurden.,
   **LY517717**: *N*-{(1*R*)-2-[4-(1-Methylpiperidin-4-yl)piperazin-1-yl]-2-oxo-1-phenylethyl}-1*H-*indol-6-carboxamid [WO 2000/76971, WO 2002/100847] 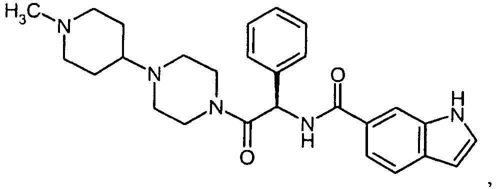 **813893** [Proteinase Inhibitor Design - Fourth SCI-RSC Symposium, Proteinase 2004: Strategies for New Medicines (Part I), London],
   6-Chlor-*N*-{(3*S*)-1-[(1*S*)-1-methyl-2-morpholin-4-yl-2-oxoethyl]-2-oxopyrrolidin-3-yl}naphtha-len-2-sulfonamid [N.S. Watson et al. Bioorg.Med.Chem.Lett. 2006, 16, 3784; WO 2002/100830; WO 2002/100886] 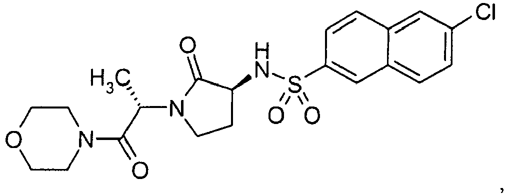 **KFA-1982 (prodrug of KFA-1829)** [T. Koizumi et al. Journal of Thrombosis and Hemostasis 2003, 1 Suppl 1, P2022],
   **EMD-503982** [Merck KGaA Annual Report 2006, 48-49],
   **EMD-495235:** 5-Chlor-*N*-[(1*R*)-1-(methoxymethyl)-2-{[3-methyl-4-(3-oxomorpholin-4-yl)-phenyl]amino}-2-oxoethyl]thiophen-2-carboxamid [Bioorg.Med.Chem.Lett. 2004, 14, 5817-5822] 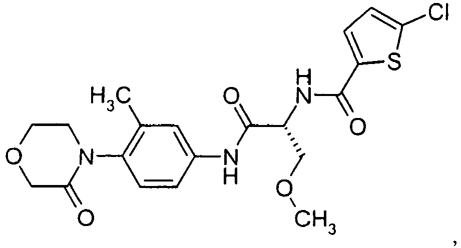 **M-55113:** 4-[(6-Chlor-2-naphthyl)sulfonyl]-1-[(1-pyridin-4-ylpiperidin-4-yl)methyl]piperazin-2-on [H. Nishida et al. Chem.Pharm.Bull. 2001, 49, 1237-1244] 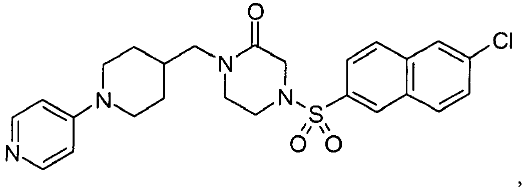 **M-55551/M-55555**: (2*R*)-4-[(6-Chlor-2-naphthyl)sulfonyl]-6-oxo-1-[(1-pyridin-4-ylpiperidin-4-yl)methyl]piperazin-2-carbonsäure [H. Nishida et al. Chem.Pharm.Bull. 2002, 50, 1187-1194] 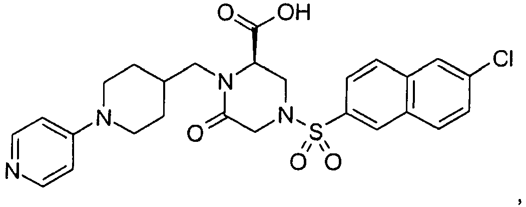 **M-55190:** (2*R*)-4-[(6-Chlor-2-naphthyl)sulfonyl]-6-oxo-1-[(1-pyridin-4-ylpiperidin-4-yl)-methyl]piperazin-2-carbonsäureethylester [H. Nishida et al. 16th Int Symp Med Chem, Bologna, 18-22 Sept 2000, Abst PA-125] 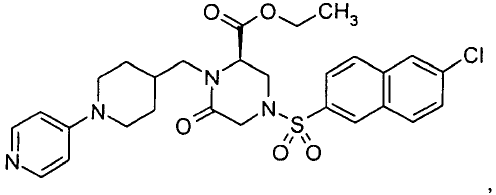 **M-55532**: 7-[(6-Chlor-2-naphthyl)sulfonyl]-8a-(methoxymethyl)-1'-pyridin-4-yltetrahydro-5*H*-spiro[1,3-oxazolo[3,2-a]pyrazin-2,4'-piperidin]-5-on [H. Nishida et al. 228th ACS National Meeting, Philadelphia, August 22-26, 2004, MEDI-251; H. Nishida et al. Chem.Pharm.Bull. 2004, 52, 406-412; *dito* 459-462] 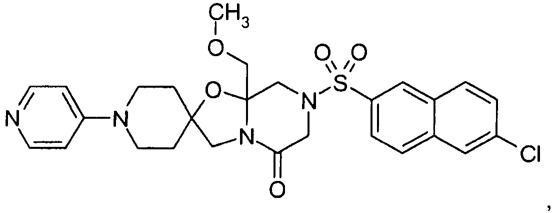 *N*-({7-[(5-Chlor-1*H*-indol-2-yl)sulfonyl]-5-oxo-1'-propionyltetrahydro-8a*H*-spiro[1,3-oxazolo-[3,2-a]pyrazin-2,4'-piperidin]-8a-yl}methyl)-*N*-methylglycin [WO 2006/106804] 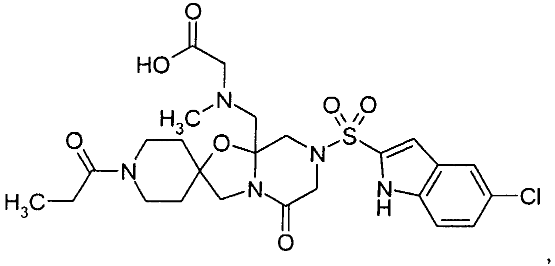 **PRT54021** [U. Sinha et al. Blood 2006, 108, abstract 907 (ASH 2006); K. Abe et al. Blood 2006, 108, abstract 901 (ASH 2006)],
   Verbindungen, die mit WO 2006/002099 offengelegt wurden.,
   **Otamixaban (FXV-673, RPR-130673)**: (2*R*,3*R*)-2-{3-[Amino(imino)methyl]benzyl}-3-{[4-(1-oxidopyridin-4-yl)benzoyl]amino}butansäuremethylester [V. Chu et al. Thrombosis Research 2001, 103, 309-324; K.R. Guertin et al. Bioorg Med.Chem.Lett. 2002, 12, 1671-1674] 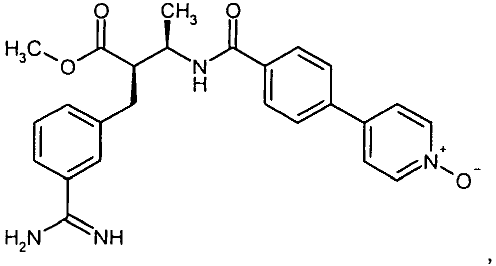 **AVE3247** [Sanofi Aventis Company Presentation, Paris 2007, February 13],
   **SAR377142 (SSR-7142)** [Sanofi Aventis Company Presentation, Paris 2007, February 13],
   **HMR-2906** [XVIIth Congress of the International Society for Thrombosis and Haemostasis, Washington D.C., USA, 14-21 Aug 1999; Generating greater value from our products and pipeline. Aventis SA Company Presentation, 05 Feb **2004**],
   **Idraparinux** [Harry R. Büller et al. Blood, 2006, 108, abstract 571 (ASH 2006)] und
   **Fondaparinux;**
- plättchenaggregationshemmende Substanzen (Plättchenaggregationshemmer, Thrombozytenaggregationshemmer) wie beispielsweise Acetylsalicylsäure (wie beispielsweise Aspirin), Ticlopidin (Ticlid), Clopidogrel (Plavix) und Prasugrel;
- Fibrinogen-Rezeptor-Antagonisten (Glycoprotein-IIb/IIIa-Antagonisten) wie beispielsweise Abciximab, Eptifibatide, Tirofiban, Lamifiban, Lefradafiban und Fradafiban;
- sowie Antiarrhythmika.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die parenterale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 5 mg/kg, vorzugsweise etwa 0.01 bis 1 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen

- CDI: Carbonyldiimidazol
- d: Dublett (bei NMR)
- DC: Dünnschicht-Chromatographie
- DCI: direkte chemische Ionisation (bei MS)
- dd: Doppeltes Dublett (bei NMR)
- DMAP: 4-Dimethylaminopyridin
- DMF: N,N-Dimethylformamid
- DMSO: Dimethylsulfoxid
- d: Tag(e)
- d. Th.: der Theorie (bei Ausbeute)
- eq.: Äquivalent(e)
- ESI: Elektrospray-Ionisation (bei MS)
- h: Stunde(n)
- HPLC: Hochdruck-, Hochleistungsflüssigchromatographie
- LC-MS: Flüssigchromatographie-gekoppelte Massenspektroskopie
- m: Multiplett (bei NMR)
- min: Minute(n)
- MS: Massenspektroskopie
- NMR: Kernresonanzspektroskopie
- RP: reverse phase (bei HPLC)
- RT: Raumtemperatur
- Rₜ: Retentionszeit (bei HPLC)
- s: Singulett (bei NMR)
- THF: Tetrahydrofuran

### LC-MS- und HPLC-Methoden

**Methode 1 (HPLC):** Instrument: HP 1100 mit DAD-Detektion; Säule: Kromasil 100 RP-18, 60 mm x 2.1 mm, 3.5 µm; Eluent A: 5 ml Perchlorsäure (70 %ig) / 1 Wasser, Eluent B: Acetonitril; Gradient: 0 min 2%B, 0.5 min 2%B, 4.5 min 90%B, 6.5 min 90%B, 6.7 min 2%B, 7.5 min 2%B; Fluß: 0.75 ml/min; Säulentemperatur: 30 °C; Detektion: UV 210 nm.

**Methode 2 (HPLC)**: Instrument: HP 1100 mit DAD-Detektion; Säule: Kromasil 100 RP-18, 60 mm x 2.1 mm, 3.5 µm; Eluent A: 5 ml Perchlorsäure (70 %ig) / 1 Wasser, Eluent B: Acetonitril; Gradient: 0 min 2%B, 0.5 min 2%B, 4.5 min 90%B, 9 min 90%B, 9.2 min 2%B, 10 min 2%B; Fluß: 0.75 ml/min; Säulentemperatur: 30 °C; Detektion: UV 210 nm.

**Methode 3 (LC-MS)**: Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

**Methode 4 (LC-MS)**: Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

**Methode 5 (LC-MS):** Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208- 400 nm.

**Methode 6 (LC-MS):** Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

**Methode 7 (LC-MS):** Instrument: Micromass Platfonn LCZ mit HPLC Agilent Serie 1100; Säule: Thermo Hypersil GOLD 3µ 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 100%A → 0.2 min 100%A → 2.9 min 30%A → 3.1 min 10%A → 5.5 min 10%A; Ofen: 50°C; Fluss: 0.8 ml/min; UV-Detektion: 210 nm.

**Methode 8 (LC-MS)**: Gerätetyp MS: Waters ZQ; Gerätetyp HPLC: Waters Alliance 2795; Säule: Phenomenex Onyx Monolithic C18, 100 mm x 3 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 210 nm.

**Methode 9 (GC-MS)**: Instrument: Micromass GCT, GC6890; Säule: Restek RTX-35MS, 30 m x 250 µm x 0.25 µm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (1.7 min halten).

**Methode 10 (GC-MS)**: Instrument: Micromass GCT, GC6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

**Methode 11 (LC-MS)**: Instrument: Micromass Quattro LCZ mit HPLC Agilent Serie 1100; Säule: Phenomenex Onyx Monolithic C18, 100 mm x 3 mm. Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 208-400 nm.

**Methode 12 (LC-MS)**: Instrument: Micromass Quattro LCZ mit HPLC Agilent Serie 1100; Säule: Phenomenex Synergi 2.5 µ MAX-RP 100A Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 0.1 min 90%A → 3.0 min 5%A → 4.0 min 5%A → 4.1 min 90%A; Fluss: 2 ml/min; Ofen: 50°C; UV-Detektion: 208- 400 nm.

**Methode 13 (LC-MS)**: Gerätetyp MS: Micromass ZQ; Gerätetyp HPLC: Waters Alliance 2795; Säule: Phenomenex Synergi 2.5 µ MAX-RP 100A Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 0.1 min 90%A → 3.0 min 5%A → 4.0 min 5%A → 4.01 min 90%A; Fluss: 2 ml/min; Ofen: 50°C; UV-Detektion: 210 nm.

**Methode 14 (LC-MS)**: Instrument: Micromass Quattro Premier mit Waters UPLC Acquity; Säule: Thermo Hypersil GOLD 1.9µ 50 mm x 1 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 0.1 min 90%A → 1.5 min 10%A → 2.2 min 10%A; Fluss: 0.33 ml/min; Ofen: 50°C; UV-Detektion: 210 nm.

### Ausgangsverbindungen

### Beispiel 1A

### 5-Chlor-N-[(2S)-oxiran-2-ylmethyl]thiophen-2-carboxamid

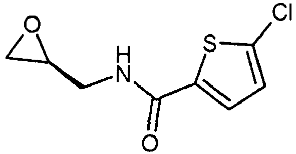

Beispiel 1A wird hergestellt, wie in WO04/101557 (Beispiel 6A) beschrieben.

### Beispiel 2A

### 3-(Hydroxymethyl)pyridin-2(1H)-on

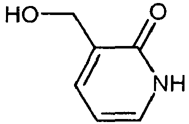

Eine Suspension von 10.0 g (71.9 mmol) 2-Hydroxynicotinsäure in 100 ml Toluol wird bei RT mit 23.2 g (144 mmol) Hexamethyldisilan und 0.781 g (7.19 mmol) Chlortrimethylsilan versetzt und es wird 30 min bei 110°C mit KPG-Rührer gerührt. Dann wird auf -40°C gekühlt und es werden 22.5 g (158 mmol) einer 1-molaren Lösung von Diisobutylaluminiumhydrid in Dichlormethan zu der Lösung getropft. Es wird auf RT aufgetaut, 18 h bei RT gerührt und schließlich bei -10°C mit verdünnter Salzsäure auf pH = 4 gebracht und mit 500 ml Methanol so versetzt, dass die Temperatur -10°C nicht übersteigt. Der gebildete Niederschlag wird abfiltriert, das Filtrat mit 100 ml Wasser versetzt, 1 h bei 50°C gerührt und der Niederschlag abfiltriert. Nach Einengen des Filtrats erhält man 8.55 g (95% d. Th.) der gewünschten Verbindung.
¹H-NMR (400 MHz, DMSO-*d₆ δ*/*ppm*): 11.53 (br. s, 1H), 7.40 (d, 1H), 7.25 (d, 1H), 6.19 (dd, 1H), 5.00 (t, 1H), 4.28 (d, 2H).
HPLC (Methode 1): Rₜ = 0.27 min.
MS (ESIpos, *m*/*z*): 148 (M+Na)⁺.

### Beispiel 3A

### 3-({[tert-Butyl(diphenyl)silyl]oxy}methyl)pyridin-2(1H)-on

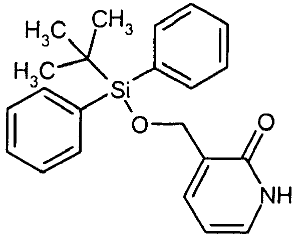

1.00 g (7.99 mmol) der Verbindung aus Beispiel 2A in 19 ml DMF werden bei RT mit 0.65 g (9.59 mmol) Imidazol, 2.42 g (8.79 mmol) t-Butyldiphenylchlorsilan und 0.10 g (0.80 mmol) DMAP versetzt und es wird 18 h bei gerührt. Dann werden 180 ml Wasser hinzugefügt und es wird 3 h bei 0°C belassen. Nach Filtration wird der erhaltene Rückstand durch Chromatographie an Kieselgel (Essigsäureethylester/Ethyldimethylamin 1000:1) aufgereinigt. Man erhält 801 mg (27% d. Th.) der gewünschten Verbindung.
¹H-NMR (400 MHz, DMSO-*d₆*, *δ*/*ppm*): 11.61 (br. s, 1H), 7.69-7.52 (m, 5H), 7.51-7.38 (m, 6H), 7.30 (d, 1H), 6.29 (dd, 1H), 4.51 (s, 2H), 1.05 (s, 9H).
HPLC (Methode 2): Rₜ = 5.27 min.
MS (DCI, *m*/*z*): 364 (M+H)⁺.

### Beispiel 4A

### 3-({[tert-Butyl(diphenyl)silyl]oxy}methyl)-1-(2-chlor-4-nitrophenyl)pyridin-2(1H)-on

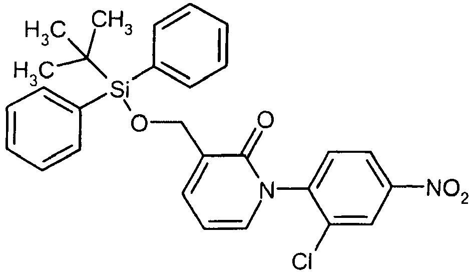

1.08 g (2.97 mmol) der Verbindung aus Beispiel 3A in 21 ml DMF werden bei 0°C mit 0.500 g (4.46 mmol) Kalium-tert.-butylat versetzt und es wird 30 min bei Raumtemperatur gerührt. 0.571 g (3.27 mmol) 2-Chlor-1-fluor-4-nitrobenzol werden zugefügt und es wird bei RT gerührt. Nach 4.5 h wird mit 200 ml Wasser versetzt und dann dreimal mit Essigsäureethylester extrahiert. Die vereinigten organischen Phasen werden mit Wasser gewaschen und anschließend über Natriumsulfat getrocknet. Nach Filtration werden die Lösungsmittel im Vakuum entfernt. Der Rückstand wird durch Chromatographie an Kieselgel (Cyclohexan/Essigsäureethylester 9:1) aufgereinigt. Man erhält 872 mg (56% d. Th.) der gewünschten Verbindung.
¹H-NMR (400 MHz, DMSO-*d₆*, *δ*/*ppm*): 8.52 (d, 1H), 8.32 (dd, 1H), 7.85 (d, 1H), 7.76 (dd, 1H), 7.68-7.63 (m, 4H), 7.59-7.55 (m, 1H), 7.54-7.41 (m, 6H), 6.54 (dd, 1H), 4.56 (br. s, 2H), 1.07 (s, 9H).
HPLC (Methode 2): Rₜ = 6.05 min.
MS (DCI, *m*/*z*): 519 (M+H)⁺.

### Beispiel 5A

### 1-(4-Amino-2-chlorphenyl)-3-({[tert-butyl(diphenyl)silyl]oxy}methyl)pyridin-2(1H)-on

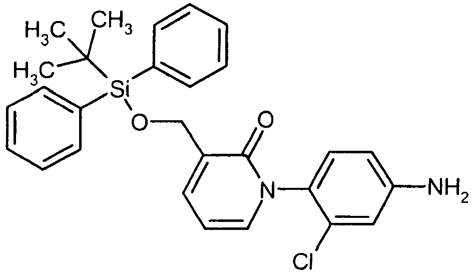

800 mg (1.54 mmol) der Verbindung aus Beispiel 4A werden in 48 ml THF gelöst. Dann werden 50 mg (0.05 mmol) Palladium auf Kohle hinzugefügt und es wird bei RT in einer Wasserstoffatmosphäre unter normalem Druck hydriert. Anschließend wird filtriert, mit THF nachgewaschen und das Filtrat vom Lösungsmittel befreit. Das Reaktionsprodukt (Reinheit: 95%) wird ohne weitere Aufreinigung weiter umgesetzt.
HPLC (Methode 1): Rₜ = 5.55 min.
MS (ESIpos, *m*/*z*): 489 (M+H)⁺.

### Beispiel 6A

### N-{[(5S)-3-{4-[3-({[tert-Butyl(diphenyl)silyl]oxy}methyl)-2-oxopyridin-1(2H)-yl]-3-chlorphenyl}-2-oxo-1,3-oxazolidin-5-yl]methyl}-5-chlorthiophen-2-carboxamid

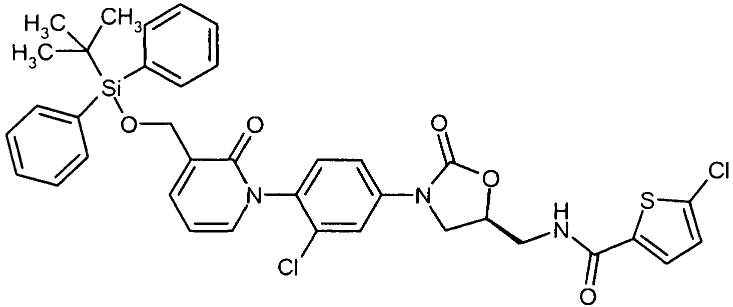

Eine Lösung von 789 mg (1.61 mmol) der Verbindung aus Beispiel 5A in 24 ml Acetonitril wird mit 386 mg (1.77 mmol) der Verbindung aus Beispiel 1A versetzt. Der Suspension werden 540 mg (2.42 mmol) Magnesiumperchlorat zugefügt. Nach 19 h bei RT wird mit 193 mg (0.952 mmol) der Verbindung aus Beispiel 1A versetzt und weitere 30 h bei RT gerührt. Dann werden 523 mg (2.46 mmol) 1,1'-Carbonyldiimidazol und 19 mg (0.09 mmol) DMAP hinzugesetzt und es wird auf 60°C erhitzt. Nach 21 h wird mit Wasser, gesättigter wässriger Natriumchlorid-Lösung und Essigsäureethylester verdünnt. Die Wasserphase wird zweimal mit Essigsäureethylester extrahiert und die vereinigten organischen Phasen über Natriumsulfat getrocknet. Nach Filtration wird vom Lösungsmittel befreit und der Rückstand durch Chromatographie an Kieselgel (Cyclohexan/Essigsäureethylester 1:1) gereinigt. Es werden 533 mg (45% d. Th.) des gewünschten Produktes erhalten.
¹H-NMR (400 MHz, DMSO-*d₆*, *δ*/*ppm*): 8.97 (t, 1H), 7.85 (dd, 1H), 7.73 (dd, 1H), 7.70-7.63 (m, 5H), 7.58 (dd, 1H), 7.53-7.38 (m, 8H), 7.19 (d, 1H), 6.47 (dd, 1H), 4.91-4.82 (m, 1H), 4.55 (br. s, 2H), 4.24 (dd, 1H), 3.89 (dd, 1H), 3.65-3.58 (m, 2H), 1.07 (s, 9H).
HPLC (Methode 2): Rₜ = 6.07 min.
MS (ESIpos, *m*/*z*): 732 (M+H)⁺.

### Beispiel 7A

### 3-({[tert-Butyl(diphenyl)silyl]oxy}methyl)-1-(2-methyl-4-nitrophenyl)pyridin-2(1H)-on

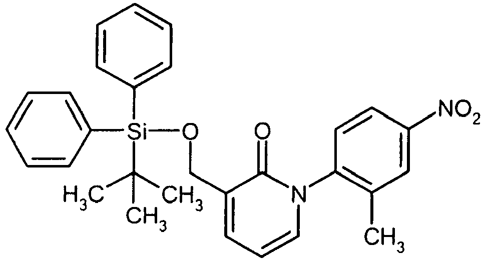

1.50 g (4.13 mmol) der Verbindung aus Beispiel 3A werden analog zu Beispiel 4A mit 704 mg (4.54 mmol) 2-Fluor-5-nitrotoluol umgesetzt. Man erhält 570 mg (28% d. Th.) der Titelverbindung.
¹H-NMR (400 MHz, DMSO-*d₆*, *δ*/*ppm*): 8.29 (d, 1H), 8.17 (dd, 1H), 7.76 (dd, 1H), 7.67-7.63 (m, 4H), 7.57 (d, 1H), 7.53 (dd, 1H), 7.51-7.41 (m, 6H), 6.52 (t, 1H), 4.63-4.51 (m, 2H), 2.12 (s, 3H), 1.07 (s, 9H).
HPLC (Methode 4): Rₜ = 3.39 min.
MS (ESIpos, *m*/*z*): 499 (M+H)⁺.

### Beispiel 8A

### 1-(4-Amino-2-methylphenyl)-3-({[tert-butyl(diphenyl)silyl]oxy}methyl)pyridin-2(1H)-on

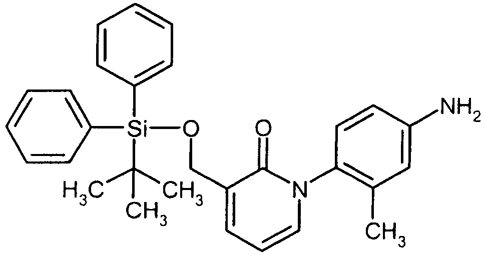

555 mg (1.11 mmol) der Verbindung aus Beispiel 7A werden in 15 ml THF gelöst, mit 150 mg Palladium auf Aktivkohle versetzt und in einer Wasserstoffatmosphäre bei normalem Druck hydriert, bis die theoretische Menge Wasserstoff aufgenommen worden ist. Man filtriert vom Katalysator ab und erhält nach dem Einengen im Vakuum 520 mg (99% d. Th.) der Titelverbindung.
¹H-MMR (400 MHz, DMSO-*d*₆, *δ*/*ppm*): 7.70-7.63 (m, 5H), 7.51-7.41 (m, 6H), 7.40-7.36 (m, 1H), 6.78 (d, 1H), 6.47-6.41 (m, 2H), 6.38 (t, 1H), 5.22 (s, breit, 2H), 4.59-4.48 (m, 2H), 1.81 (s, 3H), 1.06 (s, 9H).
HPLC (Methode 5): Rₜ = 3.20 min.
MS (ESIpos, *m*/*z*): 469 (M+H)⁺.

### Beispiel 9A

### N-[((5S)-3-{4-[3-({[tert-Butyl(diphenyl)silyl]oxy}methyl)-2-oxopyridin-1(2H)-yl]-3-methylphenyl}-2-oxo-1,3-oxazolidin-5-yl)methyl]-5-chlorthiophen-2-carboxamid

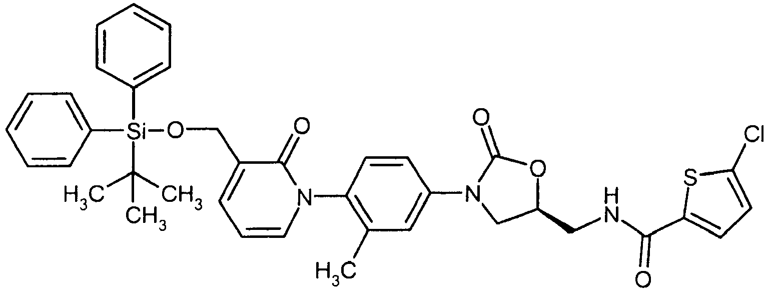

522 mg (1.11 mmol) der Verbindung aus Beispiel 8A werden in 10 ml Acetonitril gelöst und bei 0°C mit 266 mg (1.22 mmol) der Verbindung aus Beispiel 1A versetzt. Man gibt 373 mg (1.67 mmol) Magnesiumperchlorat hinzu und läßt 20 h bei RT rühren. Dann werden 271 mg (1.67 mmol) 1,1'-Carbonyldiimidazol und 14 mg (0.11 mmol) DMAP hinzugefügt, und man erwärmt die Reaktionsmischung 20 h auf 60 °C. Man engt dann im Vakuum ein und gibt Wasser und *tert*-Butylmethylether hinzu. Es wird zweimal mit Essigsäureethylester extrahiert. Die vereinten organischen Phasen werden über Natriumsulfat getrocknet und eingeengt. Man reinigt den Rückstand mittels präparativer HPLC. Es werden 562 mg (71% d. Th.) des gewünschten Produktes erhalten.
¹H-NMR (400 MHz, DMSO-*d₆*, *δ*/*ppm*): 8.97 (t, 1H), 7.74-7.63 (m, 6H), 7.58-7.41 (m, 9H), 7.23 (d, 1H), 7.19 (d, 1H), 6.45 (t, 1H), 4.89-4.80 (m, 1H), 4.58-4.49 (m, 2H), 4.21 (t, 1H), 3.90-3.83 (m, 1H), 3.63-3.58 (m, 2H), 1.98 (s, 3H), 1.07 (s, 9H).
HPLC (Methode 4): Rₜ = 3.39 min.
MS (ESIpos, *m*/*z*): 712/714 (³⁵Cl/³⁷Cl) (M+H)⁺.

### Beispiel 10A

### 5-Chlor-N-[((5S)-3-{4-[3-(hydroxymethyl)-2-oxopyridin-1(2H)-yl]-3-methylphenyl}-2-oxo-1,3-oxazolidin-5-yl)methyl]thiophen-2-carboxamid

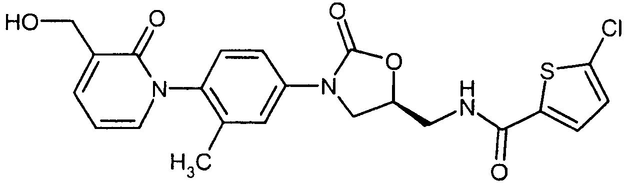

530 mg (0.74 mmol) der Verbindung aus Beispiel 9A werden in 9 ml THF gelöst. Man setzt 1.5 ml einer 1-molaren Lösung von Tetrabutylammoniumfluorid in THF hinzu und läßt 30 min bei RT rühren. Es wird etwas Wasser hinzugesetzt, eingeengt und mittels präparativer HPLC gereinigt. Es werden 335 mg (93% d. Th.) des gewünschten Produktes erhalten.
¹H-NMR (400 MHz, DMSO-d₆, *δ*/*ppm*): 9.00 (t, 1H), 7.70 (d, 1H), 7.55-7.49 (m, 3H), 7.39 (d, 1H), 7.23 (d, 1H), 7.20 (d, 1H), 6.36 (t, 1H), 5.14 (s, breit, 1H), 4.90-4.82 (m, 1H), 4.38-4.29 (m, 2H), 4.22 (t, 1H), 3.91-3.85 (m, 1H), 3.62 (t, 2H), 2.01 (s, 3H).
HPLC (Methode 3): Rₜ = 1.66 min.
MS (ESIpos, *m*/*z*): 474/476 (³⁵Cl/³⁷Cl) (M+H)⁺.

### Beispiel 11A

### N- {[(5S)-3-{4-[3-(Brommethyl)-2-oxopyridin-1(2H)-yl]-3-methylphenyl}-2-oxo-1,3-oxazolidin-5-yl]methyl}-5-chlorthiophen-2-carboxamid

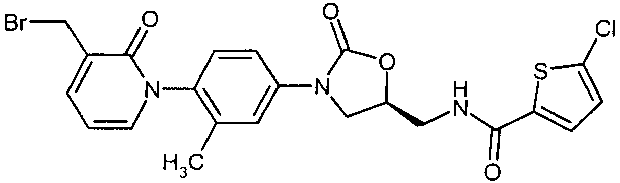

50 mg (0.11 mmol) des Produktes aus Beispiel 10A werden in 1 ml Dichlormethan gelöst. Man setzt 0.024 ml (0.32 mmol) Thionylbromid hinzu und läßt 1.5 h bei RT rühren. Es wird mit 1 ml Methanol und 3 ml Dichlormethan verdünnt und anschließend im Vakuum vom Lösemittel befreit. Das Rohprodukt wird ohne weitere Aufreinigung weiter umgesetzt.

### Beispiel 12A

### 3-Brom-1-(2-methyl-4-nitrophenyl)pyridin-2(1H)-on

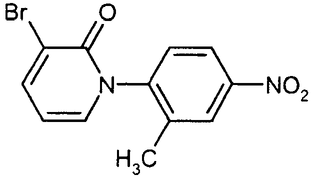

44.5 g (280 mmol) 3-Brompyridin-2(1H)-on werden in 750 ml wasserfreiem Dimethylsulfoxid gelöst und bei Raumtemperatur portionsweise mit 33.4 g (298 mmol) Kalium-tert-butylat versetzt. Die Suspension wird 1h bei dieser Temperatur gerührt, bevor 38.5 g (280 mmol) 1-Fluor-2-methyl-4-nitrobenzol zugesetzt werden und die Reaktionslösung für 20 h auf 80°C erhitzt wird. Man läßt erkalten und es wird vorsichtig mit Wasser verdünnt. Der ausgefallene kristalline Niederschlag wird abfiltriert, mit etwas Wasser gewaschen und im Vakuum getrocknet. Es werden 62 g (80% d. Th.) des gewünschten Produktes erhalten.
¹H-NMR (400 MHz, DMSO-d₆, *δ*/*ppm*): δ = 8.34 (d, 1H), 8.21 (dd, 1H), 8.10 (dd, 1H), 7.71-7.63 (m, 2H), 6.36 (t, 1H), 2.17 (s, 3H).
LC-MS (Methode 3): Rₜ = 1.72 min
MS (ESIpos): m/z = 309 (M+H)⁺

### Beispiel 13A

### 1-(2-Methyl-4-nitrophenyl)-3-vinylpyridin-2(1H)-on

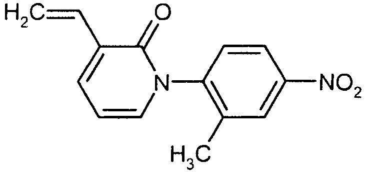

50 g (162 mmol) der Verbindung aus Beispiel 12A werden in 700 ml wasserfreiem Dioxan gelöst und mit 62 g (194 mmol) Tributylvinyl-Zinn und 4.7 g (4 mmol) Tetrakis(triphenylphosphin)-Palladium versetzt und es wird 15 h am Rückfluss erhitzt. Man lässt erkalten und filtriert über Kieselgur. Es wird mit Essigsäureethylester nachgewaschen und die vereinigten Filtrate werden im Vakuum zur Trockne eingeengt. Der Rückstand wird auf Kieselgel aufgezogen und an 800 g Kieselgel mit einem Gradienten aus Cyclohexan und Essigsäureethylester chromatographiert. Es werden 27 g (62% d. Th.) des gewünschten Produktes erhalten.
¹H-NMR (400 MHz, DMSO-d₆, *δ*/*ppm*): δ = 8.35 (d, 1H), 8.2 (dd, 1H), 7.75 (dd, 1H), 7.60 (d, 1H), 7.55 (dd, 1H), 6.75 (dd, 1H), 6.45 (t, 1H), 6.15 (dd, 1H), 5.30 (dd, 1H), 2.17 (s, 3H).
LC-MS (Methode 4): Rₜ = 1.86 min
MS (ESIpos): m/z = 257 (M+H)⁺

### Beispiel 14A

### 3-(2-Hydroxyethyt)-1-(2-methyl-4-nitrophenyl)pyridin-2(1H)-on

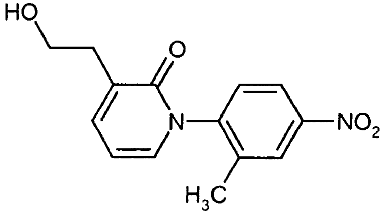

38 g (148 mmol) der Verbindung aus Beispiel 13A werden unter Eiskühlung innerhalb von 45 min mit einer Lösung von 40 g (326 mmol) 9-Borabicyclo[3.3.1]nonan in 650 ml Tetrahydrofuran versetzt. Bei dieser Temperatur wird eine weitere Stunde nachgerührt, bevor mit einer Lösung von 30 g (747 mmol) Natriumhydroxid in 740 ml Wasser innerhalb von 15 min versetzt wird. Es werden 151 ml einer 30%igen Wasserstoffperoxidlösung so zugegeben, dass die Temperatur 30°C nicht übersteigt. Nach beendeter Zugabe wird die Kühlung entfernt und es werden weitere 30 min nachgerührt. Es wird mehrmals mit Essigsäureethylester extrahiert, die vereinigten organischen Phasen werden mit einer Lösung aus 780 g (1.63 mol) Natriumdisulfit gewaschen, die organische Phase abgetrennt und die wässrige Phase erneut mit Essigsäureethylester extrahiert. Die vereinigten organischen Phasen werden mit gesättigter Natriumchloridlösung gewaschen, über Magnesiumsulfat getrocknet und im Vakuum zur Trockene eingedampft. Der Rückstand wird auf Kieselgel aufgezogen und mit einem Gradienten aus Cyclohexan und Essigsäureethylester chromatographiert. Die produkthaltigen Fraktionen werden vereinigt und im Vakuum zur Trockene eingeengt. Es werden 38 mg (93% d. Th.) des gewünschten Produktes erhalten.
¹H-NMR (400 MHz, DMSO-d₆, δ/ppm): δ = 8.33 (d, 1H), 8.18 (d, 1H), 7.57 (d, 1H), 7.48-7.40 (m, 2H), 6.33 (t, 1H), 4.58 (t, 1H), 3.62-3.50 (m, 2H), 2.62 (t, 2H), 2.15 (s, 3H).
LC-MS (Methode 6): Rₜ = 1.57 min
MS (ESIpos): m/z = 275 (M+H)⁺

### Beispiel 15A

### 3-(2-{[tert-Butyl(diphenyl)silyl]oxy}ethyl)-1-(2-methyl-4-nitrophenyl)pyridin-2(1H)-on

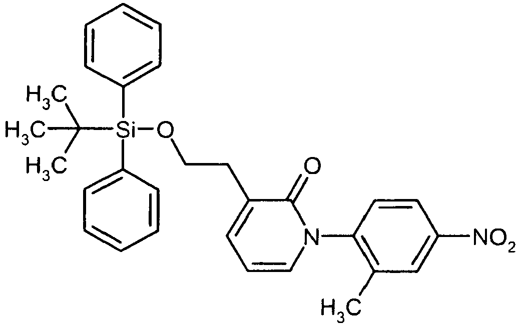

38 g (138 mmol) der Verbindung aus Beispiel 14A werden in 200 ml wasserfreiem N,N-Dimethylformamid gelöst und bei 0°C mit 12.2 g (198 mmol) Imidazol und portionsweise mit 46 g (135 mmol) *tert*-Butyl(chlor)diphenylsilan versetzt. Nach Rühren über Nacht wird mit Wasser verdünnt und dreimal mit Essigsäureethylester extrahiert. Die vereinigten organischen Phasen werden zweimal mit gesättigter Natriumchloridlösung gewaschen, über Magnesiumsulfat getrocknet, filtriert und im Vakuum eingedampft. Der Rückstand wird auf Kieselgel aufgezogen und mit einem Gradienten aus Cyclohexan und Essigsäureethylester chromatographiert. Die produkthaltigen Fraktionen werden vereinigt und im Vakuum zur Trockene eingeengt. Es werden 62 g (88% d. Th.) des gewünschten Produktes erhalten.
LC-MS (Methode 5): Rₜ = 3.18 min
MS (ESIpos): m/z = 483 (M+H)⁺

### Beispiel 16A

### 1-(4-Amino-2-methylphenyl)-3-(2-{[tert-butyl(diphenyl)silyl]oxy}ethyl)pyridin-2(1H)-on

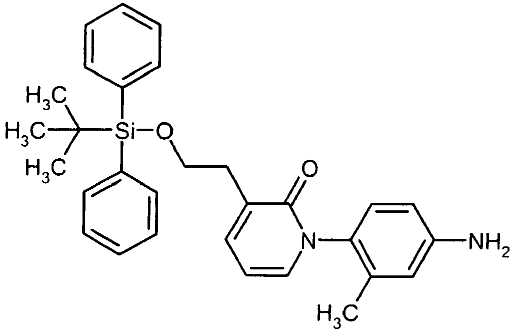

62 g (121 mmol) der Verbindung aus Beispiel 15A werden in 2 1 einer 1:1 1 Mischung aus Ethanol und Essigsäureethylester gelöst und mit 46 g (726 mmol) Ammoniumformiat und 0.6 g Palladium auf Kohle versetzt. Es wird auf 80°C erhitzt. Nach 45 min lässt man erkalten und filtriert über Kieselgel. Es wird mit Essigsäureethylester nachgewaschen und das Filtrat im Vakuum zur Trockene eingedampft. Es werden 36 g (61 % d. Th.) des gewünschten Produktes erhalten.
LC-MS (Methode 7): Rₜ = 1.84 min
MS (ESIpos): m/z = 221 (M+H)⁺

### Beispiel 17A

### N-[((5S)-3-{4-[3-(2-{[tert-Butyl(diphenyl)silyl]oxy}ethyl)-2-oxopyridin-1(2H)-yl]-3-methylphenyl}-2-oxo-1,3-oxazolidin-5-yl)methyl]-5-chlorthiophen-2-carboxamid

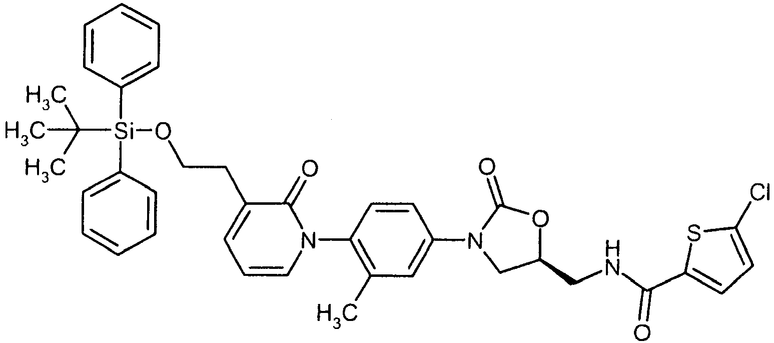

35.6 g (74.1 mmol) der Verbindung aus Beispiel 16A werden in 800 ml wasserfreiem Acetonitril gelöst und bei 0°C mit 19 g (89 mmol) der Verbindung aus Beispiel 1A versetzt. Es werden 25 g (110 mmol) Magnesiumperchlorat zugegeben, die Kühlung entfernt und 15 h bei Raumtemperatur gerührt. Es wird mit 24 mg (148 mmol) 1,1-Carbonyldiimidazol und 180 mg (1.4 mmol) N,N-Dimethylaminopyridin versetzt und 2 h am Rückfluss erhitzt. Man lässt erkalten und destilliert das Lösemittel im Vakuum ab. Dann wird in Essigsäureethylester aufgenommen und mit Wasser sowie dreimal mit gesättigter Natriumchloridlösung gewaschen. Nach Trocknen über Magnesiumsulfat wird filtriert und im Vakuum zur Trockene eingedampft. Der Rückstand wird auf Kieselgel aufgezogen und mit einem Gradienten aus Cyclohexan und Essigsäureethylester chromatographiert. Die produkthaltigen Fraktionen werden vereinigt und im Vakuum zur Trockene eingeengt. Es werden 46.4 g (84% d. Th.) des gewünschten Produktes erhalten.
LC-MS (Methode 5): Rₜ = 3.31 min
MS (ESIpos): m/z = 700 (M+H)⁺

### Beispiel 18A

### 5-Chlor-N-[((5S)-3-{4-[3-(2-hydroxyethyl)-2-oxopyridin-1(2H)-yl]-3-methylphenyl}-2-oxo-1,3-oxazolidin-5-yl)methyl]thiophen-2-carboxamid

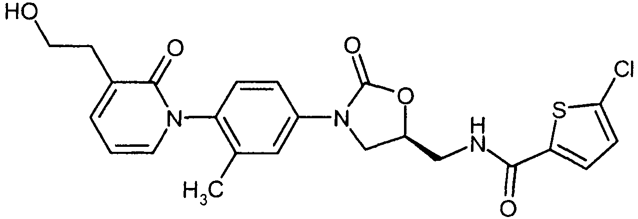

43.8 g (60.3 mmol) der Verbindung aus Beispiel 17A werden unter Eiskühlung mit 400 ml 1.25N Salzsäure in Methanol versetzt. Nach 1h wird mit Dichlormethan verdünnt und die wässrige Phase anschließend abgetrennt. Die organische Phase wird zweimal mit Wasser gewaschen, über Natriumsulfat getrocknet und nach Filtration wird im Vakuum zur Trockene eingeengt. Der Rückstand wird auf Kieselgel aufgezogen und mit einem Gradienten aus Cyclohexan und Essigsäureethylester chromatographiert. Die produkthaltigen Fraktionen werden vereinigt und im Vakuum zur Trockene eingeengt. Es werden 19.6 g (66% d. Th.) des gewünschten Produktes erhalten.
¹H-NMR (400 MHz, DMSO-d₆, *δ*/*ppm*): δ = 8.98 (t, 1H), 7.70 (d, 1H), 7.55-7.47 (m, 2H), 7.40 (dd, 1H), 7.35 (dd, 1H), 7.25-7.17 (m, 2H), 6.26 (t, 1H), 4.90-4.82 (m, 1H), 4.60 (t, 1H), 4.22 (t, 1H), 3.92-3.84 (m, 1H), 3.66-3.54 (m, 4H), 2.60 (t, 2H), 2.01 (s, 3H).
LC-MS (Methode 5): Rₜ = 1.87 min
MS (ESIpos): m/z = 488 (M+H)⁺

### Beispiel 19A

### 3-Brom-1-(2-methoxy-4-nitrophenyl)pyridin-2(1H)-on

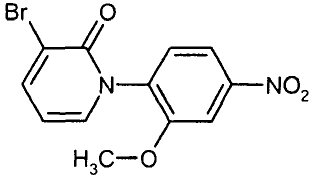

70 g (403 mmol) 3-Brompyridin-2(1H)-on werden in 11 wasserfreiem Dimethylsulfoxid gelöst und bei Raumtemperatur mit 54 g (484 mmol) Kalium-tert.-butylat versetzt. Die Suspension wird 1h bei dieser Temperatur gerührt. 69 g (403 mmol) 1-Fluor-2-methoxy-4-nitrobenzol werden zugesetzt und die Reaktionslösung für 20 h auf 80°C erhitzt. Es wird vorsichtig mit 5 l Wasser verdünnt. Der ausgefallene Feststoff wird abfiltriert, mit Wasser gewaschen und im Vakuum getrocknet. Es werden 103 g (72% d. Th.) des gewünschten Produktes erhalten.
¹H-NMR (400 MHz, DMSO-d₆, δ/ppm): δ = 8.05 (dd, 1H), 8.1 (d, 1H), 7.95 (dd, 1H), 7.7 (d, 1H), 7.6 (dd, 1H), 6.3 (t, 1H), 3.9 (s, 3H).
MS (ESIpos): m/z = 342 (M+NH₄)⁺

### Beispiel 20A

### 1-(2-Methoxy-4-nitrophenyl)-3-vinylpyridin-2(1H)-on

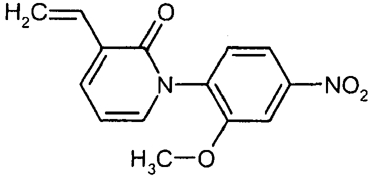

100 g (308 mmol) der Verbindung aus Beispiel 19A werden in 1.4 wasserfreiem Dioxan gelöst und mit 8.9 g (7.7 mmol) Tetrakis(triphenylphospin)Palladium und 117 g (370 mmol) Tributylvinyl-Zinn versetzt. Es wird 16 h am Rückfluß erhitzt. Dann läßt man erkalten und filtriert die Reaktionslösung durch Kieselgur. Die Filtrate werden im Vakuum zur Trockene eingeengt. Der Rückstand wird an Kieselgel unter Verwendung eines Gradienten aus Cyclohexan und Essigsäureethylester chromatographiert. Die produkthaltigen Fraktionen werden vereinigt und im Vakuum zur Trockene eingeengt. Man versetzt mit Petrolether bis die Kristallisation einsetzt. Die Kristalle werden abfiltriert und im Vakuum getrocknet. Es werden 37 g (41% d. Th.) des gewünschten Produktes erhalten.
¹H-NMR (400 MHz, DMSO-d₆, δ/ppm): δ = 8.0 (m, 2H), 7.7 (m, 2H), 7.5 (dd, 1H), 6.7 (q, 1H), 6.4 (t, 1H), 6.1 (dd, 1H), 5.3 (dd, 1H), 3.9 (s, 3H).
MS (ESIpos): m/z = 273 (M+H)⁺

### Beispiel 21A

### 3-(2-Hydroxyethyl)-1-(2-methoxy-4-nitrophenyl)pyridin-2(1H)-on

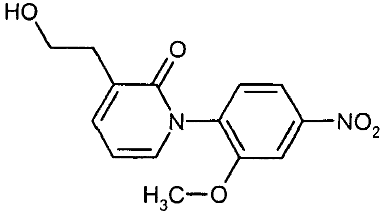

37 g (136 mmol) der Verbindung aus Beispiel 20A werden bei 0°C innerhalb von 45 min mit einer Lösung von 36 g (299 mmol) 9-Borabicycio[3.3.1]nonan in 600 ml Tetrahydrofuran versetzt. Nach einer weiteren Stunde bei dieser Temperatur wird innerhalb von 15 min mit einer Lösung von 27 g (680 mmol) Natriumhydroxid (IN in Wasser) versetzt. Man rührt weitere 5 min nach bevor 120 ml einer 30%igen Wasserstoffperoxidlösung so zugegeben werden, daß die Temperatur nicht über 30°C ansteigt. Die Kühlung wird entfernt und es werden weitere 30 min nachgerührt. Es wird mehrmals mit Essigsäureethylester extrahiert, die vereinigten organischen Phasen werden mit einer Lösung aus 730 g (1.50 mol) Natriumdisulfit gewaschen, die organische Phase abgetrennt und die wässrige Phase erneut mit Essigsäureethylester extrahiert. Die vereinigten organischen Phasen werden mit gesättigter Natriumchloridlösung gewaschen, über Magnesiumsulfat getrocknet und im Vakuum zur Trockene eingedampft. Der Rückstand wird auf Kieselgel absorbiert und unter Verwendung eines Gradienten aus Cyclohexan und Essigsäureethylester chromatographiert. Die Produktfraktionen werden vereinigt und im Vakuum zur Trockene eingedampft. Zur Kristallisation wird mit tert-Butylmethylether versetzt. Die Kristalle werden abfiltriert und im Vakuum getrocknet. Es werden 24 g (60% d. Th.) des gewünschten Produktes erhalten.
¹H-NMR (400 MHz, DMSO-d₆, δ/ppm): δ = 8.0 (d, 1H), 7.95 (dd, 1H), 7.6 (d, 1H), 7.4 (d, 1H), 6.35 (t, 1H), 4.6 (t, 1H), 3.9 (s, 3H), 3.55 (m, 2H), 2.6 (m, 2H).
MS (ESIpos): m/z = 291 (M+H)⁺

### Beispiel 22A

### 3-(2-{[tert-Butyl(diphenyl)silyl]oxy}ethyl)-1-(2-methoxy-4-nitrophenyl)pyridin-2(1H)-on

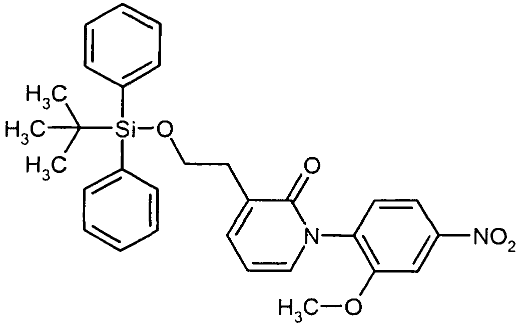

24 g (81 mmol) der Verbindung aus Beispiel 21A werden in 200 ml wasserfreiem N,N-Dimethytformamid gelöst und mit 7.2 g (106 mmol) Imidazol und 27 g (98 mmol) tert-Butyl(chlor)diphenylsilan versetzt. Nach 16 h wird mit 1.2 1 Wasser verdünnt und dreimal mit Essigsäureethylester extrahiert. Die vereinigten organischen Phasen werden zweimal mit Wasser gewaschen, über Magnesiumsulfat getrocknet, filtriert und im Vakuum eingedampft. Zur Kristallisation wird mit tert-Butylmethylether versetzt und die erhaltenen Kristalle werden abfiltriert und im Vakuum getrocknet. Es werden 30 g (67% d. Th.) des gewünschten Produktes erhalten.
¹H-NMR (400 MHz, DMSO-d₆, δ/ppm): δ = 8.0 (d, 1H), 7.95 (dd, 1H), 7.6-7.5 (m, 5H), 7.5-7.4 (m, 8H), 6.35 (t, 1H), 3.8 (m, 5H), 2.7 (m, 2H), 1.0 (s, 9H).

### Beispiel 23A

### 1-(4-Amino-2-methoxyphenyl)-3-(2-{[tert-butyl(diphenyl)silyl]oxy}ethyl)pyridin-2(1H)-on

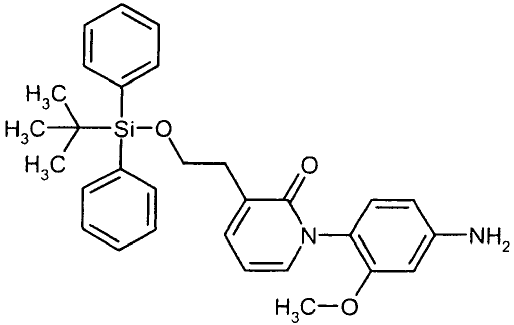

25 g (48 mmol) der Verbindung aus Beispiel 22A werden in 800 ml einer 1:1 Mischung aus Ethanol und Essigsäureethylester gelöst und mit 18 g (286 mmol) Ammoniumformiat und 800 mg Palladium auf Kohle versetzt. Es wird auf 80°C erhitzt. Nach 60 min lässt man erkalten und filtriert über Kieselgel. Es wird mit Essigsäureethylester nachgewaschen und das Filtrat im Vakuum zur Trockene eingedampft. Es werden 27 g (98% d. Th.) des gewünschten Produktes erhalten.
¹H-NMR (400 MHz, DMSO-d₆, δ/ppm): δ = 7.6 (m, 4H), 7.4 (m, 6H), 7.3 (dd, 1H), 7.25 (dd, 1H), 6.75 (d, 1H), 6.3 (d, 1H), 6.2 (dd, 1H), 6.1 (t, 1H), 5.3 (b, 2H), 3.8 (m, 2H), 3.6 (s, 3H), 2.7 (m, 2H), 1.0 (s, 9H).
MS (ESIpos): m/z = 499 (M+H)⁺

### Beispiel 24A

### N-{[(5S)-3-{4-[3-(2-{[tert-Buty(diphenyl)silyl]oxy}ethyl)-2-oxopyridin-1(2H)-yl]-3-methoxyphenyl}-2-oxo-1,3-oxazolidin-5-yl]methyl}-5-chlorthiophen-2-carboxamid

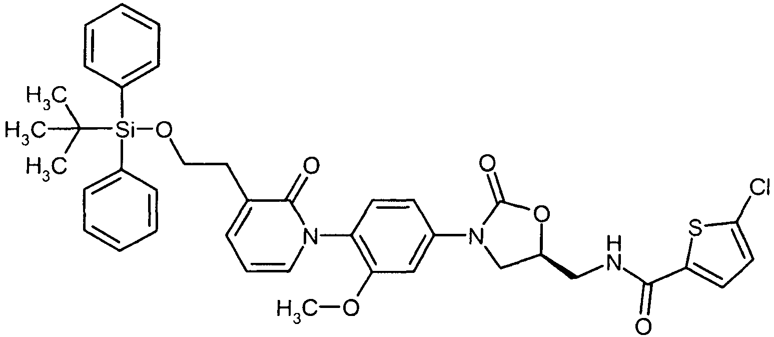

29 g (58 mmol) der Verbindung aus Beispiel 23A werden in 600 ml wasserfreiem Acetonitril gelöst und bei 0°C mit 15 g (69 mmol) der Verbindung aus Beispiel 1A versetzt. Es werden 19 g (87 mmol) Magnesiumperchlorat zugegeben, die Kühlung entfernt und 15 h bei RT gerührt. Dann wird mit 19.0 g (116 mmol) 1,1-Carbonyldiimidazol und 141 mg (1.21 mmol) N,N-Dimethylaminopyridin versetzt und am Rückfluß erhitzt. Nach 2 h lässt man erkalten und destilliert das Lösemittel im Vakuum ab. Der Rückstand wird in Essigsäureethylester angelöst und mit Wasser sowie dreimal mit gesättigter Natriumchloridlösung gewaschen. Nach Trocknen über Magnesiumsulfat wird filtriert und im Vakuum zur Trockene eingedampft. Der Rückstand wird an Kieselgel unter Verwendung eines Gradienten aus Cyclohexan und Essigsäureethylester chromatographiert. Die produkthaltigen Fraktionen werden vereinigt und im Vakuum zur Trockene eingeengt. Es werden 37 g (85% d. Th.) des gewünschten Produktes erhalten.
¹H-NMR (400 MHz, DMSO-d₆): δ = 9.0 (t, 1H), 7.7 (d, 1H), 7.6 (m, 4H), 7.5-7.3 (m, 9H), 7.2 (m, 2H), 7.1 (m, 1H), 6.2 (t, 1H), 4.8 (m, 1H), 4.4 (t, 1H), 3.9 (m, 1H), 3.8 (b, 2H), 3.7 (s, 3H), 3.65 (m, 2H), 2.7 (m, 2H), 1.0 (s, 9H).
LC-MS (Methode 8): Rt = 4.53 min
MS (ESIpos): m/z = 742 (M+H)⁺

### Beispiel 25A

### 5-Chlor-N-{[(5S)-3-{4-[3-(2-hydroxyethyl)-2-oxopyridin-1(2H)-yl]-3-methoxyphenyl}-2-oxo-1,3-oxazolidin-5-yl]methyl}thiophen-2-carboxamid

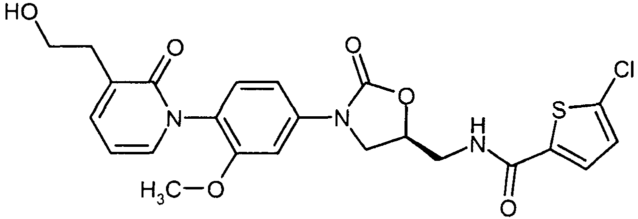

37 g (49 mmol) der Verbindung aus Beispiel 24A werden bei 0°C in 313 ml 1.25N Salzsäure in Methanol gelöst. Nach 1 h wird die Lösung im Vakuum eingedampft und mit Dichlormethan verdünnt. Die organische Phase wird zweimal mit Wasser gewaschen, über Magnesiumsulfat getrocknet und nach Filtration im Vakuum zur Trockene eingedampft. Der Rückstand wird an Kieselgel unter Verwendung eines Gradienten aus Dichlormethan und Methanol chromatographiert. Die Produktfraktionen werden vereinigt und im Vakuum zur Trockene eingedampft. Es werden 19 g (78% d. Th.) des gewünschten Produktes erhalten.
¹H-NMR (400 MHz, DMSO-d₆, δ/*ppm*): δ = 9.00 (t, 1H), 7.70 (d, 1H), 7.45 (d, 1H), 7.35 (dd, 1H), 7.30 (dd, 1H), 7.25 (d, 1H), 7.20 (d, 1H), 7.15-7.08 (m, 1H), 6.19 (t, 1H), 4.91-4.83 (m, 1H), 4.60 (t, 1H), 4.25 (t, 1H), 3.94-3.87 (m, 1H), 3.73 (s, 3H), 3.66-3.53 (m, 4H), 2.62-2.55 (m, 2H).
MS (ESIpos): m/z = 504 (M+H)⁺

### Beispiel 26A

### 2-(Brommethyl)-1-fluor-4-nitrobenzol

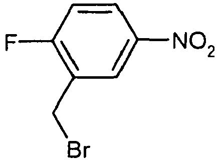

186 g (1.20 mol) 2-Fluor-5-nitro-toluol werden in 1.2 1 Tetrachlormethan gelöst und mit 214 g (1.20 mol) N-Brom-succinimid versetzt. 19.7 g (120 mmol) Azo-diisobutyronitril werden zugegeben und es wird unter Rückfluß erhitzt. Nach 16 h lässt man erkalten, filtriert und dampft im Vakuum zur Trockene ein. Der Rückstand wird in 300 ml Dichlormethan gelöst und mit 300 g Seesand versetzt. Dann wird erneut im Vakuum zur Trockene eingeengt und der Rückstand auf eine 1 kg Kieselgelsäule gegeben. Es wird mit einer 20:1 Mischung aus Cyclohexan und Essigsäureethylester chromatographiert und die Produktfraktionen werden im Vakuum zur Trockene eingedampft. Der Rückstand wird mit Cyclohexan kristallisiert und im Vakuum getrocknet. Es werden 92 g (32% d. Th.) des gewünschten Produktes erhalten.
¹H-NMR (400 MHz, DMSO-d₆, δ/*ppm*): δ = 8.57-8.52 (m, 1H), 8.33-8.27 (m, 1H), 7.56 (t, 1H), 4.62 (s, 2H).
GC-MS (Methode 9): Rₜ = 7.79 min
MS (ESIpos): m/z = 154 (M-Br)⁺

### Beispiel 27A

### 1-Fluor-2-(methoxymethyl)-4-nitrobenzol

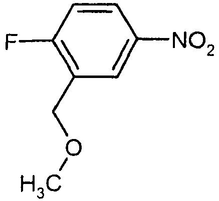

30 g (128 mmol) der Verbindung aus Beispiel 26A werden in 1.31 wasserfreiem Toluol gelöst und mit 45 g (192 mmol) Silber(I)oxid und 24.6 g (769 mmol) wasserfreiem Methanol versetzt. Es wird 16 h auf 60°C erhitzt. Dann lässt man erkalten, und filtriert über Kieselgel. Das Produkt wird mit einem Gradienten von Cyclohexan und Cyclohexan/Essigsäureethylester 25:1 fraktioniert eluiert. Die Produktfraktionen werden im Vakuum zur Trockene eingedampft und im Vakuum getrocknet. Es werden 17 g (72% d. Th.) des gewünschten Produktes erhalten.
¹H-NMR (400 MHz, DMSO-d₆, δ/*ppm*): δ = 8.41-8.36 (m, 1H), 8.22-8.16 (m, 1H), 7.26 (t, 1H), 4.58 (s, 2H), 3.49 (s, 3H).
GC-MS (Methode 9): Rₜ = 6.52 min
MS (ESIpos): m/z = 154 (M-OCH₃)⁺

### Beispiel 28A

### 3-Brom-1-[2-(methoxymethyl)-4-nitrophenyl]pyridin-2(1H)-on

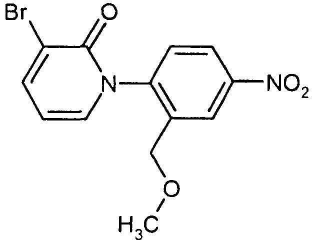

38 g (391 mmol) 3-Brom-2-hydroxypyridin werden in 1250 ml wasserfreiem Dimethylsulfoxid gelöst und portionsweise mit 53 g (469 mmol) Kalium-tert-butylat versetzt. Es wird eine Stunde nachgerührt bevor 72.4 g (391 mmol) der Verbindung aus Beispiel 27A zugegeben werden. Nach beendeter Zugabe wird 3 h auf 80°C erhitzt. Anschließend lässt man erkalten und rührt weitere 16 h bei Raumtemperatur nach. Die Reaktionslösung wird dann auf 15°C abgekühlt und bei dieser Temperatur vorsichtig mit 1N Salzsäure auf pH = 3 gebracht. Es werden 4 1 Wasser zugegeben und es wird dreimal mit 2 1 Essigsäureethylester extrahiert. Die vereinigten organischen Phasen werden mit gesättigter Natriumchloridlösung gewaschen und über Magnesiumsulfat getrocknet. Dann wird im Vakuum zur Trockene eingeengt und zur Kristallisation mit tert-Butylmethylether versetzt. Die Kristalle werden abfiltriert und im Vakuum getrocknet. Es werden 94 g (71% d. Th.) des gewünschten Produktes erhalten.
¹H-NMR (400 MHz, DMSO-d₆, δ/ppm): δ = 8.37 (d, 1H), 8.33 (dd, 1H), 8.10 (dd, 1H), 7.73-7.67 (m, 2H), 6.35 (t, 1H), 4.3 (q, 2H), 3.3 (s, 3H).
LC-MS (Methode 6): Rₜ= 1.88 min
MS (ESIpos): m/z = 339 (M+H⁺)⁺

### Beispiel 29A

### 1-[2-(Methoxymethyl)-4-nitrophenyl]-3-vinylpyridin-2(1H)-on

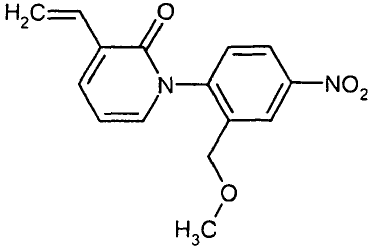

94 g (277 mmol) der Verbindung aus Beispiel 28A werden in 1.2 l wasserfreiem Dioxan gelöst und mit 8 g (6.9 mmol) Tetrakis(triphenylposphin)-Palladium (0) versetzt. Es wird bei Raumtemperatur langsam mit 105 g (333 mmol) Tributylvinyl-Zinn versetzt und nach beendeter Zugabe 21 h am Rückfluss erhitzt. Man läßt erkalten und filtriert die Reaktionslösung über Kieselgur. Es wird mit Essigsäureethylester nachgewaschen und die vereinigten organischen Filtrate werden im Vakuum zur Trockene eingeengt. Der verbleibende Rückstand wird mit Dichlormethan gelöst und auf Kieselgur aufgezogen. Es wird an 1.2 kg Kieselgel unter Verwendung eines Gradienten aus Cyclohexan und Essigsäureethylester chromatographiert. Die produkthaltigen Fraktionen werden vereinigt und im Vakuum zur Trockene eingeengt. Es werden 23 g (29% d. Th.) des gewünschten Produktes erhalten.
¹H-NMR (400 MHz, DMSO-d₆, δ/ppm): δ = 8.36 (d, 1H), 8.31 (dd, 1H), 7.77 (dd, 1H), 7.67 (d, 1H), 7.56 (dd, 1H), 6.80-6.71 (m, 1H), 6.45 (t, 1H), 6.14 (dd, 1H), 5.33 (dd, 1H), 4.37-4.22 (m, 2H), 3.26 (s, 3H).
LC-MS (Methode 6): Rₜ = 2.07 min
MS (ESIpos): m/z = 387 (M+H⁺)⁺

### Beispiel 30A

### 3-(2-Hydroxyethyl)-1-[2-(methoxymethyl)-4-nitrophenyl]pyridin-2(1H)-on

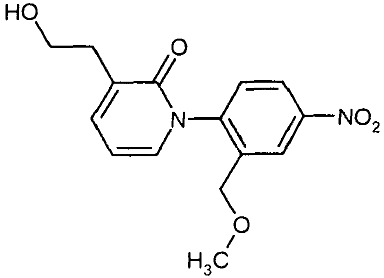

23 g (80 mmol) der Verbindung aus Beispiel 29A werden in 80 ml wasserfreiem Tetrahydrofuran gelöst und auf 5°C abgekühlt. Innerhalb von 15 min werden 21 g (176 mmol) 9-Borabicyclo[3.3.1]nonan (0.5M Lösung in Tetrahydrofuran) zugegeben. Die Kühlung wird entfernt und es wird weitere 2 h bei Raumtemperatur nachgerührt. Dann wird erneut auf 5°C abgekühlt und mit 400 ml 1N Natronlauge versetzt. Nach beendeter Zugabe wird bei dieser Temperatur portionsweise mit 81 ml 30%-iger Wasserstoffperoxid-Lösung versetzt. Nach Verdünnnen mit 500 ml Essigsäureethylester wird zur Vernichtung der Peroxide mit 32 ml 40%-iger Natriumhydrogensulfit-Lösung gewaschen. Die organische Phase wird abgetrennt und die wässrige Phase viermal mit Essigsäureethylester extrahiert. Die vereinigten organischen Phasen werden mit gesättigter Natriumchloridlösung gewaschen, über Magnesiumsulfat getrocknet und nach Filtration im Vakuum zur Trockene eingeengt. Der Rückstand wird an Kieselgel unter Verwendung eines Gradienten aus Cyclohexan und Essigsäureethylester chromatographiert. Die produkthaltigen Fraktionen werden vereinigt und im Vakuum zur Trockene eingeengt. Es werden 20 g (77% d. Th.) des gewünschten Produktes erhalten.
¹H-NMR (400 MHz, DMSO-d₆, δ/ppm): δ = 8.36 (d, 1H), 8.30 (dd, 1H), 7.62 (d, 1H), 7.45 (d, 1H), 6.33 (t, 1H), 4.60 (t, 1H), 4.35-4.20 (m, 2H), 3.62-3.55 (m, 2H), 3.32 (s, 3H), 2.62 (t, 2H).
LC-MS (Methode 8): Rₜ = 1.60 min
MS (ESIpos): m/z = 305 (M+H⁺)⁺

### Beispiel 31A

### 3-(2-{[tert-Butyl(diphenyl)silyl]oxy}ethyl)-1-[2-(methoxymethyl)-4-nitrophenyl]pyridin-2(1H)-on

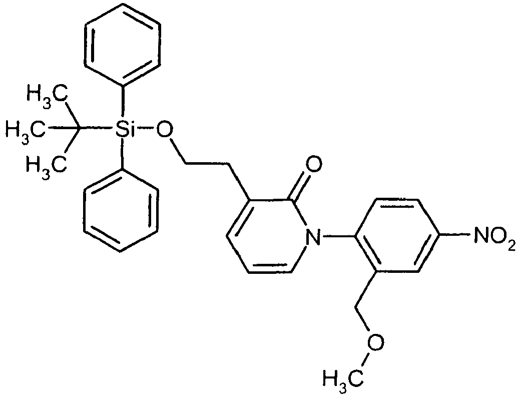

20 g (65 mmol) der Verbindung aus Beispiel 30A werden in 75 ml wasserfreiem N,N-Dimethylformamid gelöst und unter Eiskühlung zunächst mit 5.3 g (78 mmol) Imidazol und dann in Portionen über 3 min mit 19 g (72 mmol) tert-Butyldiphenylchlorsilan versetzt. Die Kühlung wird entfernt und es wird weitere 19 h bei Raumtemperatur gerührt. Die Reaktionslösung wird mit Essigsäureethylester verdünnt und dreimal mit Wasser und zweimal mit gesättigter Natriumchloridlösung gewaschen. Dann wird über Magnesiumsulfat getrocknet, filtriert und im Vakuum zur Trockene eingeengt. Der Rückstand wird mit tert-Butylmethylether versetzt, die erhaltenen Kristalle werden abfiltriert und im Vakuum getrocknet. Es werden 39 g (90% d. Th.) des gewünschten Produktes erhalten.
¹H-NMR (400 MHz, DMSO-d₆, δ/ppm): δ = 8.35 (d, 1H), 8.31 (dd, 1H), 7.65-7.35 (m, 13H), 6.35 (t, 1H), 4.32-4.14 (m, 2H), 3.92-3.80 (m, 2H), 3.32 (s, 3H), 2.78-2.71 (m, 2H), 0.97 (s, 9H).
LC-MS (Methode 8): Rₜ = 4.59 min
MS (ESIpos): m/z = 543 (M+H⁺)⁺

### Beispiel 32A

### 1-[4-Amino-2-(methoxymethyl)phenyl]-3-(2-{[tert-butyl(diphenyl)silyl]oxy}ethyl)pyridin-2(1H)-on

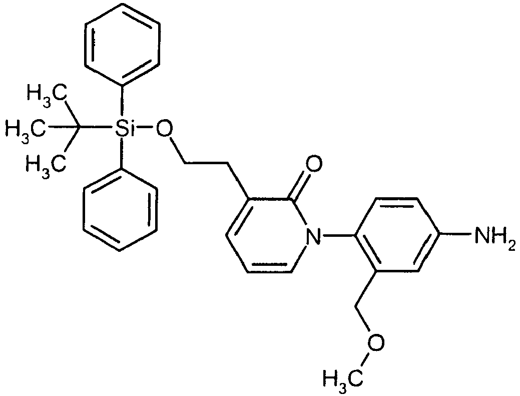

25 g (48 mmol) der Verbindung aus Beispiel 31A werden in 500 ml Essigsäureethylester und 500 ml Ethanol gelöst. Es wird 18 g (286 mmol) Ammoniumformiat und 1g Palladium auf Kohle zugegeben und 45 min am Rückfluß erhitzt. Dann lässt man erkalten und filtriert die Reaktionslösung über Kieselgel. Das Filtrat wird im Vakuum zur Trockene eingeengt. Es werden 25 g (100% d. Th.) des gewünschten Produktes erhalten.
¹H-NMR (400 MHz, DMSO-d₆, δ/ppm): δ = 7.65-7.20 (m, 12H), 6.79 (d, 1H), 6.68 (d, 1H), 6.54 (dd, 1H), 6.20 (t, 1H), 5.46-5.25 (m, 2H), 4.04-3.91 (m, 2H), 3.87-3.77 (m, 2H), 3.07 (s, 3H), 2.75-2.68 (m, 2H), 0.95 (s, 9H).
LC-MS (Methode 6): Rₜ = 3.22 min
MS (ESIpos): m/z = 513 (M+H⁺)⁺

### Beispiel 33A

### N-{[(5S)-3-{4-[3-(2-{[tert-Butyl(diphenyl)silyl]oxy}ethyl)-2-oxopyridin-1(2H)-yl]-3-(methoxymethyl)phenyl}-2-oxo-1,3-oxazolidin-5-yl]methyl}-5-chlorthiophen-2-carboxamid

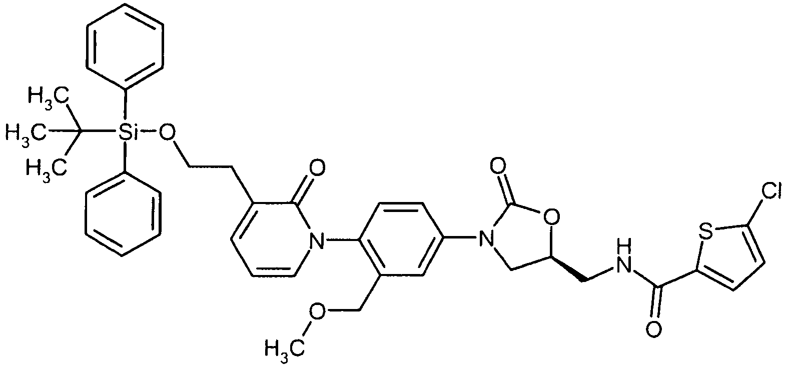

25 g (47 mmol) der Verbindung aus Beispiel 32A werden in 500 ml wasserfreiem Acetonitril gelöst und mit 15 g (61 mmol) der Verbindung aus Beispiel 1A und 16 g (71 mmol) Magnesiumperchlorat versetzt. Es wird 5 h bei Raumtemperatur gerührt bevor erneut 1 g (4.1 mmol) der Verbindung aus Beispiel 1A zugegeben wird. Nach 21 h werden 15.3 g (95 mmol) Carbonyldiimidazol und 116 mg (0.65 mmol) 4-Dimethylaminopyridin zugegeben und es wird 3.5 h am Rückfluß erhitzt. Dann wird das Lösemittel im Vakuum entfernt und der Rückstand in 800 ml Essigsäureethylester aufgenommen. Die Lösung wird mit Wasser sowie zweimal mit gesättigter Natriumchloridlösung gewaschen, über Magnesiumsulfat getrocknet und anschließend im Vakuum eingeengt. Der Rückstand wird an Kieselgel unter Verwendung eines Gradienten aus Cyclohexan und Essigsäureethylester getrennt. Die produkthaltigen Fraktionen werden vereinigt und im Vakuum zur Trockene eingeengt. Es werden 26.5 g (72% d. Th.) des gewünschten Produktes erhalten.
¹H-NMR (400 MHz, DMSO-d₆, δ/ppm): δ = 9.00 (t, 1H), 7.73-7.35 (m, 15H), 7.23 (d, 1H), 7.19 (d, 1H), 6.28 (t, 1H), 4.90-4.81 (m, 1H), 4.28-3.80 (m, 6H), 3.62 (t, 2H), 3.11 (s, 3H), 2.79-2.71 (m, 2H), 0.97 (s, 9H).
LC-MS (Methode 6): Rₜ = 3.39 min
MS (ESIpos): m/z = 756 (M+H⁺)⁺

### Beispiel 34A

### N-{[(5S)-3-{4-[3-(2-{[tert-Butyl(diphenyl)silyl]oxy}ethyl)-2-oxopyridin-1(2H)-yl]-3-(methoxymethyl)phenyl}-2-oxo-1,3-oxazolidin-5-yl]methyl}-5-chlorthiophen-2-carboxamid

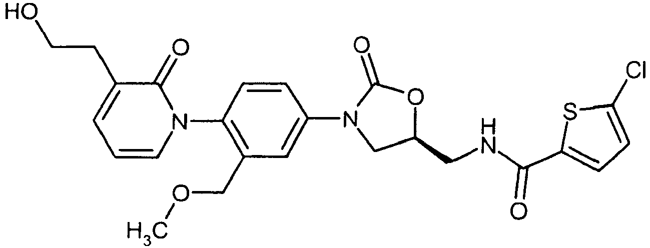

27 g (34 mmol) der Verbindung aus Beispiel 33A werden unter Eiskühlung mit 135 ml 1.25N Salzsäure in Methanol versetzt. Es wird 45 min bei dieser Temperatur weiter gerührt. Unter Eiskühlung wird mit 1N Natronlauge auf pH 7 gebracht und die kalte Lösung wird mehrmals mit Dichlormethan extrahiert. Die vereinigten organischen Phasen werden mit gesättigter Natriumchloridlösung gewaschen und über Magnesiumsulfat getrocknet. Es wird im Vakuum zur Trockene eingedampft und der Rückstand wird mit einem Gradienten aus Dichlormethan und Methanol an Kieselgel chromatographiert. Die produkthaltigen Fraktionen werden vereinigt und im Vakuum zur Trockene eingeengt. Es werden 16.4 g (89% d. Th.) des gewünschten Produktes erhalten.
¹H-NMR (400 MHz, DMSO-d₆, δ/*ppm*): δ = 8.98 (t, 1H), 7.72-7.66 (m, 2H), 7.62-7.56 (m, 1H), 7.40 (dd, 1H), 7.36 (dd, 1H), 7.27 (d, 1H), 7.20 (d, 1H), 6.25 (t, 1H), 4.90-4.82 (m, 1H), 4.60 (t, 1H), 4.27-4.08 (m, 3H), 3.94-3.87 (m, 1H), 3.65-3.55 (m, 4H), 3.18 (s, 3H), 2.60 (t, 2H).
LC-MS (Methode 6): Rₜ = 1.96 min
MS (ESIpos): m/z = 518 (M+H⁺)⁺

### Beispiel 35A

### 3-Allyl-1-(2-methyl-4-nitrophenyl)pyridin-2(1H)-on

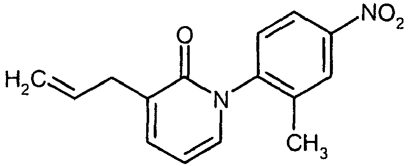

In einem ausgeheizten Kolben werden 1.50 g (4.85 mmol) der Verbindung aus Beispiel 12A, 1.44 g (9.46 mmol) Cäsiumfluorid und 0.56 g (0.48 mmol) Tetrakkis-(triphenylphosphin)-Palladium(0) in 30 ml entgastem THF vorgelegt. Man tropft eine Lösung von 2.04 g (12.1 mmol) 2-Allyl-4,4,5,5-tetramethyl-1,3,2-dioxaborolan in 5 ml entgastem THF hinzu und erhitzt über Nacht auf Rückfluß. Dann wird mit Dichlormethan verdünnt und mit Wasser versetzt. Nach Phasentrennung wird die wässrige Phase dreimal mit Dichlormethan extrahiert. Die vereinten organischen Phasen werden über Natriumsulfat getrocknet und eingeengt. Man reinigt durch Chromatographie an Kieselgel und erhält 1.18 g (62% d. Th.) der Titelverbindung.
¹H-NMR (400 MHz, *DMSO-d₆,* δ/*ppm*): 8.31 (d, 1H), 8.18 (dd, 1H), 7.57 (d, 1H), 7.46 (dd, 1H), 7.44-7.38 (m, 1H), 6.35 (t, 1H), 5.96 (dddd, 1H), 5.16-5.06 (m, 2H), 3.20 (d, 2H), 2.15 (s, 3H).
HPLC (Methode 2): Rₜ = 4.05 min.
MS (ESIpos, *mlz*): 271 (M+H)⁺.

### Beispiel 36A

### 3-(3-Hydroxypropyl)-1-(2-methyl-4-nitrophenyl)pyridin-2(1H)-on

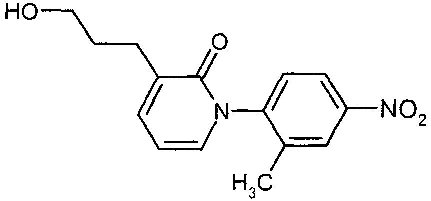

Zu 1.00 g (3.70 mmol) der Verbindung aus Beispiel 35A in 4 ml THF werden bei 0°C langsam 18.5 ml (9.25 mmol) einer 0.5 molaren Lösung von 9-Borabicyclo[3.3.1]nonan in THF getropft. Nach einer Stunde bei Raumtemperatur wird erneut auf 0°C gekühlt und es werden 18.5 ml (18.5 mmol) einer 1 molaren Lösung von Natriumhydroxid in Wasser zugetropft. Man rührt weitere 30 min bei 0°C und gibt dann 3.24 ml einer 30%-igen Wasserstoffperoxidlösung so zu, daß die Temperatur nicht über 30°C ansteigt. Man läßt 30 min bei Eiskühlung rühren und versetzt dann mit mit Essigsäureethylester sowie anschließend mit 11 g (40 mmol) Natriumhydrogensulfit-Lösung. Die organische Phase wird abgetrennt und die wässrige Phase zweimal mit Essigsäureethylester extrahiert. Die vereinigten organischen Phasen werden mit gesättigter wässriger Natriumchlorid-Lösung gewaschen. Die organische Phase wird über Natriumsulfat getrocknet und dann im Vakuum zur Trockene eingedampft. Der Rückstand wird über Kieselgel (Cyclohexan/Essigsäureethylester 1:4) chromatographiert. Man erhält 1.03 g (83% d. Th.) des gewünschten Produktes.
¹H-NMR (400 MHz, DMSO-d₆, δ/*ppm*): δ = 8.31 (d, 1H), 8.18 (dd, 1H), 7.66-7.51 (m, 1H), 7.47-7.38 (m, 2H), 6.33 (t, 1H), 4.46 (t, 1H), 3.46-3.38 (q, 2H), 2.52-2.45 (m, 2H), 2.15 (s, 3H), 1.73-1.62 (m, 2H).
HPLC (Methode 1): Rₜ = 3.51 min.
MS (DCI, m/z) = 289 (M+H)⁺

### Beispiel 37A

### 1-(4-Amino-2-methylphenyl)-3-(3-hydroxypropyl)pyridin-2(1H)-on

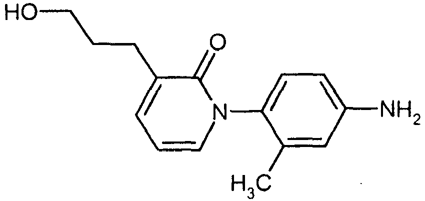

475 mg (1.65 mmol) der Verbindung aus Beispiel 36A werden in 48 ml THF gelöst. Dann werden 50 mg (0.05 mmol) Palladium auf Kohle hinzugefügt und es wird bei RT in einer Wasserstoffatmosphäre unter normalem Druck hydriert. Anschließend wird filtriert, dreimal mit THF nachgewaschen und das Filtrat vom Lösungsmittel befreit. Das Reaktionsprodukt wird ohne weitere Aufreinigung weiter umgesetzt.
HPLC (Methode 1): Rₜ = 2.82 min.
MS (DCI, *m*/*z*): 259 (M+H)⁺.

### Beispiel 38A

### 5-Chlor-N-{[(5S)-3-{4-[3-(3-hydroxypropyl)-2-oxopyridin-1(2H)-yl]-3-methylphenyl}-2-oxo-1,3-oxazolidin-5-yl]methyl}thiophen-2-carboxamid

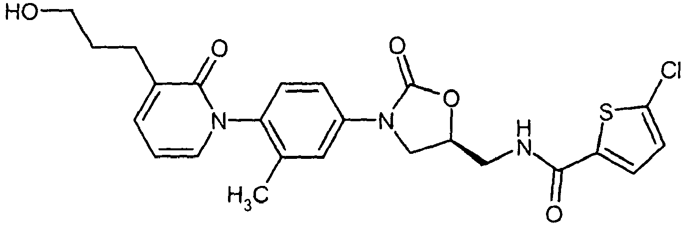

580 mg (2.24 mmol) der Verbindung aus Beispiel 37A werden in 12.7 ml wasserfreiem Acetonitril gelöst und mit 538 mg (2.47 mmol) der Verbindung aus Beispiel 1A und 751 mg (3.37 mmol) Magnesiumperchlorat versetzt. Es wird 3.5 h bei Raumtemperatur gerührt. Dann werden 437 mg (2.69 mmol) Carbonyldiimidazol und 27 mg (0.23 mmol) 4-Dimethylaminopyridin zugegeben und es wird 18 h am Rückfluß erhitzt. Anschließend wird in 100 ml Wasser gegeben und dreimal mit 50 ml Essigsäureethylester extrahiert. Die vereinigten organischen Phasen werden mit gesättigter Natriumchloridlösung gewaschen, über Natriumsulfat getrocknet und anschließend im Vakuum eingeengt. Der Rückstand wird mittels präparativer HPLC gereinigt. Es werden 175 mg (16% d. Th.) des gewünschten Produktes erhalten.
¹H-NMR (400 MHz, DMSO-d₆, δ/*ppm*): δ = 8.99 (t, 1H), 7.70 (d, 1H), 7.57-7.47 (m, 2H), 7.40-7.31 (m, 2H), 7.23 (d, 1H), 7.20 (d, 1H), 6.26 (t, 1H), 4.90-4.81 (m, 1H), 4.46 (dd, 1H), 4.22 (t, 1H), 3.91-3.85 (m, 1H), 3.62 (t, 2H), 3.42 (ddd, 2H), 3.31 (s, 1H), 2.48-2.42 (m, 1H), 2.01 (s, 3H), 1.67 (ddd, 2H).
HPLC (Methode 2): Rₜ = 3.92 min
MS (DCI, m/z) = 502 (M+H)⁺

### Beispiel 39A

### 3-Methoxy-1-(2-methyl-4-nitrophenyl)pyridin-2(1H)-on

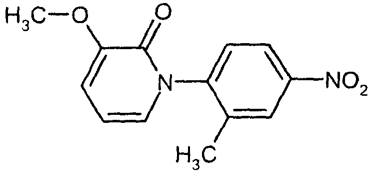

28.5 g (228 mol) 3-Methoxypyridin-2(1H)-on werden in 850 ml Dimethylsulfoxid gelöst und bei RT mit 31 g (273 mmol) Kalium-tert.-butylat versetzt. Die Suspension wird 30 min bei RT gerührt, bevor 35 g (228 mmol) 1-Fluor-2-methyl-4-nitrobenzol zugesetzt werden und die Reaktionslösung für 20 h auf 80°C erhitzt wird. Dann wird vorsichtig mit 1 1 Wasser verdünnt und mit 1N Salzsäure auf pH 1-2 gebracht. Die Lösung wird mehrmals mit Dichlormethan extrahiert. Die vereinigten organischen Extrakte werden mit Wasser und gesättigter Natriumchloridlösung gewaschen, über Natriumsulfat getrocknet, filtriert und im Vakuum eingedampft. Der erhaltene Feststoff wird mit wenig tert-Butylmethylether gewaschen, abfiltriert und im Vakuum getrocknet. Man erhält 42.8 g (72% d. Th.) der gewünschten Verbindung.
¹H-NMR (400 MHz, DMSO-*d₆*, δ/*ppm*): δ = 8.35 (d, 1H), 8.18 (dd, 1H), 7.57 (d, 1H), 7.13 (dd, 1H), 6.95 (dd, 1H), 6.32 (t, 1H), 3.75 (s, 3H), 2.25 (s, 3H).
LC-MS (Methode 3): Rₜ = 1.45 min
MS (ESIpos): m/z = 261 (M+H)⁺

### Beispiel 40A

### 3-Hydroxy-1-(2-methyl-4-nitrophenyl)pyridin-2(1H)-on

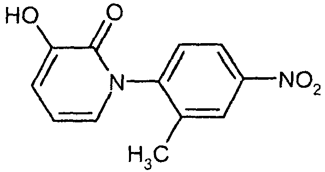

23.3 g (90 mmol) Beispiel 39A werden in 730 ml wasserfreiem Dichlormethan gelöst und auf 0°C gekühlt. Innerhalb von 10 Minuten werden 224 ml (224 mmol) einer 1N Bortribromid Lösung in Dichlormethan zugetropft, bevor weitere 1.5 h bei dieser Temperatur gerührt wird. Der Ansatz wird mit 200 ml Wasser versetzt und die Wasserphase wird dreimal mit Dichlormethan extrahiert. Die vereinigten organischen Phasen werden mit gesättigter Natriumchloridlösung gewaschen, über Natriumsulfat getrocknet und abfiltriert. Das Lösemittel wird im Vakuum entfernt und der erhaltene Feststoff wird mit tert.-Butylmethylether gewaschen und abfiltriert. Man erhält 20.1 g (91% d. Th.) der gewünschten Verbindung.
¹H-NMR (400 MHz, DMSO-*d₆*, δ/*ppm*): δ = 9.50 (s, 1H), 8.42 (d, 1H), 8.20 (dd, 1H), 7.6 (d, 1H), 7.05 (d, 1H), 6.85 (dd, 1H), 6.25 (t, 1H), 2.25 (s, 3H).
LC-MS (Methode 3): Rₜ = 1.45 min
MS (ESIpos): m/z = 246 (M+H)⁺

### Beispiel 41A

### 3-[(2-Methoxyethoxy)methoxy]-1-(2-methyl-4-nitrophenyl)pyridin-2(1H)-on

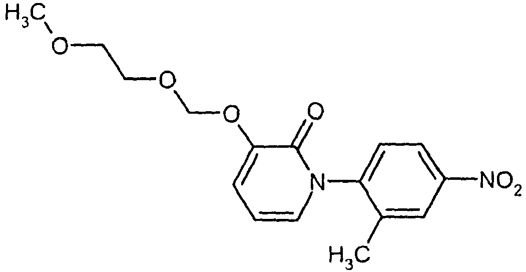

10.0 g (41 mmol) Beispiel 40A werden in wasserfreiem Dichlormethan gelöst und mit 13.6 g (89 mmol) 1,8-Diazabicyclo(5.4.0)undec-7-en versetzt. Zu dieser Lösung wird bei 25°C langsam portionsweise 8.6 g (69 mmol) 1-(Chlormethoxy)-2-methoxyethan zugegeben. Nach einer weiteren Stunde wird die Lösung über Kieselgel filtriert und im Vakuum zur Trockene eingedampft. Man erhält 20.1 g (91% d. Th.) der gewünschten Verbindung.
¹H-NMR (400 MHz, DMSO-*d*₆, δ/*ppm*): δ = 8.32 (d, 1H), 8.19 (dd, 1H), 7.6 (d, 1H), 7.22 (dd, 1H), 7.15 (dd, 1H), 6.3 (t, 1H), 5.25 (s, 2H), 3.78-3.72 (m, 2H), 3.51-3.45 (m, 2H), 3.23 (s, 3H), 2.15 (s, 3H).
LC-MS (Methode 3): Rₜ = 1.79 min
MS (ESIpos): m/z= 335 (M+H)⁺

### Beispiel 42A

### 1-(4-Amino-2-methylphenyl)-3-(2-methoxyethoxy)methoxy]pyridin-2(1H)-on

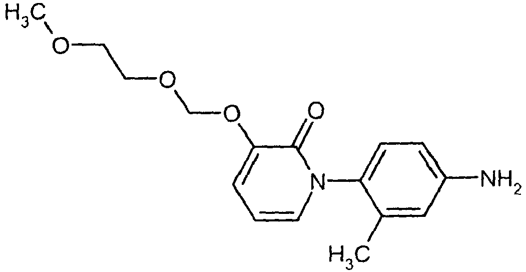

12 g (36 mmol) Beispiel 41 A werden in 1.21 eines 1: Gemische aus Essigsäureethylester und Ethanol gelöst und mit 0.1 Equivalenten Palladium auf Kohle und mit 11.3 g (180 mmol) Ammoniumformiat versetzt. Es wird zwei Stunden auf 80°C erhitzt. Man läßt erkalten, filtriert über Kieselgel und entfernt das Lösemittel im Vakuum. Man erhält 10.6 g (88% d. Th.) der gewünschten Verbindung.
¹H-NMR (400 MHz, DMSO-*d₆*, δ/*ppm*): δ = 7.12-7.pos (m, 1H), 6.80 (d, 1H), 6.51-6.42 (m, 2H), 6.17 (t, 1H), 5.21 (s, 2H), 3.76-3.71 (m, 2H), 3.49-3.45 (m, 2H), 3.23 (s, 3H), 1.85 (s, 3H).
LC-MS (Methode 3): Rₜ = 1.10 min
MS (ESIpos): m/z = 305 (M+H)⁺

### Beispiel 43A

### 5-Chlor-N-[((5S)-3- 4-[3-[(2-methoxyethoxy)methoxy]-2-oxopyridin-1(2H)-yl]-3-methylphenyl}-2-oxo-1,3-oxazolidin-5-yl)methyl]thiophen-2-carboxamid

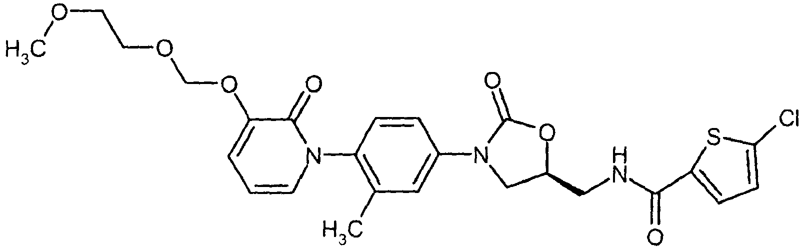

9.2 g (30 mmol) Beispiel 42A werden in 580 ml Acetonitril gelöst und auf 0°C gekühlt. Bei dieser Temperatur werden 7.3 g (34 mmol) Beispiel 1A zugegeben und dann weitere 10 Minuten gerührt. Es wird mit 10.2 g (46 mmol) Magnesiumperchlorat versetzt, die Kühlung entfernt und weiter 17 h nachgerührt. Dann werden 14.8 g (91 mmol) Carbonyldiimidaziol und 0.37 g (3 mmol) N,N-4-Dimethylaminopyridin zugegeben und man erhitzt für 4 h auf 60°C. Nach Erkalten wird weitere 16 h bei RT gerührt und dann im Vakuum zur Trockene eingedampft. Der Rückstand wird mit 1N Salzsäure und Essigsäureethylester versetzt und heftig gerührt. Nach 15 min werden die Phasen getrennt, die wässrige Phase dreimal mit Essigsäureethylester extrahiert und die organischen Phasen vereinigt. Nach Waschen mit gesättigter Natriumchloridlösung wird getrocknet und im Vakuum zur Trockene eingedampft. Man erhält 17.2 g (95% d. Th.) der gewünschten Verbindung.
¹H-NMR (400 MHz, DMSO-*d₆*, δ/*ppm*): δ = 9.0 (t, 1H), 7.70 (d, 1H), 7.56-7.49 (m, 2H), 7.26 (d, 1H), 7.20 (d, 1H), 6.95 (dd, 1H), 6.80 (dd, 1H), 6.20 (t, 1H), 4.90-4.81 (m, 1H), 4.24 (t, 1H), 3.92-3.85 (m, 1H), 3.70-3.55 (m, 3H), 3.52-3.25 (m, 2H), 2.55 (s, 3H), 2.08-2.02 (m, 3H), 1.91 (s, 3H).
LC-MS (Methode 4): Rₜ = 2.13 min
MS (ESIpos): m/z = 548 (M+H)⁺

### Beispiel 44A

### 5-Chlor-N-({(5S)-3-[4-(3-hydroxy-2-oxopyridin-1(2H)-yl)-3-methylphenyl]-2-oxo-1,3-oxazolidin-5-yl}methyl)thiophen-2-carboxamid

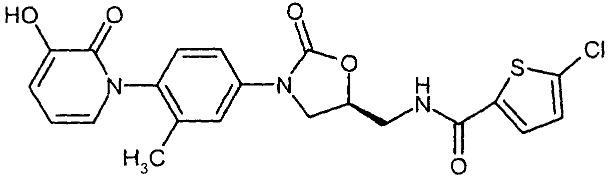

18 g (33 mmol) Beispiel 43A wird in 50 ml Trifluoressigsäure gelöst und 3 h nachgerührt, bevor im Vakuum zur Trockene eingedampft wird. Der Rückstand (22.3 g) wird in Portionen zu je 2 g in je 8.5 ml Dimethylsulfoxid gelöst und mit präparativer HPLC mit einem Wasser/Acetonitril Gradienten gereinigt. Die Produktfraktionen werden vereinigt, vom Acetonitril befreit und die enstandenen Kristalle abfiltriert. Das Filtrat wird mit Essigsäureethylester extrahiert, die Essigsäureethylesterphasen werden vereinigt und mit gesättigter Natriumchloridlösung gewaschen getrocknet und im Vakuum eingedampft. Der Rückstand wird mit den Kristallen vereinigt und im Vakuum getrocknet. Man erhält 9.95 g (65% d. Th.) der gewünschten Verbindung.
¹H-NMR (400 MHz, DMSO-*d₆*, δ/*ppm*): δ = 9.2 (br. s, 1H), 9.00 (t, 1H), 7.70 (d, 1H), 7.56-7.48 (m, 2H), 7.26 (d, 1H), 7.20 (d, 1H), 6.95 (dd, 1H), 6.86 (dd, 1H), 6.2 (t, 1H), 4.92-4.80 (m, 1H), 4.22 (t, 1H), 3.92-3.82 (m, 1H), 3.62 (t, 2H), 2.04 (s, 3H).
LC-MS (Methode 4) Rₜ = 2.09 min
MS (ESIpos): m/z = 460 (M+H)⁺

### Beispiel 45A

### 5-Chlor-N-[((5S)-3-{4-[3-(2-hydroxyethoxy)-2-oxopyridin-1(2H)-yl]-3-methylphenyl}-2-oxo-1,3-oxazolidin-5-yl)methyl]thiophen-2-carboxamid

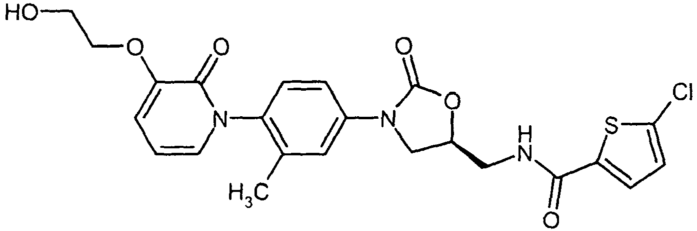

2.0 g (4.35 mmol) Beispiel 44A werden in 9 ml wasserfreiem N,N-Dimethylformamid gelöst, mit 3.6 g (26.1 mmol) Kaliumcarbonat versetzt und 30 min nachgerührt. Es wird mit weiteren 3 ml wasserfreiem N,N-Dimethylformamid verdünnt, mit 3.1 g (13.05 mmol) (2-Bromethoxy)(tert.-butyl)dimethylsilan versetzt und 7 h auf 60°C erhitzt. Man lässt erkalten, filtriert und reinigt mit präparativer HPLC mit einem Wasser/Acetonitril Gradient. Die Produktfraktionen werden im Vakuum zur Trockene eingedampft und der Rückstand im Vakuum getrocknet. Man erhält 1.17 g (53% d. Th.) der gewünschten Verbindung.
¹H-NMR (400 MHz, DMSO-*d₆*, δ/*ppm*): δ = 9.00 (t, 1H), 7.70 (d, 1H), 7.55-7.48 (m, 2H), 7.25-7.18 (m, 2H), 7.05 (dd, 1H), 6.93 (dd, 1H), 6.22 (t, 1H), 4.91 (t, 1H), 4.89-4.82 (m, 1H), 4.22 (t, 1H), 4.00-3.85 (m, 3H), 3.75-3.70 (m, 2H), 3.61 (t, 2H), 2.0 (s, 3H).
LC-MS (Methode 5): Rₜ = 1.93 min
MS (ESIpos): m/z = 504 (M+H)⁺

### Beispiel 46A

### 5-Chlor-N-{[(5S)-3-{4-[3-(2-chlorethoxy)-2-oxopyridin-1(2H)-yl]-3-methylphenyl}-2-oxo-1,3-oxazolidin-5-yl]methyl}thiophen-2-carboxamid

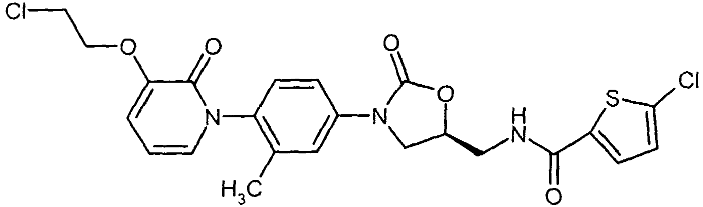

4.5 g (7.5 mmol) Beispiel 44A und 1.4 g (9.8 mmol) 2-Chlor-ethylbromid werden in 45 ml absolutem N,N-Dimethylformamid gelöst und mit 3.1 g (22.6 mmol) Kaliumcarbonat versetzt. Die Reaktion wird auf drei Reaktionsvials aufgeteilt und bei 80°C 40 Minuten in der Mikrowelle (300 W) gerührt. Zu den abgekühlten Reaktionvials werden in drei Portionen insgesamt 1.4 g (9.8 mmol) 2-Chlor-ethylbromid gegeben und für weitere 60 Minuten bei 80°C in der Mikrowelle (300 W) gerührt. Die Reaktion wird mit Wasser versetzt, mit Dichlormethan extrahiert und die separierte organische Phase mit 1N Salzsäure und gesättigter Natriumchlorid gewaschen. Über Natriumsulfat wird die Dichlormethan-Phase getrocknet, das Trockenmittel abfiltriert und das Lösungsmittel am Rotationsverdampfer abdestilliert. Danach chromatographiert man den Rückstand über Kieselgel 60 mit dem Laufmittel Dichlormethan/Acetonitril 10/1 und spült mit Dichlormethan/Methanol 10/1 nach. Die produkthaltigen Fraktionen werden bis zur Trockne eingeengt. Nach der Feinreinigung über präparative RP-HPLC mit einem Acetonitril/WasserGemisch wird 1.4 g Produkt (35% d. Th.) erhalten.
¹H-NMR (400 MHz, DMSO-*d₆*, δ/*ppm*): 8.96 (t, 1H), 7.69 (d, 1H), 7.55-7.46 (m, 2H), 7.23 (d, 1H), 7.20 (d, 1H), 7.12 (d, 1H), 6.98 (d, 1H), 6.23 (t, 1H), 4.86 (m, 1H), 4.32-4.17 (m, 3H), 3.97 (m, 2H), 3.92-3.84 (m, 1H), 3.62 (t, 2H), 2.01 (s, 3H).
LC-MS (Methode 6): Rₜ = 2.31 min, 2.36 min
MS (ESIpos): m/z = 522 (M+H)⁺

### Beispiel 47A

### (2-Fluor-5-nitrobenzyl)(triphenyl)phosphonium-bromid

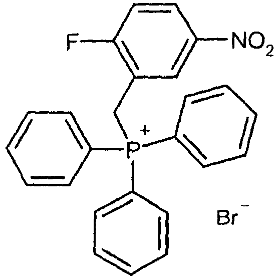

20 g (85.5 mmol) der Verbindung aus Beispiel 26A werden in 250 ml wasserfreiem Toluol gelöst und mit 22.4 g (85.5 mmol) Triphenylphosphin versetzt. Die Lösung wird 16 h unter Rückfluss erhitzt, wobei sich ein Niederschlag abscheidet. Man lässt erkalten und filtriert den Niederschlag ab. Nach Waschen mit Diethylether wird im Vakuum getrocknet. Es werden 39 g (92% d. Th.) des gewünschten Produktes erhalten.
¹H-NMR (400 MHz, DMSO-*d₆*, δ/ppm): δ = 8.30-8.23 (m, 1H), 7.98-7.88 (m, 4H), 7.81-7.70 (m, 12H), 7.45 (t, 1H), 5.32 (d, 2H).

### Beispiel 48A

### 1-Fluor-4-nitro-2-[prop-1-en-1-yl]benzol

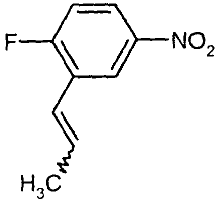

Bei 10°C werden zu einer Lösung von 13.5 g (27.3 mmol) der Verbindung aus Beispiel 47A in 145 ml Dioxan 5.99 g (32.7 mmol) Natriumbis(trimethylsiliy)amid getropft. Es wird 1 h bei dieser Temperatur gerührt. Dann wird eine Lösung von 2.40 g (54.5 mmol) Acetaldehyd in 5 ml Dioxan zugegeben und die Reaktion 1 h bei RT gerührt. Anschließend wird mit 400 ml Wasser versetzt, dreimal mit Dichlormethan extrahiert und die vereinigten organischen Phasen zweimal mit gesättigter wässriger Natriumchlorid-Lösung gewaschen. Nach Trocknen über Natriumsulfat und anschließender Filtration wird das Lösungsmittel im Vakuum entfernt. Man reinigt mittels Chromatographie an Kieselgel (Eluens: Cyclohexan/Essigsäureethylester = 40:1). Es werden 5.2 g (100% d. Th.) des gewünschten Produktes als E/Z-Isomeren-Gemisch erhalten.
¹H-NMR (400 MHz, DMSO-d₆, δ/ppm): δ = 8.47-8.05 (m, 2H), 7.58-7.42 (m, 1H), 6.70-6.05 (m, 2H), 1.90-1.78 (m, 3H).
GC-MS (Methode 10): Rₜ = 2.64 und 2.70 min
MS (ESIpos): m/z= 181 (M+H⁺)⁺

### Beispiel 49A

### 3-Methoxy-1-{4-nitro-2-[prop-1-en-1-yl]phenyl}pyridin-2(1H)-on

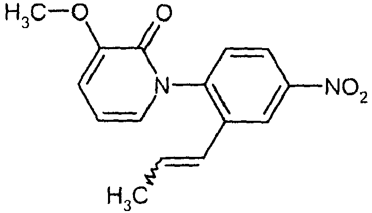

14.0 g (111.9 mmol) 3-Methoxypyridin-2(1H)-on werden in 350 ml Dimethylsulfoxid gelöst, mit 15.4 g (134.3 mmol) Kalium-tert.-butylat versetzt und die Suspension wird 30 Minuten bei Raumtemperatur nachgerührt. Zur Reaktionsmischung werden 20.2 g (111.9 mmol) Beispiel 48A gegeben. Es wird auf 80°C erwärmt und 12 Stunden bei der Temperatur nachgerührt. Die Reaktion wird mit 150 ml 1N Salzsäure unter leichter Kühlung auf pH 3 eingestellt, mit 800 ml Wasser verdünnt, mit Dichlormethan extrahiert und die organische Phase nacheinander mit Wasser und gesättigter Natriumchlorid-Lösung gewaschen. Anschließend wird die Dichlormethan-Phase über Natriumsulfat getrocknet, das Trockenmittel abfiltriert und das Lösungsmittel vollständig am Rotationsverdampfer entfernt. Aus dem Rückstand werden nach dem Umkristallisieren mit tert-Butylmethylether 17.9 g Produkt (45% d. Th.) erhalten.
¹H-NMR (400 MHz, DMSO-*d₆*, δ/*ppm*): 8.50 (d, 0.75H), 8.17 (dd, 0.25H), 8.22-8.13 (m, 1H), 7.17 (d, 0.25H), 7.57 (d, 0.75H), 7.09 (dd, 1H), 6.96 (dd, 0.75H) 6.91 (d, 0.25H), 6.59 (m, 0.75H), 6.35-6.23 (m, 1H), 6.09-5.85 (m, 1.25H), 3.78 (s, 2.25H), 3.75 (s, 0.75H), 1.81 (dd, 2.25H), 1.73 (dd, 0.75H).
LC-MS (Methode 3): Rₜ = 2.75 min, 2.79 min
MS (ESIpos): m/z = 273 (M+H)⁺

### Beispiel 50A

### 3-Hydroxy-1-(4-nitro-2-[prop-1-en-1-yl]phenyl)pyridin-2(1H)-on

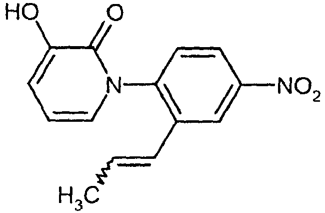

17.8 g (50.6 mmol) Beispiel 49A werden in 300 ml Dichlormethan gelöst. Der eisgekühlten Lösung werden 31.7 g (126.5 mmol) Bortribromid, gelöst in 100 ml Dichlormethan, tropfenweise zugegeben und es wird 1.5 Stunden bei 0°C nachgerührt. Zur rührenden Reaktion wird Eis gegeben. Dann wird mit Wasser verdünnt und anschließend mehrmals mit Dichlormethan/Methanol 10/1 und Chloroform extrahiert. Die vereinigten organischen Phasen werden über Natriumsulfat getrocknet, das Trockenmittel wird abfiltriert und das Lösungsmittel am Rotationsverdampfer abdestilliert. Aus dem Rückstand werden nach dem Umkristallisieren mit tert.-Butylmethylether/ Dichlormethan (10/1) 9.4 g Produkt (67% d. Th.) erhalten.
¹H-NMR (400 MHz, DMSO-*d₆*, δ/*ppm*): 9.43 (br. s, 1H), 8.50 (d, 0.75H), 8.27 (dd, 0.25H), 8.22-8.13 (m, 1H), 7.19 (d, 0.25H), 7.59 (d, 0.75H), 6.99 (dd, 1H), 6.85 (d, 0.75H) 6.81 (d, 0.25H), 6.58 (m, 0.75H), 6.31-6.19 (m, 1H), 6.11-5.85 (m, 1.25H), 1.81 (dd, 2.25H), 1.73 (dd, 0.75H).
LC-MS (Methode 3): Rₜ = 1.78 min
MS (ESIpos): m/z = 287 (M+H)⁺

### Beispiel 51A

### 3-[(2-Methoxyethoxy)methoxy]-1-{4-nitro-2-[prop-1-en-1-yl]phenyl}pyridin-2(1H)-on

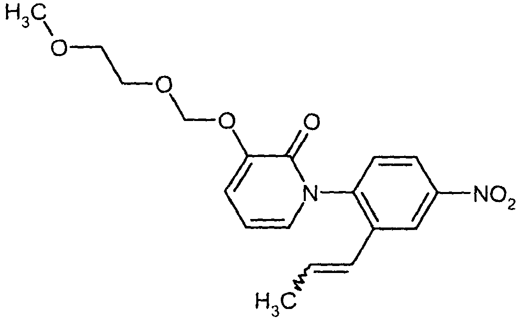

15.0 g (48.9 mmol) Beispiel 50A werden in 700 ml Dichlormethan gelöst und mit 16.4 g (107 mmol) 1,8-Diazabicyclo[5.4.0]undec-7-en versetzt. Zur wassergekühlten Mischung wird eine Lösung aus 100 ml Dichlormethan und 10.4 g (83.2 mmol) 1-(Chlormethoxy)-2-methoxyethan getropft und es wird 4 Stunden bei Raumtemperatur gerührt. Zur Aufarbeitung wird die Reaktionslösung direkt auf Kieselgel 60 aufgegeben und mit einem Laufmittelgradienten aus Cyclohexan und Essigsäureethylester (2/1 → 1/2) chromatographiert. 9.6 g (43% d. Th.) Produkt werden erhalten.
¹H-NMR (400 MHz, DMSO-*d₆*, δ/*ppm*): 8.50 (d, 0.5H), 8.27 (dd, 0.5H), 8.22-8.13 (m, 1H), 7.19 (d, 0.5H), 7.59 (d, 0.5H), 7.23-7.10 (m, 2H), 6.58 (m, 0.5H) 6.85-6.25 (m, 1H), 6.60-5.85 (m, 1.5H), 5.28 (s, 1H), 5.23 (s, 1H), 3.75 (m, 2H), 3.48 (m, 2H), 3.25 (s, 3H), 1.80 (dd, 1.5H), 1.72 (dd, 1.5H).
LC-MS (Methode 6): Rₜ = 2.09 min
MS (ESIpos): m/z = 361 (M+H)⁺

### Beispiel 52A

### 1-(4-Amino-2-propylphenyl)-3-[(2-methoxyethoxy)methoxy]pyridin-2(1H)-on

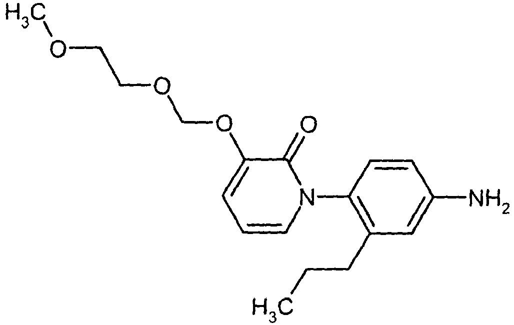

2.9 g (7.0 mmol) Beispiel 51A werden in einer Mischung aus 56 ml Essigsäureethylester und 35 ml Ethanol gelöst und nacheinander mit 2.2 g (35.0 mmol) Ammoniumformiat und einer katalytischen Menge 10%ige Palladium auf Kohle versetzt. Die Reaktion wird 2 Stunden bei 80°C gerührt. Zur Aufarbeitung wird die abgekühlte Suspension über Kieselgel 60 filtriert, mit Ethanol gewaschen und das Filtrat wird am Rotationsverdampfer bis zur Trockne eingeengt. Der Rückstand wird mit einer Mischung aus 56 ml Essigsäureethylester und 35 ml Ethanol gelöst und mit 2.2 g (35.0 mmol) Ammoniumformiat sowie einer katalytischen Menge 10%ige Palladium auf Kohle versetzt. Bei einer Reaktionszeit von 24 Stunden und einer Temperatur von 80°C wird das Edukt vollständig reduziert. Nochmals wird die abgekühlte Suspension über Kieselgel 60 filtriert, mit Ethanol eluiert und das Lösungsmittel am Rotationsverdampfer vollständig entfernt. Es werden 3.0 g Rohprodukt erhalten.
LC-MS (Methode 6): Rₜ = 1.73 min, 1.81 min
MS (ESIpos): m/z = 333 (M+H)⁺

### Beispiel 53A

### 5-Chlor-N-({(5S)-3-[4-(3-hydroxy-2-oxopyridin-1(2H)-yl)-3-propylphenyl]-2-oxo-1,3-oxazolidin-5-yl}methyl)thiophen-2-carboxamid

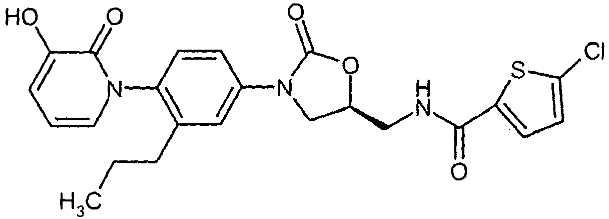

3.0 g (9.0 mmol) Beispiel 52A werden in 125 ml Acetonitril gelöst. Die eisgekühlte Lösung wird mit 2.6 g (9.9 mmol) Beispiel 1A versetzt, 10 Minuten gerührt und anschließend werden portionsweise 3.0 g (13.5 mmol) Magnesiumperchlorat zugegeben. Man rührt bei Raumtemperatur über Nacht und versetzt dann mit 4.4 g (27.1 mmol) 1,1-Carbodiimidazol und 0.1 g (0.9 mmol) 4-Dimethylaminopyridin. Nach 4 Stunden bei 60°C wird die Suspension filtriert, der Filterrückstand mit Acetonitril gewaschen und das Filtrat am Rotationsverdampfer bis zur Trockne eingeengt. Der Rückstand wird mit Essigsäureethylester aufgenommen, mit 50 ml 1N Salzsäure extrahiert, die wässrige Säurephase mehrmals mit Essigsäureethylester extrahiert und die vereinigten organischen Phasen mit gesättigter Natriumchlorid-Lösung gewaschen. Anschließend wird die Essigsäureethylester-Phase über Natriumsulfat getrocknet, das Trockenmittel abfiltriert und das Lösungsmittel am Rotationsverdampfer vollständig entfernt. Nach der Feinreinigung über präparative RP-HPLC mit einem Acetonitril/Wasser-Gemisch werden 1.1 g (19% d. Th.) Produkt erhalten.
LC-MS (Methode 11): Rₜ = 3.26 min
MS (ESIpos): m/z = 488 (M+H)⁺

### Beispiel 54A

### 5-Chlor-N-{[(5S)-3-{4-[3-(2-chlorethoxy)-2-oxopyridin-1(2H)-yl]-3-propy)phenyl}-2-oxo-1,3-oxazolidin-5-yl]methyl}thiophen-2-carboxamid

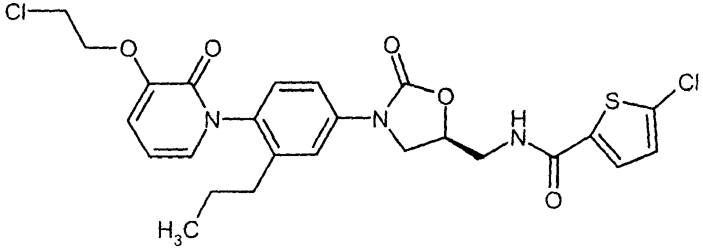

241 mg (0.50 mmol) Beispiel 53A und 270 mg (1.4 mmol) 1-Chlor-2-iodethan werden in 9 ml N,N-Dimethylformamid gelöst und mit 131 mg (0.90 mmol) Kaliumcarbonat versetzt. Die Reaktion wird 4 Stunden bei 60°C gerührt. Zur Aufarbeitung wird der Suspension Wasser zugegeben und mit Dichlormethan extrahiert. Die abgetrennte organische Phase wird nacheinander mit 1N Salzsäure und gesättigter Natriumchlorid-Lösung gewaschen, anschließend über Natriumchlorid getrocknet, das Trockenmittel abfiltriert und das Lösungsmittel am Rotationsverdampfer vollständig entfernt. Nach dem Trocknen des Rückstandes im Vakuum werden 30 mg (12% d. Th.) Produkt erhalten.
LC-MS (Methode 6): Rₜ = 2.36 min
MS (ESIpos): m/z = 550 (M+H)⁺

### Beispiel 55A

### 1-(4-Amino-2-propylphenyl)-3-methoxypyridin-2(1H)-on

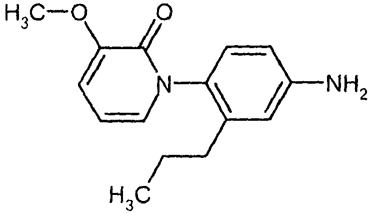

27.0 g (87.7 mmol) Beispiel 49A werden in 702 ml Essigsäureethylester und 441 ml Ethanol gelöst, mit 16.6 g (263 mmol) Ammoniumformiat und 0.093 g (0.09 mmol)Palladium/Kohle (10%ig) versetzt und 16 h bei 80°C gerührt. Aufgrund unvollständiger Umsetzung werden 5.5 g (87 mmol) Ammoniumformiat und 0.03 g (0.03 mmol) Palladium/Kohle (10%ig) zugegeben. Nach weiteren 16 h Rühren bei 80°C werden erneut diese Mengen zugegeben und es wird 16 h bei 80°C gerührt. Zur Aufarbeitung wird die Reaktionslösung auf Raumtemperatur gebracht und über eine Kieselgelfritte gegeben. Es wird mit Ethanol nachgewaschen, das Filtrat im Vakuum eingeengt und der Rückstand über Kieselgel 60 mit einem Gradienten aus Dichlormethan und Methanol (100/l → 30/l) chromatographiert. 17.3 g Produkt werden erhalten, die jedoch noch Signale im ¹H-NMR-Spektrum für das doppelbindungshaltige Zwischenprodukt enthalten. Das verunreinigte Material wird daher in 360 ml Essigsäureethylester und 226 ml Ethanol gelöst, mit 12.8 g (202 mmol) Ammoniumformiat und 0.072 g (0.07 mmol) Palladium/Kohle (10%ig) versetzt und 36 h bei 80°C gerührt. Nach dem Abkühlen wird über eine Kieselgelfritte filtriert, mit Ethanol nachgewaschen und im Vakuum eingeengt. Der Rückstand wird über Kieselgel 60 mit einem Gradienten aus Dichlormethan und Methanol (100/1 → 10/1) chromatographiert. 8.35g Produkt (47% d. Th.) werden erhalten.
¹H-NMR (400 MHz, DMSO-*d₆*, δ/*ppm*): 6.97 (dd, 1H), 6.85 (dd, 1H), 6.75 (d, 1H), 6.50 (d, 1H), 6.45 (dd, 1H), 6.15 (t, 1H), 5.22 (s, 2H) 3.71 (s, 3H), 2.12 (t, 2H), 1.45-1.30 (m, 2H), 0.76 (t, 3H).
LC-MS (Methode 6): Rₜ = 1.53 min
MS (ESIpos): m/z = 259 (M+H)⁺.

### Beispiel 56A

### 5-Chlor-N-({(5S)-3-[4-(3-methoxy-2-oxopyridin-1(2H)-yl)-3-propylphenyl]-2-oxo-1,3-oxazolidin-5-yl}methyl)thiophen-2-carboxamid

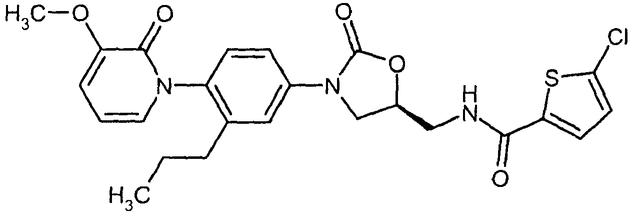

1.00 g (3.87 mmol) Beispiel 55A werden in 23.5 ml wasserfreiem Acetonitril gelöst und mit 926 mg (4.26 mmol) Beispiel 1A und 1.30 g (5.81 mmol) Magnesiumperchlorat versetzt. Es wird 3.5 h bei Raumtemperatur gerührt. Dann werden 1.57 g (9.68 mmol) Carbonyldiimidazol und 47 mg (0.38 mmol) 4-Dimethylaminopyridin zugegeben und es wird 4 h bei 60°C und dann 18 h bei RT gerührt. Anschließend wird mit 300 ml Wasser und 150 ml Essigsäureethylester verdünnt und die wässrige Phase zweimal mit 150 ml Essigsäureethylester extrahiert. Die vereinigten organischen Phasen werden über Natriumsulfat getrocknet, im Vakuum eingeengt und der erhaltene Rückstand durch Chromatographie an Kieselgel (Dichlormethan7Methanol 20:1) gereinigt. Es werden 1.81 g (92% d. Th.) des gewünschten Produktes erhalten.
¹H-NMR (400 MHz, DMSO-*d₆*, δ/*ppm*): δ = 8.99 (t, 1H), 7.70 (d, 1H), 7.58-7.47 (m, 2H), 7.26-7.15 (m, 2H), 7.04 (dd, 1H), 6.90 (dd, 1H), 6.23 (dd, 1H), 4.91-4.81 (m, 1H), 4.23 (dd, 1H), 3.93-3.85 (m, 1H), 3.74 (s, 3H), 3.62 (dd, 2H), 2.31-2.23 (m, 2H), 1.50-1.36 (m, 2H), 0.78 (t, 3H).
HPLC (Methode 2): Rₜ = 4.19 min
MS (ESI pos, m/z) = 502 (M+H)⁺

### Beispiel 57A

### 5-Chlor-N-({(5S)-3-[4-(3-hydroxy-2-oxopyridin-1(2H)-yl)-3-propylphenyl]-2-oxo-1,3-oxazolidin-5-yl} methyl)thiophen-2-carboxamid

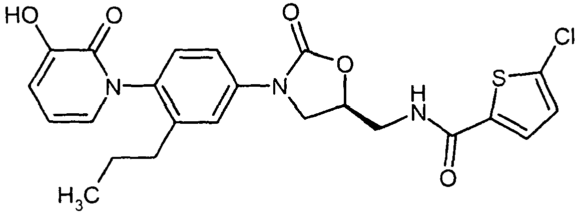

1.79 g (3.56 mmol) Beispiel 56A werden in 104 ml wasserfreiem Dichlormethan gelöst und auf - 78°C gekühlt. Bei dieser Temperatur werden 28.5 ml (28.5 mmol) einer 1N Bortribromid-Lösung in Dichlormethan so zugetropft, dass die Temperatur -65°C nicht überschreitet. Es wird 2 h bei - 78°C und dann 1 h bei RT gerührt, ehe vorsichtig auf 200 ml gesättigte wässrige Natriumhydrogencarbonat-Lösung gegeben wird. Nach Phasentrennung wird die Wasserphase dreimal mit Dichlormethan extrahiert, die vereinigten organischen Phasen mit gesättigter Natriumchloridlösung gewaschen, über Natriumsulfat getrocknet, filtriert und im Vakuum zur Trockene eingedampft. Man erhält 1.75 g (97% d. Th.) der gewünschten Verbindung.
¹H-NMR (400 MHz, DMSO-*d₆*, δ/*ppm*): δ = 9.21 (s, 1H), 8.99 (t, 1H), 7.70 (d, 1H), 7.56-7.47 (m, 2H), 7.29-7.15 (m, 2H), 6.94 (dd, 1H), 6.80 (dd, 1H), 6.19 (dd, 1H), 4.91-4.81 (m, 1H), 4.23 (dd, 1H), 3.93-3.85 (m, 1H), 3.62 (dd, 2H), 2.35-2.26 (m, 2H), 1.50-1.36 (m, 2H), 0.78 (t, 3H).
HPLC (Methode 2): Rₜ = 4.21 min
MS (DCI): m/z = 488 (M+H)⁺

### Beispiel 58A

### 5-Chlor-N-{[(5S)-3-{4-[3-(2-chlorethoxy)-2-oxopyridin-1(2H)-yl]-3-propylphenyl}-2-oxo-1,3-oxazolidin-5-yl]methyl}thiophen-2-carboxamid

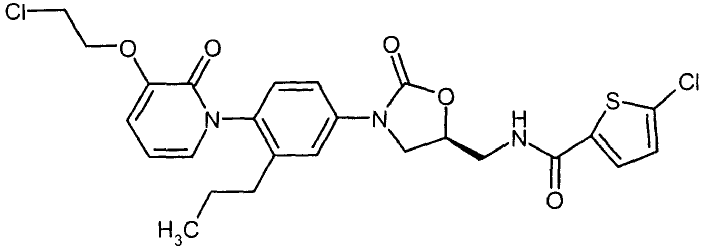

1.70 g (3.48 mmol) Beispiel 57A und 2.49 g (17.4 mmol) 1-Brom-2-chlorethan werden in 55 ml 1-Methyl-2-pyrrolidon gelöst. Die Lösung wird mit 2.27 g (6.96 mmol) Cäsiumcarbonat versetzt und 2 Stunden bei 60°C gerührt. Dann werden 500 ml Wasser, 100 ml gesättigte wässrige Natriumchlorid-Lösung und 200 ml tert-Butylmethylether und 200 ml Essigsäureethylester hinzugefügt. Nach Phasentrennung wird die wässrige Phase zweimal mit 200 ml Essigsäureethylester extrahiert. Die vereinigten organischen Phasen werden nacheinander mit 1N Natronlauge und gesättigter Natriumchlorid-Lösung gewaschen, anschließend über Natriumsulfat getrocknet, das Trockenmittel abfiltriert und das Lösungsmittel am Rotationsverdampfer vollständig entfernt. Der Rückstand wird durch Chromatographie an Kieselgel (Dichlormethan/Ethanol 40:1) gereinigt. Es werden 1.78 g (85% d. Th.) des gewünschten Produktes erhalten.
¹H-NMR (400 MHz, DMSO-*d₆*, δ/*ppm*): δ = 8.99 (t, 1H), 7.70 (d, 1H), 7.56-7.47 (m, 2H), 7.29-7.13 (m, 2H), 7.12 (dd, 1H), 6.99 (dd, 1H), 6.22 (dd, 1H), 4.91-4.82 (m, 1H), 4.30-4.14 (m, 1H), 3.98-3.85 (m, 1H), 3.61 (dd, 2H), 2.35-2.26 (m, 2H), 2.18 (t, 2H), 1.95-1.85 (m, 2H), 1.50-1.36 (m, 2H), 0.78 (t, 3H).
HPLC (Methode 2): Rₜ = 4.44 min
MS (ESIpos): m/z = 550 (M+H)⁺

### Beispiel 59A

### 3-Brom-1-(2,6-dimethyl-4-nitrophenyl)pyridin-2(1H)-on

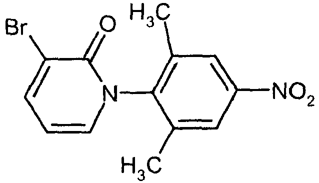

2.81 g (16.1 mmol) 3-Brompyridin-2(1*H*)-on (O. S. Tee, M. Pavent, J. Am. Chem. Soc. 1982, 104, 4142-4146.) werden in 100 ml DMF gelöst. Man kühlt auf 0°C und versetzt mit 2.71 g (24.2 mmol) Kalium-tert.-butylat. Das Eisbad wird entfernt und es wird 30 min bei Raumtemperatur gerührt. 3.00 g (17.7 mmol) 1-Fluor-2,5-dimethyl-4-nitrobenzol werden hinzugegeben und es wird 18 h bei 80°C und 36 h bei 100°C und 18 h bei 120°C gerührt. Dann gibt man auf Wasser und extrahiert dreimal mit Essigsäureethylester. Die vereinten organischen Phasen werden über Natriumsulfat getrocknet. Der Rückstand wird durch Chromatographie an Kieselgel (Cyclohexan/Essigsäure-ethylester 4:1) gereinigt. Man erhält 2.04 g (38% d. Th.) der gewünschten Verbindung.
¹H-NMR (400 MHz, DMSO-*d₆*, δ/*ppm*): δ = 8.19 (s, 2H), 8.14 (dd, 1H), 7.62 (dd, 1H), 6.43 (t, 1H), 2.11 (s, 6H).
HPLC (Methode 1): Rₜ = 4.13 min.
MS (ESIpos, *m*/*z*): 323 (M+H)⁺.

### Alternative Synthese:

150 g (750 mmol) 3-Brompyridin-2(1*H*)-on (O. S. Tee, M. Pavent, J. Am. Chem. Soc. 1982, 104, 4142-4146.) und 207 g (1.50 mol) Kaliumcarbonat werden in 2.9 1 Dimethylsulfoxid gelöst und auf 120°C erhitzt. Bei dieser Temperatur wird eine Lösung von 317 g (750 mmol) 1-Fluor-2,5-dimethyl-4-nitrobenzol in 700 ml Dimethylsulfoxid innerhalb von 60 min zugetropft und es wird 3.5 h bei 120°C gerührt. Nach Erkalten wird die Reaktionslösung in ein Wasser/Salzsäure-Gemisch eingerührt. Es wird mit Essigsäureethylester extrahiert, die Phasen werden getrennt und die wässrige Phase wird nochmals mit Essigsäureethylester extrahiert. Die vereinigten organischen Phasen werden anschließend mit Wasser gewaschen. Die Phasen werden getrennt und die organische Phase wird über Natriumsulfat getrocknet. Nach Einengen im Vakuum wird der Rückstand durch Chromatographie an Kieselgel (Dichlormethan, dann Essigsäureethylester/Dichlormethan 1:20) gereinigt. Die Produkt enthaltenden Fraktionen werden vereint, die Lösungsmittel werden entfernt und der Rückstand in Diethylether verrührt. Man erhält 112 g (46% d. Th.) der gewünschten Verbindung.
¹H-NMR (400 MHz, DMSO-*d₆*, δ/*ppm*): δ = 8.19 (s, 2H), 8.14 (dd, 1H), 7.62 (dd, 1H), 6.43 (t, 1H), 2.11 (s, 6H).
HPLC (Methode 13): Rₜ = 1.59 min.
MS (ESIpos, *m*/*z*): 323 (M+H)⁺.

### Beispiel 60A

### 1-(2,6-Dimethyl-4-nitrophenyl)-3-vinylpyridin-2(1H)-on

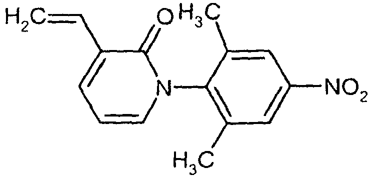

2.00 g (6.19 mmol) der Verbindung aus Beispiel 59A werden in 31 ml wasserfreiem Dioxan gelöst und mit 2.36 g (7.42 mmol) Tributylvinyl-zinn und 143 mg (0.124 mmol) Tetrakis(triphenylphosphin)palladium versetzt und es wird 5 h bei 100°C gerührt. Man lässt erkalten und filtriert über Kieselgur. Es wird dreimal mit Essigsäureethylester nachgewaschen und die vereinigten Filtrate werden im Vakuum zur Trockene eingeengt. Der Rückstand wird durch Chromatographie an Kieselgel (Cyclohexan/Essigsäureethylester 4:1) gereinigt. Es werden 846 mg (51% d. Th.) des gewünschten Produktes erhalten.
¹H-NMR (400 MHz, DMSO-*d₆*, δ/*ppm*): δ = 8.17 (s, 2H), 7.78 (dd, 1H), 7.48 (dd, 1H), 6.75 (dd, 1 H), 6.48 (dd, 1H), 6.14 (dd, 1H), 5.33 (dd, 1H), 2.10 (s, 6H).
HPLC (Methode 1): Rₜ = 4.25 min
MS (ESIpos): m/z = 271 (M+H)⁺

### Alternative Synthese:

132 g (408 mmol) der Verbindung aus Beispiel 59A (Alternativsynthese) werden in 1.27 wasserfreiem Dioxan gelöst und mit 136 g (428 mmol) Tributylvinyl-zinn und 9.44 g (8.17 mmol) Tetrakis(triphenyl-phosphin)palladium versetzt und es wird 4 h bei 100°C gerührt. Man lässt erkalten und filtriert über Kieselgur. Es wird im Vakuum zur Trockene eingeengt. Der Rückstand wird in Dichlormethan gelöst und durch Chromatographie an Kieselgel (Petrolether/Essigsäureethylester 9:1, dann 8:2, dann 7:3) gereinigt. Die Produkt enthaltenden Fraktionen werden vereint, die Lösungsmittel werden entfernt und der Rückstand in Petrolether/Diethylether (10:1) verrührt. Es werden 83 g (75% d. Th.) des gewünschten Produktes erhalten.
¹H-NMR (400 MHz, DMSO-*d₆*, δ/*ppm*): δ = 8.17 (s, 2H), 7.78 (dd, 1H), 7.48 (dd, 1H), 6.75 (dd, 1H), 6.48 (dd, 1H), 6.14 (dd, 1H), 5.33 (dd, 1H), 2.10 (s, 6H).
HPLC (Methode 13): Rₜ = 1.70 min
MS (ESIpos): m/z = 271 (M+H)⁺

### Beispiel 61A

### 1-(2,6-Dimethyl-4-nitrophenyl)-3-(2-hydroxyethyl)pyridin-2(1H)-on

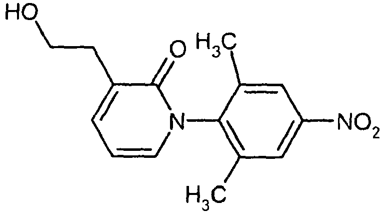

800 mg (2.96 mmol) der Verbindung aus Beispiel 60A werden unter Eiskühlung mit einer Lösung von 902 mg (7.40 mmol) 9-Borabicyclo[3.3.1]nonan in 14.8 ml Tetrahydrofuran versetzt. Es wird 3 h bei Raumtemperatur nachgerührt, dann auf 0°C gekühlt und mit einer wässrigen Lösung von 591 mg (14.8 mmol) Natriumhydroxid innerhalb von 15 min versetzt wird. Es werden 2.60 ml einer 30%-igen Wasserstoffperoxidlösung so zugegeben, dass die Temperatur 30°C nicht übersteigt. Nach beendeter Zugabe wird 30 min bei 0°C nachgerührt. Das Reaktionsgemisch wird unter Eiskühlung mit einer Lösung aus 8.73 g (32.6 mol) Natriumhydrogensulfit in 12 ml Wasser gegeben. Es wird mit 50 ml Essigsäureethylester verdünnt, die organische Phase abgetrennt und die wässrige Phase zweimal mit Essigsäureethylester extrahiert. Die vereinigten organischen Phasen werden mit gesättigter Natriumchloridlösung gewaschen, über Natriumsulfat getrocknet und im Vakuum zur Trockene eingedampft. Der Rückstand wird durch Chromatographie an Kieselgel (Cyclohexan/Essigsäureethylester 1:2) gereinigt. Es werden 765 mg (89% d. Th.) des gewünschten Produktes erhalten.
¹H-NMR (400 MHz, DMSO-d₆, δ/*ppm*): δ = 8.15 (s, 2H), 7.46 (dd, 1H), 7.35 (dd, 1H), 6.37 (dd, 1H), 4.62 (dd, 1H), 4.25 (d, 1H), 2.62 (dd, 2H), 2.08 (s, 6H).
HPLC (Methode 1): Rₜ = 3.59 min
MS (ESIpos): m/z = 289 (M+H)⁺

### Alternative Synthese:

70.7 g (262 mmol) der Verbindung aus Beispiel 60A (Alternativsynthese) werden unter Eiskühlung so mit 1.15 1 (575 mmol) einer 0.5 molaren Lösung von 9-Borabicyclo[3.3.1]nonan in Tetrahydrofuran versetzt, dass die Innentemperatur zwischen 10-15°C beträgt. Dann wird auf RT erwärmt und es wird 1.5 h bei Raumtemperatur nachgerührt. Anschließend wird gekühlt und bei 0°C-5°C mit 653 ml (1.31 mol) einer 2-molaren wässrigen Lösung von Natriumhydroxid versetzt. Es wird kurz nachgerührt und dann werden 296 g (2.51 mol) einer 30%-igen Wasserstoffperoxidlösung so zugegeben, dass die Temperatur 30°C nicht übersteigt und 25°C nicht unterschreitet. Nach beendeter Zugabe wird 30 min nachgerührt. Das Reaktionsgemisch wird mit Wasser und Essigsäureethylester versetzt. Nach Phasentrennung wird die wäßrige Phase erneut mit Essigsäureethylester extrahiert. Die vereinigten organischen Phasen werden mit einer wäßrigen Natriumhydrogensulfit-Lösung gewaschen. Die organische Phase wird abgetrennt und im Vakuum zur Trockene eingedampft. Der Rückstand wird in Dichlormethan gelöst und durch Chromatographie an Kieselgel (Cyclohexan/Essigsäureethylester 1:2, dann Essigsäureethylester) gereinigt. Es werden 66.5 g (88% d. Th.) des gewünschten Produktes erhalten.
¹H-NMR (400 MHz, CDCl₃, δ/*ppm*): δ = 8.07 (s, 2H), 7.43 (dd, 1H), 6.98 (ddd, 1H), 6.38 (dd, 1H), 3.88 (ddd, 2H), 3.55 (dd, 1H), 2.89 (dd, 2H), 2.19 (s, 6H).
HPLC (Methode 14): Rₜ = 0.83 min
MS (ESIpos): m/z = 289 (M+H)⁺

### Beispiel 62A

### 3-(2-{[tert-Butyl(diphenyl)silyl]oxy}ethyl)-1-(2,6-dimethyl-4-nitrophenyl)pyridin-2(1H)-on

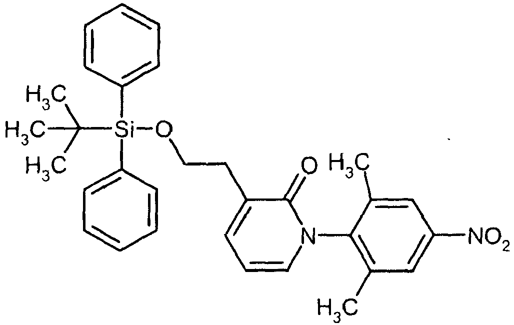

760 mg (2.64 mmol) der Verbindung aus Beispiel 61A und 0.55 ml (3.9 mmol) Triethylamin werden in 7 ml wasserfreiem N,N-Dimethylformamid gelöst. 16 mg (0.13 mmol) 4-Dimethylaminopyridin und 1.09 g (3.95 mmol) tert-Butyl(chlor)diphenylsilan werde hinzugefügt und es wird 2 h bei RT gerührt. Dann wird in Wasser gegeben und nach Phasentrennung wird dreimal mit Essigsäureethylester extrahiert. Die vereinigten organischen Phasen werden zweimal mit Wasser gewaschen, über Natriumsulfat getrocknet, filtriert und im Vakuum eingedampft. Der Rückstand wird durch Chromatographie an Kieselgel (Cyclohexan/Essigsäureethylester 5:1) gereinigt. Es werden 971 mg (58% d. Th.) des gewünschten Produktes erhalten.
HPLC (Methode 2): Rₜ = 5.97 min
MS (ESIpos): m/z = 527 (M+H)⁺

### Alternative Synthese:

100 g (346 mmol) der Verbindung aus Beispiel 61 A (Alternativsynthese) und 30.7 g (450 mmol) Imidazol werden in 11 wasserfreiem N,N-Dimethylformamid gelöst. Eine Lösung von 117 g (416 mmol) tert-Butyl(chlor)diphenylsilan in 150 ml N,N-Dimethylformamid wird hinzugetropft und es wird 3 h bei RT gerührt. Dann wird mit Wasser und Essigsäureethylester versetzt, die Phasen werden getrennt und die organische Phase wird mit Wasser gewaschen, über Natriumsulfat getrocknet, filtriert und im Vakuum eingedampft. Der Rückstand wird in Petrolether/Diethylether (10:1) verrührt. Nach Filtration wird mit Petrolether gewaschen und anschließend an der Luft getrocknet. Es werden 150 g (82% d. Th.) des gewünschten Produktes erhalten.
¹H-NMR (400 MHz, CDCl₃, δ/*ppm*): δ = 8.04 (s, 2H), 7.68-7.62 (m, 4H), 7.46-7.33 (m, 7H), 6.92 (dd, 1H), 6.30 (dd, 1H), 3.95 (dd, 2H), 2.86 (dd, 2H), 2.16 (s, 6H), 1.02 (s, 9H).
HPLC (Methode 13): Rₜ = 2.98 min
MS (ESIpos): m/z = 527 (M+H)⁺

### Beispiel 63A

### 1-(4-Amino-2,6-dimethylphenyl)-3-(2-{[tert-butyl(diphenyl)silyl]oxy}ethyl)pyridin-2(1H)-on

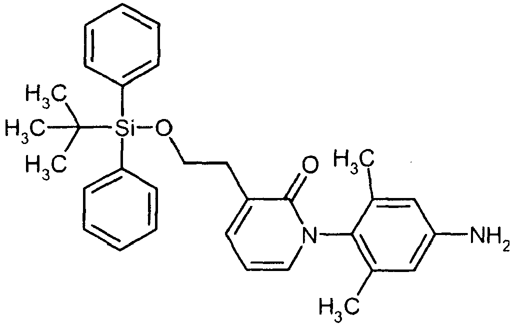

970 mg (1.84 mmol) der Verbindung aus Beispiel 62A werden in 20 ml THF gelöst. Dann werden 200 mg Palladium auf Kohle hinzugefügt und es wird bei RT in einer Wasserstoffatmosphäre unter normalem Druck hydriert. Anschließend wird über Kieselgur filtriert, dreimal mit THF nachgewaschen und das Filtrat vom Lösungsmittel befreit. Das Reaktionsprodukt (1.00 g) wird ohne weitere Aufreinigung weiter umgesetzt.
HPLC (Methode 2): Rₜ = 4.99 min
MS (ESIpos): m/z = 497 (M+H)⁺

### Alternative Synthese:

143 g (271 mmol) der Verbindung aus Beispiel 62A (Alternativsynthese) werden in 1.43 1 Tetrahydrofuran gelöst und mit Argon durchspült. Es werden 17 g Palladium auf Kohle (50%ig wasserfeucht) hinzugefügt und dann wird bei RT in einer Wasserstoffatmosphäre unter normalem Druck hydriert. Anschließend wird über Kieselgur filtriert, mit Tetrahydrofuran nachgewaschen und das Filtrat vom Lösungsmittel befreit. Das Reaktionsprodukt (134 g) wird ohne weitere Aufreinigung weiter umgesetzt.
HPLC (Methode 6): Rₜ = 3.11 min
MS (ESIpos): m/z = 497 (M+H)⁺

### Beispiel 64A

### N-[((5S)-3-{4-[3-(2-{[tert-Butyl(diphenyl)silyl]oxy}ethyl)-2-oxopyridin-1(2H)-yl]-3,5-dimethylphenyl}-2-oxo-1,3-oxazolidin-5-yl)methyl]-5-chlorthiophen-2-carboxamid

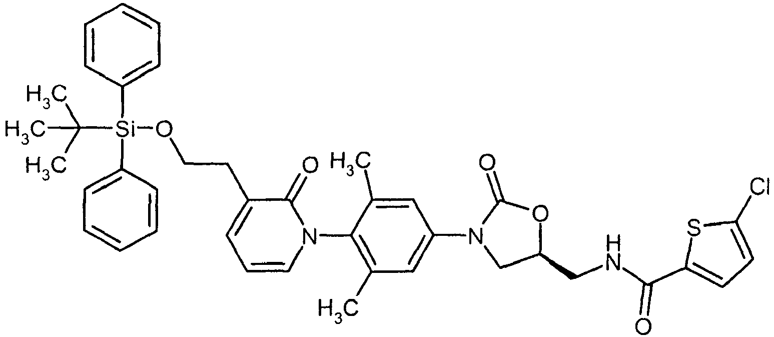

800 mg (1.61 mmol) der Verbindung aus Beispiel 63A werden in 15 ml wasserfreiem Acetonitril gelöst und mit 385 g (1.77 mmol) der Verbindung aus Beispiel 1A sowie 539 mg (2.41 mmol) Magnesiumperchlorat versetzt. Es wird 5.5 h bei Raumtemperatur gerührt. Dann wird mit 652 mg (4.03 mmol) 1,1-Carbonyldiimidazol und 19 mg (0.16 mmol) N,N-Dimethylaminopyridin versetzt und 18 h am Rückfluss erhitzt. Man lässt erkalten und gibt in 100 ml Wasser und 100 ml Essigsäureethylester. Nach Phasentrennung wird die wässrige Phase zweimal mit Essigsäureethylester extrahiert und die vereinigten organischen Phasen über Natriumsulfat getrocknet. Nach Filtration wird im Vakuum zur Trockene eingedampft. Der Rückstand wird durch Chromatographie an Kieselgel (Cyclohexan/Essigsäureethylester 1:2) gereinigt. Es werden 664 mg (55% d. Th.) des gewünschten Produktes erhalten.
¹H-NMR (400 MHz, DMSO-d₆, *δ*/*ppm*): δ = 8.99 (t, 1H), 7.70 (d, 1H), 7.63-7.45 (m, 4H), 7.48-7.31 (m, 9H), 7.28 (dd, 1H), 7.20 (d, 1H), 6.32 (t, 1H), 4.90-4.81 (m, 1H), 4.22 (dd, 1H), 3.89-3.82 (m, 3H), 3.62 (t, 2H), 2.76 (dd, 2H), 1.94 (s, 6H), 0.95 (s, 9H).
HPLC (Methode 2): Rₜ = 5.92 min
MS (ESIpos): m/z = 740 (M+H)⁺

### Alternative Synthese:

102 g (171 mmol) der Verbindung aus Beispiel 68A werden in 1.45 1 Dichlormethan gelöst und mit 44.7 ml (257 mmol) N,N-Diisopropylethylamin versetzt. Eine Lösung von 37.1 g (205 mmol) 5-Chlorthiophen-2-carbonylchlorid in wenig Dichlormethan wird langsam zugetropft und es wird eine Stunde bei Raumtemperatur gerührt. Dann wird mit Wasser versetzt, die Phasen werden getrennt und die organische Phase wird über Natriumsulfat getrocknet. Nach Filtration wird im Vakuum zur Trockene eingedampft. Der Rückstand wird ohne weitere Aufreinigung weiter umgesetzt.
HPLC (Methode 6): Rₜ = 3.33 min
MS (ESIpos): m/z = 740 (M+H)⁺

### Beispiel 65A

### 5-Chlor-N-[((5S)-3-{4-[3-(2-hydroxyethyl)-2-oxopyridin-1(2H)-yl]-3,5-dimethylphenyl}-2-oxo-1,3-oxazolidin-5-yl)methyl]thiophen-2-carboxamid

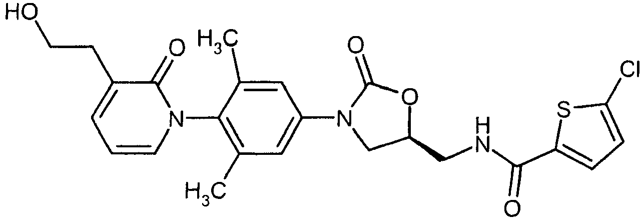

660 mg (0.891 mmol) der Verbindung aus Beispiel 64A werden in 20 ml THF gelöst und mit 512 mg (1.96 mmol) Tetrabutylammoniumfluorid versetzt. Nach 1 h wird im Vakuum zur Trockene eingeengt. Der Rückstand wird durch Chromatographie an Kieselgel (Dichlormethan/Methanol 10:1; 1% Triethylamin) gereinigt. Es werden 396 mg (85% d. Th.) des gewünschten Produktes erhalten.
¹H-NMR (400 MHz, DMSO-d₆, δ/*ppm*): δ = 8.98 (t, 1H), 7.69 (d, 1H), 7.42 (dd, 1H), 7.38 (d, 1H), 7.25 (dd, 1H), 7.19 (d, 1H), 6.29 (t, 1H), 4.90-4.81 (m, 1H), 4.60 (t, 1H), 4.20 (t, 1H), 3.86 (dd, 1H), 3.64-3.52 (m, 4H), 2.62 (t, 2H), 1.95 (s, 6H).
HPLC (Methode 1): Rₜ = 3.92 min
MS (ESIpos): m/z = 502 (M+H)⁺

### Alternative Synthese:

157 g (174 mmol) der Verbindung aus Beispiel 64A (Alternativsynthese) werden in 1.44 l Dichlormethan gelöst. Bei 15-20°C werden 453 ml (5.32 mol) konzentrierte Salzsäure zugetropft und es wird 1 h bei Raumtemperatur gerührt. Die Phasen werden getrennt, die organische Phase wird verworfen. Die wäßrige Phase wird zweimal mit Dichlormethan gewaschen. Anschließend wird die wäßrige Phase mit Dichlormethan versetzt und es wird mit 1:1 verdünnter Natronlauge unter Kühlung pH = 10 eingestellt. Die Phasen werden getrennt, die organische Phase wird zweimal mit Wasser gewaschen und dann über Natriumsulfat getrocknet. Nach Filtration wird das Filtrat im Vakuum zur Trockene eingeengt. Der Rückstand wird in Aceton verrührt, auf 10°C abgekühlt, bei dieser Temperatur abfiltriert und mit kaltem Aceton gewaschen. Nach Trocknen werden 75 g (85% d. Th.) des gewünschten Produktes erhalten.
¹H-NMR (400 MHz, DMSO-d₆, δ/*ppm*): δ = 8.98 (t, 1H), 7.69 (d, 1H), 7.42 (dd, 1H), 7.42-7.38 (m, 2H), 7.25 (dd, 1H), 7.19 (d, 1H), 6.29 (t, 1H), 4.90-4.81 (m, 1H), 4.60 (t, 1H), 4.20 (t, 1H), 3.86 (dd, 1H), 3.64-3.52 (m, 4H), 2.62 (t, 2H), 1.95 (s, 6H).
HPLC (Methode 6): Rₜ = 1.93 min
MS (ESIpos): m/z = 502 (M+H)⁺

### Beispiel 66A

### 2-[(2R)-3-({4-[3-(2-{[tert-Butyl(diphenyl)silyl)oxy}ethyl)-2-oxopyridin-1(2H)-yl]-3,5-dimethylphenyl}amino)-2-hydroxypropyl]-1H-isoindol-1,3(2H)-dion

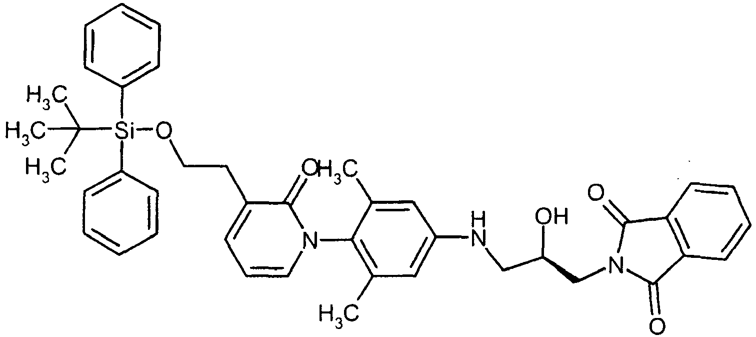

65 g (131 mmol) der Verbindung aus Beispiel 63A (Alternativsynthese) werden in 1.3 1 Acetonitril gelöst und mit 27.9 g (137 mmol) (S)-Epoxyphthalimid sowie 43.8 g (196 mmol) Magnesiumperchlorat versetzt. Es wird 15 h bei Raumtemperatur gerührt. Dann werden Wasser und Dichlormethan hinzugefügt, die Phasen werden getrennt und die wässrige Phase wird über Natriumsulfat getrocknet. Nach Filtration wird im Vakuum zur Trockene eingedampft. Der Rückstand wird ohne weitere Aufreinigung weiter umgesetzt.
HPLC (Methode 14): Rₜ = 1.73 min
MS (ESIpos): m/z = 701 (M+H)⁺

### Beispiel 67A

### 2-{[(5S)-3-{4-[3-(2-{[tert-Butyl(diphenyl)silyl]oxy}ethyl)-2-oxopyridin-1(2H)-yl]-3,5-dimethylphenyl}-2-oxo-1,3-oxazolidin-5-yl]methyl}-1H-isoindol-1,3(2H)-dion

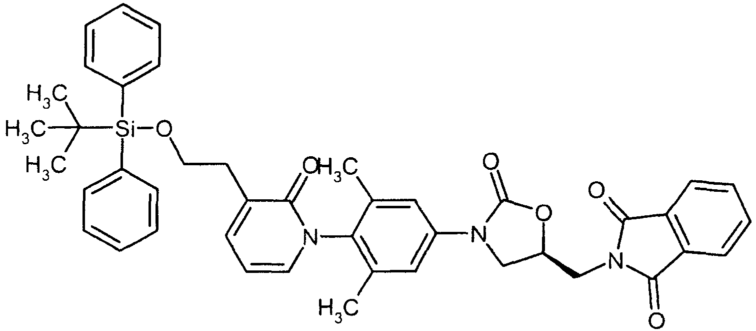

190 g (204 mmol) der Verbindung aus Beispiel 66A werden in 1.011 Toluol gelöst. Dann werden 66.0 g (407 mmol) 1,1'-Carbonyldiimidazol dazugegeben und es wird eine Stunde zum Rückfluß erhitzt. Dann wird auf Raumtemperatur abgekühlt und mit Wasser und Dichlormethan versetzt.

Die Phasen werden getrennt und die organische Phase wird über Natriumsulfat getrocknet. Nach Filtration wird im Vakuum zur Trockene eingedampft. Der Rückstand wird in Methanol verrührt, der verbleibende Feststoff abfiltriert und mit Methanol gewaschen. Man erhält 130 g (87% d. Th.) des gewünschten Produktes.
¹H-NMR (400 MHz, DMSO-d₆, *δ*/*ppm*): δ = 7.99-7.82 (m, 4H), 7.65-7.56 (m, 4H), 7.48-7.28 (m, 10H), 6.32 (t, 1H), 5.03-4.93 (m, 1H), 4.24 (dd, 1H), 4.00 (dd, 1H), 3.97-3.88 (m, 2H), 3.85 (t, 2H), 2.76 (t, 2H), 1.94 (s, 6H), 0.95 (s, 9H).
HPLC (Methode 14): Rₜ = 1.74 min
MS (ESIpos): m/z = 726 (M+H)⁺

### Beispiel 68A

### 1-{4-[(5S)-5-(Aminomethyl)-2-oxo-1,3-oxazolidin-3-yl]-2,6-dimethylphenyl}-3-(2-{[tert-butyl-(diphenyl)silyl]oxy}ethyl)pyhdin-2(1H)-on

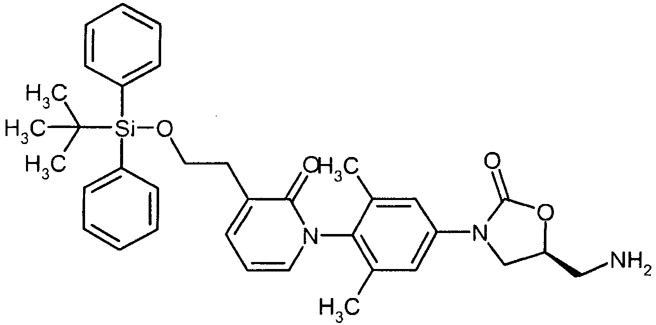

130 g (179 mmol) der Verbindung aus Beispiel 67A werden in 650 ml Ethanol gelöst, mit 193 ml (2.68 mol) 40%-iger wäßriger Methanamin-Lösung versetzt und es wird eine Stunde auf 50-55°C erhitzt. Nach Abkühlen auf Raumtemperatur wird mit Wasser und Dichlormethan versetzt, die Phasen werden getrennt und die organische Phase wird über Natriumsulfat getrocknet. Nach Filtration wird im Vakuum zur Trockene eingedampft. Der Rückstand (117 g, 89% d. Th.) wird ohne weitere Aufreinigung weiter umgesetzt.
HPLC (Methode 6): Rₜ = 2.05 min
MS (ESIpos): m/z = 597 (M+H)⁺

### Beispiel 69A

### 1-(2,6-Dimethyl-4-nitrophenyl)-3-methoxypyridin-2(1H)-on

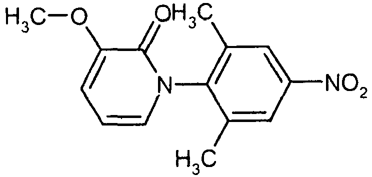

336 mg (2.69 mmol) 3-Methoxypyridinon in 10 ml DMF werden bei 0°C mit 452 mg (4.03 mmol) Kalium-tert.-butylat versetzt und es wird 30 min bei Raumtemperatur gerührt. 500 mg (2.96 mmol) 1-Fluor-2,5-dimethyl-4-nitrobenzol werden zugefügt und es wird bei 80°C gerührt. Nach 22 h wird auf 120°C erhitzt und weitere 20 h gerührt. Dann wird abgekühlt und in 100 ml Wasser und 15 ml gesättigte wässrige Natriumchlorid-Lösung gegeben. Es wird dreimal mit je 300 ml Essigsäureethylester extrahiert und die vereinigten organischen Phasen werden über Natriumsulfat getrocknet. Nach Filtration werden die Lösungsmittel im Vakuum entfernt und der Rückstand durch Chromatographie an Kieselgel (Cyclohexan/Essigsäureethylester 1:1) gereinigt. Man erhält 383 mg (52% d. Th.) der gewünschten Verbindung.
¹H-NMR (300 MHz, DMSO-*d₆*. *δ*/*ppm*): δ = 8.16 (s, 2H), 7.04 (dd, 1H), 6.97 (dd, 1H), 6.38 (dd, 1H), 3.77 (s, 3H), 2.08 (s, 6H).
HPLC (Methode 1): Rₜ = 2.84 min.
MS (DCI, m/z): 275 (M+H)⁺.

### Beispiel 70A

### 1-(4-Amino-2,6-dimethylphenyl)-3-methoxypyridin-2(1H)-on

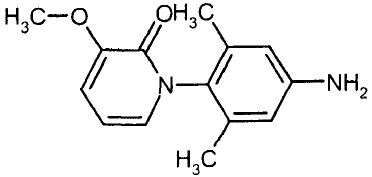

2.05 g (7.47 mmol) der Verbindung aus Beispiel 69A werden in 70 ml Essigsäureethylester und 70 ml Ethanol gelöst, mit 2.35 g (37.4 mmol) Ammoniumformiat und 0.39 g (0.37 mmol) Palladium/Kohle (10%ig) versetzt und 2 h bei 80°C gerührt. Zur Aufarbeitung wird die Reaktionslösung auf Raumtemperatur gebracht und über eine Kieselgelfritte gegeben. Es wird mit Ethanol nachgewaschen und das Filtrat wird im Vakuum eingeengt. Das Reaktionsprodukt (1.65 g) wird ohne weitere Aufreinigung umgesetzt.
¹H-NMR (300 MHz, DMSO-*d₆*. δ/*ppm*): δ = 6.93-6.83 (m, 2H), 6.33 (s, 2H), 6.19 (dd, 1H), 5.12 (br.s, 2H), 3.72 (s, 3H), 1.78 (s, 6H).
LC-MS (Methode 1): Rₜ = 2.90 min.
MS (DCI, *m*/*z*): 245 (M+H)⁺.

### Beispiel 71A

### 5-Chlor-N-({(5S)-3-[4-(3-methoxy-2-oxopyridin-1(2H)-yl)-3,5-dimethylphenyl]-2-oxo-1,3-oxazolidin-5-yl}methyl)thiophen-2-carboxamid

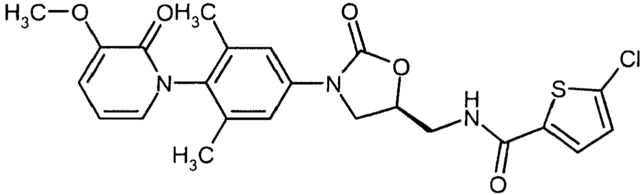

Eine Lösung von 555 mg (2.27 mmol) der Verbindung aus Beispiel 70A in 28 ml Acetonitril wird mit 544 mg (2.50 mmol) der Verbindung aus Beispiel 1A versetzt. Der Suspension werden 761 mg (3.41 mmol) Magnesiumperchlorat zugefügt, wonach man 5.5 h bei RT rühren lässt. Dann werden 736 mg (4.54 mmol) 1,1'-Carbonyldiimidazol und 28 mg (0.23 mmol) DMAP hinzugesetzt und es wird auf 60°C erhitzt. Nach 18 h wird erneut mit 28 mg (0.23 mmol) DMAP versetzt. Nach 2 h bei 70°C wird abgekühlt und mit 100 ml Wasser verdünnt. Die Wasserphase wird dreimal mit je 100 ml Essigsäureethylester extrahiert und die vereinigten organischen Phasen über Natriumsulfat getrocknet. Nach Filtration wird vom Lösungsmittel befreit und der Rückstand durch Chromatographie an Kieselgel (Dichlormethan/Ethanol 20:1) gereinigt. Nach Entfernen der Lösungsmittel erhält man 840 mg (69% d. Th.) des gewünschten Produktes.
¹H-NMR 300 MHz, DMSO-*d₆,* δ/*ppm*): δ = 8.98 (t, 1H), 7.69 (d, 1H), 7.42-7.32 (m, 2H), 7.20 (dd, 1H), 7.00-6.88 (m, 2H), 6.28 (dd, 1H), 4.91-4.80 (m, 1H), 4.20 (dd, 1H), 3.90-3.82 (m, 1H), 3.75 (s, 3H), 3.60 (dd, 2H), 1.95 (s, 6H).
HPLC (Methode 14): Rₜ = 1.02 min.
MS (ESIpos, *m*/*z*): 488 (M+H)⁺.

### Beispiel 72A

### 5-Chlor-N-({(5S)-3-[4-(3-hydroxy-2-oxopyridin-1(2H)-yl)-3,5-dimethylphenyl]-2-oxo-1,3-oxazolidin-5-yl}methyl)thiophen-2-carboxamid

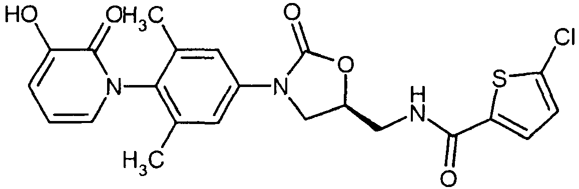

830 mg (1.70 mmol) der Verbindung aus Beispiel 71A werden in 50 ml wasserfreiem Dichlormethan gelöst und auf -78°C gekühlt. Bei dieser Temperatur werden 3.40 ml (3.40 mmol) einer 1 normalen Bortribromid-Lösung in Dichlormethan so zugetropft, dass die Temperatur -65°C nicht überschreitet. Es wird 2 h bei -78°C, dann 2.5 h bei RT gerührt und 15 h bei -20°C belassen. Die Lösung wird bei Raumtemperatur vorsichtig auf gesättigte wässrige Natriumhydrogencarbonat-Lösung gegeben. Nach Phasentrennung wird die Wasserphase dreimal mit Dichlormethan extrahiert, die vereinigten organischen Phasen mit gesättigter Natriumchloridlösung gewaschen, über Natriumsulfat getrocknet, filtriert und im Vakuum zur Trockene eingedampft. Man erhält 907 mg (69% d. Th., LC-MS Reinheit 61%) der gewünschten Verbindung.
LC-MS (Methode 6): Rₜ = 2.09 min.
MS (ESIpos, *m*/*z*) = 474 (M+H)⁺

### Beispiel 73A

### 5-Chlor-N-{[(5S)-3-{4-[3-(2-chlorethoxy)-2-oxopyridin-1(2H)-yl]-3,5-dimethylphenyl}-2-oxo-1,3-oxazolidin-5-yl]methyl} thiophen-2-carboxamid

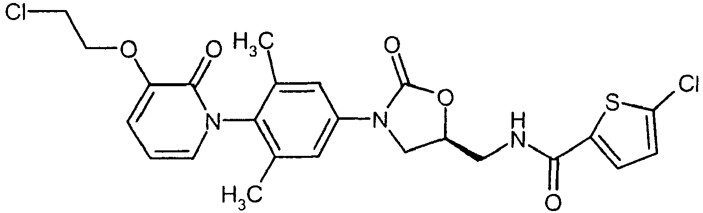

900 mg (1.89 mmol) der Verbindung aus Beispiel 72A und 1.36 g (9.49 mmol) 1-Brom-2-chlorethan werden in 30 ml 1-Methyl-2-pyrrolidon gelöst. Die Lösung wird mit 1.55 g (4.75 mmol) Cäsiumcarbonat versetzt und 15 Stunden bei 60°C gerührt. Dann wird mit Wasser versetzt und nach Phasentrennung wird die wässrige Phase dreimal mit tert-Butylmethylether und dreimal mit Dichlormethan extrahiert. Die vereinigten organischen Phasen werden nacheinander mit 1N Natronlauge und gesättigter Natriumchlorid-Lösung gewaschen, anschließend über Natriumsulfat getrocknet. Das Trockenmittel wird abfiltriert und das Lösungsmittel am Rotationsverdampfer vollständig entfernt. Der Rückstand wird durch Chromatographie an Kieselgel (Cylcohexan/Essigsäureethylester 1:5) gereinigt. Es werden 464 mg (45% d. Th., LC-MS Reinheit 74%) des gewünschten Produktes erhalten.
HPLC (Methode 6): Rₜ = 2.26 min
MS (ESIpos): m/z = 536 (M+H)⁺

### Ausführungsbeispiele

### Beispiel 1

### 5-Chlor-N-{[(5S)-3-{4-[3-{[(2-hydroxyethyl)amino]methyl}-2-oxopyridin-1(2H)-yl]-3-methylphenyl}-2-oxo-1,3-oxazolidin-5-yl]methyl} thiophen-2-carboxamid

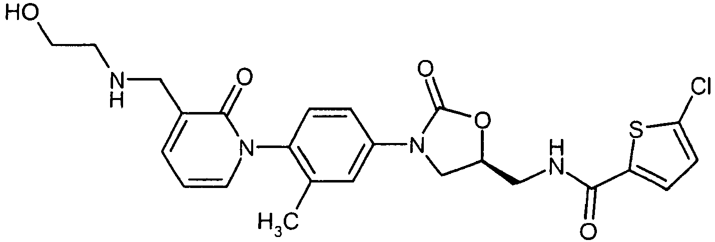

Zu einer Lösung von 56 mg (0.11 mmol) der Verbindung aus Beispiel 11A in 1 ml DMF werden bei -10°C 38 mg (0.63 mmol) Aminoethanol gegeben. Nach 30 min bei -10°C wird auf Wasser gegeben und anschließend dreimal mit Essigsäureethylester extrahiert. Die organischen Phasen werden vereinigt und anschließend im Vakuum vom Lösemittel befreit. Es wird mittels präparativer HPLC unter Verwendung eines Acetonitril/Wasser Gemisches gereinigt. Es werden 14.5 mg (26% d. Th.) des gewünschten Produktes erhalten.
¹H-NMR (400 MHz, DMSO-*d₆*, δ/*ppm*): 9.00 (t, 1H), 8.28-8.15 (m, 2H), 7.70 (d, 1H), 7.60-7.48 (m, 3H), 7.45 (d, 1H), 7.23 (d, 1H), 7.20 (d, 1H), 6.35 (dd, 1H), 4.90-4.82 (m, 1H), 4.22 (dd, 1H), 3.88 (dd, 1H), 3.71-3.45 (m, 6H), 2.68 (dd, 2H), 2.02 (s, 3H).
HPLC (Methode 2): Rₜ = 3.71 min.
MS (ESIpos, *m*/*z*): 517/519 (³⁵Cl/³⁷Cl) (M+H)⁺.

### Beispiel 2

### 5-Chlor-N-{[(5S)-3-{3-methyl-4-[3-(2-morpholin-4-ylethyl)-2-oxopyridin-1(2H)-yl]phenyl}-2-oxo-1,3-oxazolidin-5-yl]methyl}thiophen-2-carboxamid

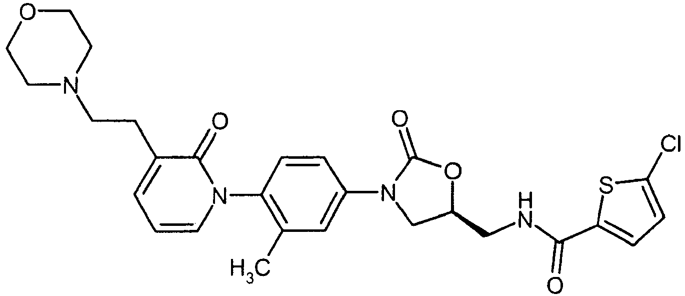

100 mg (0.21 mmol) Beispiel 18A werden in 5 ml wasserfreiem Dichlormethan gelöst und auf -78°C abgekühlt. 66 mg (0.62 mmol) 2,6-Dimethylpyridin und 69 mg (0.25 mmol) Trifluormethansulfonsäureanhydrid werden bei dieser Temperatur zugegeben und es wird weitere 10 min nachgerührt. Dann werden 179 mg (2.05 mmol) Morpholin zugegeben, nach 5 min die Kühlung entfernt und 16 h bei Raumtemperatur gerührt. Das Lösemittel wird im Vakuum entfernt, der Rückstand in wenig Methanol gelöst und mittels präparativer HPLC unter Verwendung eines Gradienten aus Acetonitril und Wasser gereinigt. Die produkthaltigen Fraktionen werden vereinigt und im Vakuum zur Trockene eingeengt. Man erhält 104 mg (91% d. Th.) der gewünschten Verbindung.
¹H-NMR (400 MHz, DMSO-*d₆,* δ/*ppm*): δ = 9.0 (t, 1H), 7.7 (d, 1H), 7.5 (m, 2H), 7.45 (dd, 1H), 7.35 (dd, 1H), 7.2 (m, 2H), 6.25 (t, 1H), 4.85 (m, 1H), 4.2 (t, 1H), 3.9 (m, 1H), 3.65 (t, 2H), 3.55 (m, 4H), 2.3-2.7 (m, 8H), 2.0 (s, 3H).
LC-MS (Methode 3): Rₜ = 1.30 min
MS (ESIpos): m/z = 556 (M+H⁺)⁺

Analog zu Beispiel 2 werden die Beispiele der folgenden Tabelle unter Verwendung des entsprechenden Amins hergestellt.

| **Beispiel** | **Struktur** | **Charakterisierung** |
|---|---|---|
| **3** | 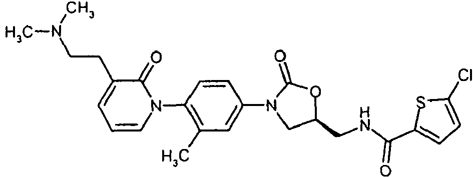 | LC-MS (Methode 6): Rₜ = 1.42 min MS (ESIpos): m/z = 515 (M+H⁺)^{+ 1}H-NMR (400 MHz, DMSO-d₆, δ/*ppm*): δ = 9.0 (t, 1H), 7.7 (d, 1H), 7.5 (m, 2H), 7.45 (dd, 1H), 7.35 (dd, 1H), 7.2 (m, 2H), 6.25 (t, 1H), 4.85 (m, 1H), 4.2 (t, 1H), 3.9 (m, 1H), 3.65 (t, 2H), 2.45 (m, 4H), 2.15 (s, 6H), 2.0 (s, 3H). |
| **4** | 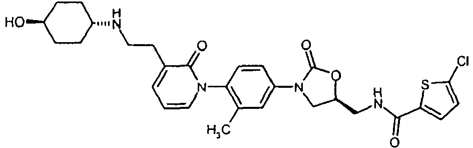 | LC-MS (Methode 6): Rₜ = 1.44 min MS (ESIpos): m/z = 585 (M+H⁺)^{+ 1}H-NMR (400 MHz, DMSO-d₆, δ/*ppm*): δ = 9.0 (t, 1H), 8.3 (s, 1H), 7.7 (d, 1H), 7.3-7.55 (m, 4H), 7.2 (m, 2H), 6.3 (t, 1H), 4.85 (m, 1H), 4.2 (t, 1H), 3.9 (m, 1H), 3.65 (t, 2H), 3.3 (m, 1H), 2.9 (t, 2H), 2.7 (m, 4H), 2.0 (s, 3H), 1.7-1.9 (m, 4H), 1.2 (m, 4H). |
| **5** | 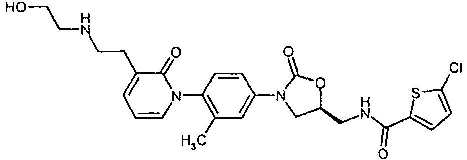 | LC-MS (Methode 11): Rₜ = 1.80 min MS (ESIpos): m/z = 531 (M+H⁺)^{+ 1}H-NMR (400 MHz, DMSO-*d₆*, δ/*ppm*): δ = 9.0 (t, 1H), 8.3 (s, 1H), 7.7 (d, 1H), 7.55 (m, 2H), 7.45 (dd, 1H), 7.4 (dd, 1H), 7.2 (m, 2H), 6.3 (t, 1H), 4.85 (m, 1H), 4.2 (t, 1H), 3.9 (m, 1H), 3.65 (t, 2H), 3.55 (t, 2H), 2.95 (m, 2H), 2.8 (t, 2H), 2.75 (m, 2H), 2.0 (s, 3H). |

### Beispiel 6

### 5-Chlor-N-{[(5S)-3-{4-[3-{2-[(trans-4-hydroxycyclohexyl)amino]ethyl}-2-oxopyridin-1(2H)-yl]-3-methoxyphenyl}-2-oxo-1,3-oxazolidin-5-yl]methyl}thiophen-2-carboxamid

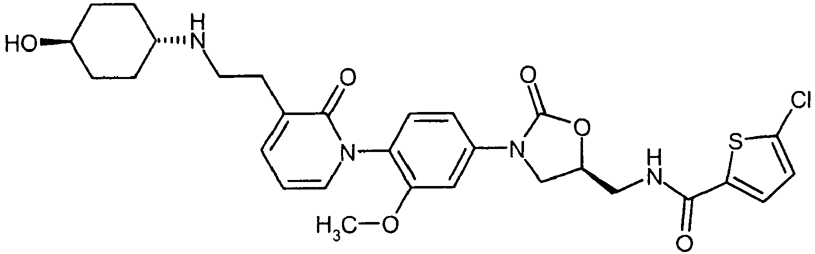

19 g (38 mmol) Beispiel 25A werden in 1000 ml wasserfreiem Dichlormethan gelöst und auf -78°C abgekühlt. Es werden 12.3 g (115 mmol) 2,6-Dimethylpyridin und 16 g (57 mmol) Trifluormethansulfonsäureanhydrid bei dieser Temperatur zugegeben. Es wird 1.5 h bei -78°C nachgerührt bevor 44 g (383 mmol) trans-4-Amino-cyclohexanol gelöst in 250 ml Dichlormethan und 50 ml N,N-Dimethylformamid zugegeben werden. Die Kühlung wird entfernt, es wird 16 h bei Raumtemperatur gerührt und das Lösemittel anschließend im Vakuum entfernt. Der Rückstand wird in wenig Methanol gelöst und mittels präparativer HPLC unter Verwendung eines Gradienten aus Acetonitril und Wasser gereinigt. Die produkthaltigen Fraktionen werden vereinigt und im Vakuum zur Trockene eingeengt. Man erhält 15.2 g (73% d. Th.) der gewünschten Verbindung.
¹H-NMR (400 MHz, CDCl₃, δ/*ppm*): δ = 7.72-7.52 (m, 1H), 7.50-7.37 (m, 2H), 7.32 (dd, 1H), 7.21 (d, 1H), 7.10(dd, 1H), 6.92-6.84 (m, 2H), 6.20 (t, 1H), 4.81-4.66 (m, 1H), 4.10-3.95 (m, 1H), 3.87-3.66 (m, 6H), 3.64-3.50 (m, 2H), 2.95-2.84 (m, 2H), 2.78-2.68 (m, 2H), 2.52-2.41 (m, 1H), 1.98-1.78 (m, 5H), 1.33-1.05 (m, 4H).
LC-MS (Methode 6): Rₜ = 1.40 min
MS (ESIpos): m/z = 602 (M+H⁺)⁺

Analog zu Beispiel 6 werden die Beispiele der folgenden Tabelle unter Verwendung des entsprechenden Amins hergestellt.

| **Beispiel** | **Struktur** | **Charakterisierung** |
|---|---|---|
| **7** | 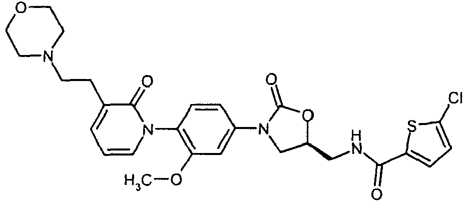 | LC-MS (Methode 6): Rₜ = 1.41 min MS (ESIpos): m/z = 573 (M+H)^{+ 1}H-NMR (400 MHz, DMSO-d₆, δ/*ppm*): δ = 9.0 (t, 1H), 7.7 (d, 1H), 7.45 (d, 1H), 7.35 (dd, 1H), 7.3 (dd, 1H), 7.25 (d, 1H), 7.2 (d, 1H), 7.1 (d, 1H), 6.2 (t, 1H), 4.85 (m, 1H), 4.25 (t, 1H), 3.9 (m, 1H), 3.75 (s, 3H), 3.65 (t, 2H), 3.5 (m, 4H), 2.4-2.6 (m, 6H), 2.4 (m, 2H). |
| **8** | 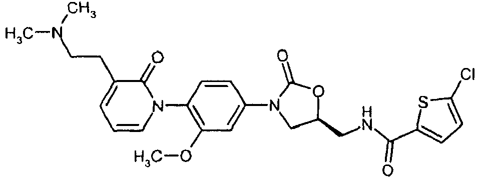 | LC-MS (Methode 6): Rₜ = 1.40 min MS (ESIpos): m/z = 531 (M+H⁺)^{+ 1}H-NMR (400 MHz, DMSO-*d₆,* δ/*ppm*): δ = 9.0 (t, 1H), 7.7 (d, 1H), 7.45 (d, 1H), 7.35 (dd, 1H), 7.3 (dd, 1H), 7.25(d, 1H), 7.2 (d, 1H), 7.1 (d, 1H), 6.2 (t, 1H), 4.85 (m, 1H), 4.25 (t, 1H), 3.9 (m, 1H), 3.75 (s, 3H), 3.65 (t, 2H), 2.4-2.6 (m, 4H), 2.1 (s, 6H). |

### Beispiel 9

### 5-Chlor-N-{[(5S)-3-{4-[3-{2-[(2-hydroxyethyl)amino]ethyl}-2-oxopyridin-l(2H)-yl]-3-(methoxymethyl)phenyl}-2-oxo-1,3-oxazolidin-5-yl]methyl}thiophen-2-carboxamid

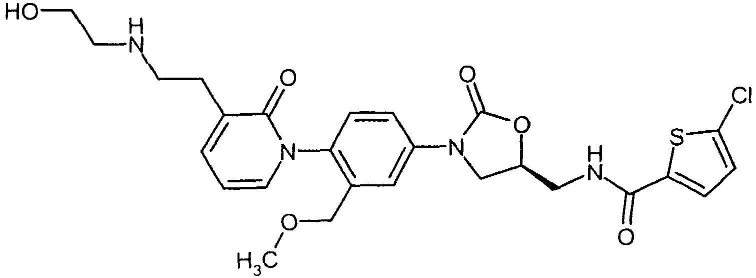

14 g (27 mmol) Beispiel 34A werden in 660 ml wasserfreiem Dichlormethan gelöst und auf - 78°C abgekühlt. Es werden 8.7 g (81 mmol) 2,6-Dimethylpyridin und 13 g (46 mmol) Trifluormethansulfonsäureanhydrid bei dieser Temperatur zugegeben. Nach 10 min bei -78°C wird mit 8.2 g (135 mmol) 2-Aminoethanol versetzt, nach weiteren 5 min die Kühlung entfernt und dann 16 h bei Raumtemperatur gerührt. Das Lösemittel wird im Vakuum entfernt, der Rückstand in wenig Methanol gelöst und mittels präparativer HPLC unter Verwendung eines Gradienten aus Acetonitril und Wasser gereinigt. Die produkthaltigen Fraktionen werden vereinigt und im Vakuum zur Trockene eingeengt. Man erhält 11.1 g (73% d. Th.) der gewünschten Verbindung.
¹H-NMR (400 MHz, DMSO-*d₆,* δ/*ppm*): δ = 8.98 (t, 1H), 7.71-7.65 (m, 2H), 7.62-7.55 (m, 1H), 7.40 (dd, 1H), 7.35 (dd, 1H), 7.28 (d, 1H), 7.20 (d, 1H), 6.25 (t, 1H), 4.90-4.82 (m, 1H), 4.42 (t, 1H), 4.27-4.08 (m, 3H), 3.94-3.88 (m, 1H), 3.63 (t, 2H), 3.45 (dt, 2H), 3.18 (s, 3H), 2.75-2.68 (m, 2H), 2.61-2.43 (m, 4H).
LC-MS (Methode 6): Rₜ = 1.34 min
MS (ESIpos): m/z = 561 (M+H⁺)⁺

### Beispiel 10

### 5-Chlor-N-{[(5S)-3-{4-[3-{2-[(trans-4-hydroxycyclohexyl)amino]ethyl}-2-oxopyridin-1(2H)-yl]-3-(methoxymethyl)phenyl}-2-oxo-1,3-oxazolidin-5-yl]methyl}thiophen-2-carboxamid

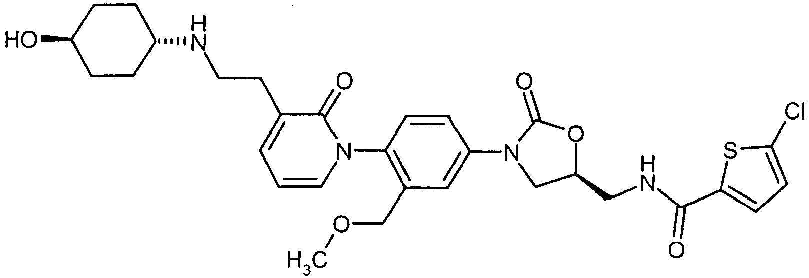

900 mg (1.7 mmol) Beispiel 34A werden in 42 ml wasserfreiem Dichlormethan gelöst und auf -78°C abgekühlt. 560 mg (5.2 mmol) 2,6-Dimethylpyridin und 833 mg (2.9 mmol) Trifluormethansulfonsäureanhydrid werden zugegeben und es wird 1.5 h bei -78°C gerührt. 1 g (8.7 mmol) trans-4-Aminocyclohexanol werden zugefügt, nach 5 min die Kühlung entfernt und dann 18 h bei Raumtemperatur gerührt. Man verdünnt mit Wasser und Dichlormethan, trennt die organische Phase ab, trocknet über Natriumsulfat, filtriert und dampft im Vakuum ein. Der Rückstand wird in wenig Methanol aufgenommen und mittels präparativer HPLC unter Verwendung eines Gradienten aus Acetonitril und Wasser gereinigt. Die produkthaltigen Fraktionen werden vereinigt und im Vakuum zur Trockene eingeengt. Man erhält 655 mg (61% d. Th.) der gewünschten Verbindung.
¹H-NMR (400 MHz, DMSO-*d₆*, δ/*ppm*): δ = 8.98 (t, 1H), 7.71-7.65 (m, 2H), 7.62-7.55 (m, 1H), 7.40 (dd, 1H), 7.35 (dd, 1H), 7.27 (d, 1H), 7.20 (d, 1H), 6.25 (t, 1H), 4.90-4.82 (m, 1H), 4.44 (d, 1H), 4.27-4.07 (m, 3H), 3.93-3.83 (m, 1H), 3.62 (t, 2H), 3.18 (s, 3H), 2.76-2.65 (m, 2H), 2.57-2.48 (m, 2H), 2.36-2.27 (m, 2H), 1.84-1.72 (m, 4H), 1.19-1.06 (m, 2H), 1.03-0.91 (m, 2H).
LC-MS (Methode 6): Rₜ = 1.68 min
MS (ESIpos): m/z = 615 (M+H⁺)⁺

Analog zu Beispiel 10 werden die Beispiele der folgenden Tabelle unter Verwendung des entsprechenden Amins hergestellt.

| **Beispiel** | **Struktur** | **Charakterisierung** |
|---|---|---|
| **11** | 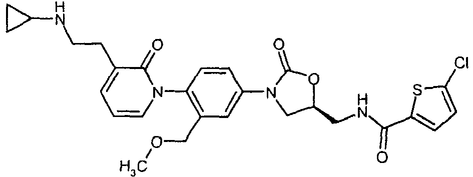 | LC-MS (Methode 11): Rₜ = 1.94 min MS (ESIpos): m/z = 557 (M+H⁺)^{+ 1}H-NMR (400 MHz, DMSO-*d₆*, δ/*ppm*): δ = 9.0 (t, 1H), 7.7 (m, 2H), 7.6 (m, 1H), 7.4 (dd, 1H), 7.35 (dd, 1H), 7.25 (d, 1H), 7.2 (d, 1H), 6.25 (t, 1H), 4.85 (m, 1H), 4.2 (m, 2H), 4.1 (d, 1H), 3.9 (m, 1H), 3.6 (t, 2H), 3.3 (m, 2H), 3.2 (s, 3H), 2.8 (m, 2H), 2.1 (m, 2H), 0.45 (m, 2H), 0.2 (m, 2H). |
| **12** | 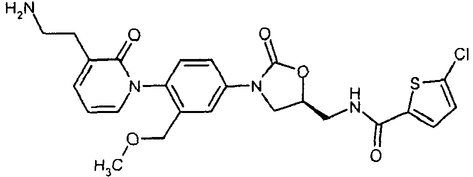 | LC-MS (Methode 11): Rₜ = 1.86 min MS (ESIpos): m/z = 517 (M+H⁺)^{+ 1}H-NMR (400 MHz, DMSO-*d₆*. δ/*ppm*): δ = 9.0 (t, 1H), 7.7 (m, 2H), 7.6 (m, 1H), 7.4 (m, 2H), 7.25 (d, 1H), 7.2 (d, 1H), 6.25 (t, 1H), 4.85 (m, 1H), 4.2 (m, 2H), 4.1 (d, 1H), 3.9 (m, 1H), 3.6 (t, 2H), 3.2 (s, 3H), 2.8 (m, 2H), 2.55 (m, 2H). |
| **13** | 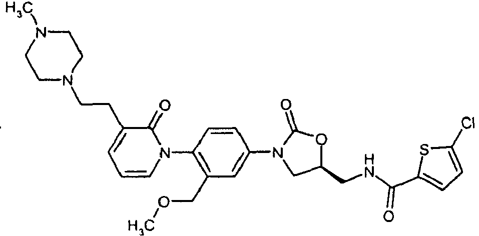 | LC-MS (Methode 11): Rₜ = 1.69 min MS (ESIpos): m/z = 600 (M+H⁺)^{+ 1}H-NMR (400 MHz, DMSO-*d₆*, δ/*ppm*): δ = 9.0 (t, 1H), 7.7 (m, 2H), 7.6 (m, 1H), 7.4 (dd, 1H), 7.35 (dd, 1H), 7.25 (d, 1H), 7.2 (d, 1H), 6.25 (t, 1H), 4.85 (m, 1H), 4.2 (m, 2H), 4.1 (d, 1H), 3.9(m, 1H), 3.6 (t, 2H), 3.2 (s, 3H), 2.2-2.8 (m, 12H), 2.15 (s, 3H). |
| **14** | 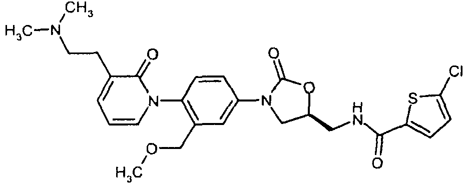 | LC-MS (Methode 11): Rₜ = 1.91 min MS (ESIpos): m/z = 545 (M+H⁺)^{+ 1}H-NMR (400 MHz, DMSO-*d₆*, δ/*ppm*): δ = 9.0 (t, 1H), 7.7 (m, 2H), 7.6 (m, 1H), 7.4 (m, 2H), 7.25 (d, 1H), 7.2 (d, 1H), 6.25 (t, 1H), 4.85 (m, 1H), 4.2 (m, 2H), 4.1 (d, 1H), 3.9 (m, 1H), 3.6 (t, 2H), 3.2 (s, 3H), 2.3-2.7 (m, 4H), 2.15 (s, 6H). |
| **15** | 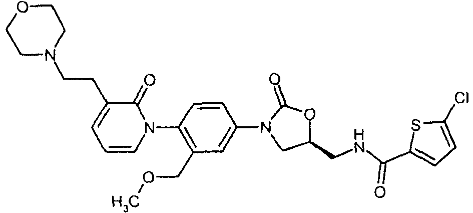 | LC-MS (Methode 11): Rₜ = 1.87 min MS (ESIpos): m/z = 587 (M+H⁺)^{+ 1}H-NMR (400 MHz, DMSO-*d₆*, δ/*ppm*): δ = 9.0 (t, 1H), 7.7 (m, 2H), 7.6 (m, 1H), 7.4 (dd, 1H), 7.35 (dd, 1H), 7.25 (d, 1H), 7.2 (d, 1H), 6.25 (t, 1H), 4.85 (m, 1H), 4.2 (m, 2H), 4.1 (d, 1H), 3.9 (m, 1H), 3.6 (t, 2H), 3.55 (m, 4H), 3.2 (s, 3H), 2.3-2.8 (m, 8H). |
| **16** | 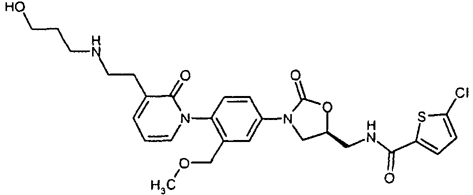 | LC-MS (Methode 11): Rₜ = 1.89 min MS (ESIpos): m/z = 575 (M+H⁺)^{+ 1}H-NMR (400 MHz, DMSO-*d₆*, δ/*ppm*): δ = 9.0 (t, 1H), 7.7 (m, 2H), 7.6 (m, 1H), 7.4 (dd, 1H), 7.35 (dd, 1H), 7.25 (d, 1H), 7.2 (d, 1H), 6.25 (t, 1H), 4.85 (m, 1H), 4.2 (m, 2H), 4.1 (d, 1H), 3.9 (m, 1H), 3.6 (t, 2H), 3.45 (t, 2H), 3.2 (s, 3H), 2.7 (t, 2H), 2.4-2.6 (m, 6H), 1.5 (m, 2H). |
| **17** | 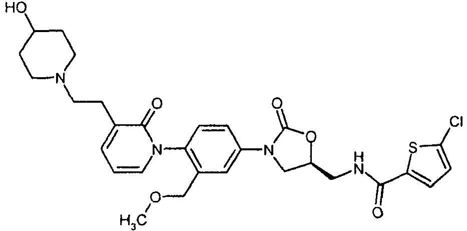 | LC-MS (Methode 11): Rₜ = 1.89 min MS (ESIpos): m/z = 601 (M+H⁺)^{+ 1}H-NMR (400 MHz, DMSO-*d₆*, δ/*ppm*): δ = 9.0 (t, 1H), 7.7 (m, 2H), 7.6 (m, 1H), 7.4 (dd, 1H), 7.35 (dd, 1H), 7.25 (d, 1H), 7.2 (d, 1H), 6.25 (t, 1H), 4.85 (m, 1H), 4.2 (m, 2H), 4.1 (d, 1H), 3.9 (m, 1H), 3.6 (t, 2H), 3.45 (m, 1H), 3.2 (s, 3H), 2.7 (m, 2H), 2.3-2.6 (m, 4H), 2.0 (t, 2H), 1.7 (m, 2H), 1.3 (m, 2H). |

### Beispiel 18

### 5-Chlor-N-{[(5S)-3-{4-[3-{3-[(trans-4-hydroxycyclohexyl)amino]propyl}-2-oxopyridin-1(2H)-yl]-3-methylphenyl}-2-oxo-1,3-oxazolidin-5-yl]methyl}thiophen-2-carboxamid

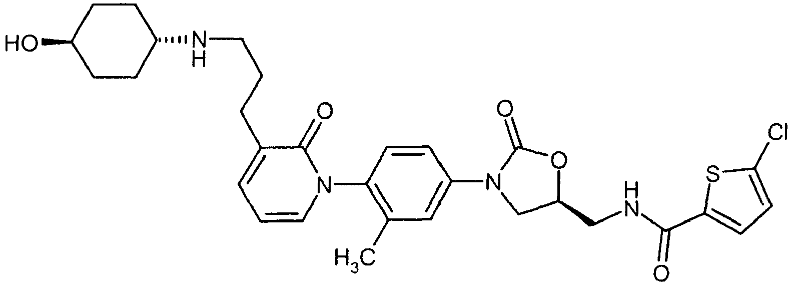

92.4 mg (0.18 mmol) Beispiel 38A werden in 4.5 ml wasserfreiem Dichlormethan gelöst und auf -78°C abgekühlt und mit 67 mg (0.55 mmol) 2,6-Dimethylpyridin und 78 mg (0.28 mmol) Trifluormethansulfonsäureanhydrid veresetzt. Nach 1 h bei -78°C wird mit 106 mg (0.920 mmol) trans-4-Aminocyclohexanol versetzt, die Kühlung entfernt und 72 h bei Raumtemperatur gerührt. Nach Zugabe von 2.5 ml Methanol wird 5 min gerührt und dann das Lösemittel im Vakuum entfernt. Der Rückstand wird mittels präparativer HPLC unter Verwendung eines Gradienten aus Acetonitril und 0.2%-iger Trifluormethansulfonsäure in Wasser gereinigt. Man erhält 7.5 mg (7% d. Th.) der gewünschten Verbindung.
¹H-NMR (400 MHz, DMSO-*d₆*, δ/*ppm*): δ = 8.99 (t, 1H), 8.37-8.21 (m, 2H), 7.70 (d, 1H), 7.58-7.47 (m, 2H), 7.45-7.36 (m, 2H), 7.22 (d, 1H), 7.20 (d, 1H), 6.32 (dd, 1H), 4.92-4.78 (m, 1H), 4.21 (dd, 1H), 4.00-3.48 (m, 4H), 3.41-3.30 (m, 1H), 2.98-2.85 (m, 2H), 2.02 (s, 3H), 2.00-1.91 (m, 2H), 1.88-1.80 (m, 4H), 1.37-1.15 (m, 6H).
LC-MS (Methode 6): Rₜ = 1.45 min
MS (ESIpos): m/z = 601 (M+H)⁺

### Beispiel 19

### 5-Chlor-N-{[(5S)-3-{4-[3-{3-[(2-hydroxyethyl)amino]propyl}-2-oxopyridin-1(2H)-yl]-3-methylphenyl}-2-oxo-1,3-oxazolidin-5-yl]methyl}thiophen-2-carboxamid

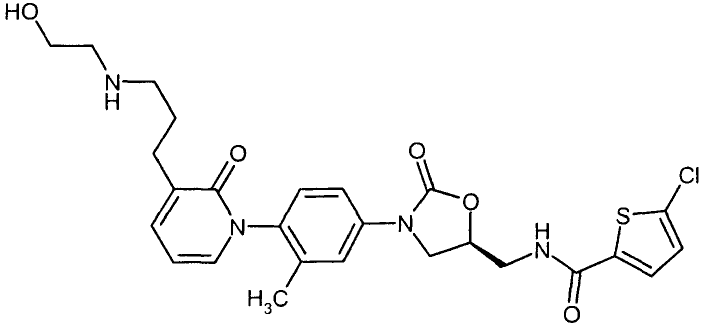

92.4 mg (0.18 mmol) Beispiel 38A werden in 4.5 ml wasserfreiem Dichlormethan gelöst und auf -78°C abgekühlt und mit 67 mg (0.55 mmol) 2,6-Dimethylpyridin und 78 mg (0.28 mmol) Trifluormethansulfonsäureanhydrid versetzt. Nach 1 h bei -78°C wird mit 112 mg (1.84 mmol) Aminoethanol versetzt, die Kühlung entfernt und 72 h bei Raumtemperatur gerührt. Nach Zugabe von 2.5 ml Methanol wird 5 min gerührt und dann das Lösemittel im Vakuum entfernt. Der Rückstand wird mittels präparativer HPLC unter Verwendung eines Gradienten aus Acetonitril und 0.2-%-iger Trifluormethansulfonsäure in Wasser gereinigt. Man erhält 14 mg (14% d. Th.) der gewünschten Verbindung.
¹H-NMR (400 MHz, DMSO-*d₆*, δ/*ppm*): δ = 8.99 (t, 1H), 8.51-8.37 (m, 2H), 7.70 (d, 1H), 7.58-7.47 (m, 2H), 7.45-7.38 (m, 2H), 7.22 (d, 1H), 7.20 (d, 1H), 6.32 (dd, 1H), 4.91-4.82 (m, 1H), 4.22 (dd, 1H), 3.95-3.50 (m, 7H), 3.05-2.89 (m, 4H), 2.02 (s, 3H), 1.93-1.82 (m, 2H).
LC-MS (Methode 6): Rₜ = 1.45 min
MS (ESIpos): m/z = 546 (M+H)⁺

### Beispiel 20

### 5-Chlor-N-{[(5S)-3-{3-methyl-4-[2-oxo-3-{2-[(tetrahydrofuran-2-ylmethyl)amino]ethoxy}pyridin-1(2H)-yl]phenyl}-2-oxo-1,3-oxazolidin-5-yl]methyl}thiophen-2-carboxamid

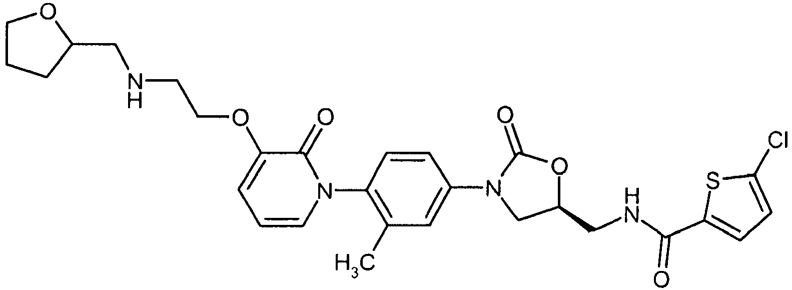

80.0 mg (153 µmol) Beispiel 46A, 23 mg (153 µmol) Natriumiodid und 105 µl (77.4 mg, 766 µmol) 1-(Tetrahydrofuran-2-yl)methanamin werden in 2 ml absolutem 1,2-Dimethoxyethan 8 Stunden bei 90°C gerührt. Zur Aufarbeitung wird die eingeengte Reaktionslösung über die präparative RP-HPLC mit einem Acetonitril/Wasser-Gemisch feingereinigt. Es werden 60 mg (67% d. Th.) Produkt erhalten.
¹H-NMR (400 MHz, DMSO-*d₆,* δ/*ppm*): 8.97 (t, 1H), 7.69 (d, 1H), 7.55-7.46 (m, 2H), 7.25-7.18 (m, 2H), 7.04 (d, 1H), 6.93 (d, 1H), 6.21 (t, 1H), 4.87 (m, 1H), 4.22 (dd, 1H), 3.97 (m, 2H), 3.90-3.81 (m, 2H), 3.72 (m, 2H), 3.66-3.55 (m, 3H), 2.90 (m, 2H), 2.62 (d, 2H), 2.01 (s, 3H), 1.95-1.80 (m, 3H), 1.57-1.96 (m, 1H).
LC-MS (Methode 3): Rₜ = 1.45 min
MS (ESIpos): m/z = 587 (M+H)⁺

### Beispiel 21

### 5-Chlor-N-{[(5S)-3-{4-[3-{2-[(trans-4-hydroxycyclohexyl)amino]ethoxy}-2-oxopyridin-1(2H)-yl]-3-methylphenyl}-2-oxo-1,3-oxazolidin-5-yl]methyl}-thiophen-2-carboxamid

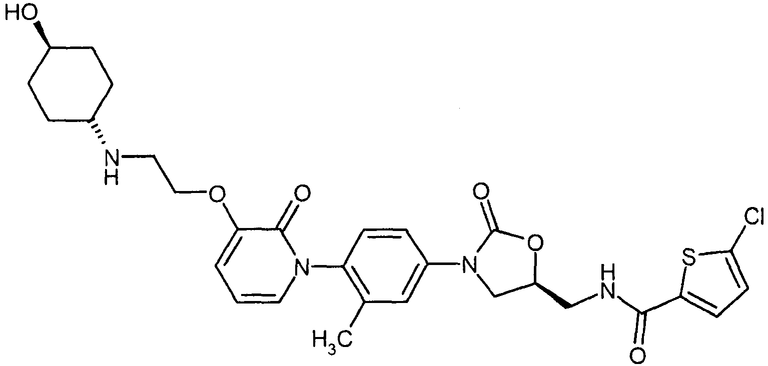

70.0 mg (134 µmol) Beispiel 46A, 20.0 mg (134 µmol) Natriumiodid und 77.2 mg (670 µmol) trans-4-Aminocyclohexanol werden in 1.75 ml absolutem 1,2-Dimethoxyethan 8 Stunden bei 90°C gerührt. Zur Aufarbeitung wird die eingeengte Reaktionslösung über die präparative RP-HPLC mit einem Acetonitril/Wasser-Gemisch feingereinigt. Es werden 57 mg (69% d. Th.) Produkt erhalten.
¹H-NMR (400 MHz, DMSO-*d₆*, δ/*ppm*): 8.97 (t, 1H), 7.69 (d, 1H), 7.53-7.50 (m, 2H), 7.23-7.18 (m, 2H), 7.04 (d, 1H), 6.92 (d, 1H), 6.22 (t, 1H), 4.89-4.81 (m, 1H), 4.44 (d, 1H), 4.22 (t, 1H), 3.99-3.82 (m, 3H), 3.62 (t, 3H), 2.87 (t, 2H), 2.41-2.31 (m, 1H), 2.01 (s, 3H), 1.80 (t, 4H), 1.53 (bs, 1H), 1.19-0.93 (m, 4H).
LC-MS (Methode 3): Rₜ = 1.41 min
MS (ESIpos): m/z = 601 (M+H)⁺.

Analog zu Beispiel 21 werden die Beispiele der folgenden Tabelle unter Verwendung des entsprechenden Amins hergestellt.

| **Beispiel** | **Struktur** | **Charakterisierung** |
|---|---|---|
| **22** | 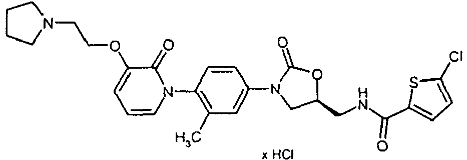 | LC-MS (Methode 3): Rₜ = 1.56 min, MS (ESIpos): m/z = 557 (M+H)^{+ 1}H-NMR (400 MHz, DMSO-*d₆*, *δppm*): 10.67 (breit s, 1H), 9.06 (t, 1H), 7.73 (d, 1H), 7.58-7.48 (m, 2H), 7.28-7.13 (m, 3H), 7.10 (d, 1H), 6.28 (t, 1H), 4.86 (m, 1H), 4.32 (m, 2H), 4.22 (t, 1H), 3.90 (m, 1H), 3.68-3.53 (m, 5H), 3.10 (m, 2H), 2.08-1.93 (m, 5H, darin 2.01 (s, 3H)), 1.92-1.80 (m, 2H). |
| **23** | 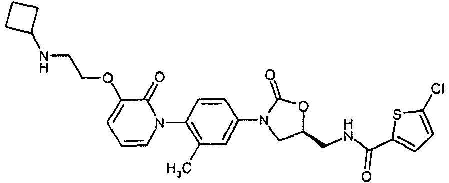 | LC-MS (Methode 11): Rₜ = 2.13 min, MS (ESIpos): m/z = 557 (M+H)^{+ 1}H-NMR (400 MHz, DMSO-*d₆*, δ/*ppm*): 8.97 (t, 1H), 7.69 (d, 1H), 7.55-7.46 (m, 2H), 7.25-7.18 (m, 2H), 7.04 (d, 1H), 6.92 (d, 1H), 6.21 (t, 1H), 4.87 (m, 1H), 4.22 (dd, 1H), 3.97-3.83 (m, 3H), 3.60 (t, 2H), 3.20 (m, 1H), 2.80 (t, 2H), 2.16-2.05 (m, 2H), 2.01 (s, 3H), 1.72-1.48 (m, 4H). |
| **24** | 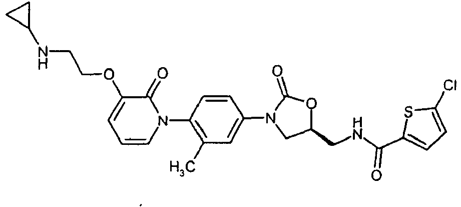 | LC-MS (Methode 11): Rₜ = 2.03 min, MS (ESIpos): m/z = 543 (M+H)^{+ 1}H-NMR (400 MHz, DMSO-*d₆*, δ/*ppm*): 8.97 (t, 1H), 7.69 (d, 1H), 7.55-7.46 (m, 2H), 7.25-7.18 (m, 2H), 7.04 (d, 1H), 6.21 (d, 1H), 6.72 (t, 1H), 4.87 (m, 1H), 4.22 (dd, 1H), 3.98 (m, 2H), 3.89 (m, 1H), 3.60 (t, 2H), 2.94 (t, 2H), 2.14 (m, 1H), 2.01 (s, 3H), 0.38 (m, 2H), 0.22 (m, 2H). |
| **25** | 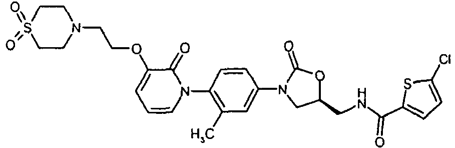 | LC-MS (Methode 11): Rₜ = 2.03 min, MS (ESIpos): m/z = 621 (M+H)^{+ 1}H-NMR (400 MHz, DMSO-*d₆*, δ/*ppm*): 8.97 (breit s, 1H), 7.70 (d, 1H), 7.55-7.46 (m, 2H), 7.25-7.18 (m, 2H), 7.06 (d, 1H), 6.95 (d, 1H), 6.21 (t, 1H), 4.86 (m, 1H), 4.22 (dd, 1H), 4.03 (m, 2H), 3.78 (m, 1H), 3.61 (t, 2H), 3.12-2.98 (m, 8H), 2.93 (t, 2H), 2.01 (s, 3H). |
| **26** | 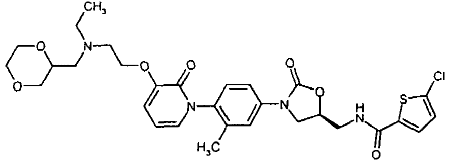 | LC-MS (Methode 11): Rₜ = 2.03 min, MS (ESIpos): m/z = 621 (M+H)^{+ 1}H-NMR (400 MHz, DMSO-*d₆*, δ/*ppm*): 8.96 (t, 1H), 7.70 (d, 1H), 7.55-7.46 (m, 2H), 7.25-7.18 (m, 2H), 7.06 (d, 1H), 6.92 (d, 1H), 6.70 (t, 1H), 4.86 (m, 1 H), 4.22 (dd, 1H), 3.95 (m, 2H), 3.88 (m, 1H), 3.78-3.50 (m, 7H), 3.41 (td, 1H), 3.18 (t, 1H), 2.82 (m, 2H), 2.65-2.45 (m, 4H), 2.02 (s, 3H), 0.95 (t, 3H). |
| **27** | 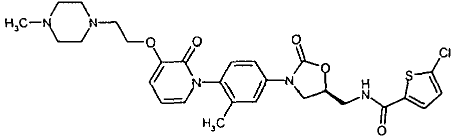 | LC-MS (Methode 6): Rₜ = 1.41 min, MS (ESIpos): m/z = 586 (M+H)^{+ 1}H-NMR (400 MHz, DMSO-*d₆*, δ/*ppm*): 8.97 (t, 1H), 7.70 (d, 1H), 7.55-7.46 (m, 2H), 7.25-7.18 (m, 2H), 7.06 (d, 1H), 6.95 (d, 1H), 6.22 (t, 1H), 4.86 (m, 1H), 4.22 (dd, 1H), 4.03 (m, 2H), 3.88 (m, 1H), 3.61 (t, 2H), 2.68 (t, 2H), 2.48 (m, 4H), 2.29 (m, 4H), 2.15 (s, 3H), 2.01 (s, 3H). |
| **28** | 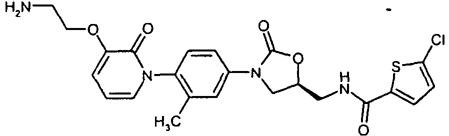 | LC-MS (Methode 6): Rₜ = 1.43 min, MS (ESIpos): m/z = 503 (M+H)^{+ 1}H-NMR (400 MHz, *DMSO-d₆,* δ/*ppm*): 8.97 (t, 1H), 7.70 (d, 1H), 7.55-7.46 (m, 2H), 7.25-7.18 (m, 2H), 7.06 (d, 1H), 6.92 (d, 1H), 6.21 (t, 1H), 4.86 (m, 1H), 4.22 (dd, 1H), 3.93-3.80 (m, 3H), 3.61 (t, 2H), 2.88 (t, 2H), 2.02 (s, 3H). |
| **29** | 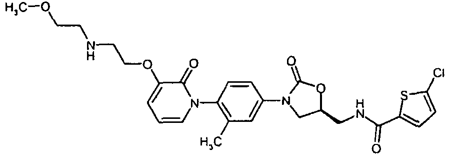 | LC-MS (Methode 6): Rₜ = 1.44 min, MS (ESIpos): m/z = 561 (M+H)^{+ 1}H-NMR (400 MHz, DMSO-*d₆*, δ/*ppm*): 8.96 (t, 1H), 7.69 (d, 1H), 7.56-7.44 (m, 2H), 7.26-7.15 (m, 2H), 7.05 (d, 1H), 6.92 (d, 1H), 6.22 (t, 1H), 4.86 (m, 1H), 4.21 (dd, 1H), 3.97 (m, 2H), 3.88 (m, 1H), 3.61 (t, 2H), 3.38 (t, 2H), 3.22 (s, 3H), 2.88 (t, 2H), 2.72 (t, 2H), 2.02 (s, 3H). |
| **30** | 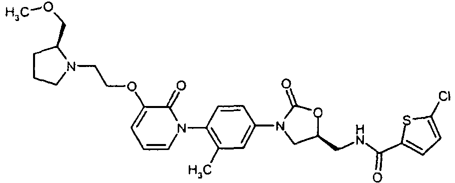 | LC-MS (Methode 11): Rₜ = 2.14 min, MS (ESIpos): m/z = 601 (M+H)^{+ 1}H-NMR (400 MHz, DMSO-*d₆*, δ/*ppm*): 8.96 (t, 1H), 7.69 (d, 1H), 7.56-7.47 (m, 2H), 7.26-7.15 (m, 2H), 7.04 (d, 1H), 6.92 (d, 1H), 6.21 (t, 1H), 4.86 (m, 1H), 4.21 (dd, 1H), 4.07-3.83 (m, 3H), 3.61 (t, 2H), 3.38-3.13 (m, 5H: darin 3.26 (s, 3H)), 3.09 (m, 1H), 2.65 (m, 2H), 2.28 (m, 1H), 2.02 (s, 3H), 1.38-1.75 (m, 1H), 1.72-1.57 (m, 2H), 1.49-1.39 (m, 1H). |
| **31** | 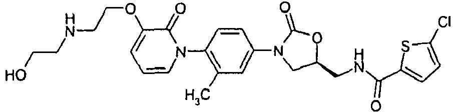 | LC-MS (Methode 3): Rₜ = 1.40 min, MS (ESIpos): m/z = 547 (M+H)^{+ 1}H-NMR (400 MHz, DMSO-*d₆*, δ/*ppm*): 8.98 (t, 1H), 7.70 (d, 1H), 7.55-7.46 (m, 2H), 7.25-7.17 (m, 2H), 7.05 (d, 1H), 6.97 (d, 1H), 6.24 (t, 1H), 4.86 (m, 1H), 4.61 (m, 1H), 4.22 (dd, 1H), 4.02 (m, 2H), 3.88 (m, 1H), 3.61 (t, 2H), 3.48 (m, 2H), 2.98 (m, 2H), 2.72 (t, 2H), 2.02 (s, 3H). |
| **32** | 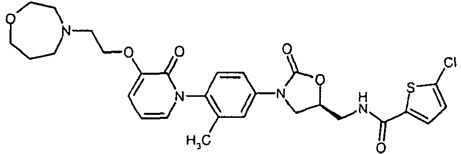 | LC-MS (Methode 3): R,= 1.33 min, MS (ESIpos): m/z = 587 (M+H)^{+ 1}H-NMR (400 MHz, DMSO-*d₆*, δ/*ppm*): 8.97 (t, 1H), 7.70 (d, 1H), 7.54-7.47 (m, 2H), 7.25-7.17 (m, 2H), 7.05 (d, 1H), 6.93 (d, 1H), 6.21 (t, 1H), 4.86 (m, 1H), 4.22 (dd, 1H), 4.02 (m, 2H), 3.70-3.57 (m, 6H), 2.89 (t, 2H), 2.75 (m, 4H), 2.02 (s, 3H), 1.78 (m, 2H). |
| **33** | 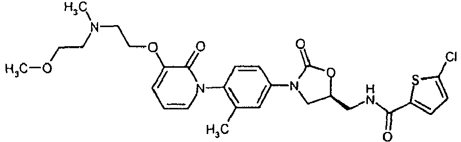 | LC-MS (Methode 6): Rₜ = 1.46 min, MS (ESIpos): m/z = 575 (M+H)^{+ 1}H-NMR (400 MHz, DMSO-*d₆*, δ/*ppm*): 8.96 (t, 1H), 7.69 (d, 1H), 7.55-7.46 (m, 2H), 7.25-7.17 (m, 2H), 7.05 (d, 1H), 6.93 (d, 1H), 6.21 (t, 1H), 4.86 (m, 1H), 4.22 (dd, 1H), 4.02 (m, 2H), 3.88 (m, 1H), 3.62 (t, 3H), 3.41 (t, 2H), 3.25 (s, 3H), 2.75 (m, 2H), 2.58 (t, 2H), 2.28 (s, 3H), 2.02 (s, 3H). |

### Beispiel 34

### 5-Chlor-N-{[(5S)-3-(4-{3-[2-(4-hydroxypiperidin-1-yl)ethoxy]-2-oxopyridin-1(2H)-yl}-3-methylphenyl)-2-oxo-1,3-oxazolidin-5-yl]methyl} thiophen-2-carboxamid

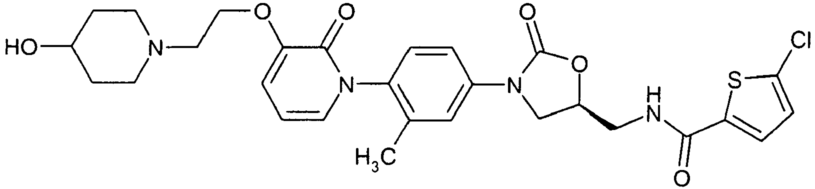

88.0 mg (175 µmol) Beispiel 45A werden in 5 ml wasserfreiem Dichlormethan gelöst, auf -78°C gekühlt und mit 42 µl (69.7 mg, 244 µmol) Trifluormethansulfonsäureanhydrid und 56 mg (524 µmol) 2,6-Dimethylpyridin versetzt. Nach einer Stunde Rühren bei -78°C werden nochmals 15 µl (24.7 mg, 88 µmol) Trifluormethansulfonsäureanhydrid zugetropft und eine weitere halbe Stunde bei dieser Temperatur nachgerührt. Die Reaktion wird abschließend mit 180 mg (1.74 mmol) 4-Hydroxypiperidin bei -78°C versetzt, 5 Minuten nachgerührt, auf Raumtemperatur erwärmt und über Nacht bei dieser Temperatur gerührt. Zur Aufarbeitung wird der Ansatz am Rotationsverdampfer eingeengt und der Rückstand zweimal über präparative RP-HPLC mit einem Acetonitril/Wasser-Gemisch gereinigt. Es werden 30.0 mg (29% d. Th.) des gewünschten Produktes erhalten.
¹H-NMR (400 MHz, DMSO-*d₆*, *δ*/*ppm*): 8.96 (t, 1H), 8.15 (s, 1H), 7.69 (d, 1H), 7.55-7.46 (m, 2H), 7.25-7.15 (m, 2H), 7.04 (d, 1H), 6.93 (d, 1H), 6.21 (t, 1H), 4.86 (m, 1H), 4.22 (dd, 1H), 4.01 (m, 2H), 3.92-3.84 (m, 1H), 3.62 (t, 2H), 3.45 (m, 1H), 2.80 (m, 2H), 2.70 (t, 2H), 2.17 (m, 2H), 2.01 (s, 3H), 1.75-1.64 (m, 2H), 1.45-1.32 (m, 2H).
LC-MS (Methode 3): Rₜ = 1.38 min
MS (ESIpos): m/z = 587 (M+H)⁺

### Beispiel 35

### 5-Chlor-N-{[(5S)-3-{4-[3-{2-[(trans-4-hydroxycyclohexyl)amino]ethoxy}-2-oxopyridin-1 (2H)-yl]-3-propylphenyl}-2-oxo-1,3-oxazolidin-5-yl]methyl}thiophen-2-carboxamid

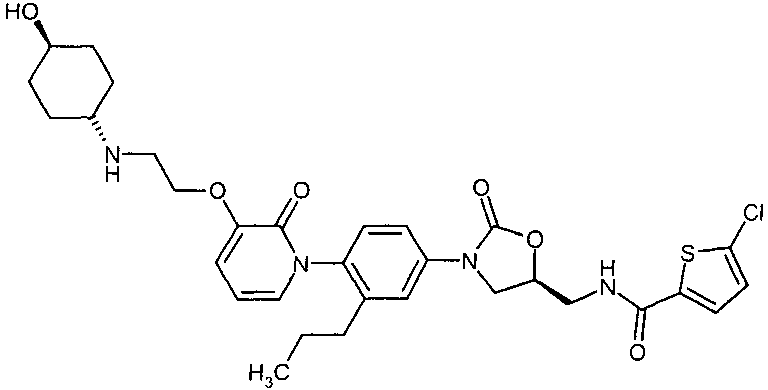

30 mg (54 µmol) Beispiel 58A, 8.2 mg (54 µmol) Natriumiodid und 31.4 mg (272 µmol) trans-4-Aminohexanol werden in 750 µl 1,2-Dimethoxyethan vorgelegt. Die Reaktion wird 8 Stunden bei 90°C gerührt. Zur Aufarbeitung wird die Reaktionslösung am Rotationsverdampfer vollständig eingeengt und über die präparative RP-HPLC mit einem Acetonitril/Wasser-Gemisch feingereinigt. Das Produkt wird mit 15.0 mg (41% d. Th.) erhalten.
¹H-NMR (400 MHz, Methanol-*d₄*, *δlppm*): 7.61 (dd, 1H), 7.52 (m, 2H), 7.20 (d, 1H), 7.07 (d, 2H), 7.01 (d, 1H), 6.92 (t, 1H), 6.42 (t, 1H), 4.27 (t, 1H), 4.08 (m, 2H), 3.98 (m, 1H), 3.73 (m, 2H), 3.58-3.45 (m, 1H), 3.02 (m, 2H), 2.50 (m, 1H), 2.35 (m, 2H), 1.97 (m, 4H), 1.50 (m, 2H), 1.38-1.11 (m, 5H), 0.83 (t, 3H).
LC-MS (Methode 8): Rₜ = 2.06 min
MS (ESIpos): m/z = 629 (M+H)⁺

### Beispiel 36

### 5-Chlor-N-{[(SS)-3-{4-[3-{2-[(2-hydroxyethyl)amino]ethoxy}-2-oxopyridin-1(2H)-yl]-3-propylphenyl}-2-oxo-1,3-oxazolidin-5-yl]methyl}thiophen-2-carboxamid

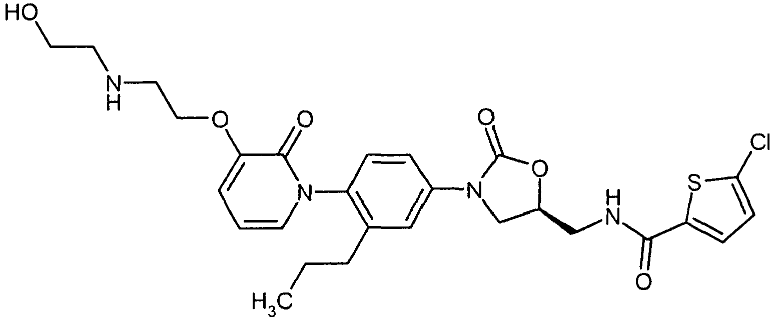

500 mg (0.908 mmol) Beispiel 58A, 142 mg (0.954 mmol) Natriumiodid und 277 mg (4.54 mmol) 2-Aminoethanol werden in 8 ml 1,2-Dimethoxyethan gelöst und 4 Stunden bei 90°C gerührt. Nach Einengen der Reaktionslösung wird in 100 ml Dichlormethan und Wasser aufgenommen und 1 h bei RT gerührt. Die Phasen werden getrennt und die wässrige Phase mit Dichlormethan extrahiert. Die vereinigten organischen Phasen werden mit gesättigter Natriumchlorid-Lösung gewaschen und anschließend über Natriumsulfat getrocknet. Das Trockenmittel wird abfiltriert und das Lösungsmittel am Rotationsverdampfer vollständig entfernt. Der Rückstand wird durch Chromatographie an Kieselgel (Dichlormethan/Ethanol 5:1; Zusatz von 0.1 Vol% Ethyldimethylamin) gereinigt. Es werden 241 mg (46% d. Th.) des gewünschten Produktes erhalten.
¹H-NMR (400 MHz, DMSO-*d₆*, *δlppm*): δ = 9.01 (t, 1H), 7.70 (d, 1H), 7.58-7.45 (m, 2H), 7.23-7.17 (m, 2H), 7.06 (dd, 1H), 6.95 (dd, 1H), 6.22 (dd, 1H), 4.91-4.82 (m, 1H), 4.65-4.55 (m, 1H), 4.24 (dd, 1H), 4.10-3.85 (m, 3H), 3.62 (dd, 2H), 3.52-3.43 (m, 2H), 2.93 (t, 2H), 2.68 (t, 2H), 2.35-2.20 (m, 2H), 1.50-1.35 (m, 2H), 0.78 (t, 3H).
HPLC (Methode 2): Rₜ = 4.00 min
MS (ESIpos): m/z = 575 (M+H)⁺

### Beispiel 37

### 5-Chlor-N-{[(5S)-3-{4-[3-{2-[(2-hydroxyethyl)amino]ethyl}-2-oxopyridin-1 (2H)-yl]-3,5-dimethylphenyl}-2-oxo-1,3-oxazolidin-5-yl]methyl} thiophen-2-carboxamid

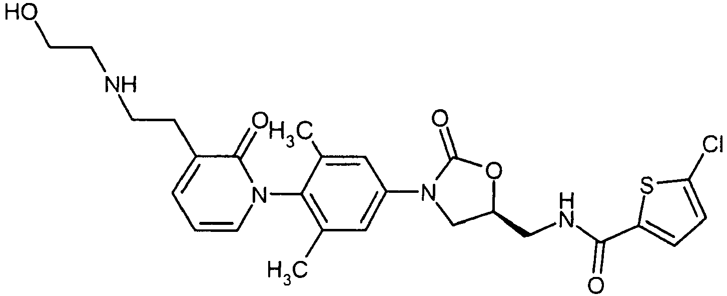

100 mg (0.20 mmol) Beispiel 65A werden in 3 ml wasserfreiem Dichlormethan gelöst, auf -78°C abgekühlt und mit 64 mg (0.59 mmol) 2,6-Dimethylpyridin und 84 mg (0.29 mmol) Trifluormethansulfonsäureanhydrid versetzt. Nach 1h bei -78°C werden 121 mg (1.99 mmol) 2-Aminoethanol zugefügt, die Kühlung nach 5 min entfernt und 18 h bei Raumtemperatur gerührt. Dann wird das Lösemittel im Vakuum entfernt und der Rückstand zweimal mittels präparativer HPLC unter Verwendung eines Gradienten aus Acetonitril und 0.3%-iger Ameisensäure in Wasser gereinigt. Man erhält 27 mg (25% d. Th.) der gewünschten Verbindung.
¹H-NMR (400 MHz, *DMSO-d₆, δ*/*ppm*): δ = 9.01 (t, 1H), 8.30 (s, 1H), 7.70 (d, 1H), 7.46 (d, 1H), 7.41-7.34 (m, 2H), 7.31 (d, 1H), 7.19 (d, 1H), 6.33 (dd, 1H), 4.91-4.82 (m, 1H), 4.20 (dd, 1H), 3.86 (dd, 1H), 3.61 (t, 2H), 3.54 (t, 2H), 2.94 (t, 2H), 2.81 (t, 2H), 2.72 (t, 2H), 1.96 (s, 6H).
LC-MS (Methode 12): Rₜ = 1.22 min
MS (ESIpos): m/z = 545 (M+H)⁺

### Beispiel 38

### 5-Chlor-N-{[(5S)-3-{4-[3-{2-[(trans-4-hydroxycyclohexyl)amino]ethyl}-2-oxopyridin-1(2H)-yl]-3,5-dimethylphenyl}-2-oxo-1,3-oxazolidin-5-yl]methyl} thiophen-2-carboxamid

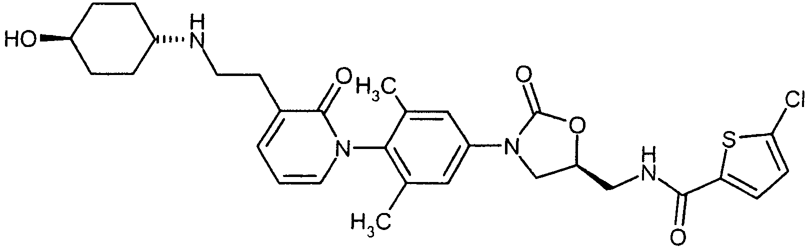

100 mg (0.20 mmol) Beispiel 65A werden in 3 ml wasserfreiem Dichlormethan gelöst, auf -78°C abgekühlt und mit 64 mg (0.59 mmol) 2,6-Dimethylpyridin und 84 mg (0.29 mmol) Trifluormethansulfonsäureanhydrid versetzt. Nach 1 h bei -78°C werden 114 mg (0.97 mmol) trans-4-Aminohexanol zugefügt, die Kühlung nach 5 min entfernt und 18 h bei Raumtemperatur gerührt. Dann wird das Lösemittel im Vakuum entfernt und der Rückstand zweimal mittels präparativer HPLC unter Verwendung eines Gradienten aus Acetonitril und 0.3%-iger Ameisensäure in Wasser gereinigt. Man erhält 15 mg (13% d. Th.) der gewünschten Verbindung.
¹H-NMR (400 MHz, DMSO-*d₆*, δ/*ppm*): δ = 9.00 (t, 1H), 8.28 (s, 1H), 7.70 (d, 1H), 7.51-7.42 (m, 1H), 7.41-7.34 (m, 2H), 7.31 (d, 1H), 7.19 (d, 1H), 6.33 (dd, 1H), 4.91-4.82 (m, 1H), 4.20 (t, 1H), 3.86 (dd, 1H), 3.64-3.55 (m, 2H), 3.52 (dd, 1H), 3.38-3.29 (m, 2H), 2.95-2.86 (m, 2H), 2.78 (dd, 1H), 2.74-2.60 (m, 3H), 1.96 (s, 6H), 1.94-1.75 (m, 2H), 1.20-1.10 (m, 3H). Das Spektrum enthält Signale von Ameisensäure und/oder dem Formiat der Titelverbindung.
LC-MS (Methode 12): Rₜ = 1.24 min
MS (ESIpos): m/z = 599 (M+H)⁺

### Alternative Synthese 1:

600 mg (1.19 mmol) Beispiel 65A werden in 18 ml wasserfreiem Dichlormethan gelöst, auf -78°C abgekühlt und mit 384 mg (3.58 mmol) 2,6-Dimethylpyridin und 573 mg (2.03 mmol) Trifluormethansulfonsäureanhydrid versetzt. Nach 1 h bei -78°C werden 688 mg (5.97 mmol) trans-4-Aminohexanol zugefügt, die Kühlung nach 5 min entfernt und 18 h bei Raumtemperatur gerührt. Dann wird das Lösemittel im Vakuum entfernt und der Rückstand mittels präparativer HPLC unter Verwendung eines 1:1 1 Gemisches von Acetonitril / 0.1 %-igem Diisopropylethlyamin in Wasser gereinigt. Man erhält 263 mg (36% d. Th.) der gewünschten Verbindung.
¹H-NMR (400 MHz, DMSO*-d₆*, δ/*ppm*): δ = 8.98 (t, 1H), 7.70 (d, 1H), 7.48-7.33 (m, 3H), 7.25 (d, 1H), 7.19 (d, 1H), 6.28 (dd, 1H), 4.91-4.82 (m, 1H), 4.44 (d, 1H), 4.20 (t, 1H), 3.86 (dd, 1H), 3.64-3.55 (m, 2H), 2.72 (dd, 2H), 2.64-2.50 (m, 3H), 2.35-2.23 (m, 1H), 1.96 (s, 6H), 1.82-1.71 (m, 4H), 1.20-0.89 (m, 4H).
LC-MS (Methode 14): R, = 0.83 min
MS (ESIpos): m/z = 599 (M+H)⁺

### Alternative Synthese 2:

39 g (77 mmol) Beispiel 65A (Alternativsynthese) werden in 1.17 1 wasserfreiem Dichlormethan gelöst und auf unter -60°C abgekühlt. Es wird erst mit 24.9 g (233 mmol) 2,6-Dimethylpyridin versetzt und anschließend werden 22.3 ml (132 mmol) Trifluormethansulfonsäureanhydrid zugetropft. Nach 15 min bei -70°C werden eine Lösung von 46.1 g (388 mmol) trans-4-Aminohexanol in 360 ml Dichlormethan und 360 ml Isopropanol unter -50°C schnell hinzugefügt. Es wird für 15 min im Trockeneisbad nachgerührt und dann das Kühlbad entfernt. Nach 3 h wird das Lösemittel im Vakuum entfernt und der Rückstand durch Chromatographie an Kieselgel (Dichlormethan/Methanol/25%ige Ammoniklösung 9:1:0.2) gereinigt. Die Produkt enthaltenden Fraktionen werden vereint, vom Lösungsmittel befreit und nochmals durch Chromatographie an Kieselgel (Dichlormethan/Methanol/25%ige Ammoniklösung 9:1:0.2) nachgereinigt. Die Produkt enthaltenden Fraktionen werden vereint und im Vakuum vom Lösungsmittel befreit. Um von noch enthaltenem Methanol zu befreien, wird zwcimal mit je 130 ml Wasser versetzt, dann bei 50°C im Vakuum vom Lösungsmittel befreit und am Hochvakuum getrocknet. Es werden 34.1 g (73% d. Th.) des gewünschten Produktes erhalten.
¹H-NMR (400 MHz, DMSO-*d₆*, δ/*ppm*): δ = 8.98 (t, 1H), 7.70 (d, 1H), 7.48-7.33 (m, 3H), 7.25 (d, 1H), 7.19 (d, 1H), 6.28 (dd, 1H), 4.91-4.82 (m, 1H), 4.44 (d, 1H), 4.20 (t, 1H), 3.86 (dd, 1H), 3.64-3.55 (m, 2H), 2.72 (dd, 2H), 2.64-2.50 (m, 3H), 2.35-2.23 (m, 1H), 1.96 (s, 6H), 1.82-1.71 (m, 4H), 1.20-0.89 (m, 4H).

### Beispiel 39

### 5-Chlor-N-{[(5S)-3- {4-[3-{2-[(trans-4-hydroxycyclohexyl)amino]ethoxy} -2-oxopyridin-1 (2H)-yl]-3,5-dimethylphenyl}-2-oxo-1,3-oxazolidin-5-yl]methyl}thiophen-2-carboxamid

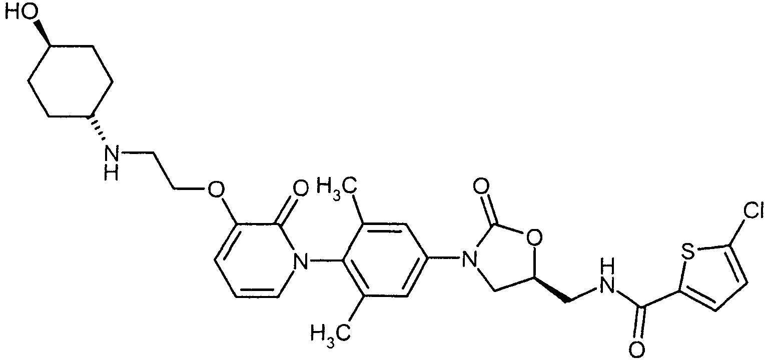

211 mg (0.393 mmol) Beispiel 73A, 62 mg (0.41 mmol) Natriumiodid und 226 mg (1.97 mmol) trans-4-Aminohexanol werden in 3.5 ml 1,2-Dimethoxyethan vorgelegt. Die Reaktion wird 15 Stunden bei 90°C gerührt. Zur Aufarbeitung wird die Reaktionslösung am Rotationsverdampfer vollständig eingeengt, dann in einem Dichlormethan/Wasser-Gemisch aufgenommen und 10 min bei RT gerührt. Nach Phasentrennung wird die wässrige Phase mit Dichlormethan extrahiert. Die vereinigten organischen Phasen werden mit einer gesättigten wässrigen Natriumchlorid-Lösung gewaschen. Nach Trocknen über Natriumsulfat wird das Trockenmittel abfiltriert und das Lösungsmittel am Rotationsverdampfer vollständig entfernt. Der Rückstand wird durch Chromatographie an Kieselgel (Dichlormethan/Ethanol 10:1, Zusatz von 1% Ethyldimethylamin) vorgereinigt. Anschließende Aufreinigung mittels präparativer HPLC mit einem Acetonitril/Wasser-Gemisch liefert 104 mg (43% d. Th.) des gewünschten Produktes.
¹H-NMR (400 MHz, DMSO-*d₆*, δ/*ppm*): δ = 8.99 (t, 1H), 7.61 (dd, 1H), 7.43-7.36 (m, 2H), 7.20 (d, 1H), 7.15-7.07 (m, 2H), 6.34 (t, 1H), 4.88-4.80 (m, 1H), 4.23-4.15 (m, 3H), 3.92-3.65 (m, 1H), 3.61 (dd, 4H), 3.40-3.31 (m, 3H), 3.13-3.03 (m, 1H), 2.07-1.95 (m, 2H), 1.96 (s, 6H), 1.88-1.80 (m, 2H), 1.41-130 (m, 2H), 1.23-1.12 (m, 2H).
HPLC (Methode 2): Rₜ = 3.84 min
MS (ESIpos): m/z = 615 (M+H)'

### Beispiel 40

### 5-Chlor-N-{[(5S)-3-{4-[3-{2-[(2-hydroxyethyl)amino]ethoxy}-2-oxopyridin-1(2H)-yl]-3,5-dimethylphenyl}-2-oxo-1,3-oxazolidin-5-yl]methyl}thiophen-2-carboxamid

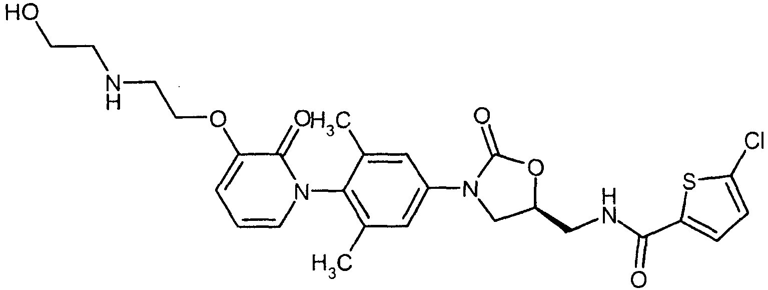

255 mg (0.475 mmol) Beispiel 73A, 74 mg (0.50 mmol) Natriumiodid und 145 mg (2.38 mmol) 2-Aminoethanol werden in 4.2 ml 1,2-Dimethoxyethan gelöst und 15 Stunden bei 90°C gerührt. Zur Aufarbeitung wird die Reaktionslösung am Rotationsverdampfer vollständig eingeengt, der Rückstand in 100 ml eines Dichlormethan/Wasser-Gemisches aufgenommen und 10 min bei RT gerührt. Nach Phasentrennung wird die wässrige Phase mit Dichlormethan extrahiert. Die vereinigten organischen Phasen werden mit einer gesättigten wässrigen Natriumchlorid-Lösung gewaschen. Nach Trocknen über Natriumsulfat wird das Trockenmittel abfiltriert und das Lösungsmittel am Rotationsverdampfer vollständig entfernt. Der Rückstand wird durch Chromatographie an Kieselgel (Dichlormethan/Ethanol 10:1 -> 5:1, Zusatz von 1 % Ethyldimethylamin) gereinigt. Es werden 168 mg (58% d. Th.) des gewünschten Produktes erhalten.
¹H-NMR (400 MHz, DMSO-*d₆*, δ/*ppm*): δ = 9.07 (t, 1H), 7.78-7.72 (m, 1H), 7.41-7.35 (m, 2H), 7.20 (d, 1H), 7.08-7.02 (m, 2H), 6.31 (dd, 1H), 4.92-4.81 (m, 1H), 4.22-4.12 (m, 2H), 3.92-3.86 (dd, 1H), 3.63-3.56 (m, 4H), 3.40-3.27 (m, 3H), 3.23-3.17 (m, 2H), 2.97-2.92 (m, 2H), 1.96 (s, 6H).
HPLC (Methode 2): Rₜ = 3.80 min
MS (ESIpos): m/z = 561 (M+H)⁺

### B. Bewertung der pharmakologischen Wirksamkeit

Die Eignung der erfindungsgemäßen Verbindungen zur Behandlung von thromboembolischen Erkrankungen kann in folgenden Assaysystemen gezeigt werden:

### a) Testbeschreibungen (in vitro)

### a.1) Messung der Faktor Xa-Hemmung in Puffer

Zur Bestimmung der Faktor Xa-Hemmung der oben aufgeführten Substanzen wird ein biochemisches Testsystem aufgebaut, in dem die Umsetzung eines Faktor Xa-Substrates zur Ermittlung der enzymatischen Aktivität von humanem Faktor Xa benutzt wird. Dabei spaltet Faktor Xa aus dem peptischen Substrat Aminomethylcoumarin ab, das fluoreszent gemessen wird. Die Bestimmungen werden in Mikrotiterplatten durchgeführt.

Zu testende Substanzen werden in unterschiedlichen Konzentrationen in Dimethylsulfoxid gelöst und 30 min mit humanem Faktor Xa (1.3 nmol/l gelöst in 50 mmol/l Tris-Puffer [C,C,C-Tris(hydroxymethyl)-aminomethan], 100 mmol/l Natriumchlorid, 5 mmol/l Calciumchlorid, 0.1% BSA [bovines Serumalbumin], ph 7.4) bei 22°C inkubiert. Anschließend wird das Substrat (5 µmol/l Boc-Ile-Glu-Gly-Arg-AMC von der Firma Bachem) hinzugefügt. Nach einer Inkubation von 30 min wird die Probe bei einer Wellenlänge von 360 nm angeregt und die Emission bei 460 nm gemessen. Die gemessenen Emissionen der Testansätze mit Prüfsubstanz werden mit den Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet.

### a.2) Messung der Thrombin-Hemmung in Puffer

Zur Bestimmung der Thrombin-Hemmung der oben aufgeführten Substanzen wird ein biochemisches Testsystem aufgebaut, in dem die Umsetzung eines Thrombin-Substrates zur Ermittlung der enzymatischen Aktivität von humanem Thrombin benutzt wird. Dabei spaltet Thrombin aus dem peptischen Substrat Aminomethylcoumarin ab, das fluoreszent gemessen wird. Die Bestimmungen werden in Mikrotiterplatten durchgeführt.

Zu testende Substanzen werden in unterschiedlichen Konzentrationen in Dimethylsulfoxid gelöst und 15 min mit humanem Thrombin (0.06 nmol/l gelöst in 50 mmol/l Tris-Puffer [C,C,C-Tris(hydroxymethyl)-aminomethan], 100 mmol/l Natriumchlorid, 0.1% BSA [bovines Serumalbumin], ph 7.4) bei 22°C inkubiert. Anschließend wird das Substrat (5 µmol/l Boc-Asp(OBzl)-Pro-Arg-AMC von der Firma Bachem) hinzugefügt. Nach einer Inkubation von 30 min wird die Probe bei einer Wellenlänge von 360 nm angeregt und die Emission bei 460 nm gemessen. Die gemessenen Emissionen der Testansätze mit Prüfsubstanz werden mit den Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet.

### a.3) Bestimmung der Selektivität

Zum Nachweis der Selektivität der Substanzen bezüglich Thrombin- und Faktor Xa-Hemmung werden die Prüfsubstanzen auf ihre Hemmung anderer humaner Serinproteasen wie Faktor XIIa, Faktor XIa, Trypsin und Plasmin hin untersucht. Zur Bestimmung der enzymatischen Aktivität von Faktor XIIa (10 nmol/l von Kordia), Faktor XIa (0.4 nmol/l von Kordia), Trypsin (83 mU/ml von Sigma) und Plasmin (0.1 µg/ml von Kordia) werden diese Enzyme gelöst (50 mmol/l Tris-Puffer [C,C,C-Tris(hydroxymethyl)-aminomethan], 100 mmol/l Natriumchlorid, 0.1% BSA [bovines Serumalbumin], 5 mmol/l Calciumchlorid, pH 7.4) und für 15 min mit Prüfsubstanz in verschiedenen Konzentrationen in Dimethylsulfoxid sowie mit Dimethylsulfoxid ohne Prüfsubstanz inkubiert. Anschließend wird die enzymatische Reaktion durch Zugabe der entsprechenden Substrate gestartet (5 µmol/l H-Pro-Phe-Arg-AMC von Bachem für Faktor XIIa, 5 µmol/l Boc-Ile-Glu-Gly-Arg-AMC von Bachem für Trypsin, 5 µmol/l Boc-Glu(OBzl)-Ala-Arg-AMC von Bachem für Faktor XIa, 50 µmol/l MeOSuc-Ala-Phe-Lys-AMC von Bachem für Plasmin). Nach einer Inkubationszeit von 30 min bei 22°C wird die Fluoreszenz gemessen (Anregung: 360 nm, Emission: 460 nm). Die gemessenen Emissionen der Testansätze mit Prüfsubstanz werden mit den Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet.

### a.4) Bestimmung der Faktor Xa-inhibitorischen Wirkung der potentiellen Inhibitoren in Plasmaproben

Zur Bestimmung der Hemmung von Faktor Xa in Plasmaproben wird der im Plasma vorhandene Faktor X durch eine Protease aus dem Gift der Klapperschlange aktiviert. Anschließend wird die Faktor Xa-Aktivität bzw. deren Hemmung durch potentielle Inhibitoren mittels Zugabe eines chromogenen Substrats gemessen.

Die zu testenden Substanzen werden in unterschiedlichen Konzentrationen in Dimethylsulfoxid gelöst und mit einer wässrigen Refludan-Lösung (10 µg/ml) verdünnt. In klaren 96-Loch-Flachbodenplatten werden 30 µl Citratplasma (Octapharma) mit 10 µl der Substanzverdünnung gemischt. Dann werden entweder 20 µl einer Lösung eines Klapperschlangerigifts (Russel viper venom (RVV); RVV Reagenz: Pentapharm 121-06, Endkonzentration 0.6 mU) in einem wässrigen Calciumchlorid-Lösung-Puffer (Endkonzentration Calciumchlorid 0.05 *M*) oder 20 µl der wässrigen Calciumchlorid-Lösung (Endkonzentration Calciumchlorid 0.05 *M*) ohne RVV Reagenz (als Referenz für eine unstimmulierte Probe) hinzugegeben. Nach Zugabe von 20 µl ChromozymXSubstrat (Endkonzentration 1.6 mmol/l, Bachem L-1565, verdünnt in Wasser) wird im SpectraFluor Reader mit einem Messfilter von 405 nm über 20 Minuten jede Minute gemessen. Die Ermittlung des IC₅₀-Wertes erfolgt, wenn ca. 70% des Maximalsignals erreicht ist (ca. 12 min).

Repräsentative Wirkdaten aus diesem Test sind in der folgenden Tabelle 1 aufgeführt:

**Tabelle 1**

| **Beispiel Nr.** | **IC₅₀ [nM]** |
|---|---|
| 6 | 31 |
| 9 | 11 |
| 10 | 15 |
| 13 | 17 |
| 16 | 18 |
| 21 | 11 |
| 36 | 29 |
| 37 | 29 |
| 38 | 34 |

### a.5) Bestimmung der Thrombin-inhibitorischen Wirkung der potentiellen Inhibitoren in Plasmaproben

Die zu testenden Substanzen werden in unterschiedlichen Konzentrationen in Dimethylsulfoxid gelöst und mit Wasser verdünnt. In weissen 96-Loch-Flachbodenplatten werden 20 µl Substanzverdünnung mit 20 µl Ecarin-Lösung (Ecarin Reagenz, Firma Sigma E-0504, Endkonzentration 20 mU pro Ansatz) in Ca-Puffer (200 mM Hepes + 560 mM Natriumchlorid + 10 mM Calciumchlorid + 0.4% PEG) bzw. mit 20 µl Ca-Puffer (als unstimulierte Kontrolle) gemischt. Desweiteren werden 20 µl fluorogenes Thrombinsubstrat (Firma Bachem I-1120, Endkonzentration 50 µmol/l) und 20 µl Citratplasma (Firma Octapharma) zugegeben und gut homogenisiert. Die Platte wird im SpectraFluorplus Reader mit einem Excitationsfilter 360 nm und Emissionsfilter 465 nm über 20 Minuten jede Minute gemessen. Die Ermittlung des IC₅₀-Wertes erfolgt, wenn ca. 70% des Maximalsignals erreicht ist (ca. 12 min).

Repräsentative Wirkdaten aus diesem Test sind in der folgenden Tabelle 2 aufgeführt:

**Tabelle 2**

| **Beispiel Nr.** | **IC₅₀ [nM]** |
|---|---|
| 6 | 143 |
| 9 | 10 |
| 10 | 24 |
| 13 | 16 |
| 16 | 10 |
| 21 | 34 |
| 36 | 36 |
| 37 | 15 |
| 38 | 30 |

### a.6) Thrombin Generation Assay (Thrombogramm)

Die Wirkung der Prüfsubstanzen auf das Thrombogramm (Thrombin Generation Assay nach Hemker) wird in vitro in Humanplasma (Octaplas® von der Firma Octapharma) bestimmt. Im Thrombin Generation Assay nach Hemker wird die Aktivität von Thrombin in gerinnendem Plasma durch die Messung der fluoreszenten Spaltprodukte des Substrats I-1140 (Z-Gly-Gly-Arg-AMC, Bachem) bestimmt. Um die Gerinnungsreaktion zu starten werden Reagenzien der Firma Thrombinoscope verwendet (PPP Reagenz: 30 pM recombinant tissue factor, 24 µM phospholipids in HEPES). Die Reaktion wird in Gegenwart variierender Konzentrationen an Prüfsubstanz oder dem entsprechenden Lösungsmittel durchgeführt. Außerdem wird ein Thrombin-Kalibrator der Firma Thrombinoscope verwendet, dessen amidolytische Aktivität zur Berechnung der Thrombinaktivität in einer Plasmaprobe benötigt wird.

Die Testdurchführung erfolgt nach Herstellerangaben (Thrombionsocpe BV): 4 µl der Prüfsubstanz oder des Lösungsmittels, 76 µl Plasma und 20 µl PPP-Reagenz oder Thrombin Calibrator werden 5 min bei 37°C inkubiert. Nach Zugabe von 20 µl 2.5 mM Thrombinsubstrat in 20 mM Hepes, 60 mg/ml BSA, 102 mM Calciumchlorid wird die Thrombin-Generierung über 120 min alle 20 s gemessen. Die Messung wird mit einem Fluorometer (Fluoroskan Ascent) der Firma Thermo Electron durchgeführt, der mit einem 390/460 nM Filterpaar und einem Dispenser ausgestattet ist. Durch die Verwendung der "thrombinoscope software" wird das Thrombogramm berechnet und graphisch dargestellt. Die folgenden Parameter werden berechnet: lag time, time to Peak, Peak, ETP (endogenous thrombin potential) und start tail.

### a.7) Bestimmung der antikoagulatorischen Wirkung

Die antikoagulatorische Wirkung der Prüfsubstanzen wird in vitro in Human-, Kaninchen- und Rattenplasma bestimmt. Dazu wird Blut unter Verwendung einer 0.11 molaren Natriumcitrat-Lösung als Vorlage in einem Mischungsverhältnis Natriumcitrat/Blut 1/9 abgenommen. Das Blut wird unmittelbar nach der Abnahme gut gemischt und 15 Minuten bei ca. 4000 g zentrifugiert. Der Überstand wird abpipettiert.

Die Prothrombinzeit (PT, Synonyme: Thromboplastinzeit, Quick-Test) wird in Gegenwart variierender Konzentrationen an Prüfsubstanz oder dem entsprechenden Lösungsmittel mit einem handelsüblichen Testkit (Neoplastin® von der Firma Boehringer Mannheim oder Hemoliance® RecombiPlastin von der Firma Instrumentation Laboratory) bestimmt. Die Testverbindungen werden 3 Minuten bei 37°C mit dem Plasma inkubiert. Anschließend wird durch Zugabe von Thromboplastin die Gerinnung ausgelöst und der Zeitpunkt des Gerinnungseintritts bestimmt. Es wird die Konzentration an Prüfsubstanz ermittelt, die eine Verdoppelung der Prothrombinzeit bewirkt.

Die Thrombinzeit (TT) wird in Gegenwart variierender Konzentrationen an Prüfsubstanz oder dem entsprechenden Lösungsmittel mit einem handelsüblichen Testkit (Thrombin Reagent von der Firma Roche) bestimmt. Die Testverbindungen werden 3 Minuten bei 37°C mit dem Plasma inkubiert. Anschließend wird durch Zugabe des Thrombin Reagenz die Gerinnung ausgelöst und der Zeitpunkt des Gerinnungseintritts bestimmt. Es wird die Konzentration an Prüfsubstanz ermittelt, die eine Verdoppelung der Thrombinzeit bewirkt.

Die aktivierte partielle Thromboplastinzeit (APTT) wird in Gegenwart variierender Konzentrationen an Prüfsubstanz oder dem entsprechenden Lösungsmittel mit einem handelsüblichen Testkit (PTT Reagent von der Firma Roche) bestimmt. Die Testverbindungen werden 3 Minuten bei 37°C mit dem Plasma und dem PTT Reagenz (Cephalin, Kaolin) inkubiert. Anschließend wird durch Zugabe von 25 mM Calciumchlorid die Gerinnung ausgelöst und der Zeitpunkt des Gerinnungseintritts bestimmt. Es wird die Konzentration an Prüfsubstanz ermittett, die eine Verdoppelung der APTT bewirkt.

### a.8) Thromboelastographie (Thromboelastogramm)

Die Thromboelastographie wird mit Hilfe des Thromboelastographen ROTEM der Firma Pentapharm und dem dazu gehörenden Zubehör Cup and pin durchgeführt. Die Messung erfolgt in Vollblut, welches zuvor in Natrium-Citrat Monovetten der Firma Sarstedt entnommen wird. Das Blut wird in den Monovetten mit Hilfe eines Schüttlers in Bewegung gehalten und bei 37°C für 30 min. vorinkubiert. Es wird eine 2 molare Stammlösung von Calciumchlorid in Wasser erstellt. Diese wird 1:10 mit einer wässrigen 0.9%igen Natriumchlorid-Lösung verdünnt. Zur Messung werden 20 µl dieser 200 mM Calciumchlorid-Lösung in die Cups vorgelegt (Endkonzentration Calciumchlorid 12.5 mM). Es werden 3.2 µl Substanz oder Lösemittel zugegeben. Die Messung wird durch Zugabe von 300 µl Vollblut gestartet. Nach der Zugabe wird kurz mit der Pipettenspitze auf und ab pipettiert ohne Luftblasen zu erzeugen. Die Messung erfolgt über 2.5 Stunden oder wird bei Beginn der Fibrinolyse gestoppt. Zur Auswertung werden folgende Parameter bestimmt: CT(clotting time/ [sec.]), CFT (clotting formation time/ [sec.]), MCF (maximum clot firmness / [mm]) und der alpha-Winkel [°]. Die Messpunkte werden alle 3 Sekunden erhoben und graphisch über die y-Achse als MCF [mm] und auf der x-Achse als Zeit [sec.] dargestellt.

### a.9) Inhibierung der an Thrombus gebundenen Gerinnungsfaktoren Thrombin und Faktor Xa

Blutgerinnsel, die sich entweder vor Therapiebeginn mit Antikoagulantien, während Therapiepausen oder trotz Therapie bilden, enthalten große Mengen Gerinnungsfaktoren, die eine fortschreitende Thrombusbildung begünstigen können. Diese Gerinnungsfaktoren sind fest an den Thromus gebunden und können nicht ausgewaschen werden. In bestimmten klinischen Situationen kann hieraus ein Risiko für den Patienten entstehen. In den unten aufgeführten Versuchen lassen sich in humanen Thromben sowohl Thrombin als auch FXa mit biologischer (prokoagulatorischer) Aktivität nachweisen.

### In vitro gebildete Thromben

Thromben werden *in vitro* aus humanem Plasma gebildet und auf Aktivität der gebundenen Gerinnungsfaktoren Thrombin und FXa untersucht. Hierzu werden 300µl Plasma mit 30 µl Lipidvesikeln und 30 µl einer wässrigen Calciumchlorid-Lösung in einer 48 MTP Platte gemischt, und 30 min inkubiert. Dieser und die folgenden Schritte werden bei 37°C und unter konstanter Bewegung durchgeführt (300 U/min). Die gebildeten Thromben werden in eine neue 48 MTP Platte transferiert und in 0.9%iger Natriumchlorid-Lösung zweimal 10 min gewaschen, wobei der Thrombus zwischen den Waschgängen auf Filterpapier abgetupft wird. Der Thrombus wird in Puffer B transferiert (Owens Veronal Puffer, 1% BSA) und 15 min inkubiert, auf Filterpapier abgetupft und 30 min in Testsubstanz in verschiedenen Konzentrationen in Puffer B inkubiert. Anschließend werden die Clots wie oben beschrieben zweimalig gewaschen. Die Thromben werden abgetupft und in Puffer D transferiert: (240 µl Owren's veronal Puffer, 1% BSA und 15.6 mM Calciumchlorid) und mit oder ohne 0.6 µM Prothrombin 45 min inkubiert. Die Reaktion wird durch 75 µl 1 % EDTA-Lösung gestoppt. Thrombinaktivität wird separat im Thrombus in Puffer A (7.5 mM Na₂EDTAx2H₂O, 175 mM Natriumchlorid, 1% BSA, pH 8.4) oder im Überstand aus dem letzten Schritt gemessen. Hierzu wird das Substrat I-1120 in der Endkonzentration 50 µM eingesetzt, und die resultierende Fluoreszenz im Fluoreszenz-Plattenausleser ausgemessen (360/465nm).

Die Aktivität dieses thrombusgebundenen Thrombins kann durch einen selektiven FXa-Inhibitor in therapeutisch relevanten Konzentrationen nicht unterdrückt werden. Hingegen kann diese mit dualen FIIa/FXa Inhibitoren oder einem FIIa-Referenz-Inhibitor gehemmt werden.

Nach Zugabe von Prothrombin wird bei Vorhandensein von thrombusgebundenem FXa (Prothrombinase-Komplex) neues Thrombin gebildet, das durch das fluoreszierende Substrat nachgewiesen wird. Diese Neubildung von Thrombin kann durch einen reinen Thrombininhibitor nicht verhindert werden, jedoch durch duale FIIa/FXa Inhibitoren oder den selektiven FXa-Inhibitor Referenzinhibitor.

Die biologische Wirksamkeit der Thrombus-gebundenen Thrombinaktivität wird durch Zugabe von fluoreszierend markiertem Fibrinogen überprüft, das durch aktives Thrombin in Fibrin umgewandelt und an den Thrombus gebunden wird. Hierzu wird der Thrombus wie oben beschrieben gebildet und in 250 µl einer Alexa488-markiertem Fibrinogen-Lösung (100 µg/ml) und 30 µl einer wässrigen 100 mM Calciumchlorid-Lösung inkubiert (mit oder ohne Testsubstanzen in verschiedenen Konzentrationen). Die Fluoreszenz des Überstandes wird in einem Fluoreszenz-Plattenausleser bei geeigneter Wellenlänge gemessen. Außerdem werden die Thromben viermal jeweils 15 min gewaschen, und fluoreszenz-mikroskopisch evaluiert. Der Abfall der Fluoreszenz aus dem Überstand und die Zunahme der Fluoreszenz der Thromben kann durch duale FIIa/FXa Inhibitoren, nicht jedoch durch den FXa-Referenzinhibitor gehemmt werden.

### In vivo entstandene, intrakardiale Thromben (Patientenmaterial)

Die Untersuchungen werden wiederholt an Thromben, die Patienten im Rahmen von Herzoperationen aus dem linken Ventrikel entnommen worden waren. Hierzu werden die Thromben aufgetaut und in Stücke unterteilt (10 - 100mg Feuchtgewicht). Die Thromben werden, je nach Protokoll, mehrmalig gewaschen oder ungewaschen eingesetzt und die Thrombinaktivität mit dem Substrat I-1120 (Endkonzentration 100 µM) analog dem oben beschrieben Verfahren gemessen.

### a.10) Spezielle Diagnostik der Gerinnungsstörung und Organsfunktion bei endotoxämischen Mäusen und Ratten

### Thrombin-Antithrombin-Komplexe

Thrombin-Antithrombin-Komplexe (nachfolgend als "TAT" bezeichnet) sind ein Maß für das durch Gerinnungsaktivierung endogen gebildete Thrombin. TAT werden mittels eines ELISA-Assays bestimmt (Enzygnost TAT micro, Dade-Behring). Aus Zitratblut wird durch Zentrifugation Plasma gewonnen. Zu 50 µl Plasma wird 50 µl TAT-Probenpuffer gegeben, kurz geschüttelt und 15 min bei Raumtemperatur inkubiert. Die Proben werden abgesaugt, und das Well 3-malig mit Waschpuffer gewaschen (300 µl/Well). Die Platte wird zwischen den Waschgängen abgeklopft. Es wird Konjugatlösung (100 µl) hinzugegeben und 15 min bei Raumtemperatur inkubiert. Die Proben werden abgesaugt, und das Well 3-malig mit Waschpuffer gewaschen (300 µl/Well). Anschließend wird chromogenes Susbtrat hinzugegeben (100 µl/Well), 30 min im Dunkeln bei Raumtemperatur inkubiert, Stopplösung hinzugegeben (100 µl/ Well), und die Farbbildung bei 492 nm gemessen (Saphire Plate reader).

### Parameter für Organfunktion

Es werden verschiedene Parameter bestimmt, aufgrund derer Rückschlüsse auf die Funktionseinschränkung verschiedener innerer Organe durch die LPS-Gabe gezogen werden können, und der therapeutische Effekt von Prüfsubstanzen abgeschätzt werden kann. Zitratblut oder ggf. Lithium-Heparin-Blut wird zentrifugiert, und die Parameter aus dem Plasma bestimmt. Folgende Parameter werden typischerweise erhoben: Kreatinin, Harnstoff, Aspartat-Aminotransferase (AST), Alanin-Aminotransferase (ALT), Gesamt-Bilirubin, Laktatdehydrogenase (LDH), Gesamt-Protein, Gesamt-Albumin und Fibrinogen. Die Werte geben Aufschluss auf die Funktion der Niere, der Leber, des Kreislaufes und der Gefäße.

### Parameter für Entzündung

Das Ausmaß der durch Endotoxin ausgelösten Entzündungsreaktion lässt sich aus dem Anstieg von Entzündungsmediatoren im Plasma nachweisen, z.B. Interleukine (1, 6, 8 und 10), Tumornekrosefaktor alpha oder Monocyte Chemoattractant Protein-1. Hierzu können ELISAs oder das Luminex-System verwendet werden.

### b) Bestimmung der antithrombotischen Wirkung (in vivo)

### b.1) Arteriovenöses Shunt- und Blutungs-Modell (Kombi-Modell Ratte)

Nüchterne männliche Ratten (Stamm: HSD CPB:WU) mit einem Gewicht von 300-350 g werden mit Inactin (150 - 180 mg/kg) narkotisiert. Die Thrombusbildung wird in einem arteriovenösen Shunt in Anlehnung an die von Christopher N. Berry et al., Br. J. Pharmacol. (1994), 113, 1209-1214 beschriebene Methode ausgelöst. Dazu werden die linke Vena jugularis und die rechte Arteria carotis freipräpariert. Ein extracorporaler Shunt wird mittels eines 10 cm langen Polyethylenschlauchs (PE 60) zwischen den beiden Gefäßen gelegt. Dieser Polyethylenschlauch ist in der Mitte in einen weiteren 3 cm langen Polyethylenschlauch (PE 160), der zur Erzeugung einer thrombogenen Oberfläche einen aufgerauhten und zu einer Schlinge gelegten Nylonfaden enthält, eingebunden. Der extrakorporale Kreislauf wird 15 Minuten lang aufrechterhalten. Dann wird der Shunt entfernt und der Nylonfaden mit dem Thrombus sofort gewogen. Das Leergewicht des Nylonfadens ist vor Versuchsbeginn ermittelt worden.

Zur Bestimmung der Blutungszeit wird unmittelbar nach Öffnung des Shunt-Kreislaufs die Schwanzspitze der Ratten mit einer Rasierklinge um 3 mm kupiert. Der Schwanz wird in 37°C temperierte physiologische Kochsalzlösung gelegt und die Blutung aus der Schnittwunde über 15 Minuten beobachtet. Es werden die Zeit bis zum Sistieren der Blutung für mind. 30 Sekunden (initiale Blutungszeit), die Gesamtblutungszeit innerhalb von 15 Minuten (kumulative Blutungszeit) sowie die Menge des Blutverlusts über die photometrische Bestimmung des aufgefangenen Hämoglobins ermittelt.

Die Prüfsubstanzen werden vor Anlegung des extrakorporalen Kreislaufs und des Schwanzspitzenschnitts entweder intravenös über die kontralaterale Jugularvene als Einzelbolus bzw. als Bolus mit anschließender Dauerinfusion oder oral mittels Schlundsonde wachen Tieren verabreicht.

### c) Löslichkeitsassay

### Benötigte Reagenzien:

- PBS-Puffer pH 7.4: 90.00 g NaCl p.a. (z.B. Merck Art. Nr. 1.06404.1000), 13.61 g KH₂PO₄ p.a. (z.B. Merck Art. Nr. 1.04873.1000) und 83.35 g 1N NaOH (z.B. Bernd Kraft GmbH Art. Nr. 01030.4000) in einen 11 Messkolben einwiegen, mit Wasser auffüllen und ca. 1 Stunde rühren.
- Acetatpuffer pH 4.6: 5.4 g Natriumacetat x 3 H₂O p.a. (z.B. Merck Art. Nr. 1.06267.0500) in einen 100 ml Messkolben einwiegen, in 50 ml Wasser lösen, mit 2.4 g Eisessig versetzen, auf 100 ml mit Wasser auffüllen, pH-Wert überprüfen und falls notwendig auf pH 4.6 einstellen.
- Dimethylsulfoxid (z.B. Baker Art. Nr. 7157.2500)
- destilliertes Wasser

### Herstellung der Kalibrierlösungen:

*Herstellung der Ausgangslösung für Kalibrierlösungen (Stamm*/*ösung):* In ein 2 ml Eppendorf-Safe-Lock Tube (Eppendorf Art. Nr. 0030 120.094) werden ca. 0.5 mg des Wirkstoffes genau eingewogen, zu einer Konzentration von 600 µg/ml mit DMSO versetzt (z.B. 0.5 mg Wirkstoff + 833 µl DMSO) und bis zur vollständigen Lösung mittels eines Vortexers geschüttelt.

*Kalibrierlösung 1 (20 µg*/*ml):* 34.4 µl der Stammlösung werden mit 1000 µl DMSO versetzt und homogenisiert.

*Kalibrierlösung 2 (2.5 µg*/*m*/*):* 100 µl der Kalibrierlösung 1 werden mit 700 µl DMSO versetzt und homogenisiert.

### Herstellung der Probenlösungen:

*Prohenlösung für Löslichkeit bis 10 g*/*l in PBS-Puffer pH 7.4:* In ein 2 ml Eppendorf-Safe-Lock Tube (Eppendorf Art. Nr. 0030 120.094) werden ca. 5 mg des Wirkstoffes genau eingewogen und zu einer Konzentration von 5 g/l mit PBS-Puffer pH 7.4 versetzt (z.B. 5 mg Wirkstoff + 500 µl PBS-Puffer pH 7.4).

*Probenlösung für Löslichkeit bis 10 g*/*l in Acetatpuffer pH 4.6:* In ein 2 ml Eppendorf-Safe-Lock Tube (Eppendorf Art. Nr. 0030 120.094) werden ca. 5 mg des Wirkstoffes genau eingewogen und zu einer Konzentration von 5 g/l mit Acetatpuffer pH 4.6 versetzt (z.B. 5 mg Wirkstoff + 500 µl Acetatpuffer pH 4.6).

*Probenlösung für Löslichkeit bis 10 g*// *in Wasser:* In ein 2 ml Eppendorf-Safe-Lock Tube (Eppendorf Art. Nr. 0030 120.094) werden ca. 5 mg des Wirkstoffes genau eingewogen und zu einer Konzentration von 5 g/l mit Wasser versetzt (z.B. 5 mg Wirkstoff + 500 µl Wasser).

### Durchführung:

Die so hergestellten Probenlösungen werden 24 Stunden bei 1400 rpm mittels eines temperierbaren Schüttlers (z.B. Eppendorf Thermomixer comfort Art. Nr. 5355 000.011 mit Wechselblock Art. Nr. 5362.000.019) bei 20°C geschüttelt. Von diesen Lösungen werden jeweils 180 µl abgenommen und in Beckman Polyallomer Centrifuge Tubes (Art. Nr. 343621) überführt. Diese Lösungen werden 1 Stunde mit ca. 223.000 *g zentrifugiert (z.B. Beckman Optima L-90K Ultracentrifuge mit Type 42.2 Ti Rotor bei 42.000 rpm). Von jeder Probenlösung werden 100 µl des Überstandes abgenommen und 1:5, 1:100 und 1:1000 mit dem jeweils verwendeten Lösungsmittel (Wasser, PBS-Puffer 7.4 oder Acetatpuffer pH 4.6) verdünnt. Es wird von jeder Verdünnung eine Abfüllung in ein geeignetes Gefäß für die HPLC-Analytik vorgenommen.

### Analytik:

Die Proben werden mittels RP-HPLC analysiert. Quantifiziert wird über eine Zwei-Punkt-Kalibrationskurve der Testverbindung in DMSO. Die Löslichkeit wird in mg/l ausgedrückt.

### Analysensequenz:

1. Kallibrierlösung 2.5 mg/ml
2. Kallibrierlösung 20 µg/ml
3. Probenlösung 1:5
4. Probenlösung 1:100
5. Probenlösung 1:1000

### HPLC-Methode für Säuren:

Agilent 1100 mit DAD (G1315A), quat. Pumpe (G1311A), Autosampler CTC HTS PAL, Degaser (G1322A) and Säulenthermostat (G1316A); Säule: Phenomenex Gemini C18, 50 x 2 mm, 5 µ; Temperatur: 40°C; Eluent A: Wasser/Phosphorsäure pH 2; Eluent B: Acetonitril; Flussrate: 0.7 ml/min; Gradient: 0-0.5 min 85% A, 15% B; Rampe: 0.5-3 min 10% A, 90% B; 3-3.5 min 10% A, 90% B; Rampe: 3.5-4 min 85% A, 15% B; 4-5 min 85% A, 15% B.

### HPLC-Methode für Basen:

Agilent 1100 mit DAD (G1315A), quat. Pumpe (G1311A), Autosampler CTC HTS PAL, Degaser (G1322A) and Säulenthermostat (G1316A); Säule: VDSoptilab Kromasil 100 C18, 60 x 2.1 mm, 3.5 µ; Temperatur: 30°C; Eluent A: Wasser + 5 ml Perchlorsäure/1; Eluent B: Acetonitril; Flussrate: 0.75 ml/min; Gradient: 0-0.5 min 98% A, 2% B; Rampe: 0.5-4.5 min 10% A, 90% B; 4.5-6 min 10% A, 90% B; Rampe: 6.5-6.7 min 98% A, 2% B; 6.7-7.5 min 98% A, 2% B.

### d) Bestimmung der Pharmakokinetik (in vivo)

Zur Bestimmung der *in vivo* Pharmakokinetik werden die Testsubstanzen in verschiedenen Formulierungsmitteln (z.B. Plasma, Ethanol, DMSO, PEG400 etc.) oder Gemischen dieser Lösungsvermittler gelöst Mäusen, Ratten, Hunden oder Affen intravenös oder peroral appliziert. Die intravenöse Applikation erfolgt wahlweise als Bolus oder als Infusion. Die applizierten Dosen liegen im Bereich von 0.1 bis 5 mg/kg. Blutproben werden mittels eines Katheters oder als Tötungsplasma zu verschiedenen Zeitenpunkten über ein Intervall von bis zu 26 h entnommen. Des weiteren werden teilweise auch Organ-, Gewebs- und Urinproben gewonnen. Die quantitative Bestimmung der Substanzen in den Versuchsproben erfolgt mittels Eichproben die in der jeweiligen Matrix eingestellt werden. In den Proben enthaltene Proteine werden durch Fällung mit Acetonitril oder Methanol entfernt. Anschließend werden die Proben mittels HPLC auf einer 2300 HTLC Anlage (Cohesive Technologies, Franklin, MA, USA) unter Verwendung von Reversed Phase Säulen aufgetrennt. Das HPLC System ist über ein Turbo Ion Spray Interface an ein Triple Quadropole Massenspektrometer API 3000 (Applied Biosystems, Darmstadt, Deutschland) gekoppelt. Die Auswertung des Plasmakonzentrations-Zeitverlaufs erfolgt unter Verwendung eines validierten Kinetikauswerteprogramms.

Die Affinität einer Substanz für ein Transportprotein wird mittels *in vitro* Testung in einem Flux Assay unter Verwendung von Caco-2 Zellen oder Zellen die ein spezifischen Transporter überexpremieren untersucht (Troutman MD, Thakker DR, Pharm. Res. 20 (8) 1210- 1224 (2003); Schwab D, Fischer H, Tabatabaei A, Poli S, Huwyler J, J. Med. Chem. 46, 1716-1725 (2003); Merino G, Jonker JW, Wagenaar E, Pulido MM, Molina AJ, Alvarez AI, Schinkel AH, Drug Metab. Dispos. 33 (5) 614- 618 (2005)). Hierzu werden die Zellen auf 24- oder 96-Loch-Filter-Platten für 4 bis 15 Tage kultiviert. Zur Bestimmung der Permeation werden die Substanzen in einem HEPES-Puffer entweder apikal (A) oder basal (B) auf die Zellen gegeben und für 2 h inkubiert. Nach 0 h und 2 h werden Proben aus den Cis- und Transkompartimenten gezogen und mittels LC-MS/MS analysiert. Der Papp-Wert wird mittels der von Schwab et al. publizierten Formel berechnet. Eine Substanz wird als aktiv transportiert klassifiziert, wenn das Ratio von Papp (B-A)/Papp (A-B) > 2 oder < 0.5 ist.

### e) Bestimmung der Wirkung bei Endotoxinämie (in vivo)

Die Untersuchung wird an Ratten oder Mäusen durchgeführt. Im Modell an Mäusen (NMRI, männlich) wird LPS (*Escherichia coli* Serotyp 055:B5, Sigma-Aldrich) 50 mg/kg intraperitoneal injiziert. Die Prüfsubstanzen werden bis zu einer Stunde vor LPS-Injektion entweder intravenös über die Schwanzvene, subkutan, intraperitoneal oder oral mittels Schlundsonde verabreicht. Vier Stunden nach LPS-Applikation wird das Tier narkotisiert (Ketavet/Rompun) und das Abdomen operativ eröffnet. Natrium-Zitratlösung (3.2% w/v) (Formel: Körpergewicht in g / 13 mal 100 µl) wird in die untere Hohlvene injiziert, und nach 30 Sek. Blut entnommen (ca. 1 ml). Aus dem Blut werden verschiedene Parameter bestimmt, z.B. die zellulären Blutbestandteile (insbesondere Erythrozyten, Leukozyten und Thrombozyten), der Laktatspiegel, die Gerinnungsaktivierung (TAT) oder Parameter der Organdysfunktion oder des Organversagens und Sterblichkeit.

### f) Methodikbeschretbung zu DIC-Versuchen an der Ratte

Bei männlichen Wistar-Ratten wird LPS (E. coli 055 B5, Hersteller Sigma, gelöst in PBS) in einer Dosierung von 250 µg/ kg intravenös in die Schwanzvene injiziert (Applikationsvolumen 2 ml/kg). Die Prüfsubstanz wird in PEG 4001H₂O 60%/40% gelöst und oral (Applikationsvolumen 5 ml/kg) 30 Minuten vor LPS-Injektion appliziert. 1, 5 oder 4 Stunden nach LPS-Injektion werden die Tiere in Terminalnarkose (Trapanal^{®} 100 mg/kg i.p.) durch Herzpunktion entblutet und Citratplasma für die Bestimmung von Fibrinogen, PT, TAT und Plättchenzahl gewonnen. Optional wird Serum zur Bestimmung von Leberenzymen, Nierenfunktionsparamertern und Cytokinen gewonnen. TNFα und IL-6 werden mit kommerziell erhältlichen ELISAs (R&D Systems) bestimmt.

Es können auch direkte Parameter der Organfunktion gemessen werden, z.B. links- und rechtsventrikuläre Drucke, arterielle Drucke, Urinausscheidung, Nierendurchblutung und Blutgase und Säure-/Basenstatus.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einher Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Natriumchloridlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel 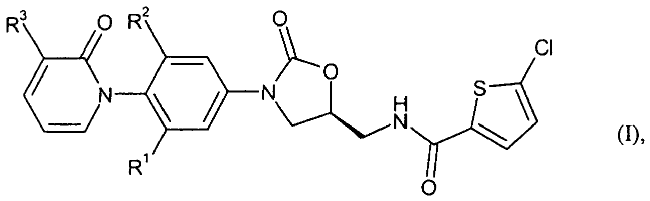 in welcher
R¹ für Chlor, Trifluormethoxy, Methyl, Ethyl, n-Propyl, Methoxy, Methoxymethyl oder Ethoxymethyl steht,
R² für Wasserstoff oder Methyl steht,
und
R³ für eine Gruppe der Formel 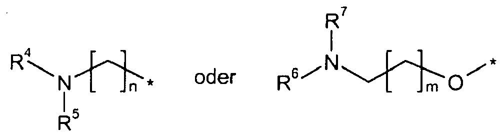
steht,
wobei
* die Anknüpfstelle an den Oxopyridin-Ring ist,
n für die Zahl 1, 2, 3 oder 4 steht,
m für die Zahl 1 oder 2 steht,
R⁴ für Wasserstoff, Methyl, Ethyl, Cyclopropyl, Cyclobutyl, 2-Hydroxyeth-1-yl, 3-Hydroxyprop-1-yl, 2-Methoxyethy-1-yl, 3-Methoxyprop-1-yl, 4-Hydroxycyclohex-1-yl, Tetrahydrofuran-2-ylmethyl oder 1,4-Dioxan-2-ylmethyl steht,
R⁵ für Wasserstoff, Methyl oder Ethyl steht,
oder
R⁴ und R⁵ zusammen mit dem Stickstoffatom an das sie gebunden sind einen Pyrrolidin-1-yl-Ring, einen 2-Methoxymethyl-pyrrolidin-1-yl-Ring, einen Morpholin-4-yl-Ring, einen 1,1-Dioxo-thiomorpholin-4-yl-Ring, einen 1,4-Oxazepan-4-yl-Ring, einen 4-Methyl-piperazin-1-yl oder einen 4-Hydroxypiperidin-1-yl-Ring bilden,
R⁶ für Wasserstoff, Methyl, Ethyl, Cyclopropyl, Cyclobutyl, 2-Hydroxyeth-1-yl, 3-Hydroxyprop-1-yl, 2-Methoxyethy-1-yl, 3-Methoxyprop-1-yl, 4-Hydroxycyclohex-1-yl, Tetrahydrofuran-2-ylmethyl oder 1,4-Dioxan-2-ylmethyl steht,
R⁷ für Wasserstoff, Methyl oder Ethyl steht,
oder
R⁶ und R⁷ zusammen mit dem Stickstoffatom an das sie gebunden sind einen Pyrrolidin-1-yl-Ring, einen 2-Methoxymethyl-pyrrolidin-1-yl-Ring, einen Morpholin-4-yl-Ring, einen 1,1-Dioxo-thiomorpholin-4-yl-Ring, einen 1,4-Oxazepan-4-yl-Ring, einen 4-Methyl-piperazin-1-yl oder einen 4-Hydroxypiperidin-1-yl-Ring bilden,
oder eines ihrer Salze, ihrer Solvate oder der Solvate ihrer Salze.

2. Verbindung nach Anspruch 1, **dadurch gekennzeichnet, dass**
R¹ für Chlor, Trifluormethoxy, Methyl, Ethyl, n-Propyl, Methoxy oder Methoxymethyl steht,
R² für Wasserstoff oder Methyl steht,
und
R³ für eine Gruppe der Formel 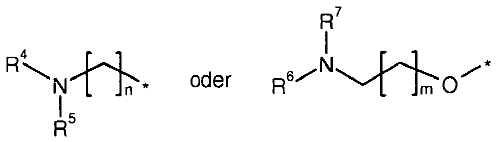
steht.
wobei
* die Anknüpfstelle an den Oxopyridin-Ring ist,
n für die Zahl 1, 2 oder 3 steht,
m für die Zahl 1 oder 2 steht,
R⁴ für Wasserstoff, Methyl, Ethyl, Cyclopropyl, Cyclobutyl, 2-Hydroxyeth-1-yl, 3-Hydroxyprop-1-yl, 2-Methoxyethy-1-yl, 3-Methoxyprop-1-yl, 4-Hydroxycyclohex-1-yl, Tetrahydrofuran-2-ylmethyl oder 1,4-Dioxan-2-ylmethyl steht,
R⁵ für Wasserstoff, Methyl oder Ethyl steht,
oder
R⁴ und R⁵ zusammen mit dem Stickstoffatom an das sie gebunden sind einen Pyrrolidin-1-yl-Ring, einen 2-Methoxymethyl-pyrrolidin-1-yl-Ring, einen Morpholin-4-yl-Ring, einen 1,1-Dioxo-thiomorpholin-4-yl-Ring, einen 1,4-Oxazepan-4-yl-Ring, einen 4-Methyl-piperazin-1-yl oder einen 4-Hydroxypiperidin-1-yl-Ring bilden,
R⁶ für Wasserstoff, Methyl, Ethyl, Cyclopropyl, Cyclobutyl, 2-Hydroxyeth-1-yl, 3-Hydroxyprop-1-yl, 2-Methoxyethy-1-yl, 3-Methoxyprop-1-yl, 4-Hydroxycyclohex-1-yl, Tetrahydrofuran-2-ylmethyl oder 1,4-Dioxan-2-ylmethyl steht,
R⁷ für Wasserstoff, Methyl oder Ethyl steht,
oder
R⁶ und R⁷ zusammen mit dem Stickstoffatom an das sie gebunden sind einen Pyrrolidin-1-yl-Ring, einen 2-Methoxymethyl-pyrrolidin-1-yl-Ring, einen Morpholin-4-yl-Ring, einen 1,1-Dioxo-thiomorpholin-4-yl-Ring, einen 1,4-Oxazepan-4-yl-Ring, einen 4-Methyl-piperazin-1-yl oder einen 4-Hydroxypiperidin-1-yl-Ring bilden,
oder eines ihrer Salze, ihrer Solvate oder der Solvate ihrer Salze.

3. Verbindung nach einem der Ansprüche 1 oder 2, **dadurch gekennzeichnet, dass**
R¹ für Methyl, Ethyl, n-Propyl, Methoxy oder Methoxymethyl steht,
R² für Wasserstoff oder Methyl steht,
und
R³ für eine Gruppe der Formel 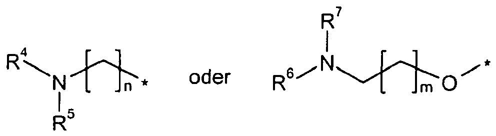
steht,
wobei
* die Anknüpfstelle an den Oxopyridin-Ring ist,
n für die Zahl 1, 2 oder 3 steht,
m für die Zahl 1 oder 2 steht,
R⁴ für Wasserstoff, Methyl, Ethyl, Cyclopropyl, Cyclobutyl, 2-Hydroxyeth-1-yl, 3-Hydroxyprop-1-yl, 2-Methoxyethy-1-yl, 3-Methoxyprop-1-yl, 4-Hydroxycyclohex-1-yl, Tetrahydrofuran-2-ylmethyl oder 1,4-Dioxan-2-ylmethyl steht,
R⁵ für Wasserstoff, Methyl oder Ethyl steht,
R⁶ für Wasserstoff, Methyl, Ethyl, Cyclopropyl, Cyclobutyl, 2-Hydroxyeth-1-yl, 3-Hydroxyprop-1-yl, 2-Methoxyethy-1-yl, 3-Methoxyprop-1-yl, 4-Hydroxycyclohex-1-yl, Tetrahydrofuran-2-ylmethyl oder 1,4-Dioxan-2-ylmethyl steht,
R⁷ für Wasserstoff, Methyl oder Ethyl steht,
oder eines ihrer Salze, ihrer Solvate oder der Solvate ihrer Salze.

4. Verbindung nach einem der Ansprüche 1 bis 3, **dadurch gekennzeichnet, dass**
R¹ für Methyl oder Methoxy steht,
R² für Wasserstoff steht,
oder
R¹ für Methyl steht,
R² für Methyl steht,
und
R³ für eine Gruppe der Formel 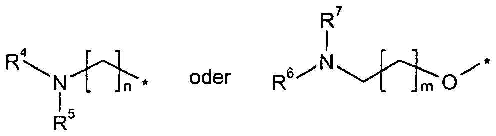
steht,
wobei
* die Anknüpfstelle an den Oxopyridin-Ring ist,
n für die Zahl 2 steht,
m für die Zahl 1 steht,
R⁴ für 2-Hydroxyeth-1-yl oder 4-Hydroxycyclohex-1-yl steht,
R⁵ für Wasserstoff steht,
R⁶ für 2-Hydroxyeth-1-yl oder 4-Hydroxycyclohex-1-yl steht,
R⁷ für Wasserstoff steht,
oder eines ihrer Salze, ihrer Solvate oder der Solvate ihrer Salze.

5. Verbindung nach einem der Ansprüche 1 bis 3, **dadurch gekennzeichnet, dass**
R¹ für Methoxymethyl steht,
R² für Wasserstoff steht,
oder
R¹ für Methyl steht,
R² für Methyl steht,
und
R³ für eine Gruppe der Formel 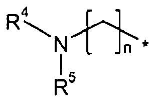
steht,
wobei
* die Anknüpfstelle an den Oxopyridin-Ring ist,
n für die Zahl 2 steht,
R⁴ für 2-Hydroxyeth-1-yl oder 4-Hydroxycyclohex-1-yl steht,
R⁵ für Wasserstoff steht,
oder eines ihrer Salze, ihrer Solvate oder der Solvate ihrer Salze.

6. Verbindung nach einem der Ansprüche 1 bis 4, **dadurch gekennzeichnet, dass**
R¹ für Methyl steht,
R² für Wasserstoff steht,
oder
R¹ für Methyl steht,
R² für Methyl steht,
und
R³ für eine Gruppe der Formel 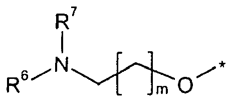
steht,
wobei
* die Anknüpfstelle an den Oxopyridin-Ring ist,
in für die Zahl 1 steht,
R⁶ für 2-Hydroxyeth-1-yl oder 4-Hydroxycyclohex-1-yl steht,
R⁷ für Wasserstoff steht,
oder eines ihrer Salze, ihrer Solvate oder der Solvate ihrer Salze.

7. Verfahren zur Herstellung einer Verbindung der Formel (I) oder eines ihrer Salze, ihrer Solvate oder der Solvate ihrer Salze nach Anspruch 1, **dadurch gekennzeichnet, dass**
[A] eine Verbindung der Formel 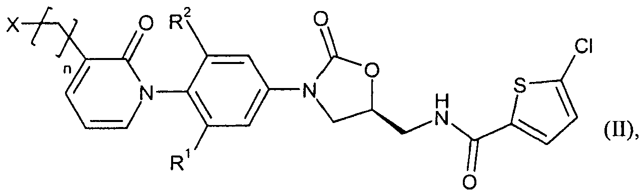 in welcher n, R¹ und R² die in Anspruch 1 angegebene Bedeutung haben, und
X für Hydroxy oder Brom steht,
mit einer Verbindung der Formel 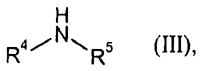 in welcher R⁴ und R⁵ die in Anspruch 1 angegebene Bedeutung haben,
zu einer Verbindung der Formel 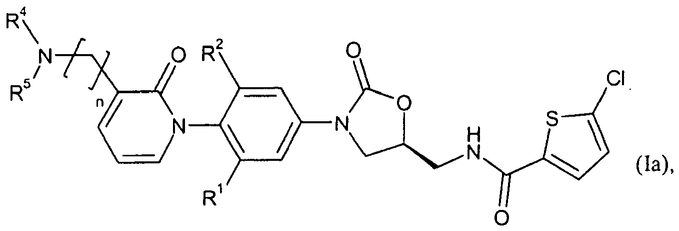 in welcher n, R¹, R², R⁴ und R⁵ die in Anspruch 1 angegebene Bedeutung haben, umgesetzt wird,
oder
[B] eine Verbindung der Formel 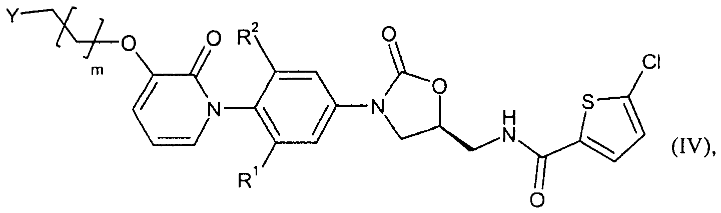 in welcher m, R¹ und R² die in Anspruch 1 angegebene Bedeutung haben, und
Y für Hydroxy oder Chlor steht,
mit einer Verbindung der Formel 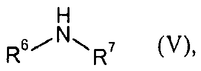 in welcher R⁶ und R⁷ die in Anspruch 1 angegebene Bedeutung haben, zu einer Verbindung der Formel 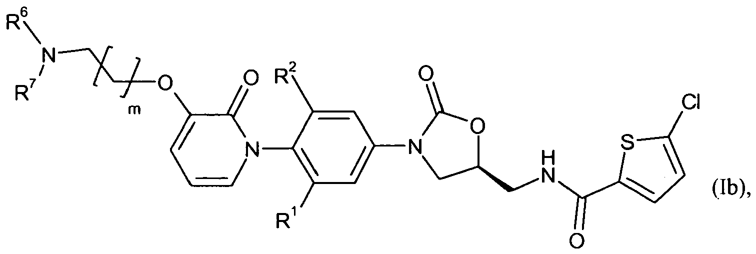 in welcher m, R¹, R², R⁵ und R⁷ die in Anspruch 1 angegebene Bedeutung haben,
umgesetzt wird.

8. Verbindung nach einem der Ansprüche 1 bis 6 zur Behandlung und/oder Prophylaxe von Krankheiten.

9. Verwendung einer Verbindung nach einem der Ansprüche 1 bis 6 zur Herstellung eines Arzneimittels zur Behandlung und/oder Prophylaxe von Krankheiten.

10. Verwendung einer Verbindung nach einem der Ansprüche 1 bis 6 zur Herstellung eines Arzneimittels zur Behandlung und/oder Prophylaxe von thromboembolischen Erkrankungen.

11. Verwendung einer Verbindung nach einem der Ansprüche 1 bis 6 zur Verhinderung der Blutkoagulation in vitro.

12. Arzneimittel enthaltend eine Verbindung nach einem der Ansprüche 1 bis 6 in Kombination mit einem inerten, nichttoxischen, pharmazeutisch geeigneten Hilfsstoff.

13. Arzneimittel enthaltend eine Verbindung nach einem der Ansprüche 1 bis 6 in Kombination mit einem weiteren Wirkstoff.

14. Arzneimittel nach Anspruch 12 oder 13 zur Behandlung und/oder Prophylaxe von thromboembolischen Erkrankungen.

15. Verfahren zur Verhinderung der Blutkoagulation in vitro, **dadurch gekennzeichnet, dass** eine antikoagulatorisch wirksame Menge einer Verbindung nach einem der Ansprüche 1 bis 6 zugegeben wird.

16. Verwendung einer Verbindung nach einem der Ansprüche 1 bis 6 zur Herstellung eines Arzneimittels zur Behandlung und/oder Prophylaxe von pulmonaler Hypertonie.

17. Verwendung einer Verbindung nach einem der Ansprüche 1 bis 6 zur Herstellung eines Arzneimittels zur Behandlung und/oder Prophylaxe von Sepsis, Systemic Inflammatory Syndrome (SIRS), septische Organdysfunktion, septisches Organversagen und Muliorganversagen, Acute Respiratory Distress Syndrome (ARDS), Acute Lung Injury (ALI), Septischer Schock, DIC ("Disseminated Intravascular coagulation") und/oder des septischen Organversagens.

18. Verbindung, wie in einem der Ansprüche 1 bis 6 definiert, zur Verwendung in einem Verfahren zur Behandlung und/oder Prophylaxe von thromboembolischen Erkrankungen.

## Claims

1. Compound of the formula 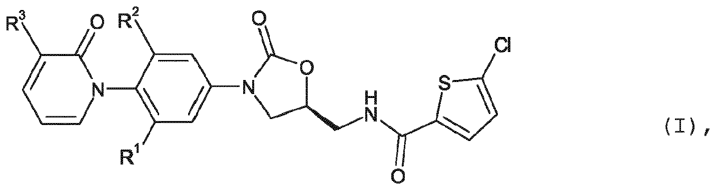 in which
R¹ represents chlorine, trifluoromethoxy, methyl, ethyl, n-propyl, methoxy, methoxymethyl or ethoxymethyl,
R² represents hydrogen or methyl,
and
R³ represents a group of the formula 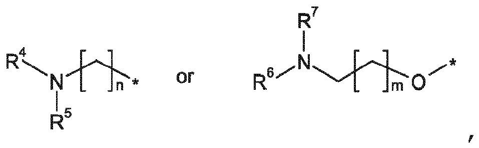
where
* is the point of attachment to the oxopyridine ring,
n represents the number 1, 2, 3 or 4,
m represents the number 1 or 2,
R⁴ represents hydrogen, methyl, ethyl, cyclopropyl, cyclobutyl, 2-hydroxyeth-1-yl, 3-hydroxyprop-1-yl, 2-methoxyeth-1-yl, 3-methoxyprop-1-yl, 4-hydroxycyclohex-1-yl, tetrahydrofuran-2-ylmethyl or 1,4-dioxan-2-ylmethyl,
R⁵ represents hydrogen, methyl or ethyl,
or
R⁴ and R⁵ together with the nitrogen atom to which they are attached form a pyrrolidin-1-yl ring, a 2-methoxymethylpyrrolidin-1-yl ring, a morpholin-4-yl ring, a 1,1-dioxothiomorpholin-4-yl ring, a 1,4-oxazepan-4-yl ring, a 4-methylpiperazin-1-yl or a 4-hydroxypiperidin-1-yl ring,
R⁶ represents hydrogen, methyl, ethyl, cyclopropyl, cyclobutyl, 2-hydroxyeth-1-yl, 3-hydroxyprop-1-yl, 2-methoxyeth-1-yl, 3-methoxyprop-1-yl, 4-hydroxycyolohex-1-yl, tetrahydrofuran-2-ylmethyl or 1,4-dioxan-2-ylmethyl,
R⁷ represents hydrogen, methyl or ethyl,
or
R⁶ and R⁷ together with the nitrogen atom to which they are attached form a pyrrolidin-1-yl ring, a 2-methoxymethylpyrrolidin-1-yl ring, a morpholin-4-yl ring, a 1,1-dioxothiomorpholin-4-yl ring, a 1,4-oxazepan-4-yl ring, a 4-methylpiperazin-1-yl or a 4-hydroxypiperidin-1-yl ring,
or one of its salts, its solvates or the solvates of its salts.

2. Compound according to Claim 1, **characterized in that**
R¹ represents chlorine, trifluoromethoxy, methyl, ethyl, n-propyl, methoxy or methoxymethyl,
R² represents hydrogen or methyl,
and
R³ represents a group of the formula 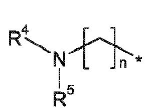
or 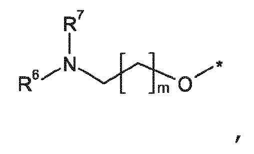
where
* is the point of attachment to the oxopyridine ring,
n is the number 1, 2 or 3,
m represents the number 1 or 2,
R⁴ represents hydrogen, methyl, ethyl, cyclopropyl, cyclobutyl, 2-hydroxyeth-1-yl, 3-hydroxyprop-1-yl, 2-methoxyeth-1-yl, 3-methoxyprop-1-yl, 4-hydroxycyclohex-1-yl, tetrahydrofuran-2-ylmethyl or 1,4-dioxan-2-ylmethyl,
R⁵ represents hydrogen, methyl or ethyl,
or
R⁴ and R⁵ together with the nitrogen atom to which they are attached form a pyrrolidin-1-yl ring, a 2-methoxymethylpyrrolidin-1-yl ring, a morpholin-4-yl ring, a 1,1-dioxothiomorpholin-4-yl ring, a 1,4-oxazepan-4-yl ring, a 4-methylpiperazin-1-yl or a 4-hydroxypiperidin-1-yl ring,
R⁶ represents hydrogen, methyl, ethyl, cyclopropyl, cyclobutyl, 2-hydroxyeth-1-yl, 3-hydroxyprop-1-yl, 2-methoxyeth-1-yl, 3-methoxyprop-1-yl, 4-hydroxycyclohex-1-yl, tetrahydrofuran-2-ylmethyl or 1,4-dioxan-2-ylmethyl,
R⁷ represents hydrogen, methyl or ethyl,
or
R⁶ and R⁷ together with the nitrogen atom to which they are attached form a pyrrolidin-1-yl ring, a 2-methoxymethylpyrrolidin-1-yl ring, a morpholin-4-yl ring, a 1,1-dioxothiomorpholin-4-yl ring, a 1,4-oxazepan-4-yl ring, a 4-methylpiperazin-1-yl or a 4-hydroxypiperidin-1-yl ring,
or one of its salts, its solvates or the solvates of its salts.

3. Compound according to Claim 1 or 2, **characterized in that**
R¹ represents methyl, ethyl, n-propyl, methoxy or methoxymethyl,
R² represents hydrogen or methyl,
and
R³ represents a group of the formula 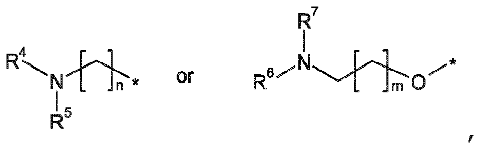
where
* is the point of attachment to the oxopyridine ring,
n is the number 1, 2 or 3,
m represents the number 1 or 2,
R⁴ represents hydrogen, methyl, ethyl, cyclopropyl, cyclobutyl, 2-hydroxyeth-1-yl, 3-hydroxyprop-1-yl, 2-methoxyeth-1-yl, 3-methoxyprop-1-yl, 4-hydroxycyolohex-1-yl, tetrahydrofuran-2-ylmethyl or 1,4-dioxan-2-ylmethyl,
R⁵ represents hydrogen, methyl or ethyl,
R⁶ represents hydrogen, methyl, ethyl, cyclopropyl, cyclobutyl, 2-hydroxyeth-1-yl, 3-hydroxyprop-1-yl, 2-methoxyeth-1-yl, 3-methoxyprop-1-yl, 4-hydroxycyclohex-1-yl, tetrahydrofuran-2-ylmethyl or 1,4-dioxan-2-ylmethyl,
R⁷ represents hydrogen, methyl or ethyl,
or one of its salts, its solvates or the solvates of its salts.

4. Compound according to any of Claims 1 to 3, **characterized in that**
R¹ represents methyl or methoxy,
R² represents hydrogen,
or
R¹ represents methyl,
R² represents methyl,
and
R³ represents a group of the formula 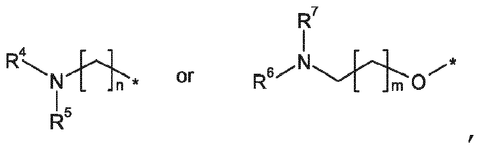
where
* is the point of attachment to the oxopyridine ring,
n represents the number 2,
m represents the number 1,
R⁴ represents 2-hydroxyeth-1-yl or 4-hydroxycyclohex-1-yl,
R⁵ represents hydrogen,
R⁶ represents 2-hydroxyeth-1-yl or 4-hydroxycyclohex-1-yl,
R⁷ represents hydrogen,
or one of its salts, its solvates or the solvates of its salts.

5. Compound according to any of Claims 1 to 3, **characterized in that**
R¹ represents methoxymethyl,
R² represents hydrogen,
or
R¹ represents methyl,
R² represents methyl,
and
R³ represents a group of the formula 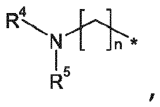
where
* is the point of attachment to the oxopyridine ring,
n represents the number 2,
R⁴ represents 2-hydroxyeth-1-yl or 4-hydroxycyclohex-1-yl,
R⁵ represents hydrogen,
or one of its salts, its solvates or the solvates of its salts.

6. Compound according to any of Claims 1 to 4, **characterized in that**
R¹ represents methyl,
R² represents hydrogen,
or
R¹ represents methyl,
R² represents methyl,
and
R³ represents a group of the formula 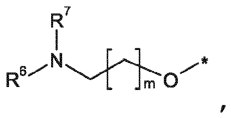
where
* is the point of attachment to the oxopyridine ring,
m represents the number 1,
R⁶ represents 2-hydroxyeth-1-yl or 4-hydroxycyclohex-1-yl,
R⁷ represents hydrogen,
or one of its salts, its solvates or the solvates of its salts.

7. Process for preparing a compound of the formula (I) or one or its salts, its solvates or the solvates of its salts according to Claim 1, **characterized in that**
[A] a compound of the formula 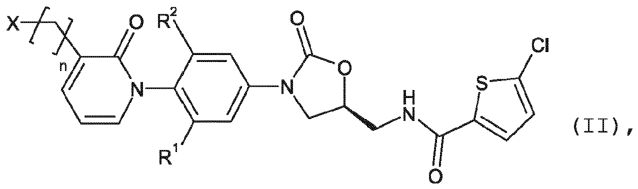 in which n, R¹ and R² have the meaning given in Claim 1, and
X represents hydroxyl or bromine,
is reacted with a compound of the formula 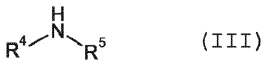 in which R⁴ and R⁵ have the meaning given in Claim 1,
to give a compound of the formula 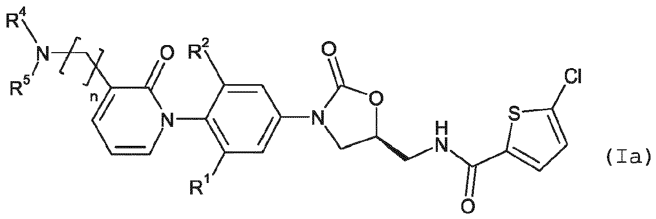 in which n, R¹, R², R⁴ and R⁵ have the meaning given in Claim 1,
or
[B] a compound of the formula 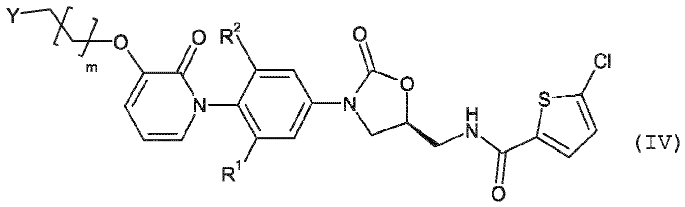 in which m, R¹ and R² have the meaning given in Claim 1, and
Y represents hydroxyl or chlorine,
is reacted with a compound of the formula 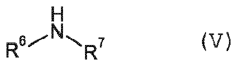 in which R⁶ and R⁷ have the meaning given in Claim 1,
to give a compound of the formula 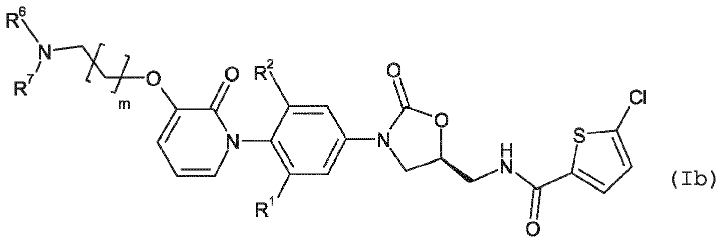 in which m, R¹, R², R⁶ and R⁷ have the meaning given in Claim 1.

8. Compound according to any of Claims 1 to 6 for the treatment and/or prophylaxis of diseases.

9. Use of a compound according to any of Claims 1 to 6 for preparing a medicament for the treatment and/or prophylaxis of diseases.

10. Use of a compound according to any of Claims 1 to 6 for preparing a medicament for the treatment and/or prophylaxis of thromboembolic disorders.

11. Use of a compound according to any of Claims 1 to 6 for preventing blood coagulation in vitro.

12. Medicament, comprising a compound according to any of Claims 1 to 6 in combination with an inert nontoxic pharmaceutically acceptable auxiliary.

13. Medicament comprising a compound according to any of Claims 1 to 6 in combination with a further active compound.

14. Medicament according to Claim 12 or 13 for the treatment and/or prophylaxis of thromboembolic disorders.

15. Method for preventing blood coagulation in vitro, **characterized in that** an anticoagulatory effective amount of a compound according to any of Claims 1 to 6 is added.

16. Use of a compound according to any of Claims 1 to 6 for preparing a medicament for the treatment and/or prophylaxis of pulmonary hypertension.

17. Use of a compound according to any of Claims 1 to 6 for preparing a medicament for the treatment and/or prophylaxis of sepsis, systemic inflammatory syndrome (SIRS), septic organ dysfunction, septic organ failure and multiorgan failure, acute respiratory distress syndrome (ARDS), acute lung injury (ALI), septic shock and/or DIC ("disseminated intravascular coagulation").

18. Compound as defined in any of Claims 1 to 6 for use in a method for the treatment and/or prophylaxis of thromboembolic disorders.

## Revendications

1. Composé de formule 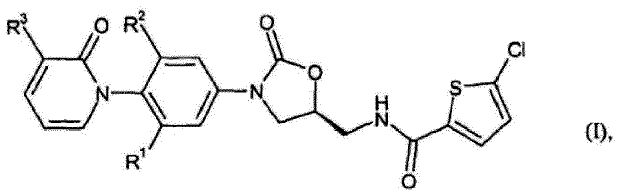 dans laquelle
R¹ représente chlore, trifluorométhoxy, méthyle, éthyle, n-propyle, méthoxy, méthoxyméthyle ou éthoxyméthyle,
R² représente hydrogène ou méthyle,
et
R³ représente un groupe de formule 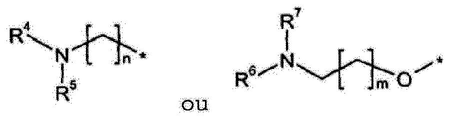 dans lesquelles
* est l'emplacement de liaison sur le cycle oxopyridine,
n représente le nombre 1, 2, 3 ou 4,
m représente le nombre 1 ou 2,
R⁴ représente hydrogène, méthyle, éthyle, cyclopropyle, cyclobutyle, 2-hydroxyéth-1-yle, 3-hydroxyprop-1-yle, 2-méthoxyéth-1-yle, 3-méthoxyprop-1-yle, 4-hydroxycyclohex-1-yle, tétrahydrofuran-2-ylméthyle ou 1,4-dioxan-2-ylméthyle,
R⁵ représente hydrogène, méthyle ou éthyle,
ou
R⁴ et R⁵ forment ensemble avec l'atome d'azote auquel ils sont reliés un cycle pyrrolidin-1-yle, un cycle 2-méthoxyméthyl-pyrrolidin-1-yle, un cycle morpholin-4-yle, un cycle 1,1-dioxo-thiomorpholine-4-yle, un cycle 1,4-oxazépan-4-yle, un 4-méthyl-pipérazin-1-yle ou un cycle 4-hydroxy-pipéridin-1-yle,
R⁶ représente hydrogène, méthyle, éthyle, cyclopropyle, cyclobutyle, 2-hydroxyéth-1-yle, 3-hydroxyprop-1-yle, 2-méthoxyéth-1-yle, 3-méthoxyprop-1-yle, 4-hydroxycyclohex-1-yle, tétrahydrofuran-2-ylméthyle ou 1,4-dioxan-2-ylméthyle,
R⁷ représente hydrogène, méthyle ou méthyle,
ou
R⁶ et R⁷ forment ensemble avec l'atome d'azote auquel ils sont reliés un cycle pyrrolidin-1-yle, un cycle 2-méthoxyméthyl-pyrrolidin-1-yle, un cycle morpholin-4-yle, un cycle 1,1-dioxo-thiomorpholine-4-yle, un cycle 1,4-oxazépan-4-yle, un 4-méthyl-pipérazin-1-yle ou un cycle 4-hydroxy-pipéridin-1-yle,
ou un de ses sels, de ses solvates ou des solvates de ses sels.

2. Composé selon la revendication 1, **caractérisé en ce que**
R¹ représente chlore, trifluorométhoxy, méthyle, éthyle, n-propyle, méthoxy ou méthoxyméthyle,
R² représente hydrogène ou méthyle,
et
R³ représente un groupe de formule 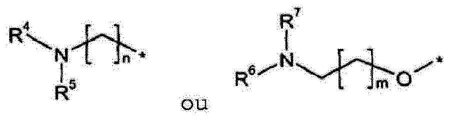 dans lesquelles
* est l'emplacement de liaison sur le cycle oxopyridine,
n représente le nombre 1, 2 ou 3,
m représente le nombre 1 ou 2,
R⁴ représente hydrogène, méthyle, éthyle, cyclopropyle, cyclobutyle, 2-hydroxyéth-1-yle, 3-hydroxyprop-1-yle, 2-méthoxyéth-1-yle, 3-méthoxyprop-1-yle, 4-hydroxycyclohex-1-yle, tétrahydrofuran-2-ylméthyle ou 1,4-dioxan-2-ylméthyle,
R⁵ représente hydrogène, méthyle ou éthyle,
ou
R⁴ et R⁵ forment ensemble avec l'atome d'azote auquel ils sont reliés un cycle pyrrolidin-1-yle, un cycle 2-méthoxyméthyl-pyrrolidin-1-yle, un cycle morpholin-4-yle, un cycle 1,1-dioxo-thiomorpholine-4-yle, un cycle 1,4-oxazépan-4-yle, un 4-méthyl-pipérazin-1-yle ou un cycle 4-hydroxy-pipéridin-1-yle,
R⁶ représente hydrogène, méthyle, éthyle, cyclopropyle, cyclobutyle, 2-hydroxyéth-1-yle, 3-hydroxyprop-1-yle, 2-méthoxyéth-1-yle, 3-méthoxyprop-1-yle, 4-hydroxycyclohex-1-yle, tétrahydrofuran-2-ylméthyle ou 1,4-dioxan-2-ylméthyle,
R⁷ représente hydrogène, méthyle ou éthyle,
ou
R⁶ et R⁷ forment ensemble avec l'atome d'azote auquel ils sont reliés un cycle pyrrolidin-1-yle, un cycle 2-méthoxyméthyl-pyrrolidin-1-yle, un cycle morpholin-4-yle, un cycle 1,1-dioxo-thiomorpholine-4-yle, un cycle 1,4-oxazépan-4-yle, un 4-méthyl-pipérazin-1-yle ou un cycle 4-hydroxy-pipéridin-1-yle,
ou un de ses sels, de ses solvates ou des solvates de ses sels.

3. Composé selon l'une quelconque des revendications 1 ou 2, **caractérisé en ce que** R¹ représente méthyle, éthyle, n-propyle, méthoxy ou méthoxyméthyle,
R² représente hydrogène ou méthyle, et
R³ représente un groupe de formule 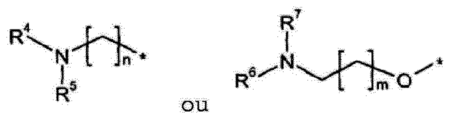 dans lesquelles
* est l'emplacement de liaison sur le cycle oxopyridine,
n représente le nombre 1, 2 ou 3,
m représente le nombre 1 ou 2,
R⁴ représente hydrogène, méthyle, éthyle, cyclopropyle, cyclobutyle, 2-hydroxyéth-1-yle, 3-hydroxyprop-1-yle, 2-méthoxyéth-1-yle, 3-méthoxyprop-1-yle, 4-hydroxycyclohex-1-yle, tétrahydrofuran-2-ylméthyle ou 1,4-dioxan-2-ylméthyle,
R⁵ représente hydrogène, méthyle ou éthyle,
R⁶ représente hydrogène, méthyle, éthyle, cyclopropyle, cyclobutyle, 2-hydroxyéth-1-yle, 3-hydroxyprop-1-yle, 2-méthoxyéth-1-yle, 3-méthoxyprop-1-yle, 4-hydroxycyclohex-1-yle, tétrahydrofuran-2-ylméthyle ou 1,4-dioxan-2-ylméthyle,
R⁷ représente hydrogène, méthyle ou éthyle,
ou un de ses sels, de ses solvates ou des solvates de ses sels.

4. Composé selon l'une quelconque des revendications 1 à 3, **caractérisé en ce que**
R¹ représente méthyle ou méthoxy,
R² représente hydrogène,
ou
R¹ représente méthyle,
R² représente méthyle,
et
R³ représente un groupe de formule 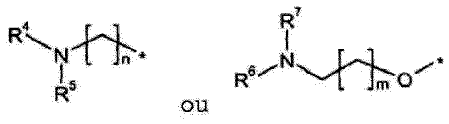 dans lesquelles
* est l'emplacement de liaison sur le cycle oxopyridine,
n représente le nombre 2,
m représente le nombre 1,
R⁴ représente 2-hydroxyéth-1-yle ou 4-hydroxycyclohex-1-yle,
R⁵ représente hydrogène,
R⁶ représente 2-hydroxyéth-1-yle ou 4-hydroxycyclohex-1-yle,
R⁷ représente hydrogène,
ou un de ses sels, de ses solvates ou des solvates de ses sels.

5. Composé selon l'une quelconque des revendications 1 à 3, **caractérisé en ce que**
R¹ représente méthoxyméthyle,
R² représente hydrogène,
ou
R¹ représente méthyle,
R² représente méthyle,
et
R³ représente un groupe de formule 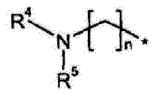 dans laquelle
* est remplacement de liaison sur le cycle oxopyridine,
n représente le nombre 2,
R⁴ représente 2-hydraxyéth-1-yle ou 4-hydroxycyclohex-1-yle,
R⁵ représente hydrogène,
ou un de ses sels, de ses solvates ou des solvates de ses sels.

6. Composé selon l'une quelconque des revendications 1 à 4, **caractérisé en ce que**
R¹ représente méthyle,
R² représente hydrogène,
ou
R¹ représente méthyle,
R² représente méthyle,
et
R³ représente un groupe de formule 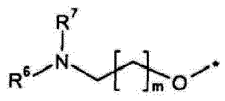 dans laquelle
* est l'emplacement de liaison sur le cycle oxopyridine,
m représente le nombre 1,
R⁶ représente 2-hydroxyéth-1-yle ou 4-hydroxycyclohex-1-yle,
R⁷ représente hydrogène,
ou un de ses sels, de ses solvates ou des solvates de ses sels.

7. Procédé de fabrication d'un composé de formule (I) ou d'un de ses sels, de ses solvates ou des solvates de ses sels selon la revendication 1, **caractérisé en ce que**
[A] un composé de formule 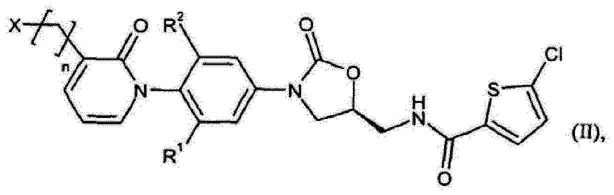 dans laquelle n, R¹ et R² ont la signification donnée dans la revendication 1, et
X représente hydroxyl ou brome,
est mis en réfaction avec un composé de formule 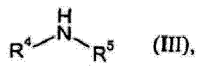 dans laquelle R⁴ et R⁵ ont la lignification donnée dans la revendication 1,
pour former un composé de formule 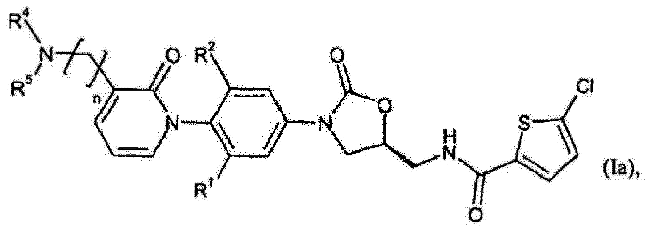 dans laquelle n, R¹, R², R⁴ et R⁵ ont la lignification donnée dans la revendication 1,
ou
[B] un composé de formule 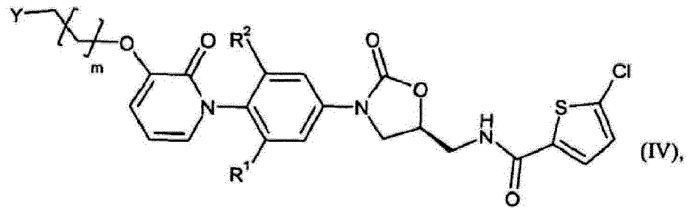 dans laquelle m, R¹ et R² ont la signification donnée dans la revendication 1, et
Y représente hydroxy ou chlore,
est mis en réaction avec un composé de formule 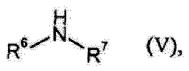 dans laquelle R⁶ et R⁷ ont la signification donnée dans la revendication 1,
pour former un composé de formule 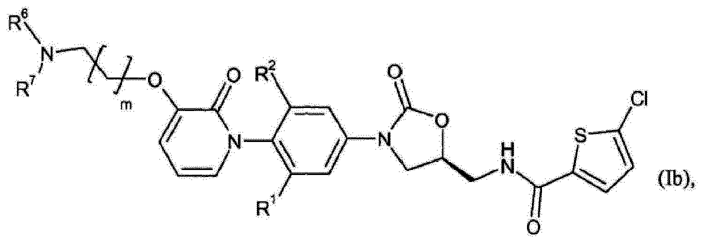 dans laquelle m, R¹, R², R⁶ et R⁷ ont la signification donnée dans la revendication 1.

8. Composé selon l'une quelconque des revendications 1 à 6 pour le traitement et/ou la prophylaxie de maladies.

9. Utilisation d'un composé selon l'une quelconque des revendications 1 à 6 pour la fabrication d'un médicament pour le traitement et/ou la prophylaxie de maladies.

10. Utilisation d'un composé selon l'une quelconque des revendications 1 à 6 pour la fabrication d'un médicament pour le traitement et/ou la prophylaxie de maladies thromboemboliques.

11. Utilisation d'un composé selon l'une quelconque des revendications 1 à 6 pour l'inhibition de la coagulation sanguine in vitro.

12. Médicament contenant un composé selon l'une quelconque des revendications 1 à 6 en combinaison avec un adjuvant inerte, non toxique, pharmaceutiquement approprié.

13. Médicament contenant un composé selon l'une quelconque des revendications 1 à 6 en combinaison avec un autre agent actif.

14. Médicament selon la revendication 12 ou 13 pour le traitement et/ou la prophylaxie de maladies thromboemboliques.

15. Procédé d'inhibition de la coagulation sanguine in vitro, **caractérisé en ce qu'**une quantité anticoagulante efficace d'un composé selon l'une quelconque des revendications 1 à 6 est ajouté.

16. Utilisation d'un composé selon l'une quelconque des revendications 1 à 6 pour la fabrication d'un médicament pour le traitement et/ou la prophylaxie de l'hypertension pulmonaire.

17. Utilisation d'un composé selon l'une quelconque des revendications 1 à 6 pour la fabrication d'un médicament pour le traitement et/ou la prophylaxie de la sepsie, du syndrome inflammatoire systémique (SIRS), de la dysfonction d'organes septique, de la défaillance d'organes septique et de la défaillance multi-organique, du syndrome de détresse respiratoire aiguë (SDRA), de la lésion pulmonaire aiguë (LPA), du choc septique, de la CIVD (coagulation intra-vasculaire disséminée) et/ou de la défaillance d'organes septique.

18. Composé tel que défini dans l'une quelconque des revendications 1 à 6, destiné à être utilisé dans un procédé de traitement et/ou de prophylaxie de maladies thromboemboliques.
